# EUROPEAN PATENT SPECIFICATION

(11) **EP 3 728 284 B1**
(45) Date of publication and mention of the grant of the patent: **21.08.2024**
(21) Application number: 18839991.9
(22) Date of filing: 21.12.2018
(51) Int. Cl.: C07J 43/00, C07J 5/00, C07J 7/00, C07J 9/00, C07J 41/00, A61K 31/57, A61K 31/58, A61K 31/56, A61K 31/575, A61P 25/00, C07J 1/00, C07J 3/00, C07J 13/00, C07J 17/00, C07J 21/00, C07J 51/00, C07J 71/00

(54) **19-HOMO, 3.ALPHA.-HYDROXY-STEROID-20-ONE COMPOUNDS FOR TREATING CNS DISORDERS**
19-HOMO, 3.ALPHA.-HYDROXYSTEROID-20-ON-VERBINDUNGEN ZUR BEHANDLUNG VON ZNS-ERKRANKUNGEN
COMPOSÉS 19-HOMO, 3.ALPHA.-HYDROXY-STÉROÏDE-20-ONES POUR LE TRAITEMENT DES TROUBLES DU SNC

(30) Priority: 22.12.2017 US 201762610069 P; 29.12.2017 US 201762611983 P; 22.01.2018 US 201862620095 P; 24.08.2018 US 201862722781 P; 21.09.2018 US 201862734718 P
(43) Date of publication of application: 28.10.2020
(73) Proprietor: Sage Therapeutics, Inc., Cambridge, MA 02142 (US)
(72) Inventor: ROBICHAUD, Albert, Jean, Boston, MA 02110 (US); SALITURO, Francesco, G., Marlborough, MA 01752 (US); BLANCO-PILLADO, Maria, Jesus, Arlington, MA 02474 (US); LA, Daniel, Randolph Road Chestnut Hill, MA 02467 (US); HARRISON, Boyd, L., Princeton, NJ 08550 (US)
(74) Representative: Coles, Andrea Birgit
(86) International application number: PCT/US2018/067277
(87) International publication number: WO 2019/126741

(56) References cited:
- WO-A1-2015/180679
- WO-A1-2015/180679
- WO-A1-2016/061527
- WO-A1-2016/061527
- WO-A1-2016/082789
- WO-A1-2016/082789
- WO-A1-2020/118060
- WO-A1-2020/118060
- WO-A1-98/05337
- WO-A2-2016/134301
- GB-A- 1 380 246
- GB-A- 1 380 246
- US-A- 4 029 777
- US-A1- 2014 249 120
- PHILLIPPS G H: "STRUCTURE-ACTIVITY RELATIONSHIPS IN STEROIDAL ANAESTHETICS", NOL. MECH. GEN. ANAESTH. GLAXO SYMPOSIUM, XX, XX, 1 January 1974 (1974-01-01), pages 32 - 47, XP000600765
- PHILLIPPS G H: "STRUCTURE-ACTIVITY RELATIONSHIPS IN STEROIDAL ANAESTHETICS", NOL. MECH. GEN. ANAESTH. GLAXO SYMPOSIUM, XX, XX, 1 January 1974 (1974-01-01), pages 32 - 47, XP000600765
- CRISTINA SUÑOL ET AL: "Activity of B-Nor Analogues of Neurosteroids on the GABA A Receptor in Primary Neuronal Cultures", JOURNAL OF MEDICINAL CHEMISTRY, vol. 49, no. 11, 1 June 2006 (2006-06-01), US, pages 3225 - 3234, XP055570705, ISSN: 0022-2623, DOI: 10.1021/jm060002f
- HAN MINGCHENG ET AL: "Neurosteroid Analogs. 4. The Effect of Methyl Substitution at the C-5 and C-10 Positions of Neurosteroids on Electrophysiological Activity at GABAA Receptors", JOURNAL OF MEDICINAL CHEMISTRY, AMERICAN CHEMICAL SOCIETY, US, vol. 39, 1 January 1996 (1996-01-01), pages 4218 - 4232, XP002176148, ISSN: 0022-2623, DOI: 10.1021/JM960304P
- ADRIANA VELEIRO ET AL: "Structure-Activity Relationships of Neuroactive Steroids Acting on the GABAA Receptor", CURRENT MEDICINAL CHEMISTRY, vol. 16, no. 4, 1 February 2009 (2009-02-01), NL, pages 455 - 472, XP055570602, ISSN: 0929-8673, DOI: 10.2174/092986709787315522

## Description

### Cross-Reference To Related Applications

This application claims priority to U.S.S.N. 62/734,718 filed September 21, 2018, U.S.S.N. 62/722,781 filed August 24, 2018, U.S.S.N. 62/620,095 filed January 22, 2018, U.S.S.N. 62/611,983 filed December, 29, 2017, and U.S.S.N. 62/610,069 filed December 22, 2017.

### Background of the Invention

Brain excitability is defined as the level of arousal of an animal, a continuum that ranges from coma to convulsions, and is regulated by various neurotransmitters. In general, neurotransmitters are responsible for regulating the conductance of ions across neuronal membranes. At rest, the neuronal membrane possesses a potential (or membrane voltage) of approximately -70 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, a change of potential from -70 mV to -50 mV occurs. This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

In the case of the GABA receptor complex (GRC), the effect on brain excitability is mediated by γ-aminobutyric acid (GABA), a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs, *i.e*., reduced neuron excitability. In other words, the higher the chloride ion concentration in the neuron, the lower the brain excitability and level of arousal.

It is well-documented that the GRC is responsible for the mediation of anxiety, seizure activity, and sedation. Thus, GABA and drugs that act like GABA or facilitate the effects of GABA (e.g., the therapeutically useful barbiturates and benzodiazepines (BZs), such as Valium^{®}) produce their therapeutically useful effects by interacting with specific regulatory sites on the GRC. Accumulated evidence has now indicated that in addition to the benzodiazepine and barbiturate binding site, the GRC contains a distinct site for neuroactive steroids. See, e.g., Lan, N. C. et al., Neurochem. Res. (1991) 16:347-356.

Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232:1004-1007 (1986); Harrison, N. L. et al., J Pharmacol. Exp. Ther. 241:346-353 (1987)).

New and improved compounds are needed that act as modulating agents for brain excitability, as well as agents for the prevention and treatment of CNS-related diseases. The compounds, compositions, and methods described herein are directed toward this end. WO-A-2015/180679, WO-A-2016/061527, WO-A-2016/082789, WO-A-98/05337, GB-A-1,380,246 and Mol. Mech. Gen. Anaesth. Glaxo Symposium pp 34-47 (1974) all disclose a number of neuroactive 3.alpha.-hydroxy steroid compounds. However, none of these has an ethyl group in position 10 of the steroid skeleton. WO-A-2016/134301 discloses two neuroactive 3.alpha.-hydroxy steroids which are substituted in position 10 by a (1'-methoxyethyl) group.

### Summary of the Invention

Provided herein are compounds designed, for example, to act as GABA modulators. In some embodiments, such compounds are envisioned to be useful as therapeutic agents for treating a CNS-related disorder. In the context of the present invention, any reference in the present description to methods of treatment of diseases by administration of a compound of the present invention is to be interpreted as indicating that the invention incorporates a compound of the present invention for use in the treatment of those same diseases.

Provided herein are compounds of Formula (II-Ia) 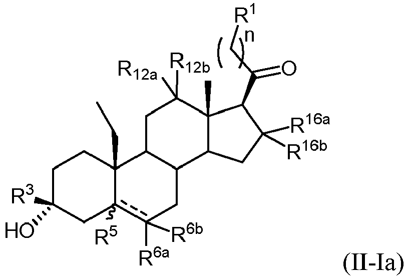 or a pharmaceutically acceptable salt thereof. Also forming part of the present invention is the compound II-A4 as identified subsequently in Table II-1, in the examples and in the claims.

In some embodiments, the compound of Formula (II-Ia) is the compound of Formula (II-Ib) 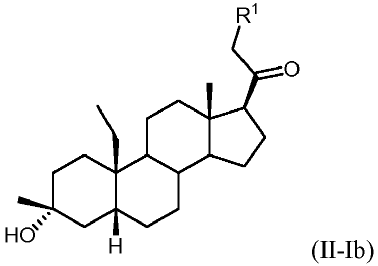 or a pharmaceutically acceptable salt thereof.

In some embodiments, the compound of Formula (II-Ia) is the compound of Formula (II-Ic) 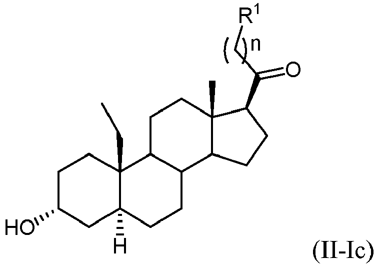 or a pharmaceutically acceptable salt thereof.

In some embodiments, the compound of Formula (II-Ia) is the compound of Formula (II-Ie) 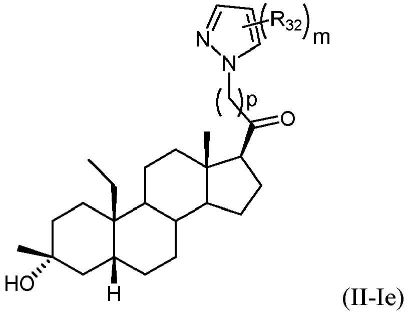 or a pharmaceutically acceptable salt thereof.

In some embodiments, the compound of Formula (II-Ia) is the compound of Formula (II-Iga) 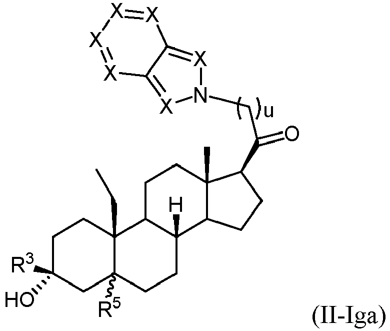 or a pharmaceutically acceptable salt thereof.

In some embodiments, the compound of Formula (II-Ia) is the compound of Formula (II-Ig) 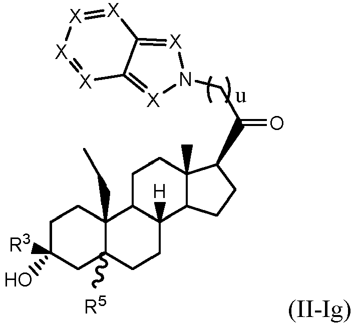 or a pharmaceutically acceptable salt thereof.

In some embodiments, the compound of Formula (II-Ia) is the compound of Formula (II-Ih) 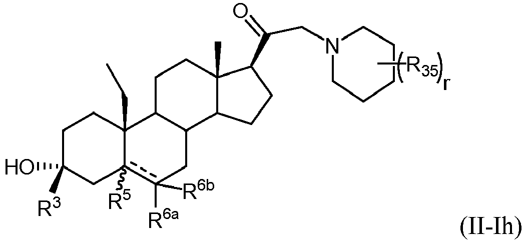 or a pharmaceutically acceptable salt thereof.

In some embodiments, the compound of Formula (II-Ia) is the compound of Formula (II-Ii) 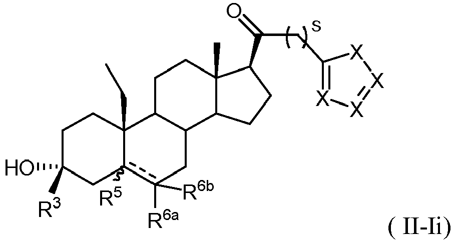 or a pharmaceutically acceptable salt thereof.

In some embodiments, a pharmaceutical composition comprises a compound of the present invention or pharmaceutically acceptable salt thereof, and a pharmaceutically acceptable excipient.

In some embodiments, a method of treating a CNS-related disorder in a subject in need thereof, comprises administering to the subject an effective amount of a compound of the present invention or a pharmaceutically acceptable salt thereof. In some embodiments, the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In some embodiments, the CNS-related disorder is depression. In some embodiments, the CNS-related disorder is postpartum depression. In some embodiments, the CNS-related disorder is major depressive disorder. In some embodiments, the major depressive disorder is moderate major depressive disorder. In some embodiments, the major depressive disorder is severe major depressive disorder.

In some embodiments, the compound is selected from the group consisting of the compounds identified in **Table II-1** herein.

In one aspect, provided herein is a pharmaceutically acceptable salt of a compound of the present invention (*e.g.,* a compound of Formula (II-Ia) or the compound II-A4).

In one aspect, provided herein is a pharmaceutical composition comprising a compound of the present invention (*e.g.,* a compound of Formula (II-Ia) or the compound II-A4) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

Compounds of the present invention as described herein, act, in certain embodiments, as GABA modulators, *e.g.,* effecting the GABA_{A} receptor in either a positive or negative manner. As modulators of the excitability of the central nervous system (CNS), as mediated by their ability to modulate GABA_{A} receptor, such compounds are expected to have CNS-activity.
Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, *e.g.,* by continuous intravenous infusion.

### Detailed Description of Certain Embodiments of the Invention

As generally described herein, the present invention provides compounds designed, for example, to act as GABA_{A} receptor modulators. In certain embodiments, such compounds are envisioned to be useful as therapeutic agents for treating a CNS-related disorder (e.g., a disorder as described herein, for example depression, such as post-partum depression or major depressive disorder).

### Definitions

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Isomers, e.g., stereoisomers, can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972).

In the compositions provided herein, an enantiomerically pure compound can be present with other active or inactive ingredients. For example, a pharmaceutical composition comprising enantiomerically pure R-position/center/ carbon compound can comprise, for example, about 90% excipient and about 10% enantiomerically pure R- compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

The term "diastereomierically pure" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of a single diastereomer. Methods for determining diastereomeric and enantiomeric purity are well-known in the art. Diastereomeric purity can be determined by any analytical method capable of quantitatively distinguishing between a compound and its diastereomers, such as high performance liquid chromatography (HPLC).

"Stereoisomers": It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are nonsuperimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The articles "a" and "an" may be used herein to refer to one or to more than one (i.e. at least one) of the grammatical objects of the article. By way of example "an analogue" means one analogue or more than one analogue.

When a range of values is listed, it is intended to encompass each value and subrange within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The following terms are intended to have the meanings presented therewith below and are useful in understanding the description and intended scope of the present invention.

"Alkyl" refers to a radical of a straight-chain or branched saturated hydrocarbon group having from 1 to 20 carbon atoms ("C₁₋₂₀ alkyl"). In some embodiments, an alkyl group has 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl", also referred to herein as "lower alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently optionally substituted, *i.e.,* unsubstituted (an "unsubstituted alkyl") or substituted (a "substituted alkyl") with one or more substituents; *e.g.,* for instance from 1 to 5 substituents, 1 to 3 substituents, or 1 substituent. In certain embodiments, the alkyl group is unsubstituted C₁₋₁₀ alkyl (*e.g.,* -CH₃). In certain embodiments, the alkyl group is substituted C₁₋₁₀ alkyl. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

"Alkylene" refers to an alkyl group wherein two hydrogens are removed to provide a divalent radical, and which may be substituted or unsubstituted. Unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Exemplary substituted alkylene groups, e.g., substituted with one or more alkyl (methyl) groups, include but are not limited to, substituted methylene (-CH(CH₃)-, (-C(CH₃)₂-), substituted ethylene (-CH(CH₃)CH₂-,-CH₂CH(CH₃)-, -C(CH₃)₂CH₂-,-CH₂C(CH₃)₂-), substituted propylene (-CH(CH₃)CH₂CH₂-, - CH₂CH(CH₃)CH₂-, -CH₂CH₂CH(CH₃)-, -C(CH₃)₂CH₂CH₂-, -CH₂C(CH₃)₂CH₂-, - CH₂CH₂C(CH₃)₂-), and the like. When a range or number of carbons is provided for a particular alkylene group, it is understood that the range or number refers to the range or number of carbons in the linear carbon divalent chain. Alkylene groups may be substituted or unsubstituted with one or more substituents as described herein.

"Alkenyl" refers to a radical of a straight-chain or branched hydrocarbon group having from 2 to 20 carbon atoms, one or more carbon-carbon double bonds (*e.g.,* 1, 2, 3, or 4 carbon-carbon double bonds), and optionally one or more carbon-carbon triple bonds (*e.g.,* 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents *e.g.,* for instance from 1 to 5 substituents, 1 to 3 substituents, or 1 substituent. In certain embodiments, the alkenyl group is unsubstituted C₂₋₁₀ alkenyl. In certain embodiments, the alkenyl group is substituted C₂₋₁₀ alkenyl.

"Alkynyl" refers to a radical of a straight-chain or branched hydrocarbon group having from 2 to 20 carbon atoms, one or more carbon-carbon triple bonds (*e.g.,* 1, 2, 3, or 4 carbon-carbon triple bonds), and optionally one or more carbon-carbon double bonds (*e.g.,* 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e.,* unsubstituted (an "unsubstituted alkynyl") or substituted (a "substituted alkynyl") with one or more substituents; *e.g.,* for instance from 1 to 5 substituents, 1 to 3 substituents, or 1 substituent. In certain embodiments, the alkynyl group is unsubstituted C₂₋₁₀ alkynyl. In certain embodiments, the alkynyl group is substituted C₂₋₁₀ alkynyl.

The term "heteroalkyl," as used herein, refers to an alkyl group, as defined herein, which further comprises 1 or more *(e.g.,* 1, 2, 3, or 4) heteroatoms *(e.g.,* oxygen, sulfur, nitrogen, boron, silicon, phosphorus) within the parent chain, wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkyl group refers to a saturated group having from 1 to 10 carbon atoms and 1, 2, 3, or 4 heteroatoms ("heteroC₁₋₁₀ alkyl"). In some embodiments, a heteroalkyl group is a saturated group having 1 to 9 carbon atoms and 1, 2, 3, or 4 heteroatoms ("heteroC₁₋₉ alkyl"). In some embodiments, a heteroalkyl group is a saturated group having 1 to 8 carbon atoms and 1, 2, 3, or 4 heteroatoms ("heteroC₁₋₈ alkyl"). In some embodiments, a heteroalkyl group is a saturated group having 1 to 7 carbon atoms and 1, 2, 3, or 4 heteroatoms ("heteroC₁₋₇ alkyl"). In some embodiments, a heteroalkyl group is a group having 1 to 6 carbon atoms and 1, 2, or 3 heteroatoms ("heteroC₁₋₆ alkyl"). In some embodiments, a heteroalkyl group is a saturated group having 1 to 5 carbon atoms and 1 or 2 heteroatoms ("heteroC₁₋₅ alkyl"). In some embodiments, a heteroalkyl group is a saturated group having 1 to 4 carbon atoms and 1or 2 heteroatoms ("heteroC₁₋₄ alkyl"). In some embodiments, a heteroalkyl group is a saturated group having 1 to 3 carbon atoms and 1 heteroatom ("heteroC₁₋₃ alkyl"). In some embodiments, a heteroalkyl group is a saturated group having 1 to 2 carbon atoms and 1 heteroatom ("heteroC₁₋₂ alkyl"). In some embodiments, a heteroalkyl group is a saturated group having 1 carbon atom and 1 heteroatom ("heteroC₁ alkyl"). In some embodiments, a heteroalkyl group is a saturated group having 2 to 6 carbon atoms and 1 or 2 heteroatoms ("heteroC₂₋₆ alkyl"). Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₁₀ alkyl. In certain embodiments, the heteroalkyl group is a substituted heteroC₁₋₁₀ alkyl.

"Aryl" refers to a radical of a monocyclic or polycyclic (*e.g.,* bicyclic or tricyclic) 4n+2 aromatic ring system (*e.g.,* having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g.,* anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

In certain embodiments, an aryl group substituted with one or more of groups selected from halo, C₁-C₈ alkyl, C₁-C₈ haloalkyl, cyano, hydroxy, C₁-C₈ alkoxy, and amino.

Examples of representative substituted aryls include the following 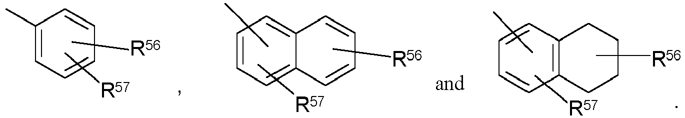 wherein one of R⁵⁶ and R⁵⁷ may be hydrogen and at least one of R⁵⁶ and R⁵⁷ is each independently selected from C₁-C₈ alkyl, C₁-C₈ haloalkyl, 4-10 membered heterocyclyl, alkanoyl, C₁-C₈ alkoxy, heteroaryloxy, alkylamino, arylamino, heteroarylamino, NR⁵⁸COR⁵⁹, NR⁵⁸SOR⁵⁹ NR⁵⁸SO₂R⁵⁹, COOalkyl, COOaryl, CONR⁵⁸R⁵⁹, CONR⁵⁸OR⁵⁹, NR⁵⁸R⁵⁹, SO₂NR⁵⁸R⁵⁹, S-alkyl, SOalkyl, SO₂alkyl, Saryl, SOaryl, SO₂aryl; or R⁵⁶ and R⁵⁷ may be joined to form a cyclic ring (saturated or unsaturated) from 5 to 8 atoms, optionally containing one or more heteroatoms selected from the group N, O, or S. R⁶⁰ and R⁶¹ are independently hydrogen, C₁-C₈ alkyl, C₁-C₄ haloalkyl, C₃-C₁₀ cycloalkyl, 4-10 membered heterocyclyl, C₆-C₁₀ aryl, substituted C₆-C₁₀ aryl, 5-10 membered heteroaryl, or substituted 5-10 membered heteroaryl .

"Fused aryl" refers to an aryl having two of its ring carbon in common with a second aryl or heteroaryl ring or with a carbocyclyl or heterocyclyl ring.

"Heteroaryl" refers to a radical of a 5-10 membered monocyclic or bicyclic 4n+2 aromatic ring system (*e.g.,* having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.,* indolyl, quinolinyl, carbazolyl, and the like) the point of attachment can be on either ring, *i.e.,* either the ring bearing a heteroatom (*e.g.,* 2-indolyl) or the ring that does not contain a heteroatom *(e.g.,* 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 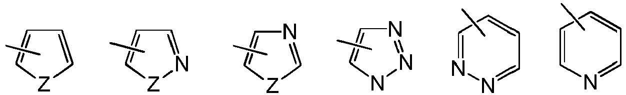 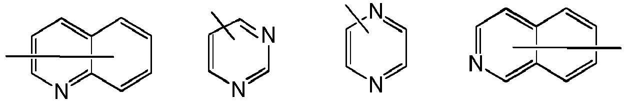 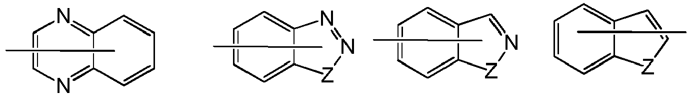 wherein each Z is selected from carbonyl, N, NR⁶⁵, O, and S; and R⁶⁵ is independently hydrogen, C₁-C₈ alkyl, C₃-C₁₀ cycloalkyl, 4-10 membered heterocyclyl, C₆-C₁₀ aryl, and 5-10 membered heteroaryl.

"Carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted C₃₋₁₀ carbocyclyl. In certain embodiments, the carbocyclyl group is a substituted C₃₋₁₀ carbocyclyl.

In some embodiments, "carbocyclyl" is a monocyclic, saturated carbocyclyl group having from 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₅). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃₋₁₀ cycloalkyl. In certain embodiments, the cycloalkyl group is substituted C₃₋₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Nitrogen-containing heterocyclyl" group means a 4- to 7- membered non-aromatic cyclic group containing at least one nitrogen atom, for example, but without limitation, morpholine, piperidine (*e.g.* 2-piperidinyl, 3-piperidinyl and 4-piperidinyl), pyrrolidine (*e.g.* 2-pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g,.* cycloheteroalkenyl, and the like having from 1 to 5, and particularly from 1 to 3 heteroatoms.

"Acyl" refers to a radical -C(O)R²⁰, where R²⁰ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, as defined herein. "Alkanoyl" is an acyl group wherein R²⁰ is a group other than hydrogen. Representative acyl groups include, but are not limited to, formyl (-CHO), acetyl (-C(=O)CH₃), cyclohexylcarbonyl, cyclohexylmethylcarbonyl, benzoyl (-C(=O)Ph), benzylcarbonyl (-C(=O)CH₂Ph), -C(O)-C₁-C₈ alkyl, -C(O)-(CH₂)ₜ(C₆-C₁₀ aryl), -C(O)-(CH₂)ₜ(5-10 membered heteroaryl), -C(O)-(CH₂)ₜ(C₃-C₁₀ cycloalkyl), and -C(O)-(CH₂)ₜ(4-10 membered heterocyclyl), wherein t is an integer from 0 to 4. In certain embodiments, R²¹ is C₁-C₈ alkyl, substituted with halo or hydroxy; or C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy.

"Alkoxy" refers to the group -OR²⁹ where R²⁹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, R²⁹ is a group that has 1 or more substituents, for instance from 1 to 5 substituents, and particularly from 1 to 3 substituents, in particular 1 substituent, selected from the group consisting of amino, substituted amino, C₆-C₁₀ aryl, aryloxy, carboxyl, cyano, C₃-C₁₀ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkylS(O)₂- and aryl-S(O)₂-. Exemplary `substituted alkoxy' groups include, but are not limited to, -O-(CH₂)ₜ(C₆-C₁₀ aryl), -O-(CH₂)ₜ(5-10 membered heteroaryl), -O-(CH₂)ₜ(C₃-C₁₀ cycloalkyl), and -O-(CH₂)ₜ(4-10 membered heterocyclyl), wherein t is an integer from 0 to 4 and any aryl, heteroaryl, cycloalkyl or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. Particular exemplary `substituted alkoxy' groups are -OCF₃, -OCH₂CF₃, -OCH₂Ph, -OCH₂-cyclopropyl, -OCH₂CH₂OH, and -OCH₂CH₂NMe₂.

"Amino" refers to the radical -NH₂.

"Oxo group" refers to -C(=O)-.

"Substituted amino" refers to an amino group of the formula -N(R³⁸)₂ wherein R³⁸ is hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of R³⁸ is not a hydrogen. In certain embodiments, each R³⁸ is independently selected from hydrogen, C₁-C₈ alkyl, C₃-C₈ alkenyl, C₃-C₈ alkynyl, C₆-C₁₀ aryl, 5-10 membered heteroaryl, 4-10 membered heterocyclyl, or C₃-C₁₀ cycloalkyl; or C₁-C₈ alkyl, substituted with halo or hydroxy; C₃-C₈ alkenyl, substituted with halo or hydroxy; C₃-C₈ alkynyl, substituted with halo or hydroxy, or - (CH₂)ₜ(C₆-C₁₀ aryl), -(CH₂)ₜ(5-10 membered heteroaryl), -(CH₂)ₜ(C₃-C₁₀ cycloalkyl), or - (CH₂)ₜ(4-10 membered heterocyclyl), wherein t is an integer between 0 and 8, each of which is substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; or both R³⁸ groups are joined to form an alkylene group.

Exemplary "substituted amino" groups include, but are not limited to, -NR³⁹-C₁-C₈ alkyl, -NR³⁹-(CH₂)ₜ(C₆-C₁₀ aryl), -NR³⁹-(CH₂)ₜ(5-10 membered heteroaryl), -NR³⁹-(CH₂)ₜ(C₃-C₁₀ cycloalkyl), and -NR³⁹-(CH₂)ₜ(4-10 membered heterocyclyl), wherein t is an integer from 0 to 4, for instance 1 or 2, each R³⁹ independently represents H or C₁-C₈ alkyl; and any alkyl groups present, may themselves be substituted by halo, substituted or unsubstituted amino, or hydroxy; and any aryl, heteroaryl, cycloalkyl, or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. For the avoidance of doubt the term `substituted amino' includes the groups alkylamino, substituted alkylamino, alkylarylamino, substituted alkylarylamino, arylamino, substituted arylamino, dialkylamino, and substituted dialkylamino as defined below. Substituted amino encompasses both monosubstituted amino and disubstituted amino groups.

"Carboxy" refers to the radical -C(O)OH.

"Cyano" refers to the radical -CN.

"Halo" or "halogen" refers to fluoro (F), chloro (Cl), bromo (Br), and iodo (I). In certain embodiments, the halo group is either fluoro or chloro.

"Haloalkyl" refers to an alkyl radical in which the alkyl group is substituted with one or more halogens. Typical haloalkyl groups include, but are not limited to, trifluoromethyl, difluoromethyl, fluoromethyl, chloromethyl, dichloromethyl, dibromoethyl, tribromomethyl, tetrafluoroethyl, and the like.

"Hydroxy" refers to the radical -OH.

"Nitro" refers to the radical -NO₂.

"Thioketo" refers to the group =S.

Alkyl, alkenyl, alkynyl, carbocyclyl, heterocyclyl, aryl, and heteroaryl groups, as defined herein, are optionally substituted (*e.g.,* "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, - OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa},-C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa},-NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂,-OP(R^{cc})₃, -B(R^{aa})₂, -B(OR^{cc})₂, -BR^{aa}(OR^{cc}), C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 R^{dd} groups; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO2H, -SO3H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, -OP(=O)(OR^{ee})₂, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl) ⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, - NHSO₂(C₁₋₆ alkyl), -SO₂N(C₁₋₆ alkyl)₂, -SO₂NH(C₁₋₆ alkyl), -SO₂NH₂,-SO₂C₁₋₆ alkyl, - SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ - C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, - OP(=O)(OC₁₋₆ alkyl)₂, C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a cationic quaternary amino group in order to maintain electronic neutrality. Exemplary counterions include halide ions (*e.g.,* F⁻, Cl⁻, Br⁻, I⁻), NO₃⁻, ClO₄⁻, OH⁻, H₂PO₄⁻, HSO₄⁻, sulfonate ions (*e.g.,* methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions *(e.g.,* acetate, ethanoate, propanoate, benzoate, glycerate, lactate, tartrate, glycolate, and the like).

These and other exemplary substituents are described in more detail in the **Detailed Description,** and **Claims**. The invention is not intended to be limited in any manner by the above exemplary listing of substituents.

### Other definitions

As used herein, the term "modulation" refers to the inhibition or potentiation of GABA_{A} receptor function. A "modulator" (*e.g.,* a modulator compound) may be, for example, an agonist, partial agonist, antagonist, or partial antagonist of the GABA_{A} receptor.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counterion of an acidic functional group. Such cations are exemplified by sodium, potassium, calcium, magnesium, ammonium, tetraalkylammonium cations, and the like. See, *e.g.,* Berge, et al., J. Pharm. Sci. (1977) 66(1): 1-79.

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed in the presence of acid or base. Tautomeric forms may be relevant to the attainment of the optimal chemical reactivity and biological activity of a compound of interest.

A "subject" to which administration is contemplated includes, but is not limited to, humans (*i.e.,* a male or female of any age group, *e.g.,* a pediatric subject (*e.g,* infant, child, adolescent) or adult subject (*e.g.,* young adult, middle-aged adult or senior adult)) and/or a non-human animal, *e.g.,* a mammal such as primates (*e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal.

In certain embodiments, the substituent present on an oxygen atom is an oxygen protecting group (also referred to as a hydroxyl protecting group). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa},-Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), 2-methoxyethoxymethyl (MEM), benzyl (Bn), triisopropylsilyl (TIPS), t-butyldimethylsilyl (TBDMS), t-butylmethoxyphenylsilyl (TBMPS), methanesulfonate (mesylate), and tosylate (Ts).

In certain embodiments, the substituent present on an sulfur atom is an sulfur protecting group (also referred to as a thiol protecting group). Sulfur protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa},-Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Sulfur protecting groups are well known in the art and include those described in detail in Protecting Groups in Organic Synthesis, T. W. Greene and P. G. M. Wuts, 3rd edition, John Wiley & Sons, 1999.

In certain embodiments, the substituent present on a nitrogen atom is an amino protecting group (also referred to herein as a nitrogen protecting group). Amino protecting groups include, but are not limited to, -OH, -OR^{aa}, -N(R^{cc})₂, -C(=O)R^{aa}, -C(=O)OR^{aa}, - C(=O)N(R^{cc})₂, -S(=O)₂R^{aa}, -C(=NR^{cc})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, C₁₋₁₀ alkyl, C₂₋₁₀ alkenyl, C₂₋₁₀ alkynyl, C₃₋₁₀ carbocyclyl, 3-14-membered heterocyclyl, C₆₋₁₄ aryl, and 5-14-membered heteroaryl groups, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined herein. Amino protecting groups are well known in the art and include those described in detail in Protecting Groups in Organic Synthesis, T. W. Greene and P. G. M. Wuts, 3rd edition, John Wiley & Sons, 1999.

Exemplary amino protecting groups include, but are not limited to amide groups (*e.g.,* -C(=O)R^{aa}), which include, but are not limited to, formamide and acetamide; carbamate groups (*e.g.,* -C(=O)OR^{aa}), which include, but are not limited to, 9-fluorenylmethyl carbamate (Fmoc), *t*-butyl carbamate (BOC), and benzyl carbamate (Cbz); sulfonamide groups (*e.g.,* -S(=O)₂R^{aa}), which include, but are not limited to, *p-*toluenesulfonamide (Ts), methanesulfonamide (Ms), and *N*-[2-(trimethylsilyl)ethoxy]methylamine (SEM).

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition In general, the "effective amount" of a compound refers to an amount sufficient to elicit the desired biological response, *e.g.,* to treat a CNS-related disorder, is sufficient to induce anesthesia or sedation. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

In an alternate embodiment, the present invention contemplates administration of the compounds of the present invention or a pharmaceutically acceptable salt or a pharmaceutically acceptable composition thereof, as a prophylactic before a subject begins to suffer from the specified disease, disorder or condition. As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, an "episodic dosing regimen" is a dosing regimen wherein a compound of Formula (II-Ia) or the compound II-A4 or a composition comprising a compound of Formula (II-Ia) or the compound II-A4 is administered to a subject for a finite period of time in response to the diagnosis of a disorder or symptom thereof, *e.g,* a diagnosis or symptom of depression. an episode of major depressive disorder, bipolar depression, anxiety, or postpartum depression. In some embodiments, the major depressive disorder is moderate major depressive disorder. In some embodiments, the major depressive disorder is severe major depressive disorder In some embodiments, the compound is formulated as individual dosage units, each unit comprising a compound of Formula (II-Ia) or the compound II-A4 and one or more suitable pharmaceutical excipients. In some embodiments, the episodic dosing regimen has a duration of a plurality of weeks, *e.g.* about 8 weeks. In contrast with chronic administration as defined herein, episodic dosing of a compound occurs over a finite period of time, e.g., from about 2 weeks to about 8 weeks, in response to a diagnosis of a disorder, *e.g.,* depression, or a symptom thereof. In some embodiments, episodic dosing occurs once per day across a plurality of weeks, *e.g.,* from about 2 weeks to about 6 weeks. In one embodiment, the episodic dosing has a duration of two weeks. In some embodiments, more than one episodic dosing regimen is administered to the subject, *e.g.,* two or more episodic regimens throughout the subject's life.

### Compounds

It should be appreciated that formulas described herein may reference particular carbon atoms, such as C17, C3, C19, etc. These references are based on the position of carbon atoms according to steroid nomenclature known and used in the industry, as shown below: 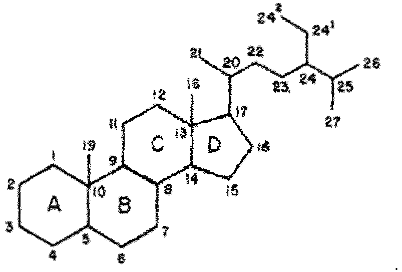 For example, C17 refers to the carbon at position 17 and C3 refers to the carbon at position 3.

In some embodiments, provided herein is a compound of Formula (II-Ia) 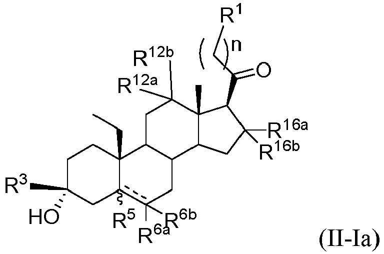 or a pharmaceutically acceptable salt thereof; wherein
n is 0, 1 or 2;
R⁵ is hydrogen or methyl, or when 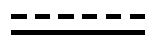 is a double bond, R⁵ is absent;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
   each of R^{6a} and R^{6b} is independently hydrogen, halogen, cyano, hydroxyl, substituted or unsubstituted alkyl, substituted or unsubstituted alkenyl, or substituted or unsubstituted alkynyl, or R^{6a} and R^{6b} are joined to form an oxo (=O) group;
   R¹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}), -OC(=O)R^{A1}, -OC(=O)OR^{A1}, -OC(=O)SR^{A1}, -OC(=O)N(R^{A1})₂, -SC(=O)R^{A2}, -SC(=O)OR^{A1}, -SC(=O)SR^{A1}, -SC(=O)N(R^{A1})₂,-NHC(=O)R^{A1}, -NHC(=O)OR^{A1}, -NHC(=O)SR^{A1}, -NHC(=O)N(R^{A1})₂, -OS(=O)₂R^{A2}, - OS(=O)₂OR^{A1}, -S-S(=O)₂R^{A2}, -S-S(=O)₂OR^{A1}, -S(=O)R^{A2}, -SO₂R^{A2}, or -S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
   each of R^{12a} and R^{12b} is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, - NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or R^{12a} and R^{12b} are joined to form an oxo (=O) group;
   each of R^{16a} and R^{16b} is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl; and wherein 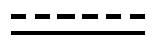 represents a single or double bond, provided if a double bond is present in Ring B, then one of R^{6a} or R^{6b} is absent.

It should be appreciated that the stereochemistry at C17 could be depicted in any of the following but equivalent ways:

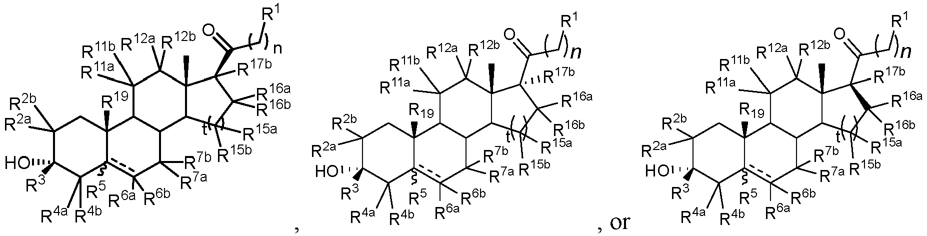

### Formula (II-Ia) Groups R^{16a} and R^{16b}

In some aspects, R^{16a} and R^{16b} is each independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, - NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl.

In some embodiments, R^{16a} and R^{16b} is each independently hydrogen, halogen, cyano, hydroxyl, substituted or unsubstituted alkyl, substituted or unsubstituted alkenyl,-OR^{D1},-OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R^{16a} and R^{16b} is each independently hydrogen, substituted or unsubstituted alkyl,-OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some further embodiments, R^{16a} and R^{16b} are both hydrogen.

In some aspects, R^{16a} and R^{16b} is each independently hydrogen or substituted or unsubstituted alkyl. In some aspects, R^{16a} and R^{16b} is independently hydrogen, C₁-C₆ alkyl, C₁-C₆ haloalkyl, C₁-C₆ alkoxy, C₁-C₆ alkoxyhalo, or -OH.

In some aspects, R^{16a} and R^{16b} is -CH₃, -CH₂CH₃, -OH, -OCH₃, or -CH(CH₃)₂.

### Formula (II-Ia) Group R⁵

In some aspects, R⁵ is hydrogen in the cis position, relative to the C19 position. In some other aspects, R⁵ is hydrogen in the trans position relative to the C19 position. In some embodiments, R⁵ is methyl in the cis position relative to the C19 position. In some further embodiments, R⁵ is methyl in the trans position relative to the C19 position.

### Formula (II-Ia) Group R³

In some embodiments, R³ is hydrogen, substituted or unsubstituted alkyl, substituted or unsubstituted alkenyl, or substituted or unsubstituted alkynyl.

In some embodiments, R³ is substituted or unsubstituted carbocyclyl, substituted or unsubstituted heterocyclyl, substituted or unsubstituted aryl, or substituted or unsubstituted heteroaryl.

In some embodiments, R³ is substituted or unsubstituted alkyl.

In some embodiments, R³ is hydrogen. In some embodiments, R³ is substituted alkyl (e.g., -CH₂OMe or -CH₂OEt, etc.). In some embodiments, R³ is unsubstituted alkyl. In some embodiments, R³ is methyl.

### Formula (II-Ia) Group R^{6a} and R^{6b}

In some embodiments, R^{6a} and R^{6b} is independently hydrogen, halogen, substituted or unsubstituted alkyl, substituted or unsubstituted alkenyl, or substituted or unsubstituted alkynyl.

In some aspects, R^{6a} and R^{6b} is independently hydrogen or substituted or unsubstituted alkyl.

In some aspects, R^{6a} and R^{6b} is independently hydrogen or substituted alkyl. In some embodiments, R^{6a} and R^{6b} is independently hydrogen or unsubstituted alkyl.

In some aspects, both R^{6a} and R^{6b} are hydrogen. In some aspects, R^{6a} is halo or alkyl and R^{6b} is hydrogen. In some embodiments, R^{6a} and R^{6b} are both halo.

In some aspects, R^{6a} and R^{6b} are both alkyl.

In some embodiments, R^{6a} and R^{6b} are joined to form an oxo group.

### Formula (II-Ia) Group R^{12a} and R^{12b}

In some embodiments, R^{12a} and R^{12b} is each independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl.

In some aspects, R^{12a} and R^{12b} is each independently hydrogen, halogen, cyano, hydroxyl, substituted or unsubstituted alkyl, substituted or unsubstituted alkenyl,-OR^{D1},-OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R^{12a} and R^{12b} is each independently hydrogen, substituted or unsubstituted alkyl,-OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R^{12a} and R^{12b} are both hydrogen.

In some further embodiments, R^{12a} and R^{12b} is each independently hydrogen or substituted or unsubstituted alkyl.

In some embodiments, R^{12a} and R^{12b} are joined together to form an oxo group (=O).

### Formula (II-Ia) n

In some embodiments, n is 1. In some other embodiments, n is 2.

### Formula (II-Ia) Group R¹

In some embodiments, R¹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl.

In some embodiments, R¹ is 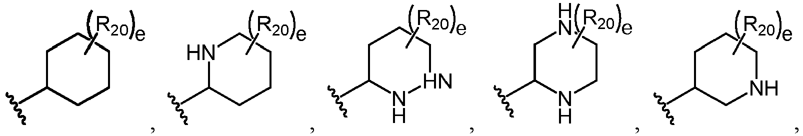 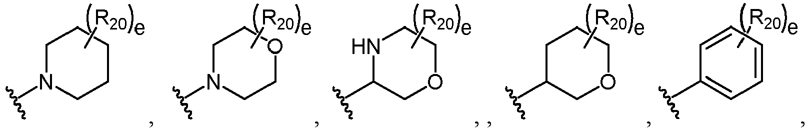 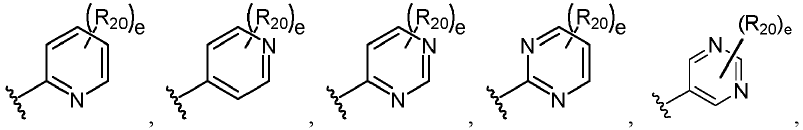 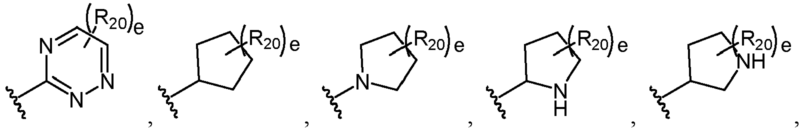 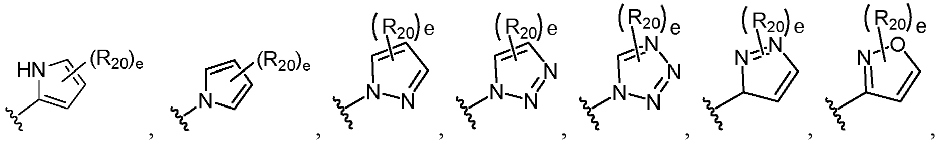 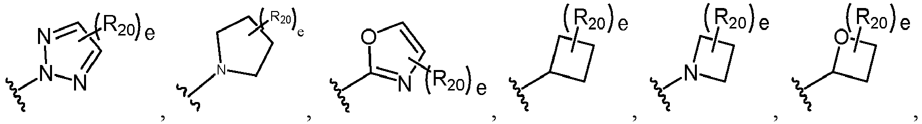 or 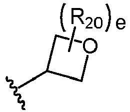 wherein each instance of R₂₀ is, independently, halogen, -NO₂, -CN, -OR^{GA}, - N(R^{GA})₂, -C(=O)R^{GA}, -C(=O)OR^{GA}, -OC(=O)R^{GA}, -OC(=O)OR^{GA}, -C(=O)N(R^{GA})₂, - N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, - OS(=O)₂R^{GA}, -S(=O)₂N(R^{GA})₂, or -N(R^{GA})S(=O)₂R^{GA}; substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocylyl, substituted or unsubstituted 3- to 4- membered heterocylyl, C₅₋₁₀ substituted or unsubstituted aryl, substituted or unsubstituted 5- to 10- membered heteroaryl, or optionally two R^{GA} are taken with the intervening atoms to form a substituted or unsubstituted 3- to 4- membered carbocyclic or heterocyclic ring; wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring; and e is 0, 1, 2, 3, 4, or 5.

In some embodiments, wherein R¹ is 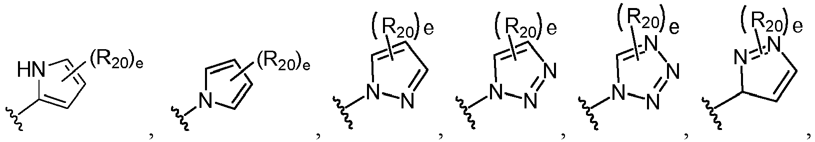 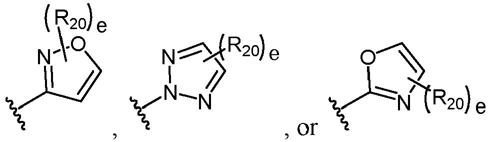 wherein each instance of R₂₀ is, independently, halogen, -NO₂, -CN, -OR^{GA}, -N(R^{GA})₂, - C(=O)R^{GA}, -C(=O)OR^{GA}, -OC(=O)R^{GA}, -OC(=O)OR^{GA}, -C(=O)N(R^{GA})₂, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, - S(=O)₂N(R^{GA})₂, or -N(R^{GA})S(=O)₂R^{GA}; substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocylyl, substituted or unsubstituted 3- to 4- membered heterocylyl, C₅₋₁₀ substituted or unsubstituted aryl, substituted or unsubstituted 5- to 10- membered heteroaryl, or optionally two R^{GA} are taken with the intervening atoms to form a substituted or unsubstituted 3- to 4- membered carbocyclic or heterocyclic ring; wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring; and e is 0, 1, 2, 3, 4, or 5.

In some embodiments, R¹ is substituted carbocyclyl, substituted heterocyclyl, substituted aryl, or substituted heteroaryl, wherein each substituted carbocyclyl, substituted heterocyclyl, substituted aryl, or substituted heteroaryl is independently substituted with an unsubstituted or substituted carbocyclyl, unsubstituted or substituted heterocyclyl, unsubstituted or substituted aryl, or unsubstituted or substituted heteroaryl.

In some embodiments, the compound of Formula (II-Ia) is the compound is of Formula (II-Ib) 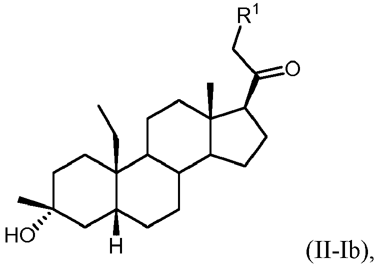 or a pharmaceutically acceptable salt thereof.

In some embodiments, the compound of Formula (II-Ia) is the compound is of Formula (II-Ic) 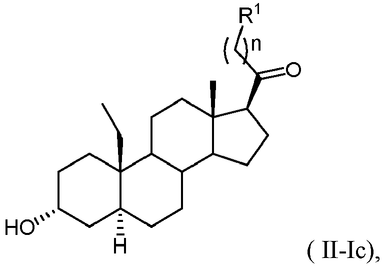 or a pharmaceutically acceptable salt thereof.

In some embodiments, the compound of Formula (II-Ia) is the compound of Formula (II-Ie), 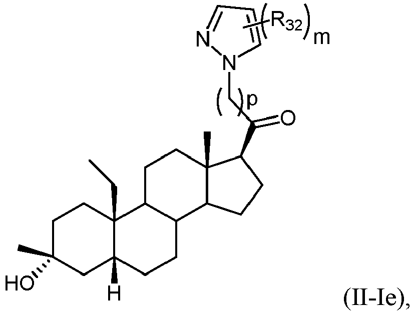 wherein m is 0, 1, 2 or 3; p is 0, 1, or 3; each R₃₂ is independently halogen, alkyl, hydroxyl, or cyano; or a pharmaceutically acceptable salt thereof.

In some embodiments, the compound of Formula (II-Ia) is the compound of Formula (II-Ig), 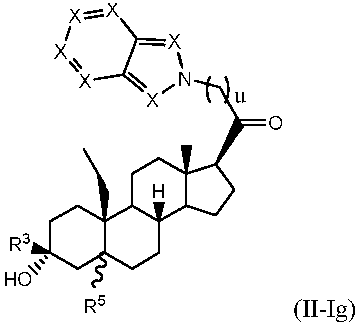 wherein u is 0, 1, or 2; each X is independently -C(R^{N})-, -C(R^{N})₂-, -O-, -S-, -N-, or N(R^{N})-wherein R^{N} is independently hydrogen, substituted or unsubstituted C₁₋₆ alkyl, C(=O)R^{GA}, - C(=O)OR^{GA}, -C(=O)N(R^{GA})₂, -S(=O)₂R^{GA}, or -S(=O)₂N(R^{GA})₂; and each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof.

In some embodiments, the compound of Formula (II-Ia) is the compound of Formula (II-Iga) 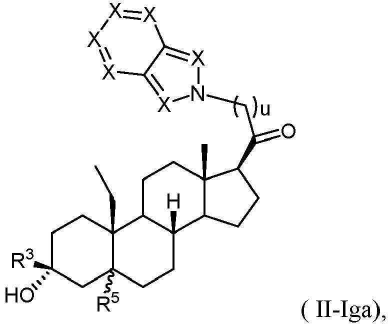 wherein u is 0, 1, or 2; each X is independently -C(R^{N})-, -C(R^{N})₂-, -O-, -S-, -N-, or N(R^{N})-wherein R^{N} is independently hydrogen, substituted or unsubstituted C₁₋₆ alkyl, C(=O)R^{GA}, - C(=O)OR^{GA}, -C(=O)N(R^{GA})₂, -S(=O)₂R^{GA}, or -S(=O)₂N(R^{GA})₂; and each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof.

In some embodiments, the compound of Formula (II-Ia) is the compound of Formula (II-Ih) 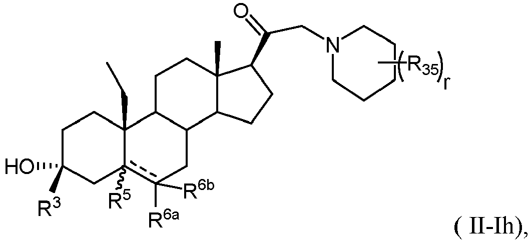 wherein each R₃₅ is independently halogen, alkyl, hydroxyl, or cyano; and r is 0, 1, 2 or 3; or a pharmaceutically acceptable salt thereof.

In some embodiments, the compound of Formula (II-Ia) is the compound of Formula (II-Ii) 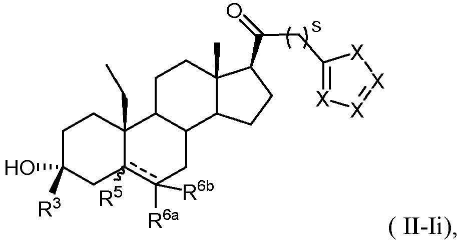 wherein s is 0, 1, or 2; each X is independently -C(R^{N})-, -C(R^{N})₂-, -O-, -S-, -N-, or N(R^{N})-wherein R^{N} is independently hydrogen, substituted or unsubstituted C1-6 alkyl, C(=O)R^{GA}, - C(=O)OR^{GA}, -C(=O)N(R^{GA})₂, -S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof.

In some embodiments, the compound is selected from the group consisting of the compounds identified in **Table II-1** below:

**Table 11-1.**

| **Compound ID** | **STRUCTURE** |
|---|---|
| II**-1** | 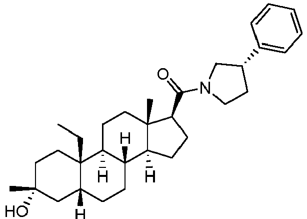 |
| II**-A4** | 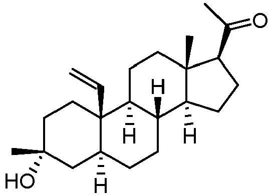 |
| II**-A5** | 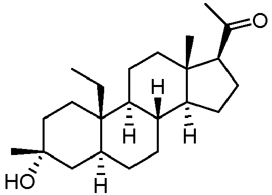 |
| II**-A7** | 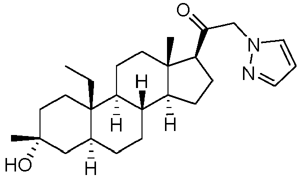 |
| II-**A8** | 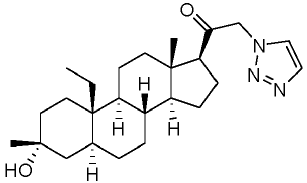 |
| II**-A8a** | 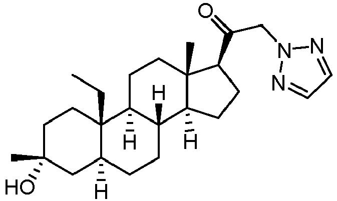 |
| II**-A9** | 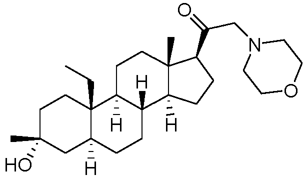 |
| II**-A10** | 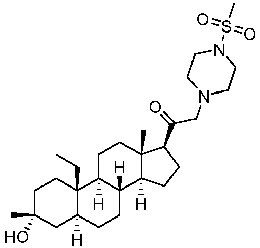 |
| II**-A11** | 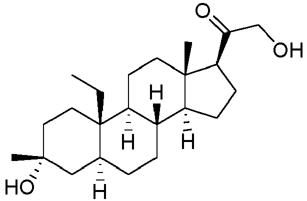 |
| II-**E12** | 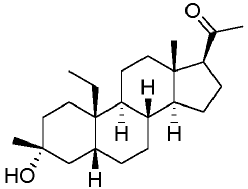 |
| II**-G3** | 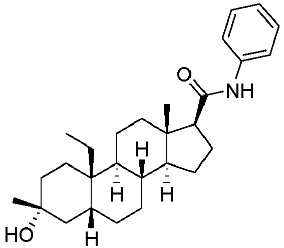 |
| II**-M2** | 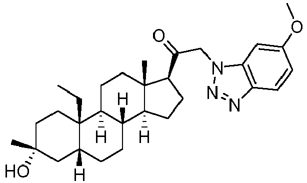 |
| II**-M2a** | 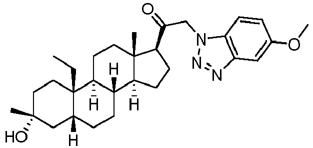 |
| II**-M2b** | 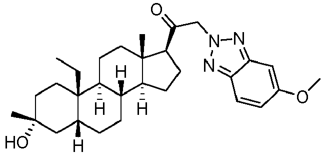 |
| II-**12** | 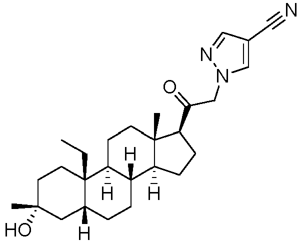 |
| II**-13** | 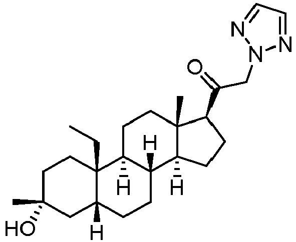 |
| II**-13a** | 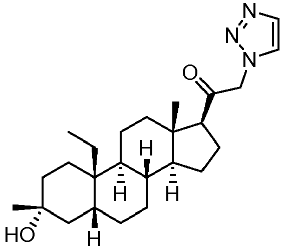 |
| II**-14** | 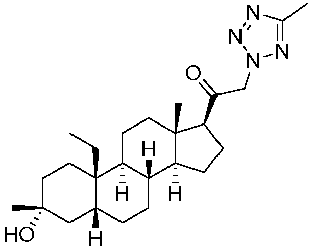 |
| II**-14a** | 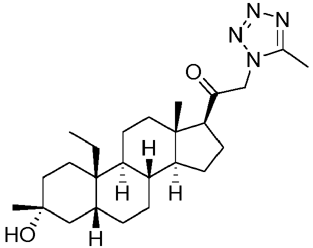 |
| II**-20** | 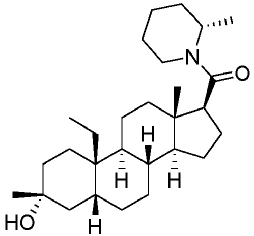 |
| II**-21** | 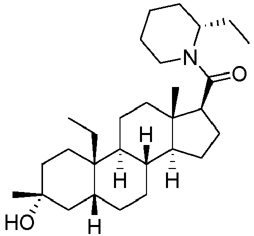 |
| II**-21a** | 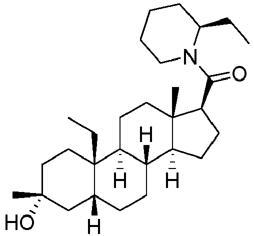 |
| II**-22** | 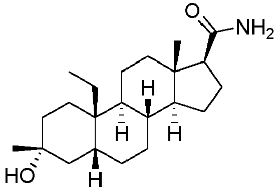 |
| II**-G5** | 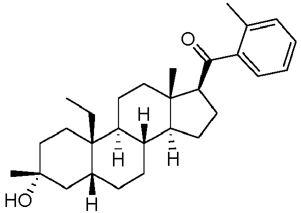 |
| II**-B9** | 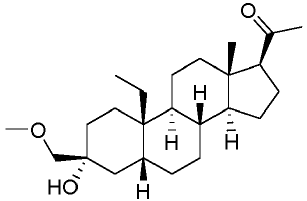 |
| II**-B11** | 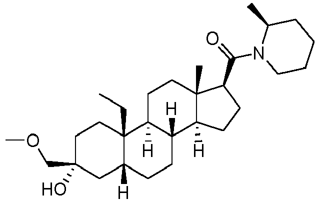 |
| II-**C4** | 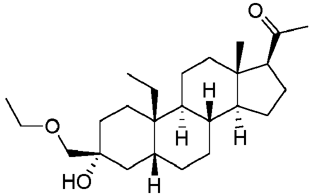 |
| II-**43** | 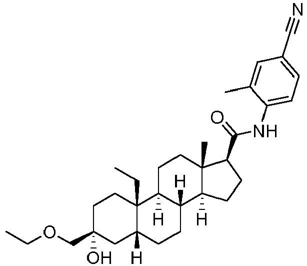 |
| II-**44** | 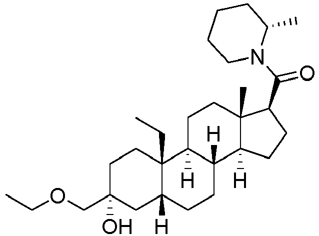 |
| II-**45** | 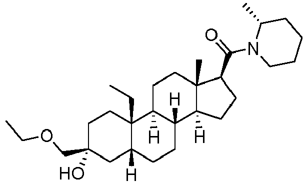 |
| II-**46** | 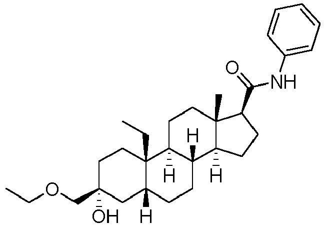 |
| II-**47** | 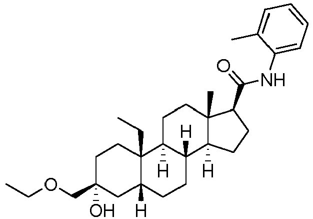 |
| II-**48** | 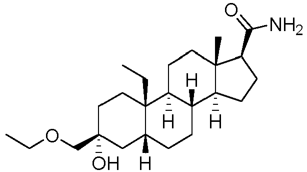 |
| II-**50** | 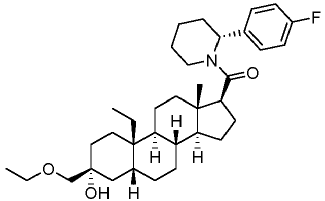 |
| II-**51** | 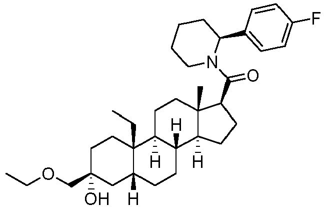 |
| II-**D9** | 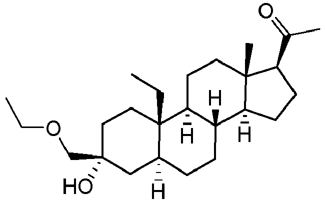 |
| II-**D11** | 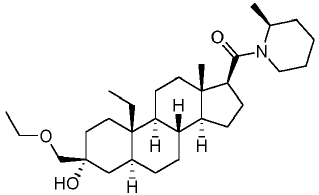 |
| II-**63** | 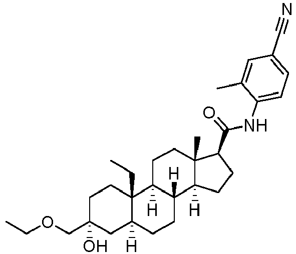 |
| II-E4 | 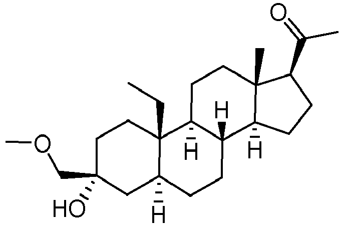 |
| II-E6 | 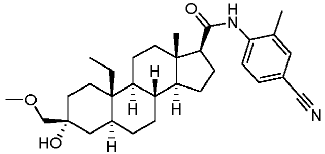 |
| II-**E8** | 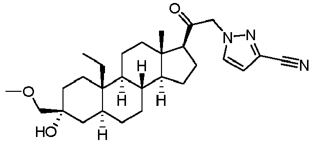 |
| II-**D13** | 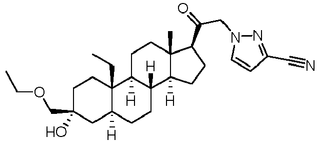 |
| II-**C8** | 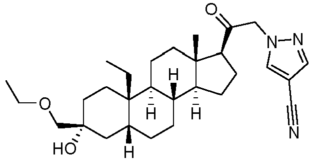 |
| II-**75** | 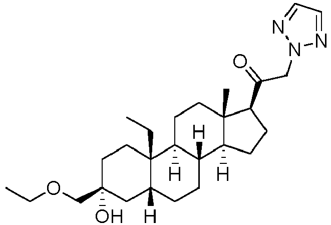 |
| II-**76** | 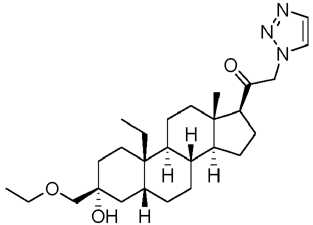 |
| II**-77** | 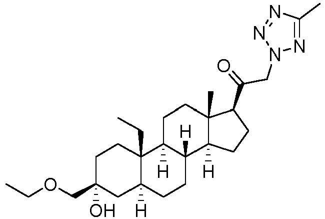 |
| II-**78** | 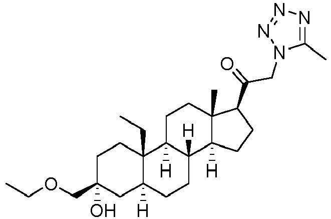 |
| II-**79** | 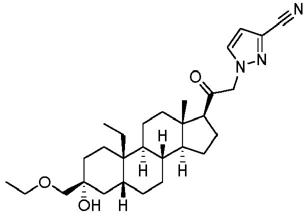 |

In one aspect, provided herein is a pharmaceutically acceptable salt of a compound of the present invention (*e.g.,* a compound of Formula (II-Ia) or compound II-A4).

In one aspect, provided herein is a pharmaceutical composition comprising a compound of the present invention (*e.g.,* a compound of Formula (II-Ia) or compound II-A4) or a pharmaceutically acceptable salt thereof, and a pharmaceutically acceptable excipient.

In certain embodiments, the compound of the present invention is provided in an effective amount in the pharmaceutical composition. In certain embodiments, the compound of the present invention is provided in a therapeutically effective amount.

Compounds of the present invention as described herein, act, in certain embodiments, as GAB_{A} modulators, *e.g.,* effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, *e.g.,* by continuous intravenous infusion.

Exemplary compounds of the invention may be synthesized from the following known starting materials using methods known to one skilled in the art or certain references, In one aspect, provided herein is a pharmaceutically acceptable salt of a compound of the present invention (*e.g.,* a compound of Formula (II-Ia) or compound II-A4).

### Alternative Embodiments

In an alternative embodiment, compounds described herein may also comprise one or more isotopic substitutions. For example, hydrogen may be ²H (D or deuterium) or ³H (T or tritium); carbon may be, for example, ¹³C or ¹⁴C; oxygen may be, for example, ¹⁸O; nitrogen may be, for example, ¹⁵N, and the like. In other embodiments, a particular isotope (e.g., ³H, ¹³C, ¹⁴C, ¹⁸O, or ¹⁵N) can represent at least 1%, at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, at least 99%, or at least 99.9% of the total isotopic abundance of an element that occupies a specific site of the compound.

### Pharmaceutical Compositions

In one aspect, provided herein is a pharmaceutical composition comprising a compound of the present invention (*e.g.,* a compound of Formula (II-Ia) or compound II-A4) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount.

In certain embodiments, the pharmaceutical composition comprises an effective amount of the active ingredient. In certain embodiments, the pharmaceutical composition comprises a therapeutically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds of the present invention are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds of the present invention will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions of the present invention can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g.,* within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g.,* through an IV drip) allows a much faster delivery which quickly raises the concentration of the active ingredient in the blood to an effective level. In other embodiments, the pharmaceutical composition may be administered as a continuous infusion, *e.g.,* by IV drip, to provide maintenance of a steady-state concentration of the active ingredient in the subject's body. Furthermore, in still yet other embodiments, the pharmaceutical composition may be administered as first as a bolus dose, followed by continuous infusion. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 20 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 5 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oilin-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds of the present invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in *Remington's Pharmaceutical Sciences.*

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

In another aspect, the invention provides a pharmaceutical composition comprising a compound of the present invention and a pharmaceutically acceptable excipient, *e.g.,* a composition suitable for injection, such as for intravenous (IV) administration.

Pharmaceutically acceptable excipients include any and all diluents or other liquid vehicles, dispersion or suspension aids, surface active agents, isotonic agents, preservatives, lubricants and the like, as suited to the particular dosage form desired, *e.g.,* injection. General considerations in the formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

For example, injectable preparations, such as sterile injectable aqueous suspensions, can be formulated according to the known art using suitable dispersing or wetting agents and suspending agents. Exemplary excipients that can be employed include, but are not limited to, water, sterile saline or phosphate-buffered saline, or Ringer's solution.

In certain embodiments, the pharmaceutical composition further comprises a cyclodextrin derivative. The most common cyclodextrins are α-, β- and γ- cyclodextrins consisting of 6, 7 and 8 α-1 ,4-linked glucose units, respectively, optionally comprising one or more substituents on the linked sugar moieties, which include, but are not limited to, substituted or unsubstituted methylated, hydroxyalkylated, acylated, and sulfoalkylether substitution. In certain embodiments, the cyclodextrin is a sulfoalkyl ether β-cyclodextrin, *e.g.,* for example, sulfobutyl ether β-cyclodextrin, also known as CAPTISOL^{®}. See, *e.g.,* U.S. 5,376,645. In certain embodiments, the composition comprises hexapropyl-β-cyclodextrin. In a more particular embodiment, the composition comprises hexapropyl-β-cyclodextrin (10-50% in water).

The injectable composition can be sterilized, for example, by filtration through a bacterial-retaining filter, or by incorporating sterilizing agents in the form of sterile solid compositions which can be dissolved or dispersed in sterile water or other sterile injectable medium prior to use.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, response of the individual patient, the severity of the patient's symptoms, and the like.

The compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include pre-filled, premeasured ampules or syringes of the liquid compositions. In such compositions, the compound is usually a minor component (from about 0.1% to about 50% by weight or preferably from about 1% to about 40% by weight) with the remainder being various vehicles or carriers and processing aids helpful for forming the desired dosing form.

The compounds of the present invention can be administered as the sole active agent, or they can be administered in combination with other active agents. In one aspect, the present invention provides a combination of a compound of the present invention and another pharmacologically active agent. Administration in combination can proceed by any technique apparent to those of skill in the art including, for example, separate, sequential, concurrent, and alternating administration.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

In one aspect, provided is a kit comprising a composition (e.g., a solid composition) comprising a compound of Formula (II-Ia) or compound II-A4).

### Methods of Use and Treatment

In an aspect, compounds described herein, *e.g.,* compounds of Formula (II-Ia) or compound II-A4, are envisioned to be useful as therapeutic agents for treating a CNS-related disorder (*e.g.,* sleep disorder, a mood disorder such as depression, a schizophrenia spectrum disorder, a convulsive disorder, epileptogenesis, a disorder of memory and/or cognition, a movement disorder, a personality disorder, autism spectrum disorder, pain, traumatic brain injury, a vascular disease, a substance abuse disorder and/or withdrawal syndrome, or tinnitus) in a subject in need (*e.g*., a subject with Rett syndrome, Fragile X syndrome, or Angelman syndrome). Exemplary CNS conditions related to GABA-modulation include, but are not limited to, sleep disorders [*e.g.,* insomnia], mood disorders [*e.g.,* depression (*e.g*., major depressive disorder (MDD)), dysthymic disorder (*e.g*., mild depression), bipolar disorder (*e.g*., I and/or II), anxiety disorders (*e.g*., generalized anxiety disorder (GAD), social anxiety disorder), stress, post-traumatic stress disorder (PTSD), compulsive disorders (*e.g*., obsessive compulsive disorder (OCD))], schizophrenia spectrum disorders [*e.g*., schizophrenia, schizoaffective disorder], convulsive disorders [*e.g*., epilepsy (*e.g*., status epilepticus (SE)), seizures], disorders of memory and/or cognition [*e.g.,* attention disorders (*e.g*., attention deficit hyperactivity disorder (ADHD)), dementia (*e.g*., Alzheimer's type dementia, Lewis body type dementia, vascular type dementia], movement disorders [*e.g*., Huntington's disease, Parkinson's disease], personality disorders [*e.g*., anti-social personality disorder, obsessive compulsive personality disorder], autism spectrum disorders (ASD) [*e.g*., autism, monogenetic causes of autism such as synaptophathy's, *e.g*., Rett syndrome, Fragile X syndrome, Angelman syndrome], pain [*e.g*., neuropathic pain, injury related pain syndromes, acute pain, chronic pain], traumatic brain injury (TBI), vascular diseases [*e.g*., stroke, ischemia, vascular malformations], substance abuse disorders and/or withdrawal syndromes [*e.g*., addition to opiates, cocaine, and/or alcohol], and tinnitus.

In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder.

In an aspect, provided is a method of alleviating or preventing seizure activity in a subject, comprising administering to the subject in need of such treatment an effective amount of a compound of the present invention. In some embodiments, the method alleviates or prevents epileptogenesis.

In yet another aspect, provided is a combination of a compound of the present invention and another pharmacologically active agent. The compounds provided herein can be administered as the sole active agent or they can be administered in combination with other agents. Administration in combination can proceed by any technique apparent to those of skill in the art including, for example, separate, sequential, concurrent and alternating administration.

In another aspect, provided is a method of treating or preventing brain excitability in a subject susceptible to or afflicted with a condition associated with brain excitability, comprising administering to the subject an effective amount of a compound of the present invention to the subject.

In yet another aspect, provided is a method of treating or preventing stress or anxiety in a subject, comprising administering to the subject in need of such treatment an effective amount of a compound of the present invention, or a composition thereof.

In yet another aspect, provided is a method of alleviating or preventing insomnia in a subject, comprising administering to the subject in need of such treatment an effective amount of a compound of the present invention, or a composition thereof.

In yet another aspect, provided is a method of inducing sleep and maintaining substantially the level of REM sleep that is found in normal sleep, wherein substantial rebound insomnia is not induced, comprising administering an effective amount of a compound of the present invention.

In yet another aspect, provided is a method of alleviating or preventing premenstrual syndrome (PMS) or postnatal depression (PND) in a subject, comprising administering to the subject in need of such treatment an effective amount of a compound of the present invention.

In yet another aspect, provided is a method of treating or preventing mood disorders in a subject, comprising administering to the subject in need of such treatment an effective amount of a compound of the present invention. In certain embodiments the mood disorder is depression.

In yet another aspect, provided is a method of cognition enhancement or treating memory disorder by administering to the subject a therapeutically effective amount of a compound of the present invention. In certain embodiments, the disorder is Alzheimer's disease. In certain embodiments, the disorder is Rett syndrome.

In yet another aspect, provided is a method of treating attention disorders by administering to the subject a therapeutically effective amount of a compound of the present invention. In certain embodiments, the attention disorder is ADHD.

In certain embodiments, the compound is administered to the subject chronically. In certain embodiments, the compound is administered to the subject orally, subcutaneously, intramuscularly, or intravenously.

### Neuroendocrine Disorders and Dysfunction

Provided herein are methods that can be used for treating neuroendocrine disorders and dysfunction. As used herein, "neuroendocrine disorder" or "neuroendocrine dysfunction" refers to a variety of conditions caused by imbalances in the body's hormone production directly related to the brain. Neuroendocrine disorders involve interactions between the nervous system and the endocrine system. Because the hypothalamus and the pituitary gland are two areas of the brain that regulate the production of hormones, damage to the hypothalamus or pituitary gland, e.g., by traumatic brain injury, may impact the production of hormones and other neuroendocrine functions of the brain. In some embodiments, the neuroendocrine disorder or dysfunction is associated with a women's health disorder or condition (e.g., a women's health disorder or condition described herein). In some embodiments, the neuroendocrine disorder or dysfunction is associated with a women's health disorder or condition is polycystic ovary syndrome.

Symptoms of neuroendocrine disorder include, but are not limited to, behavioral, emotional, and sleep-related symptoms, symptoms related to reproductive function, and somatic symptoms; including but not limited to fatigue, poor memory, anxiety, depression, weight gain or loss, emotional lability, lack of concentration, attention difficulties, loss of lipido, infertility, amenorrhea, loss of muscle mass, increased belly body fat, low blood pressure, reduced heart rate, hair loss, anemia, constipation, cold intolerance, and dry skin.

### Neurodegenerative Diseases and Disorders

The methods described herein can be used for treating neurodegenerative diseases and disorders. The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MI,S); closed head injury; coma; contusive injuries (*e.g.,* spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cyloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; Parkinson's disease; seizure; status epilecticus; stroke; tinnitus; tubular sclerosis, and viral infection induced neurodegeneration (*e.g.,* caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

### Mood disorders

Also provided herein are methods for treating a mood disorder, for example clinical depression, postnatal depression or postpartum depression, perinatal depression, atypical depression, melancholic depression, psychotic major depression, cataonic depression, seasonal affective disorder, dysthymia, double depression, depressive personality disorder, recurrent brief depression, minor depressive disorder, bipolar disorder or manic depressive disorder, depression caused by chronic medical conditions, treatment-resistant depression, refractory depression, suicidality, suicidal ideation, or suicidal behavior. In some embodiments, the method described herein provides therapeutic effect to a subject suffering from depression (e.g., moderate or severe depression). In some embodiments, the mood disorder is associated with a disease or disorder described herein (e.g., neuroendocrine diseases and disorders, neurodegenerative diseases and disorders (e.g., epilepsy), movement disorders, tremor (e.g., Parkinson's Disease), women's health disorders or conditions).

**Clinical depression** is also known as major depression, major depressive disorder (MDD), severe depression, unipolar depression, unipolar disorder, and recurrent depression, and refers to a mental disorder characterized by pervasive and persistent low mood that is accompanied by low self-esteem and loss of interest or pleasure in normally enjoyable activities. Some people with clinical depression have trouble sleeping, lose weight, and generally feel agitated and irritable. Clinical depression affects how an individual feels, thinks, and behaves and may lead to a variety of emotional and physical problems. Individuals with clinical depression may have trouble doing day-to-day activities and make an individual feel as if life is not worth living.

**Peripartum depression** refers to depression in pregnancy. Symptoms include irritability, crying, feeling restless, trouble sleeping, extreme exhaustion (emotional and/or physical), changes in appetite, difficulty focusing, increased anxiety and/or worry, disconnected feeling from baby and/or fetus, and losing interest in formerly pleasurable activities.

**Postnatal depression (PND)** is also referred to as **postpartum depression (PPD),** and refers to a type of clinical depression that affects women after childbirth. Symptoms can include sadness, fatigue, changes in sleeping and eating habits, reduced sexual desire, crying episodes, anxiety, and irritability. In some embodiments, the PND is a treatment-resistant depression (e.g., a treatment-resistant depression as described herein). In some embodiments, the PND is refractory depression (e.g., a refractory depression as described herein).

In some embodiments, a subject having PND also experienced depression, or a symptom of depression during pregnancy. This depression is referred to herein as) **perinatal depression.** In an embodiment, a subject experiencing perinatal depression is at increased risk of experiencing PND.

**Atypical depression (AD)** is characterized by mood reactivity (*e.g*., paradoxical anhedonia) and positivity, significant weight gain or increased appetite. Patients suffering from AD also may have excessive sleep or somnolence (hypersomnia), a sensation of limb heaviness, and significant social impairment as a consequence of hypersensitivity to perceived interpersonal rejection.

**Melancholic depression** is characterized by loss of pleasure (anhedonia) in most or all activities, failures to react to pleasurable stimuli, depressed mood more pronounced than that of grief or loss, excessive weight loss, or excessive guilt.

**Psychotic major depression (PMD)** or psychotic depression refers to a major depressive episode, in particular of melancholic nature, where the individual experiences psychotic symptoms such as delusions and hallucinations.

**Catatonic depression** refers to major depression involving disturbances of motor behavior and other symptoms. An individual may become mute and stuporose, and either is immobile or exhibits purposeless or bizarre movements.

**Seasonal affective disorder (SAD)** refers to a type of seasonal depression wherein an individual has seasonal patterns of depressive episodes coming on in the fall or winter.

**Dysthymia** refers to a condition related to unipolar depression, where the same physical and cognitive problems are evident. They are not as severe and tend to last longer (*e.g*., at least 2 years).

**Double depression** refers to fairly depressed mood (dysthymia) that lasts for at least 2 years and is punctuated by periods of major depression.

**Depressive Personality Disorder (DPD)** refers to a personality disorder with depressive features.

**Recurrent Brief Depression (RBD)** refers to a condition in which individuals have depressive episodes about once per month, each episode lasting 2 weeks or less and typically less than 2-3 days.

**Minor depressive disorder** or minor depression refers to a depression in which at least 2 symptoms are present for 2 weeks.

**Bipolar disorder or manic depressive disorder** causes extreme mood swings that include emotional highs (mania or hypomania) and lows (depression). During periods of mania the individual may feel or act abnormally happy, energetic, or irritable. They often make poorly thought out decisions with little regard to the consequences. The need for sleep is usually reduced. During periods of depression there may be crying, poor eye contact with others, and a negative outlook on life. The risk of suicide among those with the disorder is high at greater than 6% over 20 years, while self-harm occurs in 30-40%. Other mental health issues such as anxiety disorder and substance use disorder are commonly associated with bipolar disorder.

**Depression caused by chronic medical conditions** refers to depression caused by chronic medical conditions such as cancer or chronic pain, chemotherapy, chronic stress.

**Treatment-resistant depression** refers to a condition where the individuals have been treated for depression, but the symptoms do not improve. For example, antidepressants or physchological counseling (psychotherapy) do not ease depression symptoms for individuals with treatment-resistant depression. In some cases, individuals with treatment-resistant depression improve symptoms, but come back. **Refractory depression** occurs in patients suffering from depression who are resistant to standard pharmacological treatments, including tricyclic antidepressants, MAOIs, SSRIs, and double and triple uptake inhibitors and/or anxiolytic drugs, as well as non-pharmacological treatments (e.g., psychotherapy, electroconvulsive therapy, vagus nerve stimulation and/or transcranial magnetic stimulation).

**Post-surgical depression** refers to feelings of depression that follow a surgical procedure (e.g., as a result of having to confront one's mortality). For example, individuals may feel sadness or empty mood persistently, a loss of pleasure or interest in hobbies and activities normally enjoyed, or a persistent felling of worthlessness or hopelessness.

**Mood disorder associated with conditions or disorders of women's health** refers to mood disorders (e.g., depression) associated with (e.g., resulting from) a condition or disorder of women's health (e.g., as described herein).

**Suicidality, suicidal ideation, suicidal behavior** refers to the tendency of an individual to commit suicide. Suicidal ideation concerns thoughts about or an unusual preoccupation with suicide. The range of suicidal ideation varies greatly, from e.g., fleeting thoughts to extensive thoughts, detailed planning, role playing, incomplete attempts. Symptoms include talking about suicide, getting the means to commit suicide, withdrawing from social contact, being preoccupied with death, feeling trapped or hopeless about a situation, increasing use of alcohol or drugs, doing risky or self-destructive things, saying goodbye to people as if they won't be seen again.

**Symptoms** of depression include persistent anxious or sad feelings, feelings of helplessness, hopelessness, pessimism, worthlessness, low energy, restlessness, difficulty sleeping, sleeplessness, irritability, fatigue, motor challenges, loss of interest in pleasurable activities or hobbies, loss of concentration, loss of energy, poor self-esteem, absence of positive thoughts or plans, excessive sleeping, overeating, appetite loss, insomnia, self-harm, thoughts of suicide, and suicide attempts. The presence, severity, frequency, and duration of symptoms may vary on a case to case basis. Symptoms of depression, and relief of the same, may be ascertained by a physician or psychologist (e.g., by a mental state examination).

In some embodiments, the method comprises monitoring a subject with a known depression scale, *e.g.,* the Hamilton Depression (HAM-D) scale, the Clinical Global Impression-Improvement Scale (CGI), and the Montgomery-Åsberg Depression Rating Scale (MADRS). In some embodiments, a therapeutic effect can be determined by reduction in Hamilton Depression (HAM-D) total score exhibited by the subject. Reduction in the HAM-D total score can happen within 4, 3, 2, or 1 days; or 96, 84, 72, 60, 48, 24, 20, 16, 12, 10, 8 hours or less. The therapeutic effect can be assessed across a specified treatment period. For example, the therapeutic effect can be determined by a decrease from baseline in HAM-D total score after administering a compound-of the present invention, e.g., a compound of Formula (II-Ia) or compound II-A4 (e.g., 12, 24, or 48 hours after administration; or 24, 48, 72, or 96 hours or more; or 1 day, 2 days, 14 days, 21 days, or 28 days; or 1 week, 2 weeks, 3 weeks, or 4 weeks; or 1 month, 2 months, 6 months, or 10 months; or 1 year, 2 years, or for life).

In some embodiments, the subject has a mild depressive disorder, *e.g.,* mild major depressive disorder. In some embodiments, the subject has a moderate depressive disorder, *e.g.,* moderate major depressive disorder. In some embodiments, the subject has a severe depressive disorder, *e.g*., severe major depressive disorder. In some embodiments, the subject has a very severe depressive disorder, e*.g.*, very severe major depressive disorder. In some embodiments, the baseline HAM-D total score of the subject (i.e., prior to treatment with a compound of the present invention, *e.g*., a compound of Formula (II-Ia) or compound II-A4 is at least 24. In some embodiments, the baseline HAM-D total score of the subject is at least 18. In some embodiments, the baseline HAM-D total score of the subject is between and including 14 and 18. In some embodiments, the baseline HAM-D total score of the subject is between and including 19 and 22. In some embodiments, the HAM-D total score of the subject before treatment with a compound of the present invention, *e.g.,* a compound of Formula (II-Ia) or compound II-A4, is greater than or equal to 23. In some embodiments, the baseline score is at least 10, 15, or 20. In some embodiments, the HAM-D total score of the subject after treatment with a compound of the present invention, *e.g.,* a compound of Formula (II-Ia) or compound II-A4, is about 0 to 10 (e.g., less than 10; 0 to 10, 0 to 6, 0 to 4, 0 to 3, 0 to 2, or 1.8). In some embodiments, the HAM-D total score after treatment with a compound of the present invention, *e.g.,* a compound of Formula (II-Ia) or compound II-A4, is less than 10, 7, 5, or 3. In some embodiments, the decrease in HAM-D total score is from a baseline score of about 20 to 30 (e.g., 22 to 28, 23 to 27, 24 to 27, 25 to 27, 26 to 27) to a HAM-D total score at about 0 to 10 (e.g., less than 10; 0 to 10, 0 to 6, 0 to 4, 0 to 3, 0 to 2, or 1.8) after treatment with a compound of the present invention, *e.g.,* a compound of Formula (II-Ia) or compound II-A4. In some embodiments, the decrease in the baseline HAM-D total score to HAM-D total score after treatment with a compound of the present invention, e.g., a compound of Formula (II-Ia) or compound II-A4, is at least 1, 2, 3, 4, 5, 7, 10, 25, 40, 50, or 100 fold). In some embodiments, the percentage decrease in the baseline HAM-D total score to HAM-D total score after treatment with a compound of the present invention, *e.g.,* a compound of Formula (II-Ia) or compound II-A4, is at least 50% (e.g., 60%, 70%, 80%, or 90%). In some embodiments, the therapeutic effect is measured as a decrease in the HAM-D total score after treatment with a compound of the present invention, *e.g.,* a compound of Formula (II-Ia) or compound II-A4, relative to the baseline HAM-D total score (e.g., 12, 24, 48 hours after administration; or 24, 48, 72, 96 hours or more; or 1 day, 2 days, 14 days, or more) is at least 10, 15, or 20 points.

In some embodiments, the method of treating a depressive disorder, e.g., major depressive disorder provides a therapeutic effect (e.g., as measured by reduction in Hamilton Depression Score (HAM-D)) within 14, 10, 4, 3, 2, or 1 days, or 24, 20, 16, 12, 10, or 8 hours or less. In some embodiments, the method of treating the depressive disorder, e.g., major depressive disorder, provides a therapeutic effect (*e.g.,* as determined by a statistically significant reduction in HAM-D total score) within the first or second day of the treatment with a compound of the present invention, *e.g.,* a compound of Formula (II-Ia) or compound II-A4. In some embodiments, the method of treating the depressive disorder, e.g., major depressive disorder, provides a therapeutic effect (*e.g.,* as determined by a statistically significant reduction in HAM-D total score) within less than or equal to 14 days since the beginning of the treatment with a compound of the present invention, *e.g.,* a compound of Formula (II-Ia) or compound II-A4. In some embodiments, the method of treating the depressive disorder, e.g., major depressive disorder, provides a therapeutic effect (*e.g.,* as determined by a statistically significant reduction in HAM-D total score) within less than or equal to 21 days since the beginning of the treatment with a compound of the present invention, *e.g.,* a compound of Formula (II-Ia) or compound II-A4. In some embodiments, the method of treating the depressive disorder, e.g., major depressive disorder, provides a therapeutic effect (*e.g.,* as determined by a statistically significant reduction in HAM-D total score) within less than or equal to 28 days since the beginning of the treatment with a compound of the present invention, *e.g.,* a compound of Formula (II-Ia) or compound II-A4. In some embodiments, the therapeutic effect is a decrease from baseline in HAM-D total score after treatment with a compound of the present invention, *e.g.,* a compound of Formula (II-Ia) or compound II-A4 (e.g., treatment with a compound of the present invention, *e.g.,* a compound of Formula (II-Ia) or compound II-A4, once a day for 14 days). In some embodiments, the HAM-D total score of the subject before treatment with a compound of the present invention, *e.g.,* a compound of Formula (II-Ia) or compound II-A4, is at least 24. In some embodiments, the HAM-D total score of the subject before treatment with a compound of the present invention, *e.g.,* a compound of Formula (II-Ia) or compound II-A4, is at least 18. In some embodiments, the HAM-D total score of the subject before treatment with a compound of the present invention, *e.g.,* a compound of Formula (II-Ia) or compound II-A4, is between and including 14 and 18. In some embodiments, the decrease in HAM-D total score after treating the subject with a compound of the present invention, *e.g.,* a compound of Formula (II-Ia) or compound II-A4, relative to the baseline HAM-D total score is at least 10. In some embodiments, the decrease in HAM-D total score after treating the subject with a compound of the present invention, *e.g.,* a compound of Formula (II-Ia) or compound II-A4, relative to the baseline HAM-D total score is at least 15 (*e.g.,* at least 17). In some embodiments, the HAM-D total score associated with treating the subject with a compound of the present invention, *e.g.,* a compound of Formula (II-Ia) or compound II-A4, is no more than a number ranging from 6 to 8. In some embodiments, the HAM-D total score associated with treating the subject with a compound of the present invention, *e.g.,* a compound of Formula (II-Ia) or compound II-A4, is no more than 7.

In some embodiments, the method provides therapeutic effect (e.g., as measured by reduction in Clinical Global Impression-Improvement Scale (CGI)) within 14, 10, 4, 3, 2, or 1 days, or 24, 20, 16, 12, 10, or 8 hours or less. In some embodiments, the CNS-disorder is a depressive disorder, *e.g.,* major depressive disorder. In some embodiments, the method of treating the depressive disorder, *e.g.,* major depressive disorder provides a therapeutic effect within the second day of the treatment period. In some embodiments, the therapeutic effect is a decrease from baseline in CGI score at the end of a treatment period (e.g., 14 days after administration).

In some embodiments, the method provides therapeutic effect (e.g., as measured by reduction in Montgomery-Åsberg Depression Rating Scale (MADRS)) within 14, 10, 4, 3, 2, or 1 days, or 24, 20, 16, 12, 10, or 8 hours or less. In some embodiments, the CNS-disorder is a depressive disorder, *e.g.,* major depressive disorder. In some embodiments, the method of treating the depressive disorder, *e.g.,* major depressive disorder provides a therapeutic effect within the second day of the treatment period. In some embodiments, the therapeutic effect is a decrease from baseline in MADRS score at the end of a treatment period (e.g., 14 days after administration).

A therapeutic effect for major depressive disorder can be determined by a reduction in Montgomery-Åsberg Depression Rating Scale (MADRS) score exhibited by the subject. For example, the MADRS score can be reduced within 4, 3, 2, or 1 days; or 96, 84, 72, 60, 48, 24, 20, 16, 12, 10, 8 hours or less. The Montgomery-Åsberg Depression Rating Scale (MADRS) is a ten-item diagnostic questionnaire (regarding apparent sadness, reported sadness, inner tension, reduced sleep, reduced appetite, concentration difficulties, lassitude, inability to feel, pessimistic thoughts, and suicidal thoughts) which psychiatrists use to measure the severity of depressive episodes in patients with mood disorders.

In some embodiments, the method provides therapeutic effect (e.g., as measured by reduction in Edinburgh Postnatal Depression Scale (EPDS)) within 4, 3, 2, 1 days; 24, 20, 16, 12, 10, 8 hours or less. In some embodiments, the therapeutic effect is an improvement measured by the EPDS.

In some embodiments, the method provides therapeutic effect (e.g., as measured by reduction in Generalized Anxiety Disorder 7-Item Scale (GAD-7)) within 4, 3, 2, 1 days; 24, 20, 16, 12, 10, 8 hours or less.

### Anxiety Disorders

Provided herein are methods for treating anxiety disorders (e.g., generalized anxiety disorder, panic disorder, obsessive compulsive disorder, phobia, post-traumatic stress disorder). **Anxiety disorder** is a blanket term covering several different forms of abnormal and pathological fear and anxiety. Current psychiatric diagnostic criteria recognize a wide variety of anxiety disorders.

**Generalized anxiety disorder** is a common chronic disorder characterized by long-lasting anxiety that is not focused on any one object or situation. Those suffering from generalized anxiety experience non-specific persistent fear and worry and become overly concerned with everyday matters. Generalized anxiety disorder is the most common anxiety disorder to affect older adults.

In **panic disorder,** a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attacks' potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

**Obsessive compulsive disorder** is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of **phobia,** which includes all cases in which fear and anxiety is triggered by a specific stimulus or situation. Sufferers typically anticipate terrifying consequences from encountering the object of their fear, which can be anything from an animal to a location to a bodily fluid.

**Post-traumatic stress disorder** or **PTSD** is an anxiety disorder which results from a traumatic experience. Post-traumatic stress can result from an extreme situation, such as combat, rape, hostage situations, or even serious accident. It can also result from long term

(chronic) exposure to a severe stressor, for example soldiers who endure individual battles but cannot cope with continuous combat. Common symptoms include flashbacks, avoidant behaviors, and depression.

### Women's Health Disorders

Provided herein are methods for treating conditions or disorders related to women's health. Conditions or disorders related to women's health include, but are not limited to, gynecological health and disorders (e.g., premenstrual syndrome (PMS), premenstrual dysphoric disorder (PMDD)), **pregnancy issues** (e.g., miscarriage, abortion), infertility and related disorders (e.g., polycystic ovary syndrome (PCOS)), other disorders and conditions, and issues related to women's overall health and wellness (e.g., menopause).

**Gynecological health and disorders** affecting women include menstruation and menstrual irregularities; urinary tract health, including urinary incontinence and pelvic floor disorders; and such disorders as bacterial vaginosis, vaginitis, uterine fibroids, and vulvodynia.

**Premenstrual syndrome (PMS)** refers to physical and emotional symptoms that occur in the one to two weeks before a women's period. Symptoms vary but can include bleeding, mood swings, tender breasts, food cravings, fatigue, irritability, acne, and depression.

**Premenstrual dysphoric disorder (PMDD)** is a severe form of PMS. The symptoms of PMDD are similar to PMS but more severe and may interfere with work, social activity, and relationships. PMDD symptoms include mood swings, depressed mood or feelings of hopelessness, marked anger, increased interpersonal conflicts, tension and anxiety, irritability, decreased interest in usual activities, difficulty concentrating, fatigue, change in appetite, feeling out of control or overwhelmed, sleep problems, physical problems (e.g., bloating, breast tenderness, swelling, headaches, joint or muscle pain).

**Pregnancy issues** include preconception care and prenatal care, pregnancy loss (miscarriage and stillbirth), preterm labor and premature birth, sudden infant death syndrome (SIDS), breastfeeding, and birth defects.

**Miscarriage** refers to a pregnancy that ends on its own, within the first 20 weeks of gestation.

**Abortion** refers to the deliberate termination of a pregnancy, which can be performed during the first 28 weeks of pregnancy.

**Infertility and related disorders** include uterine fibroids, polycystic ovary syndrome, endometriosis, and primary ovarian insufficiency.

**Polycystic ovary syndrome (PCOS)** refers to an endocrine system disorder among women of reproductive age. PCOS is a set of symptoms resulting from an elevated male hormone in women. Most women with PCOS grow many small cysts on their ovaries. Symptoms of PCOS include irregular or no menstrual periods, heavy periods, excess body and facial hair, acne, pelvic pain, difficulty getting pregnant, and patches of thick, darker, velvety skin. PCOS may be associated with conditions including type 2 diabetes, obesity, obstructive sleep apnea, heart disease, mood disorders, and endometrial cancer.

**Other disorders and conditions** that affect only women include Turner syndrome, Rett syndrome, and ovarian and cervical cancers.

**Issues related to women's overall health and wellness** include violence against women, women with disabilities and their unique challenges, osteoporosis and bone health, and menopause.

**Menopause** refers to the 12 months after a woman's last menstrual period and marks the end of menstrual cycles. Menopause typically occurs in a woman's 40s or 50s. Physical symptoms such as hot flashes and emotional symptoms of menopause may disrupt sleep, lower energy, or trigger anxiety or feelings of sadness or loss. Menopause includes natural menopause and surgical menopause, which is a type of induced menopause due to an event such as surgery (e.g., hysterectomy, oophorectomy; cancer). It is induced when the ovaries are gravely damaged by, e.g., radiation, chemotherapy, or other medications.

### Epilepsy

The compound of Formula (II-Ia) or compound II-A4, or pharmaceutically acceptable salt, or a pharmaceutically acceptable composition thereof, can be used in a method described herein, for example in the treatment of a disorder described herein such as epilepsy, status epilepticus, or seizure.

Epilepsy is a brain disorder characterized by repeated seizures over time. Types of epilepsy can include, but are not limited to generalized epilepsy, *e.g.,* childhood absence epilepsy, juvenile nyoclonic epilepsy, epilepsy with grand-mal seizures on awakening, West syndrome, Lennox-Gastaut syndrome, partial epilepsy, *e.g.,* temporal lobe epilepsy, frontal lobe epilepsy, benign focal epilepsy of childhood.

### Epileptogenesis

The compounds and methods described herein can be used to treat or prevent epileptogenesis. Epileptogenesis is a gradual process by which a normal brain develops epilepsy (a chronic condition in which seizures occur). Epileptogenesis results from neuronal damage precipitated by the initial insult (e.g., status epilepticus).

### Status epilepticus (SE)

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus, *e.g.,* generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, *e.g.,* complex partial non-convulsive status epilepticus, simple partial non-convulsive status epilepticus, subtle non-convulsive status epilepticus; generalized non-convulsive status epilepticus, *e.g.,* late onset absence non-convulsive status epilepticus, atypical absence non-convulsive status epilepticus, or typical absence non-convulsive status epilepticus.

The compound of Formula (II-Ia) or compound II-A4 or pharmaceutically acceptable salt, or a pharmaceutically acceptable composition thereof, can also be administered as a prophylactic to a subject having a CNS disorder *e.g.,* a traumatic brain injury, status epilepticus, *e.g.,* convulsive status epilepticus, *e.g.,* early status epilepticus, established status epilepticus, refractory status epilepticus, super-refractory status epilepticus; non-convulsive status epilepticus, *e.g.,* generalized status epilepticus, complex partial status epilepticus; generalized periodic epileptiform discharges; and periodic lateralized epileptiform discharges; prior to the onset of a seizure.

### Seizure

A seizure is the physical findings or changes in behavior that occur after an episode of abnormal electrical activity in the brain. The term "seizure" is often used interchangeably with "convulsion." Convulsions are when a person's body shakes rapidly and uncontrollably. During convulsions, the person's muscles contract and relax repeatedly.

Based on the type of behavior and brain activity, seizures are divided into two broad categories: generalized and partial (also called local or focal). Classifying the type of seizure helps doctors diagnose whether or not a patient has epilepsy.

Generalized seizures are produced by electrical impulses from throughout the entire brain, whereas partial seizures are produced (at least initially) by electrical impulses in a relatively small part of the brain. The part of the brain generating the seizures is sometimes called the focus.

There are six types of generalized seizures. The most common and dramatic, and therefore the most well-known, is the generalized convulsion, also called the grand-mal seizure. In this type of seizure, the patient loses consciousness and usually collapses. The loss of consciousness is followed by generalized body stiffening (called the "tonic" phase of the seizure) for 30 to 60 seconds, then by violent jerking (the "clonic" phase) for 30 to 60 seconds, after which the patient goes into a deep sleep (the "postictal" or after-seizure phase). During grand-mal seizures, injuries and accidents may occur, such as tongue biting and urinary incontinence.

Absence seizures cause a short loss of consciousness (just a few seconds) with few or no symptoms. The patient, most often a child, typically interrupts an activity and stares blankly. These seizures begin and end abruptly and may occur several times a day. Patients are usually not aware that they are having a seizure, except that they may be aware of "losing time."

Myoclonic seizures consist of sporadic jerks, usually on both sides of the body. Patients sometimes describe the jerks as brief electrical shocks. When violent, these seizures may result in dropping or involuntarily throwing objects.

Clonic seizures are repetitive, rhythmic jerks that involve both sides of the body at the same time.

Tonic seizures are characterized by stiffening of the muscles.

Atonic seizures consist of a sudden and general loss of muscle tone, particularly in the arms and legs, which often results in a fall.

Seizures described herein can include epileptic seizures; acute repetitive seizures; cluster seizures; continuous seizures; unremitting seizures; prolonged seizures; recurrent seizures; status epilepticus seizures, e.g., refractory convulsive status epilepticus, non-convulsive status epilepticus seizures; refractory seizures; myoclonic seizures; tonic seizures; tonic-clonic seizures; simple partial seizures; complex partial seizures; secondarily generalized seizures; atypical absence seizures; absence seizures; atonic seizures; benign Rolandic seizures; febrile seizures; emotional seizures; focal seizures; gelastic seizures; generalized onset seizures; infantile spasms; Jacksonian seizures; massive bilateral myoclonus seizures; multifocal seizures; neonatal onset seizures; nocturnal seizures; occipital lobe seizures; post traumatic seizures; subtle seizures; Sylvan seizures; visual reflex seizures; or withdrawal seizures. In some embodiments, the seizure is a generalized seizure associated with Dravet Syndrome, Lennox-Gastaut Syndrome, Tuberous Sclerosis Complex, Rett Syndrome or PCDH19 Female Pediatric Epilepsy.

### Movement Disorders

Also described herein are methods for treating a movement disorder. As used herein, "movement disorders" refers to a variety of diseases and disorders that are associated with hyperkinetic movement disorders and related abnormalities in muscle control. Exemplary movement disorders include, but are not limited to, Parkinson's disease and parkinsonism (defined particularly by bradykinesia), dystonia, chorea and Huntington's disease, ataxia, tremor (e.g., essential tremor), myoclonus and startle, tics and Tourette syndrome, Restless legs syndrome, stiff person syndrome, and gait disorders.

### Tremor

The methods described herein can be used to treat tremor, for example the compound of Formula (II-Ia) or compound II-A4 can be used to treat cerebellar tremor or intention tremor, dystonic tremor, essential tremor, orthostatic tremor, parkinsonian tremor, physiological tremor, psychogenic tremor, or rubral tremor. Tremor includes hereditary, degenerative, and idiopathic disorders such as Wilson's disease, Parkinson's disease, and essential tremor, respectively; metabolic diseases (e.g., thyroid-parathyroid-, liver disease and hypoglycemia); peripheral neuropathies (associated with Charcot-Marie-Tooth, Roussy-Levy, diabetes mellitus, complex regional pain syndrome); toxins (nicotine, mercury, lead, CO, Manganese, arsenic, toluene); drug-induced (narcoleptics, tricyclics, lithium, cocaine, alcohol, adrenaline, bronchodilators, theophylline, caffeine, steroids, valproate, amiodarone, thyroid hormones, vincristine); and psychogenic disorders. Clinical tremor can be classified into physiologic tremor, enhanced physiologic tremor, essential tremor syndromes (including classical essential tremor, primary orthostatic tremor, and task- and position-specific tremor), dystonic tremor, parkinsonian tremor, cerebellar tremor, Holmes' tremor (i.e., rubral tremor), palatal tremor, neuropathic tremor, toxic or drug-induced tremor, and psychogenic tremor.

**Tremor** is an involuntary, at times rhythmic, muscle contraction and relaxation that can involve oscillations or twitching of one or more body parts (*e.g.,* hands, arms, eyes, face, head, vocal folds, trunk, legs).

**Cerebellar tremor** or **intention tremor** is a slow, broad tremor of the extremities that occurs after a purposeful movement. Cerebellar tremor is caused by lesions in or damage to the cerebellum resulting from, *e.g.,* tumor, stroke, disease (*e.g.,* multiple sclerosis, an inherited degenerative disorder).

**Dystonic tremor** occurs in individuals affected by dystonia, a movement disorder in which sustained involuntary muscle contractions cause twisting and repetitive motions and/or painful and abnormal postures or positions. Dystonic tremor may affect any muscle in the body. Dystonic tremors occurs irregularly and often can be relieved by complete rest.

**Essential tremor** or benign essential tremor is the most common type of tremor. Essential tremor may be mild and nonprogressive in some, and may be slowly progressive, starting on one side of the body but affect both sides within 3 years. The hands are most often affected, but the head, voice, tongue, legs, and trunk may also be involved. Tremor frequency may decrease as the person ages, but severity may increase. Heightened emotion, stress, fever, physical exhaustion, or low blood sugar may trigger tremors and/or increase their severity. Symptoms generally evolve over time and can be both visible and persistent following onset.

**Orthostatic tremor** is characterized by fast (*e.g.,* greater than 12 Hz) rhythmic muscle contractions that occurs in the legs and trunk immediately after standing. Cramps are felt in the thighs and legs and the patient may shake uncontrollably when asked to stand in one spot. Orthostatic tremor may occurs in patients with essential tremor.

**Parkinsonian tremor** is caused by damage to structures within the brain that control movement. Parkinsonian tremor is often a precursor to Parkinson's disease and is typically seen as a "pill-rolling" action of the hands that may also affect the chin, lips, legs, and trunk. Onset of parkinsonian tremor typically begins after age 60. Movement starts in one limb or on one side of the body and can progress to include the other side.

**Physiological tremor** can occur in normal individuals and have no clinical significance. It can be seen in all voluntary muscle groups. Physiological tremor can be caused by certain drugs, alcohol withdrawal, or medical conditions including an overactive thyroid and hypoglycemia. The tremor classically has a frequency of about 10 Hz.

**Psychogenic tremor** or hysterical tremor can occur at rest or during postural or kinetic movement. Patient with psychogenic tremor may have a conversion disorder or another psychiatric disease.

**Rubral tremor** is characterized by coarse slow tremor which can be present at rest, at posture, and with intention. The tremor is associated with conditions that affect the red nucleus in the midbrain, classical unusual strokes.

**Parkinson's Disease** affects nerve cells in the brain that produce dopamine. Symptoms include muscle rigidity, tremors, and changes in speech and gait. **Parkinsonism** is characterized by tremor, bradykinesia, rigidity, and postural instability. Parkinsonism shares symptoms found in Parkinson's Disease, but is a symptom complex rather than a progressive neurodegenerative disease.

**Dystonia** is a movement disorder characterized by sustained or intermittent muscle contractions causing abnormal, often repetitive movements or postures. Dystonic movements can be patterned, twisting, and may be tremulous. Dystonia is often initiated or worsened by voluntary action and associated with overflow muscle activation.

**Chorea** is a neurological disorder characterized by jerky involuntary movements typically affecting the shoulders, hips, and face. **Huntington's Disease** is an inherited disease that causes nerve cells in the brain to waste away. Symptoms include uncontrolled movements, clumsiness, and balance problems. Huntington's disease can hinder walk, talk, and swallowing.

**Ataxia** refers to the loss of full control of bodily movements, and may affect the fingers, hands, arms, legs, body, speech, and eye movements.

**Myloclonus and Startle** is a response to a sudden and unexpected stimulus, which can be acoustic, tactile, visual, or vestibular.

**Tics** are an involuntary movement usually onset suddenly, brief, repetitive, but non-rhythmical, typically imitating normal behavior and often occurring out of a background of normal activity. Tics can be classified as motor or vocal, motor tics associated with movements while vocal tics associated with sound. Tics can be characterized as simple or complex. For example simple motor tics involve only a few muscles restricted to a specific body part. **Tourette Syndrome** is an inherited neuropsychiatric disorder with onset in childhood, characterized by multiple motor tics and at least one vocal tic.

**Restless Legs Syndrome** is a neurologic sensorimotor disorder characterized by an overwhelming urge to move the legs when at rest.

**Stiff Person Syndrome** is a progressive movement disorder characterized by involuntary painful spasms and rigidity of muscles, usually involving the lower back and legs. Stiff-legged gait with exaggerated lumbar hyperlordosis typically results. Characteristic abnormality on EMG recordings with continuous motor unit activity of the paraspinal axial muscles is typically observed. Variants include "stiff-limb syndrome" producing focal stiffness typically affecting distal legs and feet.

**Gait disorders** refer to an abnormality in the manner or style of walking, which results from neuromuscular, arthritic, or other body changes. Gait is classified according to the system responsible for abnormal locomotion, and include hemiplegic gait, diplegic gait, neuropathic gait, myopathic gait, parkinsonian gait, choreiform gait, ataxic gait, and sensory gait.

### Anesthesia / Sedation

Anesthesia is a pharmacologically induced and reversible state of amnesia, analgesia, loss of responsiveness, loss of skeletal muscle reflexes, decreased stress response, or all of these simultaneously. These effects can be obtained from a single drug which alone provides the correct combination of effects, or occasionally with a combination of drugs (*e.g.,* hypnotics, sedatives, paralytics, analgesics) to achieve very specific combinations of results. Anesthesia allows patients to undergo surgery and other procedures without the distress and pain they would otherwise experience.

Sedation is the reduction of irritability or agitation by administration of a pharmacological agent, generally to facilitate a medical procedure or diagnostic procedure.

Sedation and analgesia include a continuum of states of consciousness ranging from minimal sedation (anxiolysis) to general anesthesia.

**Minimal sedation is a**lso known as anxiolysis. Minimal sedation is a drug-induced state during which the patient responds normally to verbal commands. Cognitive function and coordination may be impaired. Ventilatory and cardiovascular functions are typically unaffected.

**Moderate sedation/analgesia (conscious sedation) is a** drug-induced depression of consciousness during which the patient responds purposefully to verbal command, either alone or accompanied by light tactile stimulation. No interventions are usually necessary to maintain a patent airway. Spontaneous ventilation is typically adequate. Cardiovascular function is usually maintained.

**Deep sedation/analgesia is a** drug-induced depression of consciousness during which the patient cannot be easily aroused, but responds purposefully (not a reflex withdrawal from a painful stimulus) following repeated or painful stimulation. Independent ventilatory function may be impaired and the patient may require assistance to maintain a patent airway. Spontaneous ventilation may be inadequate. Cardiovascular function is usually maintained.

**General anesthesia is a** drug-induced loss of consciousness during which the patient is not arousable, even to painful stimuli. The ability to maintain independent ventilatory function is often impaired and assistance is often required to maintain a patent airway. Positive pressure ventilation may be required due to depressed spontaneous ventilation or drug-induced depression of neuromuscular function. Cardiovascular function may be impaired.

Sedation in the intensive care unit (ICU) allows the depression of patients' awareness of the environment and reduction of their response to external stimulation. It can play a role in the care of the critically ill patient, and encompasses a wide spectrum of symptom control that will vary between patients, and among individuals throughout the course of their illnesses. Heavy sedation in critical care has been used to facilitate endotracheal tube tolerance and ventilator synchronization, often with neuromuscular blocking agents.

In some embodiments, sedation (*e.g.,* long-term sedation, continuous sedation) is induced and maintained in the ICU for a prolonged period of time (*e.g.,* 1 day, 2 days, 3 days, 5 days, 1 week, 2 week, 3 weeks, 1 month, 2 months). Long-term sedation agents may have long duration of action. Sedation agents in the ICU may have short elimination half-life.

Procedural sedation and analgesia, also referred to as conscious sedation, is a technique of administering sedatives or dissociative agents with or without analgesics to induce a state that allows a subject to tolerate unpleasant procedures while maintaining cardiorespiratory function.

### Examples

In order that the invention described herein may be more fully understood, the following examples are set forth. The synthetic and biological examples described in this application are offered to illustrate the compounds, pharmaceutical compositions, and methods provided herein and are not to be construed in any way as limiting their scope. The following examples are only exemplifications of the present invention in as far as they relate to the compounds II-1, II-A4, II-A5, II-A7, II-A8, II-A8a, II-A9, II-A10, II-A11, II-E12, II-G3, II-M2, II-M2a, II-M2b, II-12, II-13, II-13a, II-14, II-14a, II-20, II-21, II-21a, II-22, II-G5, II-B9, II-B-11, II-C4, II-43, II-44, II-45, II-46, II-47, II-48, 11-50, II-51, II-D9, II-D11, II-63, II-E4, II-E6, II-E8, II-D13, II-C8, II-75, II-76, II-77, II-78 and II-79 (the specific compounds appearing in claim 16). Other compounds are provided as reference examples.

### Materials and Methods

The compounds provided herein can be prepared from readily available starting materials using the following general methods and procedures. It will be appreciated that where typical or preferred process conditions (*i.e*., reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include (but are not limited to) column chromatography, HPLC, or supercritical fluid chromatography (SFC). The following schemes are presented with details as to the preparation of representative oxysterols that have been listed herein. The compounds provided herein may be prepared from known or commercially available starting materials and reagents by one skilled in the art of organic synthesis. Exemplary chiral columns available for use in the separation/purification of the enantiomers/diastereomers provided herein include, but are not limited to, CHIRALPAK^{®} AD-10, CHIRALCEL^{®} OB, CHIRALCEL^{®} OB-H, CHIRALCEL^{®} OD, CHIRALCEL^{®} OD-H, CHIRALCEL^{®} OF, CHIRALCEL^{®} OG, CHIRALCEL^{®} OJ and CHIRALCEL^{®} OK.

**¹H-NMR** reported herein (e.g., for the region between δ (ppm) of about 0.5 to about 4 ppm) will be understood to be an exemplary interpretation of the NMR spectrum (e.g., exemplary peak integratations) of a compound.

Exemplary general method for preparative HPLC: Column: Waters RBridge prep 10 µm C18, 19*250 mm. Mobile phase: acetonitrile, water (NH₄HCO₃) (30 L water, 24 g NH₄HCO₃, 30 mL NH₃.H₂O). Flow rate: 25 mL/min.

Exemplary general method for analytical HPLC: Mobile phase: A: water (10 mM NH₄HCO₃), B: acetonitrile Gradient: 5%-95% B in 1.6 or 2 min Flow rate: 1.8 or 2 mL/min; Column: XBridge C18, 4.6*50mm, 3.5 µm at 45 C.

Exemplary general method for prep HPLC: Column Waters Xbridge 150*25 5u Condition water (10mM NH4HCO3)-ACN Begin B 85 End B 100 Gradient Time (min) 6.5 100%B Hold Time (min) 1 FlowRate(ml/min) 25 Injections 4).

Exemplary general method for SFC: Column: CHIRALPAK^{®} AD CSP (250 mm * 30 mm, 10 µm), Gradient: 45% B, A= NH₃H₂O, B= MeOH, flow rate: 60 mL/min. For example, AD_3_EtOH_DEA_5_40_25ML would indicate: "Column: Chiralpak AD-3 150×4.6mm I.D., 3um Mobile phase: A: CO2 B:ethanol (0.05% DEA) Gradient: from 5% to 40% ofB in 5 min and hold 40% for 2.5 min, then 5% ofB for 2.5 min Flow rate: 2.5mL/min Column temp: 35°C".

Exemplary general method for Standard LC-ELSD 30-90AB_2min_E. (Mobile Phase: 1.5mL/4L TFA in water (solvent A) and 0.75mL/4L TFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm, 3µm; Wavelength: UV 220 nm; Column temperature: 50°C; MS ionization: ESI; Detector: PDA&ELSD

Exemplary general method for SFC: Column: CHIRALPAK^{®} AD CSP (250 mm * 30 mm, 10 µm), Gradient: 45% B, A= NH₃H₂O, B= MeOH, flow rate: 60 mL/min. For example, AD_3_EtOH_DEA_5_40_25ML would indicate: "Column: Chiralpak AD-3 150×4.6mm I.D., 3um Mobile phase: A: CO2 B:ethanol (0.05% DEA) Gradient: from 5% to 40% ofB in 5 min and hold 40% for 2.5 min, then 5% ofB for 2.5 min Flow rate: 2.5mL/min Column temp: 35°C".

### Formula (I-X) or Formula (1-1) Abbreviations:

PE: petroleum ether; EtOAc: ethyl acetate; THF: tetrahydrofuran; PCC: pyridinium chlorochromate; TLC: thin layer chromatography; PCC: pyridinium chlorochromate; t-BuOK: potassium tert-butoxide; 9-BBN: 9-borabicyclo[3.3.1]nonane; Pd(*t-*Bu₃P)₂: bis(tri-tert-butylphosphine)palladium(0); AcCl: acetyl chloride; *i*-PrMgCl: Isopropylmagnesium chloride; TBSCl: tert-Butyl(chloro)dimethylsilane; (*i*-PrO)₄Ti: titanium tetraisopropoxide; BHT: 2,6-di-t-butyl-4-methylphenoxide; Me: methyl; *i*-Pr: iso-propyl; *t-*Bu: tert-butyl; Ph: phenyl; Et: ethyl; Bz: benzoyl; BzCl: benzoyl chloride; CsF: cesium fluoride; DAST: Diethylaminosulfur trifluoride; DCC: dicyclohexylcarbodiimide; DCM: dichloromethane; DMAP: 4-dimethylaminopyridine; DMP: Dess-Martin periodinane; EtMgBr: ethylmagnesium bromide; EtOAc: ethyl acetate; TEA: triethylamine; AlaOH: alanine; Boc: t-butoxycarbonyl. Py: pyridine; TBAF: tetra-n-butylammonium fluoride; THF: tetrahydrofuran; TBS: t-butyldimethylsilyl; TMS: trimethylsilyl; TMSCF₃: (Trifluoromethyl)trimethylsilane; Ts: p-toluenesulfonyl; Bu: butyl; Ti(OiPr)₄: tetraisopropoxytitanium; LAH: Lithium Aluminium Hydride; LDA: lithium diisopropylamide; LiOH.H₂O: lithium hydroxide hydrates; MAD: methyl aluminum bis(2,6-di-t-butyl-4-methylphenoxide); MeCN: acetonitrile; NBS: N-bromosuccinimide; Na₂SO₄: sodium sulfate; Na₂S₂O₃: sodium thiosulfate; PE: petroleum ether; MeCN: acetonitrile; MeOH: methanol; Boc: t-butoxycarbonyl; MTBE: methyl tert-butyl ether; K-selectride: Potassium tri(s-butyl)borohydride.

### EXAMPLE 1-1: Synthesis of 1-((3R,5R,8S,9S,10R,13S,14S,17S)-3-hydroxy-3,5,10,13-tetramethylhexadecahydro-1H-cyclopenta[a]phenanthren-17-yl)-2-(methyl(phenyl)amino)ethan-1-one (I-A2)

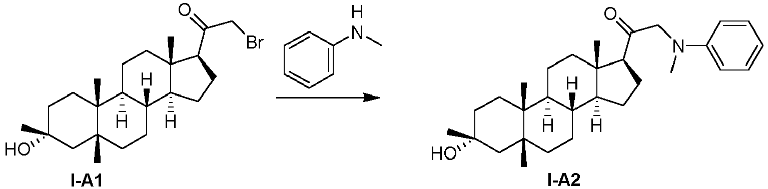

To a solution of DIPEA (78.9 mg, 0.611 mmol) in DMF (10 mL) was added N-methylaniline (100 mg, 0.94 mmol) at 10°C. After stirring at 10°C for 10 min, **I-A1** (200 mg, 0.47 mmol, described below in EXAMPLE I-4) was added. The mixture was stirred at 40°C for 16 hours. The mixture was concentrated to give an oil, which was purified by HPLC to afford **I-A2** (21 mg, 10%) as a solid.

**¹H NMR** (400 MHz, CDCl₃) δ 7.24-7.15 (m, 2H), 6.75-6.70 (m, 1H), 6.65-6.55 (m, 2H), 4.13-3.93 (m, 2H), 3.00 (s, 3H), 2.63-2.54 (m, 1 H), 2.27-2.09 (m, 2 H), 2.03-1.95 (m, 1H), 1.74-1.53 (m, 6H), 1.50-1.42 (m, 6H), 1.38-1.32 (m, 5H), 1.24-1.11 (m, 4H), 1.06-0.98 (m, 1H), 0.93 (s, 3H), 0.81 (s, 3H), 0.65 (s, 3H); LC ELSD purity 99%, MS ESI calcd. for C₃₀H₄₆NO₂ [M+H]⁺ 452, found 452.

### EXAMPLE 1-2: Synthesis of 1-((3R,5R,8S,9S,10R,13S,14S,17S)-3-hydroxy-3,5,10,13-tetramethylhexadecahydro-1H-cyclopenta[a]phenanthren-17-yl)-2-(phenylamino)ethan-1-one (I-A3)

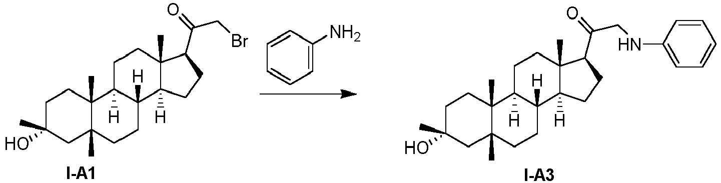

To a solution of DIPEA (78.9 mg, 0.611 mmol) in DMF (10 mL) was added aniline (100 mg, 0.94 mmol) at 10°C. After stirring at 10°C for 10 min, **I-A1** (200 mg, 0.47 mmol) was added. The mixture was stirred at 40°C for 16 hours. The mixture was concentrated to give a oil, which was purified by HPLC to afford **I-A3** (5 mg, 2%) as a solid.

**¹H NMR** (400 MHz, CDCl₃) δ_{H} 7.23-7.16 (m, 2H), 6.75-6.70 (m, 1H), 6.63-6.58 (m, 2H), 4.00- 3.86 (m, 2H), 2.60-2.56 (m, 1H), 2.29 - 2.18 (m, 2H), 2.04-1.94 (m, 2H), 1.78-1.71 (m, 2H), 1.52 - 1.43 (m, 7H), 1.42 - 1.38 (m, 2H), 1.36 (s, 4H), 1.29-1.23 (m, 3H), 1.20-1.15 (m, 2H), 1.06-0.99 (m, 1H), 0.94 (s, 3H), 0.90 - 0.85 (m, 1H), 0.80 (s, 3H), 0.62 (s, 3 H); LC-ELSD purity 99%; **MS ESI** calcd. for C₂₉H₄₄NO₂ [M+H]+ 438, found 438.

### EXAMPLE 1-3: Synthesis of 1-((3R,5R,8S,9S,10R,13S,14S,17S)-3-hydroxy-3,5,10,13-tetramethylhexadecahydro-1H-cyclopenta[a]phenanthren-17-yl)ethan-1-one (I-B6)

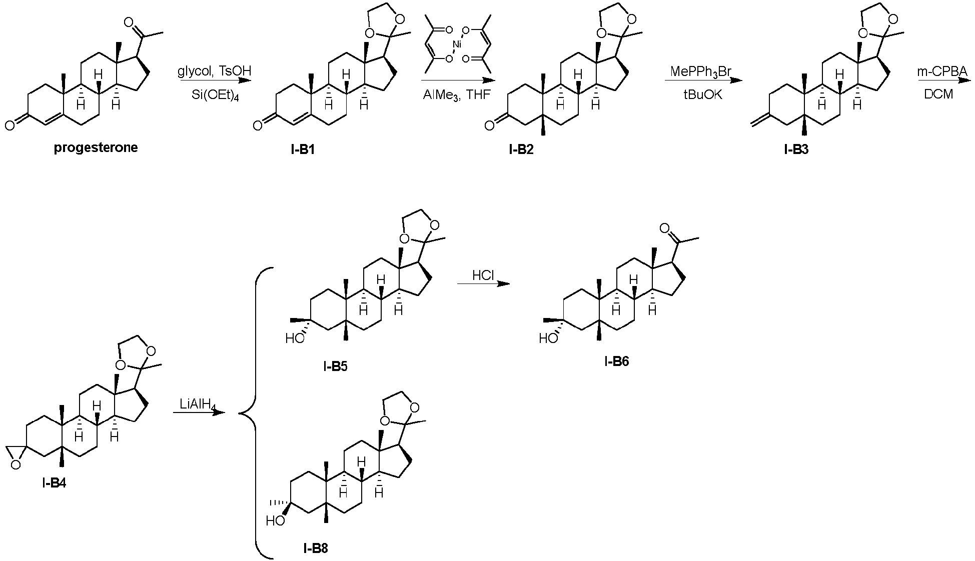

### Synthesis of I-B1

Si(OEt)₄ (132 g, 636 mmol) and a catalytic amount of TsOH (4.91 g, 28.6 mmol) were added to a solution of progesterone (100 g, 318 mmol) in ethylene glycol (2 L). After stirring 25°C for 5 hrs, the reaction mixture was quenched with NaHCO₃ (aq. 1.5 L), filtered, washed by water (2 L × 3), filtered, dried in the air, and triturated with MTBE/PE (50 mL/2.5 L) to give **I-B1** (95.0 g, 83%) as a solid.

**¹H NMR** (400 MHz, CDCl₃) δ_{H} 5.72 (s, 1H), 4.05-3.80 (m, 4H), 2.45-2.25 (m, 4H), 2.10-2.00 (m, 2H), 1.90-1.57 (m, 5H), 1.56-1.40 (m, 3H), 1.29 (s, 3H), 1.22-1.13 (m, 5H), 1.10-0.85 (m, 4H), 0.81 (s, 3H).

### Synthesis of I-B2

To a solution of **I-B1** (10 g, 27.8 mmol) and nickel(2+) diacetylacetonate (1.42 g, 5.56 mmol) in THF (300 mL) was added dropwise AlMe₃ (41.7 mL, 83.4 mmol, 2M in toluene) at 0°C under N₂. After addition, the reaction mixture was stirred at 0°C for 2 hrs. The mixture was quenched with 50% NH₄Cl (500 mL), diluted with EtOAc (300 mL), and then filtered. The organic layer was separated and the aqueous phase was extracted with EtOAc (200 mL). The combined organic phase was washed with brine (500 mL), dried over Na₂SO₄, filtered, concentrated and purified by combi-flash (0-15% of EtOAc in PE) to give **I-B2** (6 g, 57%) as a solid.

### Synthesis of I-B3

To a suspension of MePh₃PBr (17 g, 48 mmol) in THF (80 mL) was added t-BuOK (5.37 g, 48 mmol). After stirring at 40°C for 20 minutes, a solution of **I-B2** (6 g, 16 mmol) in THF (20 mL) was added dropwise at 40°C. After addition, the mixture was quenched with 50% NH₄Cl (300 mL) and extracted with EtOAc (2 × 100 mL). The combined organic phase was dried over Na₂SO₄, filtered, concentrated and purified by combi-flash (0-5% of EtOAc in PE) to give **I-B3** (5 g, 83%) as a solid.

**¹H NMR** (400 MHz, CDCl₃) δ_{H} 4.66-4.61 (m, 1H), 4.58-4.51 (m, 1H), 4.03-3.82 (m, 4H), 2.87-2.79 (m, 1H), 2.20-1.91 (m, 3H), 1.83-1.77 (m, 1H), 1.75-1.58 (m, 5H), 1.54 (s, 3H), 1.48-1.31 (m, 5H), 1.29 (s, 3H), 1.23-1.01 (m, 5H), 0.82-0.78 (m, 5H), 0.74 (s, 3H).

### Synthesis of I-B4

To a solution of **I-B3** (5 g, 13.4 mmol) in DCM (100 mL) was added K₂CO₃ (9.27 g, 67 mmol) and *m*-CPBA (6.91 g, 40.1 mmol). After stirring at 20°C for 30 minutes, the reaction mixture was quenched with sat.Na₂S₂O₃ (300 mL). After stirring at 20°C for 10 minutes, the organic phase was separated, dried over Na₂SO₄, filtered and concentrated to give **I-B4** (5 g,) as a solid.

**¹H NMR** (400 MHz, CDCl₃) δ_{H} 4.03-3.83 (m, 4H), 2.69-2.46 (m, 3H), 2.09-1.89 (m, 2H), 1.82-1.76 (m, 1H), 1.75-1.62 (m, 4H), 1.62-1.47 (m, 2H), 1.46-1.33 (m, 4H), 1.29 (s, 3H), 1.26-0.98 (m, 8H), 0.96-0.93 (m, 2H), 0.89-0.82 (m, 3H), 0.74 (s, 3H), 0.61-0.50 (m, 1H).

### Synthesis of I-B5 & I-B8

To a solution of **I-B4** (300 mg, 0.772 mmol) in THF (30 mL) was added LiAlH₄ (293 mg, 7.72 mmol) in portions during 10 minutes. After stirring at 20°C for 30 minutes, the mixture was quenched with 50% NH₄Cl (60 mL) and extracted with EtOAc (3 × 30 mL). The combined organic phase was washed with 50% NH₄Cl (100 mL), dried over Na₂SO₄, filtered, concentrated and purified by combi-flash (0-15% of EtOAc in PE) to give **I-B5** (210 mg, 69%) and **I-B8** (40 mg, 13%) as a solid.

**I-B5: ¹H NMR** (400 MHz, CDCl₃) δ_{H} 4.04-3.81 (m, 4H), 2.11-2.00 (m, 2H), 1.84-1.58 (m, 5H), 1.52-1.39 (m, 3H), 1.39-1.34 (m, 2H), 1.33-1.29 (m, 4H), 1.28-1.23 (m, 3H), 1.22-1.16 (m, 4H), 1.16-1.02 (m, 7H), 1.00-0.87 (m, 2H), 0.83 (s, 3H), 0.73 (s, 3H).

**I-B8: ¹H NMR** (400 MHz, CDCl₃) δ_{H} 4.02-3.82 (m, 4H), 2.26-2.19 (m, 1H), 2.05-2.00 (m, 1H), 1.82-1.75 (m, 1H), 1.74-1.62 (m, 3H), 1.57-1.49 (m, 2H), 1.48-1.37 (m, 7H), 1.33 (s, 3H), 1.28 (s, 3H), 1.24-1.09 (m, 5H), 1.08-0.98 (m, 3H), 0.91 (s, 3H), 0.79 (s, 3H), 0.72 (s, 3H).

### Synthesis of I-B6

To a solution of **I-B5** (210 mg, 0.537 mmol) in MeOH (8 mL) was added HCl (5.35 mL, 10.7 mmol, 2M in water). After stirring at 20°C for 5 minutes, the mixture was diluted with water (50 mL) and filtered, dried in vacuum to give **I-B6** (150 mg, 80%) as a solid. Stereochemical assignment was confirmed by x-ray crystallography.

**¹H NMR** (400 MHz, CDCl₃) δ_{H} 2.56-2.47 (m, 1H), 2.26-2.12 (m, 2H), 2.10 (s, 3H), 2.04-1.97 (m, 1H), 1.74-1.53 (m, 5H), 1.52-1.38 (m, 7H), 1.37-1.33 (m, 4H), 1.29-1.10 (m, 5H), 1.05-0.98 (m, 1H), 0.92 (s, 3H), 0.79 (s, 3H), 0.57 (s, 3H).

### EXAMPLE 1-4: Synthesis of 1-(2-((3R,5R,8S,9S,10R,13S,14S,17S)-3-hydroxy-3,5,10,13-tetramethylhexadecahydro-1H-cyclopenta[a]phenanthren-17-yl)-2-oxoethyl)-1H-pyrazole-4-carbonitrile (I-A4)

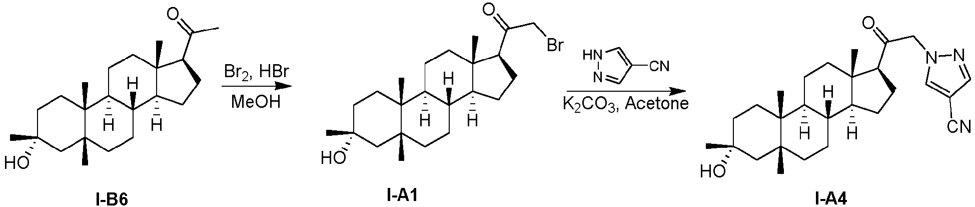

### Synthesis of I-AI

To a solution of **I-B6** (1.2 g, 3.46 mmol) in MeOH (30 mL) was added one drop of HBr (55.9 mg, 0.692 mmol) and Br₂ (829 mg, 5.19 mmol). After stirring at 20°C for 1h, the mixture was quenched with 50% NaHCOs (50 mL) and extracted with EtOAc (2 × 30 mL). The combined organic layer was washed with brine (50 mL), dried over Na₂SO₄, filtered, concentrated to give **I-AI** (1.4 g, 95%) as a solid.

**¹H NMR** (400 MHz, CDCl₃) δ_{H} 3.95-3.85 (m, 2H), 2.85-2.77 (m, 1H), 2.27-2.12 (m, 2H), 1.96-1.88 (m, 1H), 1.80-1.68 (m, 2H), 1.56-1.39 (m, 10H), 1.35 (s, 3H), 1.34-1.11 (m, 6H), 1.06-0.99 (m, 1H), 0.93 (s, 3H), 0.79 (s, 3H), 0.61 (s, 3H).

### Synthesis of I-A4

To a solution of **I-AI** (100 mg, 0.235 mmol) in acetone (2 mL) was added 1H-pyrazole-4-carbonitrile (32.7 mg, 0.352 mmol) and K₂CO₃ (64.8 mg, 0.47 mmol). After stirring at 20°C for 16 hrs, the reaction mixture was filtered and purified by prep-HPLC to give **I-A4** (46 mg, 45%) as a solid.

**¹H NMR** (400 MHz, CDCl₃) δ_{H} 7.85 (s, 1H), 7.81 (s, 1H), 5.05-4.85 (m, 2H), 2.64-2.54 (m, 1H), 2.29-2.15 (m, 2H), 2.09-1.99 (m, 1H), 1.80-1.71 (m, 2H), 1.51-1.38 (m, 9H), 1.38-1.33 (m, 5H), 1.32-1.21 (m, 3H), 1.20-1.12 (m, 2H), 1.07-1.00 (m, 1H), 0.94 (s, 3H), 0.80 (s, 3H), 0.64 (s, 3H); **LC-ELSD** purity 99%; **MS ESI** calcd. for C₂₇H₃₈N₃O [M+H-H₂O]⁺ 420, found 420.

### EXAMPLE 1-5: Synthesis of 1-((3S,5R,8S,9S,10R,13S,14S,17S)-3-hydroxy-3,5,10,13-tetramethylhexadecahydro-1H-cyclopenta[a]phenanthren-17-yl)ethan-1-one (I-A5)

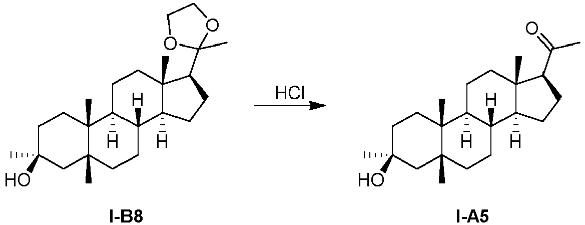

To a solution of **I-B8** (40 mg, 0.102 mmol) in MeOH (2 mL) was added HCl (1.02 mL, 2M in water). After stirring at 20°C for 5 minutes, the mixture was diluted with water (20 mL) and filtered, dried in vacuum to give **A5** (20 mg, 56%) as a solid.

**¹H NMR** (400 MHz, CDCl₃) δ_{H} 2.57-2.48 (m, 1H), 2.20-1.98 (m, 6H), 1.74-1.57 (m, 4H), 1.56-1.42 (m, 5H), 1.41-1.30 (m, 5H), 1.21 (s, 3H), 1.20-1.13 (m, 2H), 1.13-1.03 (m, 6H), 0.98-0.92 (m, 1H), 0.83 (s, 3H), 0.58 (s, 3H).

### EXAMPLE 1-6 & 1-7: Synthesis of 1-((3R,5R,8S,9S,10R,13S,14S,17S)-3-hydroxy-3,5,10,13-tetramethylhexadecahydro-1H-cyclopenta[a]phenanthren-17-yl)-2-(2H-pyrazolo[3,4-c]pyridin-2-yl)ethan-1-one (I-A6) & 1-((3R,5R,8S,9S,10R,13S,14S,17S)-3-hydroxy-3,5,10,13-tetramethylhexadecahydro-1H-cyclopenta[a]phenanthren-17-yl)-2-(1H-pyrazolo[3,4-c]pyridin-1-yl)ethan-1-one (I-A7)

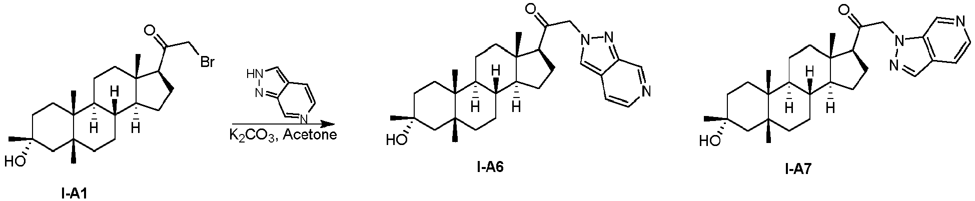

To a mixture of **I-AI** (200 mg, 0.47 mmol) and K₂CO₃ (129 mg, 0.94 mmol) in acetone (5 mL) was added 1H-pyrazolo[3,4-c]pyridine (83.9 mg, 0.705 mmol) at 25°C. After stirring at 25°C for 16 hrs, the reaction mixture was filtered and the filtrate was concentrated in vacuum to give product which was purified by prep. HPLC, and the eluents were washed with sat.NaHCO₃, dried over Na₂SO₄, filtered and concentrated to give **I-A6** (25 mg, ) and **I-A7** (40 mg, ) as a solid, which were further purified by prep-TLC (DCM: Acetone = 2:3) to give pure **I-A6** (4 mg, 1.8%) and **I-A7** (20 mg, 9%) as a solid.

**I-A6: ¹H NMR** (400 MHz, CDCl₃) δ 9.26 (s, 1H), 8.19-8.14 (m, 1H), 7.98 (s, 1H), 7.55-7.50 (m, 1H), 5.35-5.18 (m, 2H), 2.70-2.61 (m, 1H), 2.29-2.09 (m, 3H), 1.84-1.71 (m, 2H), 1.55-1.40 (m, 10H), 1.38-1.35 (m, 4H), 1.31-1.22 (m, 3H), 1.20-1.11 (m, 2H), 1.08-1.00 (m, 1H), 0.94 (s, 3H), 0.81 (s, 3H), 0.70 (s, 3H); **LC-ELSD** purity 99%; **MS ESI** calcd. for C₂₉H₄₂N₃O₂ [M+H]⁺ 464, found 464.

**I-A7: ¹H NMR** (400 MHz, CDCl₃) δ 8.79 (s, 1H), 8.36-8.31 (m, 1H), 8.09 (s, 1H), 7.67-7.62 (m, 1H), 5.31-5.19 (m, 2H), 2.70-2.62 (m, 1H), 2.29-2.11 (m, 3H), 1.81-1.69 (m, 2H), 1.64-1.60 (m, 1H), 1.53-1.39 (m, 9H), 1.36 (s, 3H), 1.35-1.21 (m, 4H), 1.20-1.13 (m, 2H), 1.08-1.01 (m, 1H), 0.94 (s, 3H), 0.82 (s, 3H), 0.72 (s, 3H); **LC-ELSD** purity 99%; **MS ESI** calcd. for C₂₉H₄₂N₃O₂ [M+H]⁺ 464, found 464.

### EXAMPLE 1-8 & 1-9: Synthesis of 1-((3R,5R,8S,9S,10R,13S,14S,17S)-3-hydroxy-3,5,10,13-tetramethylhexadecahydro-1H-cyclopenta[a]phenanthren-17-yl)-2-(5-methyl-1H-tetrazol-1-yl)ethan-1-one (I-A8) & 1-((3R,5R,8S,9S,10R,13S,14S,17S)-3-hydroxy-3,5,10,13-tetramethylhexadecahydro-1H-cyclopenta[a]phenanthren-17-yl)-2-(5-methyl-2H-tetrazol-2-yl)ethan-1-one (I-A9)

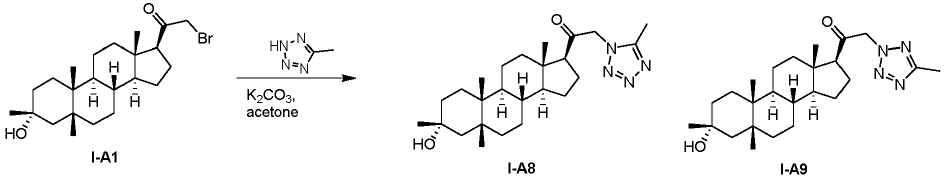

To a mixture of **I-AI** (200 mg, 0.5 mmol) and K₂CO₃ (129 mg, 0.9 mmol) in acetone (5 mL) was added 5-methyl-2H-1,2,3,4-tetrazole (59.2 mg, 0.7 mmol) at 15°C. The reaction mixture was stirred at the 15°C for 16 hrs. The reaction mixture was quenched by water (20 mL) and extracted with EtOAc (2 × 20 mL). The combined organic layer was dried over Na₂SO₄, filtered and concentrated in vacuum to give product which was purified by a flash column (EtOAc in PE, 50%-80%) to give **I-A8** (65 mg, 32%) as a solid and **I-A9** (45 mg, 22%) as a solid.

**I-A8: ¹H NMR** (400 MHz, CDCl₃) δ 5.21-4.97 (m, 2H), 2.68-2.59 (m, 1H), 2.47 (s, 3H), 2.30-2.03 (m, 3H), 1.86-1.67 (m, 2H), 1.65-1.57 (m, 2H), 1.53-1.38 (m, 9H), 1.37 (s, 3H), 1.32-1.11 (m, 5H), 1.08-1.01 (m, 1H), 0.95 (s, 3H), 0.81 (s, 3H), 0.66 (s, 3H); **LC ELSD** purity 99%; **MS ESI** calcd. for C₂₅H₃₉N₄O [M+H-H₂O]⁺ 411, found 411.

**I-A9: ¹H NMR** (400 MHz, CDCl₃) δ 5.40-5.29 (m, 2H), 2.65-2.53 (m, 4H), 2.29-2.15 (m, 2H), 2.12-2.04 (m, 1H), 1.84-1.71 (m, 2H), 1.66-1.57 (m, 2H), 1.52-1.39 (m, 7H), 1.39-1.33 (m, 5H), 1.29-1.11 (m, 5H), 1.07-0.99 (m, 1H), 0.94 (s, 3H), 0.81 (s, 3H), 0.69 (s, 3H); **LC-ELSD** purity 99%; **MS ESI** calcd. for C₂₅H₃₉N₄O [M+H-H₂O]⁺ 411, found 411.

**Formula II Abbreviations:** PE: petroleum ether; EtOAc: ethyl acetate; THF: tetrahydrofuran; PCC: pyridinium chlorochromate; TLC: thin layer chromatography; PCC: pyridinium chlorochromate; t-BuOK: potassium tert-butoxide; 9-BBN: 9-borabicyclo[3.3.1]nonane; Pd(*t*-Bu₃P)₂: bis(tri-tert-butylphosphine)palladium(0); AcCl: acetyl chloride; *i*-PrMgCl: Isopropylmagnesium chloride; TBSCl: tert-Butyl(chloro)dimethylsilane; (*i*-PrO)₄Ti: titanium tetraisopropoxide; BHT: 2,6-di-t-butyl-4-methylphenoxide; Me: methyl; *i*-Pr: iso-propyl; *t*-Bu: tert-butyl; Ph: phenyl; Et: ethyl; Bz: benzoyl; BzCl: benzoyl chloride; CsF: cesium fluoride; DCC: dicyclohexylcarbodiimide; DCM: dichloromethane; DMAP: 4-dimethylaminopyridine; DMP: Dess-Martin periodinane; EtMgBr: ethylmagnesium bromide; EtOAc: ethyl acetate; TEA: triethylamine; AlaOH: alanine; Boc: t-butoxycarbonyl. Py: pyridine; TBAF: tetra-n-butylammonium fluoride; THF: tetrahydrofuran; TBS: t-butyldimethylsilyl; TMS: trimethylsilyl; TMSCF₃: (Trifluoromethyl)trimethylsilane; Ts: p-toluenesulfonyl; Bu: butyl; Ti(OiPr)₄: tetraisopropoxytitanium; LAH: Lithium Aluminium Hydride; LDA: lithium diisopropylamide; LiOH.H₂O: lithium hydroxide hydrates; MAD: methyl aluminum bis(2,6-di-t-butyl-4-methylphenoxide); MeCN: acetonitrile; NBS: N-bromosuccinimide; Na₂SO₄: sodium sulfate; Na₂S₂O₃: sodium thiosulfate; PE: petroleum ether; MeCN: acetonitrile; MeOH: methanol; Boc: t-butoxycarbonyl; MTBE: methyl tert-butyl ether; K-selectride: Potassium tri(s-butyl)borohydride.

### General Schemes

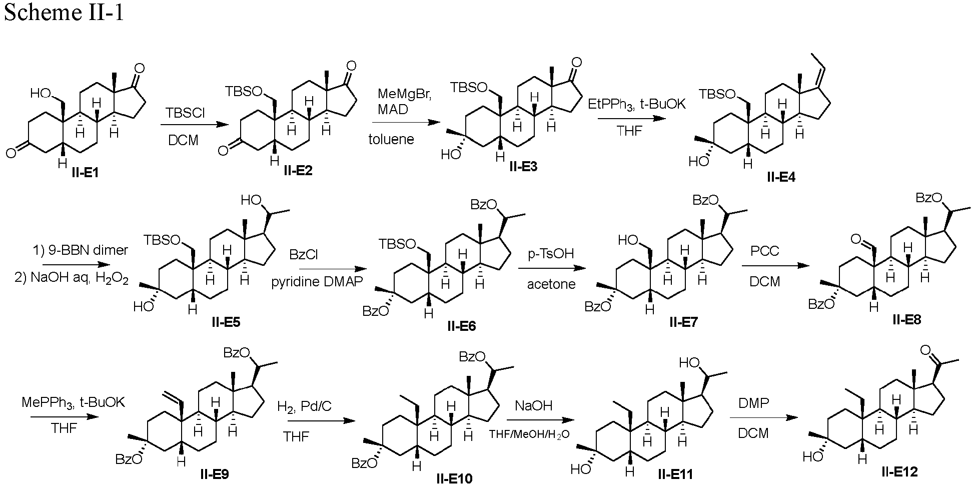

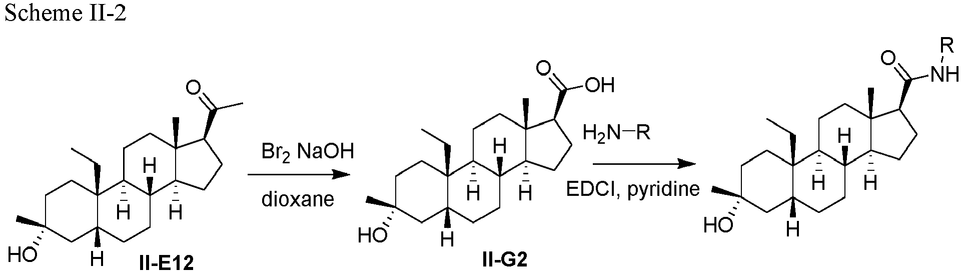

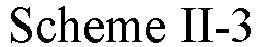

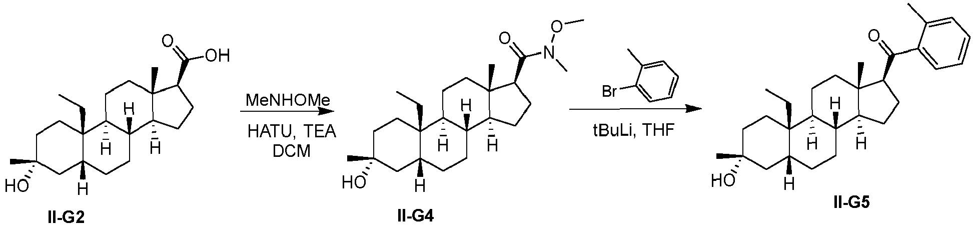

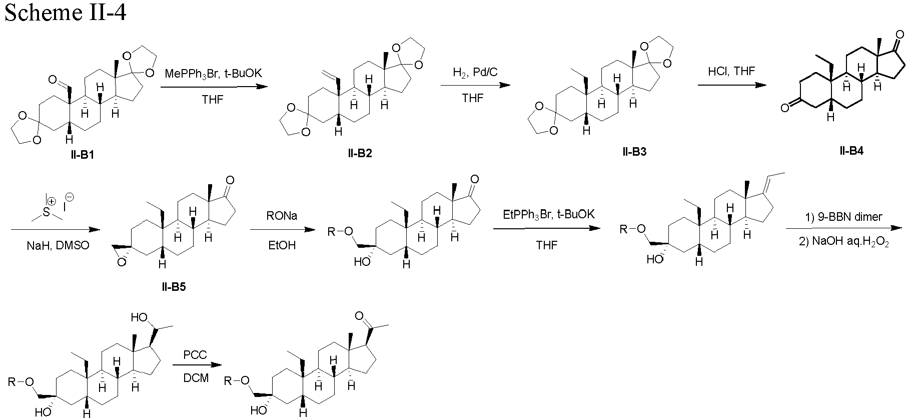

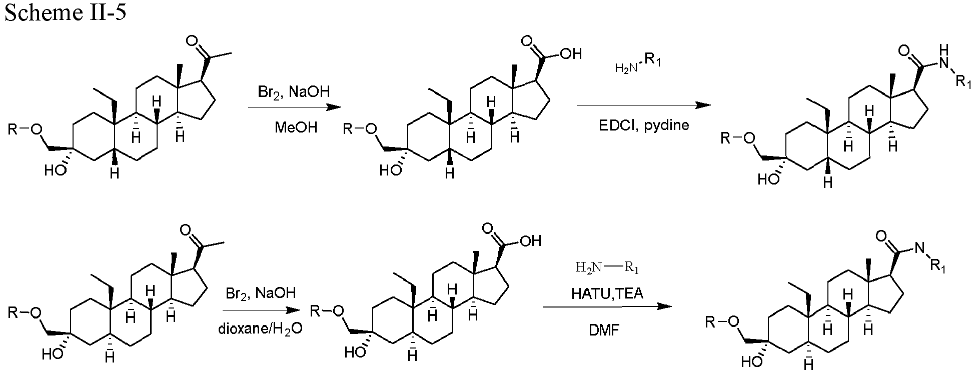

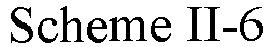

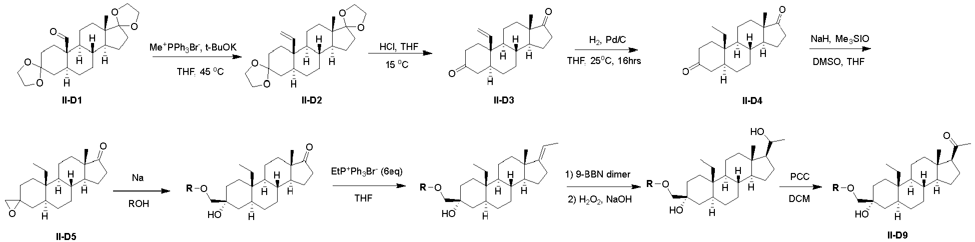

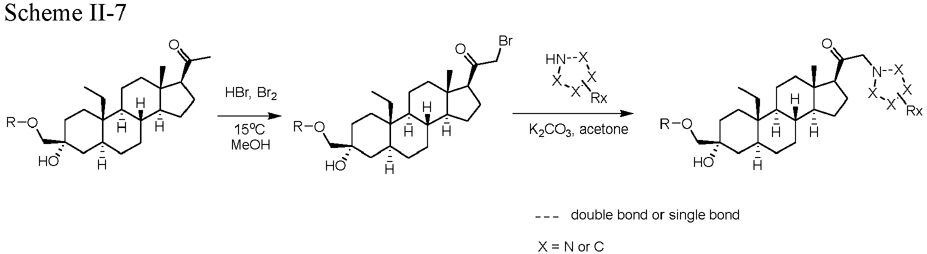

### Example II-1: Synthesis of ((3R,5R,8S,9S,10S,13S,14S,17S)-10-ethyl-3-hydroxy-3,13-dimethylhexadecahydro-1H-cyclopenta[a]phenanthren-17-yl)((S)-3-phenylpyrrolidin-1-yl)methanone (11-1)

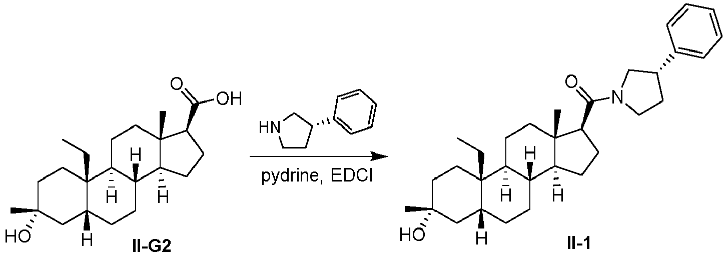

The experimental of intermediate **II-G2** could be found in Example II-10. A solution of **II-G2** (50 mg, 0.143 mmol), EDCI (54.9 mg, 0.287 mmol) and (3S)-3-phenylpyrrolidine (25.3 mg, 0.172 mmol) in pyridine (2 mL) was stirred at 35°C for 18 hrs. The mixture was treated with water (5 mL), extracted with EtOAc (2 × 8 mL). The organic layers were washed with brine (2 × 10 mL), dried over Na₂SO₄, filtered, concentrated in vacuum to give oil (35 mg). The product was purified by prep-HPLC (Instrument: BF; Column: Agela DuraShell 150mm_25mm_5um; Condition: water (0.225%FA)-ACN, Begin B: 69, End B: 99, Gradient Time (min): 8.5, 100%B Hold Time (min): 2; Flow Rate (ml/min): 30; Injections: 7) to give **II-1** (20 mg, 29%) as a solid.
**¹H NMR** (400 MHz, CDCl₃) δ_{H} 7.37-7.29 (m, 2H), 7.26-7.19 (m, 3H), 4.11-3.97 (m, 1H), 3.94-3.85 (m, 0.5H), 3.81-3.71 (m, 0.5H), 3.58-3.26 (m, 3H), 2.60-2.48 (m, 1H), 2.40-2.16 (m, 2H), 2.09-1.88 (m, 2H), 1.87-1.61 (m, 6H), 1.59-1.47 (m, 4H), 1.45-1.28 (m, 8H), 1.27-1.09 (m, 8H), 0.85-0.72 (m, 6H)
**LC-ELSD/MS** Rt = 1.252 min in 2 min chromatography, 30-90AB_2MIN_E.M, purity 99%, **MS ESI** calcd. for C₃₂H₄₈NO₂ [M+H]⁺ 478, found 478.
(Mobile Phase: 1.5ML/4LTFA in water (solvent A) and 0.75ML/4LTFA in acetonitrile (solvent B), using the elution gradient 30%-90% (solvent B) over 0.9 minutes and holding at 90% for 0.6 minutes at a flow rate of 1.2 ml/min; Column: Xtimate C18 2.1*30mm, 3um; Wavelength: UV 220 nm; Column temperature: 50°C; MS ionization: ESI; Detector: PDA&ELSD)

### Example II-2: Synthesis of 1-((3R,5S,8S,9S,10R,13S,14S,17S)-3-hydroxy-3,13-dimethyl-10-vinylhexadecahydro-1H-cyclopenta[a]phenanthren-17-yl)ethan-1-one (II-A4)

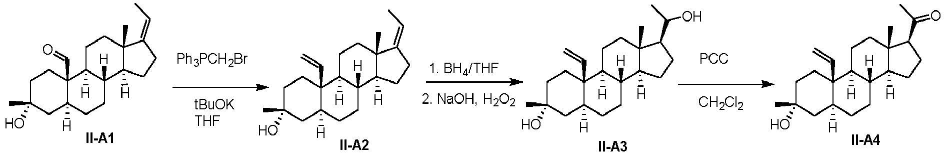

### Synthesis of II-A2

To a suspension of methyltriphenylphosphonium bromide (16.27g, 45.45 mmol) in dry THF (30mL) was added KOtBu (5.15g, 45.9mmol) under N₂ atmosphere. The mixture was heated at reflux for 1 hour. Then compound **II-A1** (5.0g, 15.15mmol) in dry THF (15 mL) was added to the above refluxing solution and stirred at reflux for 3 h. After cooling to room temperature, the solution was poured into brine (100mL). The aqueous solution was extracted with ethyl acetate (50mL × 3). The extracts were washed with brine (50mL × 2), dried over Na₂SO₄, filtered, concentrated and purified by column chromatography on silica gel (petroleum ether / ethyl acetate from 20/1 to 10/1) to get 3.82g (11.65mmol, yield 76.90%) of **II-A2** as a solid.

### Compound II-A2:

**¹H NMR** (500 MHz, CDCl₃) δ_{H} (ppm): 5.88 (1H, dd, J=11.0Hz, J=17.0Hz), 5.33 (1H, dd, J=1.5Hz, J=11.5Hz), 5.10 (1H, t×d, J=2.0Hz, J=7.5Hz), 5.00 (1H, dd, J=1.5Hz, J=11.5Hz), 1.63 (3H, t×d, J=2.0Hz, 7.0Hz), 1.15 (3H,s), 0.78 (3H, s).

### Synthesis of II-A3

To a solution of compound **II-A2** (3.82g,11.65mmol) in dry THF (50mL) was added boranetetrahydrofuran complex (15mL of 1.0 M solution in THF) and the reaction mixture was stirred at ambient temperature for 3h, and 10 % aqueous NaOH (5mL) was slowly added. The mixture was cooled in ice and 30% aqueous solution of H₂O₂ (10mL) was slowly added. The mixture was stirred at ambient temperature for 1 hour and then extracted with CH₂Cl₂ (3 × 50 mL). The combined CH₂Cl₂ extracts were washed with 10% aqueous Na₂S₂0₃ (2 × 20mL), then were directly used in the next step without further purification.

### Synthesis of II-A4

The compound **II-A3** from the last step was dissolved in 100 mL of dichloromethane cooled to 0°C and 4.0g of PCC was added at 0°C. Then the mixture was stirred for 6h. The mixture was filtered, concentrated, and purified by flash chromatography on silica gel using (petroleum ether/ethyl acetate = 12/1-6/1) elution to give 3.15g (9.15mmol,78.54%, two steps) of compound **II-A4** as a solid.

**¹H NMR** (500 MHz, CDCl₃) δ_{H} (ppm): 5.85(1H, dd, J=11.5Hz, J=17.5Hz), 5.32(1H, dd, J=2.0Hz, J=11.5Hz), 4.98(1H, dd, J=2.0Hz, J=11.5Hz), 2.51 (1H, t, J=9.5 Hz), 2.10(3H,s), 1.15 (3H, s), 0.51 (3H, s). **LC-MS**: rt=1.88min, m/z=327.0 [M -H₂O+H]⁺ ,m/z=345.1 [M +H]⁺

### Example II-3: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)ethan-1-one (II-A5)

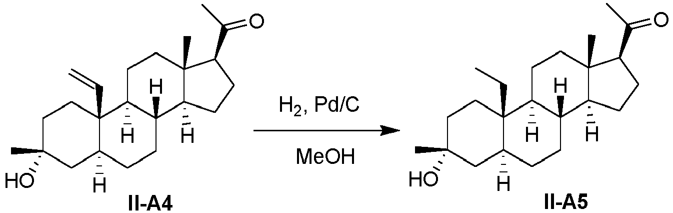

Compound **II-A4** (1.0g, 2.91 mmol) was dissolved in 20 mL of dry MeOH and 100mg of Pd/C was added. The reaction mixture was stirred at room temperature under balloon H₂ atmosphere overnight, filtered, resulting solution was concentrated and purified by column chromatography on silica gel (petroleum ether / ethyl acetate from 12/1 to 6/1) to give 896mg (2.59 mmol, yield 89.0%) of Compound **II-A5** as a solid
**¹H NMR** (500 MHz, CDCl₃) δ_{H} (ppm): 2.52(1H,t, J=8.5Hz), 2.11(3H,s),1.20(3H,s), 0.93(3H,t, J=7.0Hz), 0.63 (3H, s).

### Example 11-4: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)-2-(1H-pyrazol-1-yl)ethan-1-one (II-A7)

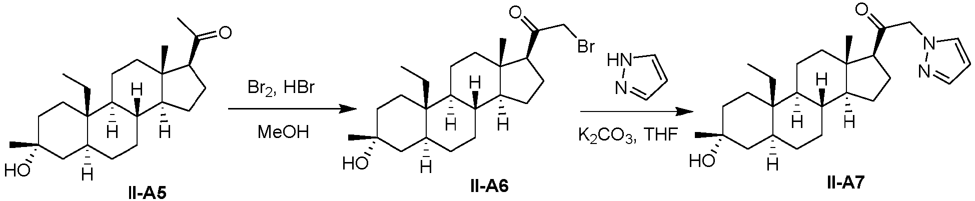

### Synthesis of II-A6:

Compound **II-A5** (100mg, 0.29 mmol) was dissolved in 10 mL of dry MeOH and 3 drops of Br₂ and 2 drops of HBr aq. were added. The reaction mixture was stirred at room temperature three hours, then the solution was poured into iced water (30mL). The aqueous solution was extracted with ethyl acetate (20mL × 2). The extracts were dried over MgSO₄, filtered, concentrated give 94mg of Compound **II-A6** as a solid.

### Synthesis of II-A7

64 mg (0. 15mmol) of compound **II-A6** was dissolved in 8 mL dry THF and 100mg (0.77mmol) of K₂CO₃, 100mg (1.47mmol) of pyrazole was added. The reaction mixture was stirred at room temperature overnight. The solution was then diluted with ethyl acetate (100 mL). The resulting solution was washed with brine (100 mL), dried over sodium sulfate and concentrated *in vacuo.* The residue was purified with reverse phase prep-HPLC to give 20 mg (0.05mmol, 32.36%) of Compound **II-A7** as a solid.

**¹H NMR** (500 MHz, CDCl₃) δ_{H} (ppm): 7.54 (1H,d, J=2.0Hz), 7.40 (1H,d, J=2.0Hz), 6.33 (1H,t, J=2.0Hz), 4.95(1H,AB, J=18.0 Hz), 4.87(1H,AB, J=17.5 Hz), 2.57(1H,t, J=9.0 Hz), 1.20(3H,s),0.93(3H,t, J=7.5Hz), 0.70 (3H, s).

### Example II-5: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)-2-(1H-1,2,3-triazol-1-yl)ethan-1-one (II-A8) and 1-((3R,5S,8S,9S,10S,13S,14S,17S)-10-ethyl-3-hydroxy-3,13-dimethylhexadecahydro-1H-cyclopenta[a]phenanthren-17-yl)-2-(2H-1,2,3-triazol-2-yl)ethan-1-one (II-A8a)

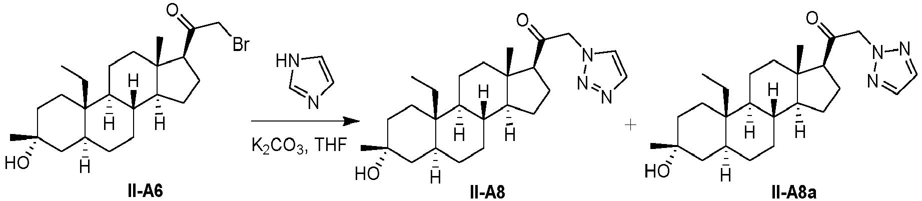

To **II-A6** (120 mg, 0.28mol) dissolved THF (8 mL) was added K₂CO₃ (200mg,1.54mmol) and 1H-1,2,3-triazole (0.5mL, 8.6 mmol). The reaction mixture was stirred at room temperature overnight. The solution was then diluted with ethyl acetate (100 mL). The resulting solution was washed with brine (100 mL), dried over sodium sulfate and concentrated *in vacuo.* The residue was purified with reverse phase prep-HPLC to give **II-A8** (24 mg, 0.06mmol, 20.75 yield%) and **II-A8a** (23 mg, 0.06mmol, 19.89% yield) as solids.

### II-A8:

**¹HNMR** (500 MHz, CDCl₃) δ(ppm): 7.75(1H,s), 7.64(1H,s), 5.26(1H,AB,J=18.0Hz), 5.13(1H,AB,J=18.0Hz), 2.64 (1H,t,J=8.5Hz), 1.21(3H,s), 0.94(3H,t,J=7.5Hz), 0.69(3H, s); **LC-MS**: rt=2.25min, m/z=414.4 [M +H]⁺

### II-A8a:

**¹HNMR** (500 MHz, CDCl₃) δ_{H} (ppm): 7.68 (2H,s),5.25(1H,AB,J=17.0Hz),5.21(1H,AB, J=17.5Hz), 2.57 (1H,t, J=9.0Hz), 1.21(3H,s),0.94(3H,t, J=7.5Hz), 0.73(3H, s); **LC-MS**: rt=2.40min, m/z=414.4 [M +H]⁺

### Example II-6: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)-2-morpholinoethan-1-one (II-A9)

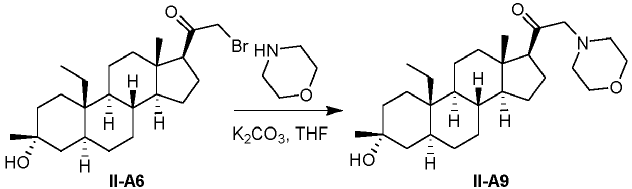

To compound **II-A6** (90 mg, 0.21mmol) dissolved inTHF (8 mL) were added K₂CO₃ (100mg, 0.77mmol) and morpholine (0.5 mL, 5.74mmol) were added. The reaction mixture was stirred at room temperature overnight. The solution was diluted with ethyl acetate (100 mL). The resulting solution was washed with brine (100 mL), dried over sodium sulfate and concentrated *in vacuo.* The residue was purified with reverse phase prep-HPLC to give 50mg (0.11mmol, 55.24%) product **II-A9** as a solid.
**¹H NMR** (500 MHz, CDCl3) δ_{H} (ppm): 3.75(4H,t, J=4.5Hz), 3.18 (1H,AB, J=17.5Hz), 3.17 (1H,AB, J=17.5Hz), 2.56(1H,t,J=8.5Hz), 2.51-2.45(4H,m), 1.20(3H,s),0.93 (3H,t, J=7.5Hz), 0.64(3H, s); **LC-MS**: rt=2.40min, m/z=432.4 [M +H]⁺

### Example II-7: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)-2-(4-(methylsulfonyl)piperazin-1-yl)ethan-1-one (II-A10)

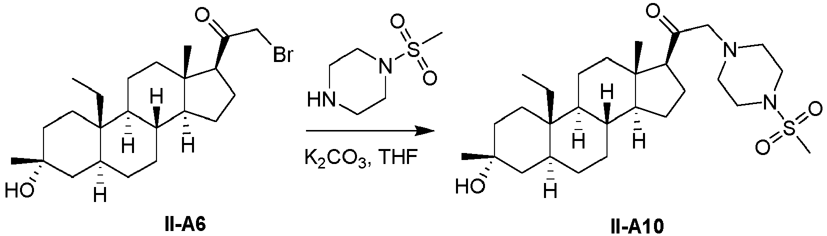

To compound **II-A6** (150 mg, 0.35 mmol) dissolved in THF (8 mL) were added K₂CO₃ (160mg, 1.15mmol) and 1-(Methylsulfonyl)piperazine (150 mg, 0.92mmol). The reaction mixture was stirred at room temperature overnight. The solution was diluted with ethyl acetate (100 mL) and the resulting solution was washed with brine (100 mL), dried over sodium sulfate and concentrated *in vacuo.* The residue was purified with reverse phase prep-HPLC to give 33mg (0.06mmol, 18.56%) product **II-A10** as a solid.
**¹H NMR** (400 MHz, CDCl₃) δ_{H} (ppm): 3.30(4H,t, J=4.0Hz), 3.25(2H, s), 2.78(3H, s),2.64∼2.57(4H,m), 2.51(1H,t, J=9.2Hz),1.21(3H,s),0.93(3H,t,J=7.2Hz), 0.64(3H, s); **LC-MS:** rt=2.33min, m/z=509.4 [M +H]⁺

### Example II-8: Synthesis of 1-((3R,5S,8S,9S,10S,13S,14S,17S)-10-ethyl-3-hydroxy-3,13-dimethylhexadecahydro-1H-cyclopenta[a]phenanthren-17-yl)-2-hydroxyethan-1-one (II-A11)

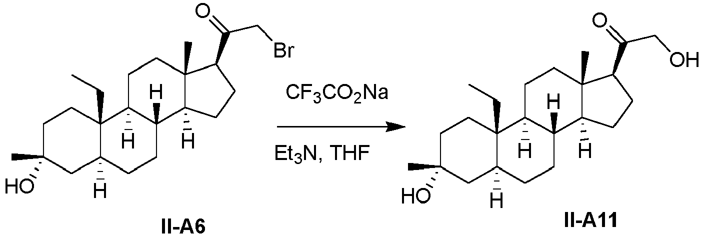

To compound **II-A6** (300 mg, 0.71 mmol) dissolved in THF (8 mL) were added triethylamine (1 mL) and 2,2,2-trifluoroacetic acid (0.5 mL). The mixture was heated at reflux for1 hour, then 400 mg (2.9mmol) of sodium 2,2,2-trifluoroacetate was added, and the resulting solution was refluxed overnight, then the solution was poured into iced water (50mL). The aqueous solution was extracted with ethyl acetate (30mL × 2). The extracts were dried over MgSO₄, and concentrated *in vacuo.* The residue was purified with reverse phase prep-HPLC to give 88 mg (0.24mmol, 34.23%) product **II-A11** as a solid.
**¹H NMR** (500 MHz, CDCl₃) δ_{H} (ppm): 4.20 (1H, AB × d, J=4.0 Hz, J=19.0 Hz), 4.15 (1H, AB × d, J=4.0 Hz, J=19.0 Hz), 3.28 (1H,t, J=4.0Hz), 2.44(1H,t, J=9.0Hz), 2.20 (1H,dd, J=11.0Hz,J=20.5Hz,), 1.20(3H,s), 0.93 (3H,AB, J=7.5Hz), 0.68(3H, s).

### Example II-9: Synthesis of 1-((3R,5R,8S,9S,10S,13S,14S,17S)-10-ethyl-3-hydroxy-3,13-dimethylhexadecahydro-1H-cyclopenta[a]phenanthren-17-yl)ethan-1-one (II-E12)

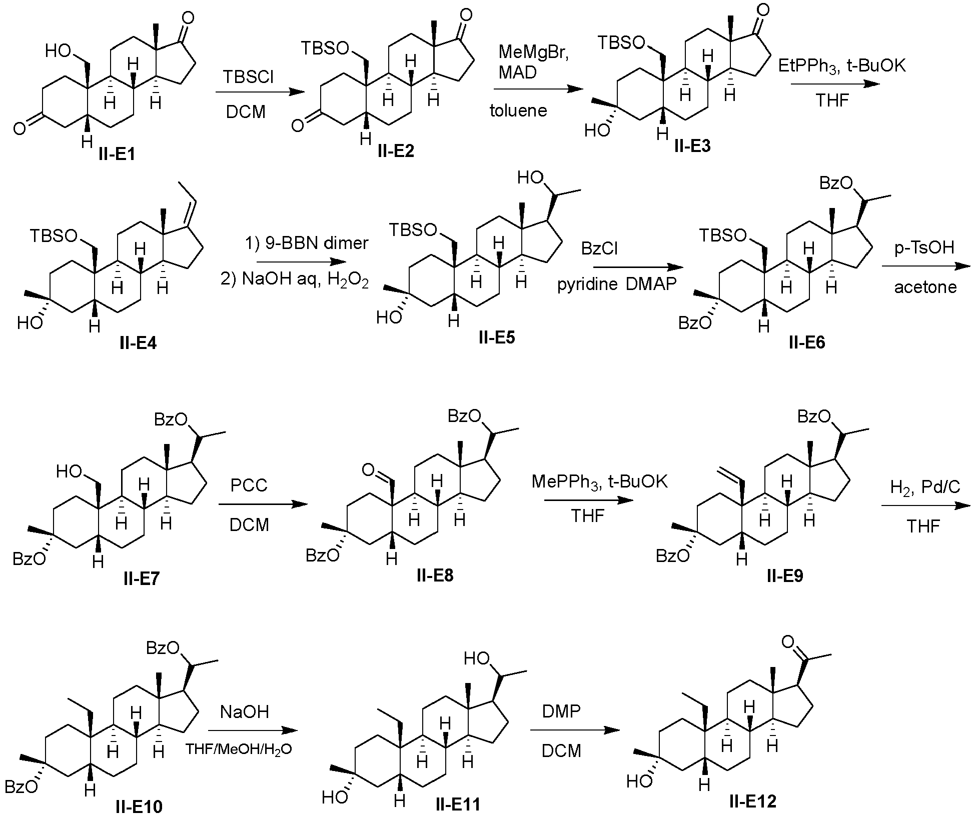

### Synthesis of II-E2

To a solution of **II-E1** (226 g, 742 mmol) in DCM (1000 mL) was added 1H-imidazole (125 g, 1.85 mol) and tert-butylchlorodimethylsilane (200 g, 1.33 mol) at 25°C under N₂. After stirring at 25°C for 4 hrs, the mixture was quenched with water (500 mL). The organic layer was separated. The aqueous phase was extracted with DCM (2 × 300 mL). The combined organic phase was washed with saturated brine (500 mL), dried with anhydrous Na₂SO₄, filtered and concentrated in vacuum.The residue was purified by silica gel chromatography (PE/EtOAc = 100/1 to 20/1) to give product **II-E2** (240 g, 77%) as a solid.

**¹H NMR** (400 MHz, CDCl₃) δ_{H} 3.80-3.75 (m, 1H), 3.60-3.50 (m, 1H), 2.65-2.50 (m, 1H), 2.48-2.40 (m, 1H), 2.40-2.25 (m, 3H), 2.20-1.65 (m, 7H), 1.65-1.10 (m, 9H), 0.81-0.80 (m, 13H), 0.00 (m, 6H).

### Synthesis of II-E3

To a solution of BHT (753 g, 3.42 mol) in toluene (2000 mL) under nitrogen at 0°C was added trimethylaluminum (855 mL, 2 M,1.71 mol) dropwise. After stirring at 0°C for 30 min, the solution was cooled to -78°C, followed by adding a solution of **II-E2** (240 g, 573 mmol) in toluene (500 mL) dropwise at -78°C. After stirring at the -78°C for 1 h, MeMgBr (476 mL, 1.43 mol, 3M in ethyl ether) was added dropwise at -78°C. The resulting solution was stirred at -78°C to -60°C for 2 hrs. The reaction mixture was poured into saturated citric acid (3000 mL) at -60°C. After stirring at 25°C for 25 mins, the aqueous layer was extracted with ethyl acetate (3 × 1000 mL). The combined organic layer was dried over Na₂SO₄ and concentrated in vacuum to give a oil, which was purified by a silica gel column (PE: EtOAc =50:1, 30:1, 15:1, 10:1, 5:1) to give **II-E3** (60 g, 24%) as a solid and **II-E3** (1.2 kg,).

**¹H NMR** (400 MHz, CDCl₃) δ_{H} 3.80-3.75 (m, 1H), 3.45-3.35 (m, 1H), 2.55-2.40 (m, 1H), 2.25-1.65 (m, 7H), 1.65-1.35 (m, 6H), 1.35-1.15 (m, 12H), 0.90-0.75 (m, 12H), 0.05 (m, 6H).

### Synthesis of II-E4

To a suspension of MePPh₃Br (170 g, 460 mmol) in THF (200 mL) was added t-BuOK (51.6 g, 460 mmol) at 25°C. After addition, the reaction mixture was heated to 45°C and stirred for 1 hour. Then a solution of **II-E3** (50 g, 115 mmol) in THF (50 mL) was added and the reaction mixture was stirred at 45°C for 16 h. The mixture was treated with NH₄Cl (100 mL, sat. aq.). The organic layer was separated. The aqueous phase was extracted with EtOAc (2 × 100 mL). The combined organic phase was washed with saturated brine (2 × 50 mL), dried over anhydrous Na₂SO₄, filtered, concentrated. The residue was purified by flash column (0-10% of EtOAc in PE) to give **II-E4** (48 g, 93%) as a solid.

**¹H NMR** (400 MHz, CDCl₃) δ_{H} 5.25-5.05 (m, 1H), 3.80-3.75 (m, 1H), 3.45-3.35 (m, 1H), 2.45-2.35 (m, 1H), 2.35-2.15 (m, 2H), 2.00-1.80 (m, 2H), 1.75-1.35 (m, 13H), 1.35-1.05 (m, 11H), 0.95-0-80 (m, 9H), 0.80-0.75 (m, 3H),0.05 (m, 6H).

### Synthesis of II-E5

To a solution of **II-E4** (48 g, 107 mmol) in THF (500 mL) was added 9-BBN dimer (104 g, 428 mmol) at 0°C under N₂. The solution was stirred at 65°C for 2 hrs. After cooling to 0°C, EtOH (49.2 g, 1.07 mol) was added. Then a solution of NaOH (214 mL, 5M, 1.07 mol) was added very slowly. After addition, H₂O₂ (121 g, 1.07 mol, 30% in water) was added slowly and the inner temperature was maintained below 10°C. The mixture was stirred at 75°C under N₂ for 1 hour. The mixture was re-cooled to 25°C. Then water (300 ml) was added. The organic layer was separated. The aqueous phase was extracted with EtOAc (3 × 100 mL). The combined organic phase was washed with saturated brine (100 mL), dried over anhydrous Na₂SO₄, filtered and concentrated under vacuum. The residue was triturated from THF/H₂O (100mL/1500 mL) at 25°C and purified by flash column (0-20% of EtOAc in PE) to give **II-E5** (45 g, 82%) as a solid.

**¹H NMR** (400 MHz, CDCl₃) δ_{H} 3.80-3.75 (m, 1H), 3.75-3.55 (m, 1H), 3.45-3.35 (m, 1H), 2.04 (s, 4H), 2.01-1.75(m, 4H), 1.75-1.55 (m, 3H), 1.55-1.25 (m, 12H), 1.25-1.05 (m, 8H), 0.89 (s, 9H), 0.63 (s, 3H),0.05-0.01 (m, 6H).

### Synthesis of II-E6

To a solution of **II-E5** (15 g, 32.2 mmol) in pyridine (200 mL) was added benzoyl chloride (22.6 g, 161 mmol) and DMAP (392 mg, 3.22 mmol). After stirring at 80°C for 4 hrs, the reaction mixture was poured into water (1000 mL). The mixture was stirred 10 mins, filtrated and the filter cake was solved in EtOAc (200 mL). The solution was washed with brine (2 × 50 mL), dried over Na₂SO₄, filtered, concentrated. The residue was purified by flash column (0∼10% of EtOAc in PE) to give **II-E6** (19 g, 84%) as a solid.

**¹H NMR** (400 MHz, CDCl₃) δ_{H} 8.05-8.00 (m, 4H), 7.65-7.50 (m, 2H), 7.45-7.35 (m, 4H), 5.25-5.15 (m, 1H), 3.80-3.75 (m, 1H), 3.45-3.35 (m, 1H), 2.30-2.20 (m, 1H), 2.25-2.00 (m, 2H), 1.95-1.75 (m, 4H), 1.75-1.25 (m, 5H), 1.25-1.00 (m, 13H), 0.95-0.75 (m, 13H), 0.70 (s, 3H), 0.06 (m, 6H).

### Synthesis of II-E7

To a solution **II-E6** (18.9 g, 28.0 mmol) in acetone (200 mL) *p*-TsOH (10.4 g, 56.0 mmol) was added. The reaction mixture was stirred at 25°C and after 5 hrs, quenched with water (200) and extracted with EtOAc (2 × 200 mL). The combined organics were washed with NaHCOs (50 mL, 10%) and brine (50 mL) and dried over Na₂SO₄, filtered and concentrated to give **II-E7** (15 g, ) as a solid.

**¹H NMR** (400 MHz, CDCl₃) δ_{H} 8.05-8.00 (m, 4H), 7.65-7.50 (m, 2H), 7.45-7.35 (m, 4H), 5.25-5.15 (m, 1H), 4.00-3.90 (m, 1H), 3.65-3.50 (m, 1H), 2.30-2.20 (m, 1H), 2.20-1.75 (m, 7H), 1.75-1.25 (m, 6H), 1.25-1.05 (m, 12H), 0.91 (s, 4H) 0.70 (s, 3H).

### Synthesis of II-E8

To a solution of **II-E7** (15 g, 26.8 mmol) in DCM (100 mL) was added PCC (11.5 g, 53.6 mmol) and silica gel (10 g) at 25°C. After stirring at 25°C for 2 hrs, the reaction mixture was filtered and the residue was washed with anhydrous DCM (2 × 50 mL). The combined filtrate was concentrated in vacuum. The residue was purified by flash column (0-10% of EtOAc in PE) to give **II-E8** (7 g, 58%) as a solid.

**¹H NMR** (400 MHz, CDCl₃) δ_{H} 0.97 (s, 1H) 8.05-8.00 (m, 4H), 7.65-7.50 (m, 2H), 7.45-7.35 (m, 4H), 5.25-5.15 (m, 1H), 2.30-2.20 (m, 1H), 2.20-1.75 (m, 12H), 1.75-1.01 (m, 14H), 1.01-0.80 (m, 4H) 0.78 (s, 3H).

### Synthesis of II-E9

To a suspension of PPh₃EtBr (17.9 g, 30.8 mmol) in THF (100mL) was added t-BuOK (3.45 g, 30.8 mmol) at 25°C. After stirring at 45°C for 1 hour, a solution of **II-E8** (7 g, 15.4 mmol) in THF (20 mL) was added at 40°C. The reaction mixture was stirred at 40°C for 10 min and treated with saturated NH₄Cl solution (50 mL) and EtOAc (50 mL). The organic layer was separated and the water phase was extracted with EtOAc (2 × 50 mL). The combined organic phase was washed with saturated brine (2 × 30 mL), dried over anhydrous Na₂SO₄, filtered concentrated. The residue was purified by flash column (0∼20% of EtOAc in PE) to give **II-E9** (4.8 g, 90%) as a solid.

**¹H NMR** (400 MHz, CDCl₃) δ_{H} 8.05-8.00 (m, 4H), 7.65-7.50 (m, 2H), 7.45-7.35 (m, 4H), 6.40-6.25 (m, 1H), 5.20-4.95 (m, 3H), 2.30-2.20 (m, 1H), 2.15-1.75 (m, 9H), 1.75-1.05 (m, 15H), 1.05-0.80 (m, 4H),0.69 (s, 3H).

### Synthesis of II-E10

To a solution of **II-E9** (2.5 g, 4.50 mmol) in THF (30 mL) was added Pd-C (dry, 10%, 0.2 g) under N₂. The suspension was degassed under vacuum and purged with H₂ for three times. The mixture was stirred under H₂ (206843 Pa = 30 psi) at 25°C for 16 hrs to give a suspension. The reaction mixture was filtered through a pad of Celite and washed with THF (3 × 20 mL). The filtrate was concentrated to give **II-E10** (2.4 g, 96%) as a solid.

**¹H NMR** (400 MHz, CDCl₃) δ_{H} 8.05-8.00 (m, 4H), 7.65-7.50 (m, 2H), 7.45-7.35 (m, 4H), 5.20-5.10 (m, 1H), 2.30-2.20 (m, 1H), 2.10-1.75 (m, 6H), 1.75-1.30 (m, 13H), 1.30-1.05 (m, 11H), 0.85-0.75 (m, 3H),0.70 (s, 3H).

### Synthesis of II-E11

To a solution of **II-E10** (2.4 g, 0.875 mmol) in THF (10 mL) and MeOH (10 mL) was added NaOH (1.72 g, 43.1 mmol) and H₂O (4 mL) at 25°C. The solution was stirred at 25°C for 48 hrs. The residue was poured into water (30 mL). The mixture was extracted with EtOAc (2 x 20 mL). The combined organic layers were dried over Na₂SO₄, filtered and concentrated in vacuum to give product which was triturated with MeCN (10 mL) to give desired product **II-E11** (1.6 g, ) as a solid.

**¹H NMR** (400 MHz, CDCl₃) δ_{H} 3.75-3.65 (m, 1H), 2.05-1.75 (m, 4H), 1.75-1.35 (m, 13H), 1.35-1.05 (m, 16H), 0.85-0.75 (m, 3H),0.64 (s, 3H).

### Synthesis of II-E12

To a solution of **II-E11** (1.6 g, 4.59 mmol) in DCM (20 mL) was added PCC (1.97 g, 1.51 mol) and silica gel (1 g) at 25°C. After stirring at 25°C for 3 hrs, the reaction mixture was filtered and the residue was washed with anhydrous DCM (2 × 10 mL). The combined filtrate was concentrated in vacuum. The residue was purified by flash column (0-50% of EtOAc in PE) to give **II-E12** (1.4 g, 88%) as a solid.

**¹H NMR** (400 MHz, CDCl₃) δ_{H} 2.60-2.50 (m, 1H), 2.25-2.05 (m, 4H), 2.05-1.90 (m, 2H), 1.80-1.65 (m, 6H), 1.65-0.30 (m, 8H), 1.30-1.05 (m, 11H), 0.85-0.70 (m, 3H), 0.59 (s, 3H). **LC-ELSD/MS** Rt = 1.223 min in 2 min chromatography, 30-90AB_2MIN_E, purity 99%, MS ESI calcd. for C₂₃H₃₇O [M+H-H₂O]⁺ 329, found 329.

(Mobile Phase: 1.5ML/4LTFA in water (solvent A) and 0.75ML/4LTFA in acetonitrile (solvent B), using the elution gradient 30% - 90% (solvent B) over 0.9 minutes and holding at 90% for 0.6 minutes at a flow rate of 1.2 ml/min; Column: Xtimate C18 2.1*30mm,3um; Wavelength: UV 220nm; Column temperature: 50°C; MS ionization: ESI; Detector: PDA & ELSD).

### Example II-10: Synthesis of (3R,5R,8S,9S,10S,13S,14S,17S)-10-ethyl-3-hydroxy-3,13-dimethyl-N-phenylhexadecahydro-1H-cyclopenta[a]phenanthrene-17-carboxamide (II-G3)

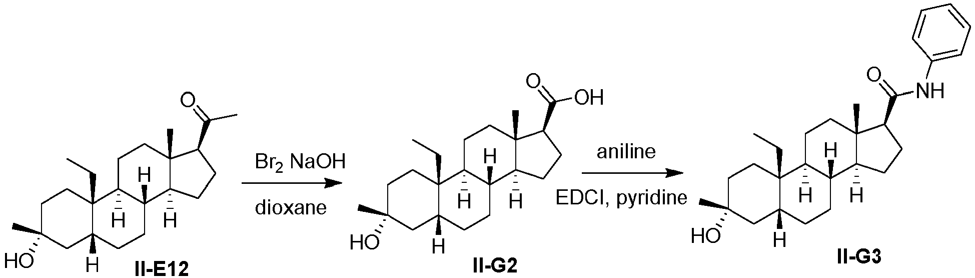

The experimental of II-E12 could be found in Example II-8.

### Synthesis of II-G2

Liquid bromine (2.28 g, 14.3 mmol) was added slowly to a vigorously stirred sodium hydroxide aqueous (5.73 mL, 3 M, 17.2 mmol) at 0°C. When all the bromine was dissolved, the mixture was diluted with cold dioxane (10 mL) and was added slowly to a stirred solution of **II-E12** (500 mg, 1.44 mmol) in dioxane (10 mL) and water (10 mL) and a precipitate was formed, and the reaction mixture was stirred at 20°C for 16 hours. The remaining oxidizing reagent was quenched by Na₂S₂O₃ aqueous (20 mL) and the mixture was then heated at 80°C until the solid material was dissolved. Acidification of the solution with hydrochloride acid (3 N) furnished a precipitate. The solid was filtered and washed with water (3 × 20 mL) to give a solid, which was dried under vacuum to afford **II-G2** (450 mg, ) as a solid.

### Synthesis of II-G3

To a solution of **II-G2** (100 mg, 0.2869 mmol) in pyridine (5 mL) was added EDCI (82.4 mg, 0.4303 mmol). After stirring at 20°C for 10 mins, aniline (29.3 mg, 0.3155 mmol) was added. The mixture was stirred at 20°C for 16 hrs. To the reaction mixture was added water (20 mL) and stirred 30 mins. The suspension was filtrated and triturated from MeCN (5 mL) at 20°C to give **II-G3** (63 mg, 52 %) as a solid.

**¹H NMR** (400 MHz, CDCl₃) δ_{H} 7.60-7.45 (m, 2H), 7.35-7.27 (m, 2H), 7.20-7.05 (m, 1H), 6.93 (s, 1H), 2.35-2.25 (m, 2H), 2.10-1.95 (m, 2H), 1.90-1.50 (m, 6H), 1.50-1.05 (m, 19H), 0.85-0.75 (m, 3H), 0.73 (s, 3H).

**LC-ELSD/MS** purity 99%, **MS ESI** calcd. for C₂₈H₄₂NO₂ [M+H]⁺ 424, found 424.

### Example II-11: Synthesis of 1-((3R,5R,8S,9S,10S,13S,14S,17S)-10-ethyl-3-hydroxy-3,13-dimethylhexadecahydro-1H-cyclopenta[a]phenanthren-17-yl)-2-(6-methoxy-1H-benzo[d][1,2,3]triazol-1-yl)ethan-1-one (II-M2), 1-((3R,5R,8S,9S,10S,13S,14S,17S)-10-ethyl-3-hydroxy-3,13-dimethylhexadecahydro-1H-cyclopenta[a]phenanthren-17-yl)-2-(5-methoxy-1H-benzo[d][1,2,3]triazol-1-yl)ethan-1-one (II-M2a) & 1-((3R,5R,8S,9S,10S,13S,14S,17S)-10-ethyl-3-hydroxy-3,13-dimethylhexadecahydro-1H-cyclopenta[a]phenanthren-17-yl)-2-(5-methoxy-2H-benzo[d][1,2,3]triazol-2-yl)ethan-1-one (II-M2b)

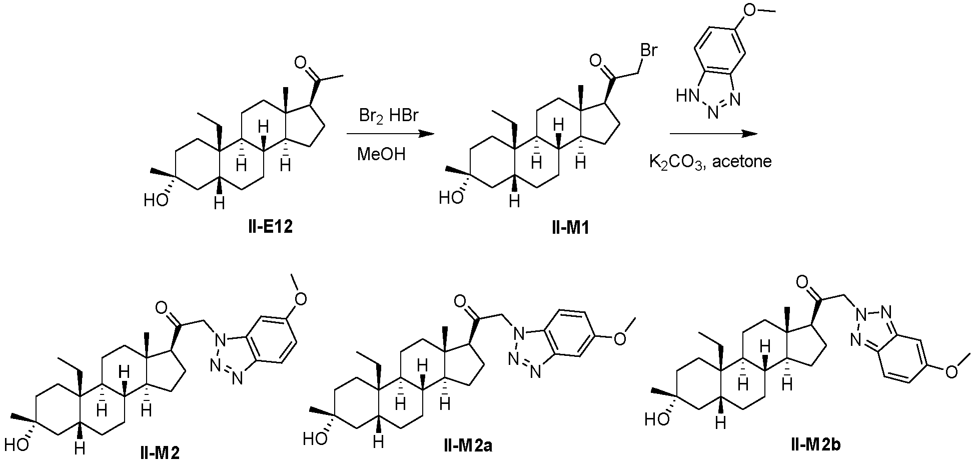

The experimental of **II-E12** could be found in Example II-8.

### Synthesis of II-M1

To a solution **of II-E12** (500 mg, 1.44 mmol) in MeOH (10 ml) was added HBr (58.2 mg, 0.288 mmol, 40% in water) and Br₂ (241 mg, 1.51 mmol) at 25°C. After stirring at 25°C for 2 hrs, the mixture was quenched by sat.aq NaHCO₃ (20 mL). The mixture was extracted with EtOAc (2 × 20 mL). The combined organic phase was washed with brine (20 mL), dried over anhydrous Na₂SO₄, filtered, concentrated in vacuum to afford **II-M1** (610 mg, ) as a solid which was used directed in the next step.

### Synthesis of II-M2, II-M2a, II-M2b

To a solution of **II-M1** (510 mg, 1.19 mmol) in Acetone (10 mL) was added K₂CO₃ (245 mg, 1.78 mmol) and 5-methyl-2H-tetrazole (211 mg, 1.42 mmol) at 20°C. The mixture was stirred at 20°C for 16 hrs. The reaction mixture was treated with water (20 mL). The mixture was extracted with EtOAc (2 × 20 mL). The combined organic phase was washed with brine (20 mL), dried over anhydrous Na₂SO₄, filtered, concentrated in vacuum. The residue was purified by HPLC (Column: YMC-Actus Triart C18 100*30mm*5um), gradient: 75-95% B (water (0.05%HCl)-ACN), flow rate: 25 mL/min) to afford the mixture **II-M2** and **II-M2a** (Peak 1, 320 mg, ) as a solid and **II-M2b** (Peak 2, 50 mg, ) as a solid.

The mixture **II-M2** and **II-M2a** (320 mg, ) was purified by SFC (column: DAICEL CHIRALCEL OJ-H(250mm*30mm,5um)), gradient: 35-35% B (0.1%NH₃H₂O, B= EtOH), flow rate: 200 mL/min) to afford **II-M2** (Peak 1, 49 mg, 8 %) as a solid and **II-M2a** (Peak 2, 62 mg, 10 %) as a solid.

The residue **II-M2b** (50 mg, ) was dissolved in MeCN (5 mL) at 25°C and concentrated in vacuum to **II-M2b** (45 mg, 8 %) as a solid.

### II-M2:

**¹H NMR** (400 MHz, CDCl₃) δ_{H} 7.90-7.80 (m, 1H), 7.05-6.95 (m, 1H), 6.75-6.55 (m, 1H), 5.45-5.25 (m, 2H), 3.86 (s, 3H), 2.75-5.60 (m, 1H), 2.25-2.05 (m, 2H), 2.01-1.85 (m, 1H), 1.80-1.50 (m, 6H), 1.50-1.05 (m, 19H), 0.80-0.75 (m, 3H), 0.72 (s, 3H); **MS ESI** calcd. for C₃₀H₄₄N₃O₃ [M+H]⁺ 494, found 494.

### II-M2a:

**¹H NMR** (400 MHz, CDCl₃) δ 7.40-7.35 (m, 1H), 7.25-7.15 (m, 1H), 7.15-7.05 (m, 1H), 5.40-5.30 (m, 2H), 3.89 (m, 3H), 2.65-2.55 (m, 1H), 2.25-2.05 (m, 3H), 2.05-1.80 (m, 1H), 1.75-1.50 (m, 7H), 1.50-1.30 (m, 7H), 1.30-1.05 (m, 10H), 0.85-0.75 (m, 3H), 0.70 (s, 3H); **MS ESI** calcd. for C₃₀H₄₄N₃O₃ [M+H]⁺ 494, found 494.

### II-M2b:

**¹H NMR** (400 MHz, CDCl₃) δ_{H} 75-7.70 (m, 1H), 7.15-7.05 (m, 2H), 5.55-5.35 (m, 2H), 3.88 (m, 3H), 2.65-2.55 (m, 1H), 2.25-2.05 (m, 2H), 2.05-1.80 (m, 1H), 1.80-1.50 (m, 7H), 1.50-1.30 (m 8H), 1.30-1.05 (m, 10H), 0.85-0.75 (m, 3H), 0.72 (s, 3H); **MS ESI** calcd. for C₃₀H₄₄N₃O₃ [M+H]⁺ 494, found 494.

### Example 11-12- 11-14

The following examples were made from **II-M1** as in Example II-11 replacing 5-methyl-2H- tetrazole with the listed nucleophile.

| **Exam ple #** | **Nucleop hile** | **Name** | **¹H NMR (400 MHz, CDCl3)** | **MS ESI** |
|---|---|---|---|---|
| II-12 | 1H-pyrazole -4-carbonitr ile | 1-((3R,5R,8S,9S,10S,13 S,14S,17S)-10-ethyl-3-hydroxy-3,13-dimethylhexadecahyd ro-1H-cyclopenta[a]phenant hrene-17-carbonyl)-1H-pyrazole-4-carbonitrile (**II-12**) | δ_{H} 7.85-7.75 (m, 2H), 5.05-4.80 (m, 2H), 2.60-2.50 (m, 1H), 2.25-2.10 (m, 1H), 2.10-1.90 (m, 2H), 1.75-1.50 (m, 6H), 1.50-1.25 (m, 8H), 1.25-1.05 (m, 11H), 0.85-0.70 (m, 3H), 0.65 (s, 3H). | calcd. for C₂₇H₃₈N₃O [M+H-H2O]⁺ 420, found 420 |
| II-13 | *2H-*1,2,3-triazole | 1-((3R,5R,8S,9S,10S,13 S,14S,17S)-10-ethyl-3-hydroxy-3,13-dimethylhexadecahyd ro-1H-cyclopenta[a]phenant hren-17-yl)-2-(2H-1,2,3-triazol-2-yl)ethan-1-one (**II-13**) | δ_{H} 7.67 (s, 2H), 5.28-5.17 (m, 2H), 2.56 (t, *J* = 7.2 Hz, 1H), 2.24-2.14 (m, 1H), 2.10-2.04 (m, 1H), 1.99-1.90 (m, 1H), 1.80-1.50 (m, 9H), 1.47-1.36 (m, 5H), 1.35-1.12 (m, 11H), 0.79 (t, *J* = 7.2 Hz, 3H), 0.68 (s, 3H). | calcd. for C₂₅H₃₉N₃O ₂ [M+H]⁺ 413.3, found 414.3. |
| II-13a | *2H-*1,2,3-triazole | 1-((3R,5R,8S,9S,10S,13 S,14S,17S)-10-ethyl-3-hydroxy-3,13-dimethylhexadecahyd ro-1H-cyclopenta[a]phenant hren-17-yl)-2-(1H-1,2,3-triazol-1-yl)ethan-1-one (**II-13a**) | δ_{H} 7.76 (d, *J* = 0.8 Hz, 1H), 7.64 (d, *J* = 0.8 Hz, 1H), 5.20 (dd, *J* = 18.0, 54.8 Hz, 2H), 2.64 (t, *J* = 8.8 Hz, 1H), 2.27-2.16 (m, 1H), 2.13-2.05 (m, 1H), 2.01-1.92 (m, 1H), 1.82-1.58 (m, 6H), 1.55-1.38 (m, 8H), 1.36-1.13(s, 11H), 0.80 (t, *J* = 7.6 Hz, 3H), 0.65 (s, 3H). | calcd. for C₂₅H₃₉N₃O ₂ [M+H]⁺ 413.3, found 414.3. |
| II-14 | 5-methyl-1H- | 1-((3R,5R,8S,9S,10S,13 S,14S,17S)-10-ethyl-3-hydroxy-3,13-dimethylhexadecahyd | δ_{H} 5.34 (s, 2H), 2.65-2.58 (m, 1H), 2.56 (s, 3H), 2.21 (br d, J = 10.4 Hz, 1H), 2.10-1.91 (m, 2.5H), 1.82-1.60 (m, 6.5H), 1.49-1.29 (m, 10H), 1.26 (s, | calcd. for C₂₅H₃₉N₄O [M-H₂O+H]+ |

| | | | | |
|---|---|---|---|---|
| | 1,2,3,4-tetrazole | ro-1H-cyclopenta[a]phenant hren-17-yl)-2-(5-methyl-2H-tetrazol-2-yl)ethan-1-one (**II-14**) | 10H), 0.80 (t, J = 7.2 Hz, 3H), 0.69 (s, 1H). | 411, found 411. |
| II-14a | 5-methyl-1H-1,2,3,4-tetrazole | 1-((3R,5R,8S,9S,10S,13 S,14S,17S)-10-ethyl-3-hydroxy-3,13-dimethylhexadecahyd ro-1H-cyclopenta[a]phenant hren-17-yl)-2-(5-methyl-1H-tetrazol-1-yl)ethan-1-one (**II-14a**) | δ_{H} 5.18 - 5.01 (m, 2H), 2.68 - 2.62 (m, 1H), 2.47 (s, 3H), 2.20 (br d, *J* = 9.2 Hz, 1H), 2.10 - 2.03 (m, 1H), 2.02 - 1.92 (m, 1H), 1.80 - 1.62 (m, 6H), 1.52 - 1.32 (m, 9H), 1.27 (s, 10H), 0.81 (t, *J* = 7.2 Hz, 3H), 0.66 (s, 3H) | calcd. for C₂₅H₃₉N₄O [M-H₂O+H]⁺ 411, found 411. |

### Example II-20- II-22

The following examples were made from **II-G2** as in Example II-1 replacing (3S)-3-phenylpyrrolidine with the listed nucleophile (amine).

| **Exam ple #** | **amine** | **Name** | **¹H NMR (400 MHz, CDCl₃)** | **MS ESI** |
|---|---|---|---|---|
| II-20 | (2S)-2-methylpi peridine | ((3R,5R,8S,9S,10S,13 S,14S,17S)-10-ethyl-3-hydroxy-3,13-dimethylhexadecahyd ro-1H-cyclopenta[a]phenant hren-17-yl)((S)-2-methylpiperidin-1-yl)methanone (**II-20**) | δ_{H} 5.00-4.90 (m, 0.88H), 4.60-4.45 (m, 0.35H), 4.30-4.25 (m, 0.33H), 3.80-3.75 (s, 0.90H), 3.10-2.95 (m, 1H), 2.70-2.55 (m, 1.70H), 2.40-2.20 (m, 1.40H), 2.05-1.80 (m, 1H), 1.80-1.50 (m, 4H), 1.50-1.30 (m, 9H), 1.30-1.20 (m, 9H), 1.20-1.05 (m, 10H), 0.80-0.75 (m, 5H), 0.71 (s, 3H). | calcd. For C₂₈H₄₈NO₂ [M+H]⁺ 430, found 430. |
| II-21 | 2-ethylpip eridine | ((3R,5R,8S,9S,10S,13 S,14S,17S)-10-ethyl-3-hydroxy-3,13-dimethylhexadecahyd ro-1H-cyclopenta[a]phenant hren-17-yl)((S)-2-ethylpiperidin-1-yl)methanone (**II-21**) | δ 4.78-4.50 (m, 1H), 3.95-3.75 (m, 1H), 3.12-2.80 (m, 1H), 2.76-2.50 (m, 1H), 2.30-2.10 (m, 1H), 2.09-1.87 (m, 1H), 1.83-1.70 (m, 2H),1.68-1.49 (m, 17H), 1.40-1.27 (m, 6H), 1.27-1.10 (m, 10H), 0.89-0.75 (m, 6H), 0.72 (s, 2H). | calcd. For C₂₉H₅₀NO₂ [M+H] ⁺ 444, found 444 |
| II-21a | 2-ethylpip eridine | ((3R,5R,8S,9S,10S,13 S,14S,17S)-10-ethyl-3-hydroxy-3,13-dimethylhexadecahyd ro-1H- | δ 4.82-4.56 (m, 1H), 4.05-3.85 (m, 1H), 3.04-2.95 (m, 0.5H), 2.73-2.54 (m, 1H), 2.53-2.38 (m, 0.5H), 2.37-2.30 (m, 1H), 1.99-1.92 (m, 1H),1.68-1.49 | calcd. For C₂₉H₅₀NO₂ [M+H] ⁺ 444, found 444 |

| | | | | |
|---|---|---|---|---|
| | | cyclopenta[a]phenant hren-17-yl)((R)-2-ethylpiperidin-1-yl)methanone (**II-21a**) | (m, 11H), 1.59-1.49 (m, 12H), 1.40-1.10 (m,11H), 0.86-0.75 (m, 6H),0.72-0.68 (m, 3H). | |
| II-22 | Ammoni um chloride | (3R,5R,8S,9S,10S,13 S,14S,17S)-10-ethyl-3-hydroxy-3,13-dimethylhexadecahyd ro-1H-cyclopenta[a]phenant hrene-17-carboxamide (**II-22**) | δ_{H} 5.24 (br d, *J* = 11.6 Hz, 2H), 2.21-2.08 (m, 2H), 2.02-1.91 (m, 2H), 1.84-1.59 (m, 6H), 1.51-1.30 (m, 8H), 1.29-1.09 (m, 11H), 0.79 (t, *J* = 7.6 Hz, 3H), 0.70 (s, 3H). | calcd. for C₂₂H₃₈NO₂ [M +H]⁺ 348, found 348 |

### Example II-30: Synthesis of ((3R,5R,8S,9S,10S,13S,14S,17S)-10-ethyl-3-hydroxy-3,13-dimethylhexadecahydro-1H-cyclopenta[a]phenanthren-17-yl)(o-tolyl)methanone (II-G5)

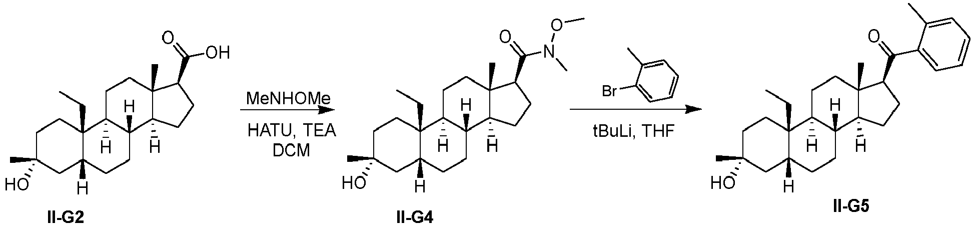

### Synthesis of II-G4

To a solution of **II-G2** (100 mg, 0.3 mmol) in DMF (3 mL) was added HATU (163 mg, 0.4 mmol), TEA (57.9 mg, 0.6 mmol) and N,O-dimethylhydroxylamine hydrochloride (26.2 mg, 0.4 mmol) at 25°C. The mixture was stirred at 25°C for 16 hrs. The reaction was poured into water (50 mL) and extracted with DCM (2 × 50 mL). The combined organic phase was washed with brine (50 mL), dried over anhydrous Na₂SO₄, filtered and concentrated in vacuum to afford product, which was washed with LiCl (2 × 50 mL), brine (2 × 20 mL), dried over anhydrous Na₂SO₄, filtered, concentrated and was purified by flash column (0∼20% of EtOAc in PE) to give (95 mg, 95%) as a **II-G4** a solid.
**¹H NMR** (400 MHz, CDCl₃) δ_{H} 3.67 (s, 3H), 3.19 (s, 3H), 1.92 (t, *J* = 8.0 Hz, 1H), 1.55-1.45 (m, 7H), 1.45-1.10 (m, 18H), 0.90-0.80 (m, 3H), 0.78 (t, *J* = 8.0 Hz, 3H), 0.72 (s, 3H).

### Synthesis of II-G5

To a solution of 1-bromo-2-methylbenzene (700 mg, 4.1 mmol) in THF (2 mL) was added t-BuLi (5.53 mL, 1.3 M, 7.2 mmol) at -70°C. A suspension was obtained. The resulting mixture was stirred at -70°C for 15 min. To the suspension of (2-methylphenyl)lithium was added **II-G4** (80 mg, 0.2 mmol) in THF (2 mL) was added at-70°C. The reaction mixture was stirred at 0°C for 10 min. The mixture was extracted with ethyl acetate (3 × 50 mL). The combined organic phase was washed with brine (50 mL), dried over Na₂SO₄, filtered and concentrated under vacuum to give a solid (95 mg). The material (20 mg, ) was purified by prep-HPLC to give **II-G5** (12 mg, 0.028 mmol) as a solid.
**¹H NMR** (400 MHz, CDCl₃) δ_{H} 7.47 (d, 1H), 7.40-7.30 (m, 1H), 7.25-7.20 (m, 2H), 3.30 (t, *J* = 8.0 Hz, 1H), 2.39 (s, 3H), 2.35-2.25 (m, 1H), 1.91 (t, *J* = 8.0 Hz, 1H), 1.75-1.05 (m, 26H), 0.76 (t, *J* = 8.0 Hz, 3H), 0.64 (s, 3H); **MS ESI** calcd. for C29H43O2 [M+H]+ 423, found 423.

### EXAMPLE II-35: Synthesis of 1-((3R,5R,8S,9S,10S,13S,14S,17S)-10-ethyl-3-hydroxy-3-(methoxymethyl)-13-methylhexadecahydro-1H-cyclopenta[a]phenanthren-17-yl)ethan-1-one (II-B9)

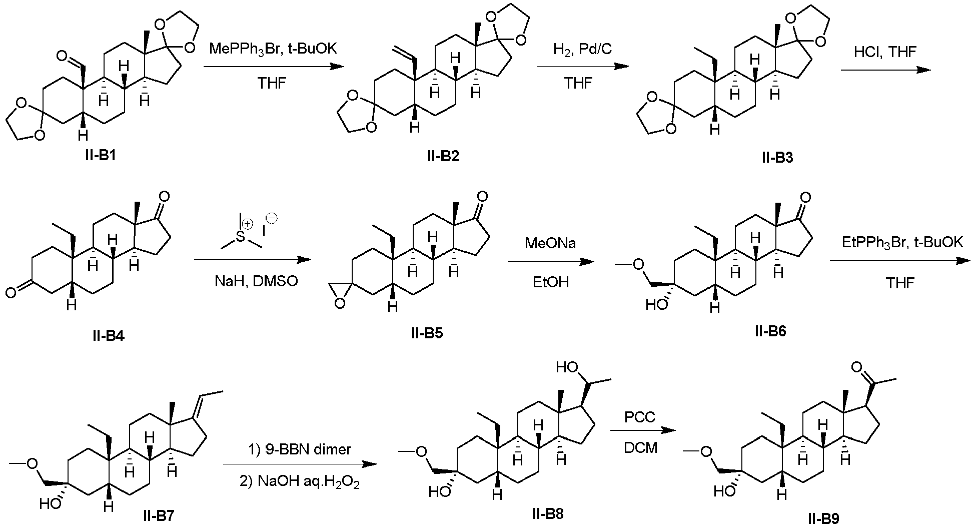

### Synthesis of II-B2

To a suspension of PPh₃MeBr (548 g, 1536 mmol) in THF (1000 mL) was added *t*-BuOK (172 g, 1536 mmol) at 15°C. After stirring at 45°C for 1 hour, a solution of **II-B1** (200 g, 512 mmol, reported in patent 'WO2016/134301, 2016, A2') in THF (500 mL) was added at 45°C and the reaction mixture was stirred at 45°C for 10 min. The mixture was added saturated NH₄Cl solution (500 mL) and EtOAc (500 mL). The organic layer was separated, and the water phase was extracted with EtOAc (2 × 300 mL). The combined organic phase was washed with saturated brine (2 × 300 mL), dried over anhydrous Na₂SO₄, filtered, concentrated and triturated from MeOH/H₂O (1000mL/1000 mL) at 15°C and purified by silica gel chromatography (PE/EtOAc = 50/1 to 20/1) to give **II-B2** (137 g, 69%) as an oil.
**¹H NMR** (400 MHz, CDCl₃) δ 6.40-6.25 (m, 1H), 5.15-5.00 (m, 2H), 3.95-3.75 (m, 9H), 2.10-1.65 (m, 9H), 1.65-1.25 (m, 5H), 1.25-1.05 (m, 6H), 0.85-0.75 (m, 4H).

### Synthesis of II-B3

To a solution **of II-B2** (70 g, 180 mmol) in THF (1000 mL) was added Pd-C (dry, 10%, 10 g) under N₂. The suspension was degassed under vacuum and purged with H₂ for three times. The mixture was stirred under H₂ (206843 Pa = 30 psi) at 25°C for 16 hours. The reaction mixture was filtered through a pad of Celite and washed with THF (3 × 500 mL). The filtrate was concentrated with a 2^{nd} batch (70g II-B2) to give **II-B3** (141 g) as an oil.
**¹H NMR** (400 MHz, CDCl₃) δ 3.95-3.75 (m, 9H), 2.10-1.90 (m, 2H), 1.90-1.65 (m, 8H), 1.65-1.25 (m, 4H), 1.25-1.05 (m, 8H), 0.80-0.45 (s, 7H).

### Synthesis of II-B4

To a soultion **of II-B3** (141 g, 361 mmol) in THF (1000 mL) was added aq.HCl (361 mL, 4M, 1444 mmol). The mixture was stirred at 25°C for 16 hrs. The mixture was poured into water (500 mL). The organic phase was separated and the aqueous was extracted with EA (2 × 300 ml). The combined organic solutions was washed with saturated aqueous NaHCO₃ (500 mL), H₂O (2 × 300 mL), brine (200 mL), dried over Na₂SO₄, filtered, concentrated in vacuum and triturated from PE (300 mL) at 15°C to give **II-B4** (82 g, 73 %) as a solid.
**¹H NMR** (400 MHz, CDCl₃) δ 2.80-2.70 (m, 1H), 2.55-2.45 (m, 3H), 2.45-2.15 (m, 1H), 2.15-2.00 (m, 3H), 2.00-1.90 (m, 1H), 1.90-1.65 (m, 6H), 1.65-1.30 (m, 2H), 1.30-1.10 (m, 7H), 0.88 (s, 3H), 1.85-0.75 (m, 3H).

### Synthesis of II-B5

To a stirred solution of Trimethylsulfonium iodide (22.2 g, 109 mmol) in DMSO/THF (100 mL/500mL) was aded NaH (4.35 g, 109 mmol, 60 % in oil) at 0°C for 1 h under N₂. The mixture was added to a solution of **II-B4** (30 g, 99.1 mmol) in DMSO (100 mL) at 15°C for 4 hrs. The reaction was treated with water (200 mL). The mixture was extracted with EtOAc (2 × 100 mL). The combined organic phase was washed with water (2 × 100 mL), brine (100 mL), dried over anhydrous Na₂SO₄, filtered and concentrated in vacuum to give **II-B5** (35 g,) as anoil.

### Synthesis of II-B6

To anhydrous methanol (300 mL) was added Na (9.83 g, 410 mmol) at 15°C in ten portions. The reaction mixture was stirred at 65°C for 1 h. To a suspension of methoxysodium was added **II-B5** (13 g, 41.0 mmol) in anhydrous methanol (50 mL) at 65°C. The reaction mixture was stirred at 65°C for 16 hrs. Water (500ml) was added. The reaction mixture was concentrated to remove most of the solvent. The mixture was extracted with EtOAc (2 × 200 mL). The combined organic phase was washed with brine (2 × 100 mL), dried over anhydrous Na₂SO₄, filtered, concentrated. The residue was purified by flash column (0-20% of EtOAc in PE) to give **II-B6** (7 g,) as an oil and **II-B6a**.

### II-B6:

**¹H NMR** (400 MHz, CDCl₃) δ 3.45-3.30 (m, 5H), 2.50-2.30 (m, 1H), 2.15-1.95 (m, 2H), 1.95-1.80 (m, 2H), 1.80-1.50 (m, 5H), 1.50-1.30 (m, 7H), 1.30-1.05 (m, 8H), 0.90-0.65 (m, 6H).

### Synthesis of II-B7

To a suspension of bromo(ethyl)triphenylphosphorane (29.6 g, 80.0 mmol) in THF (200 mL) was added *t*-BuOK (8.96 g, 80.0 mmol) at 15°C under N₂. The mixture was heated to 45°C and stirred for 1 h. A solution of **II-B6** (7 g, 20.0 mmol) in THF (50 mL) was added. The mixture was stirred at 45°C for 16 hrs. The mixture was treated with NH₄Cl (100 mL). The organic layer was separated. The aqueous phase was extracted with EtOAc (2 × 50 mL). The combined organic phase was washed with brine (2 × 30 mL), dried over anhydrous Na₂SO₄, filtered, concentrated in vacuum. The residue was triturated from MeOH/H₂O (50mL/50 mL) at 15°C to give **II-B7** (7.5 g,) as a solid.

### Synthesis of II-B8

To a solution of **II-B7** (5 g, 13.8 mmol) in THF (50 mL) was added 9-BBN dimer (6.73 g, 27.6 mmol) at 15°C. The mixture was stirred at 15°C for 48 hrs. After cooling to 0°C, to the reaction mixture was added ethanol (6.34 g, 138 mmol) and NaOH (27.6 mL, 5 M, 138 mmol) very slowly. After the addition was completed, H₂O₂ (13.8 mL, 138 mmol, 30%) was added slowly and the inner temperature was maintained below 15°C. The resulting solution was stirred at 75°C for 1 hrs. Saturated aqueous Na₂S₂O₃ (50 mL) was added and the mixture was stirred at 0°C for another 1 hour. The reaction was checked by potassium iodide-starch test paper to confirm excess H₂O₂ was destroyed. The mixture was cooled and added to Water (500 mL). The mixture was filtered. The filter cake was dissolved in EtOAc (100 mL). The organic phase was washed with brine (30 mL), dried over anhydrous Na₂SO₄, filtered and concentrated under vacuum to give **II-B8** (7 g,) as an oil.

### Synthesis of II-B9

To a solution **of II-B8** (7 g, 18.4 mmol) in DCM (100 mL) was added PCC (7.91 g, 36.8 mmol) and silica gel (8 g) at 15°C. The solution was stirred at 15°C for 3 hrs. The reaction mixture was filtered and the residue was washed with anhydrous DCM (2 × 100 mL). The combined filtrate was concentrated in vacuum. The residue was purified by flash column (0∼50% of EtOAc in PE) to give **II-B9** (3.8 g, 55 %) as a solid.
**¹H NMR** (400 MHz, CDCl₃) δ 3.45-3.30 (m, 5H), 2.63 (s, 1H), 2.60-2.45 (m, 1H), 2.20-2.05 (m, 4H), 2.05-1.80 (m, 2H), 1.80-1.50 (m, 7H), 1.50-1.30 (m, 7H), 1.30-1.05 (m, 7H), 0.80-0.65 (m, 3H), 0.58 (s, 3H); **MS ESI** calcd. for C₂₄H₃₉O₂ [M+H-H₂O]⁺ 359, found 359.

### Example II-36: Synthesis of ((3R,5R,8S,9S,10S,13S,14S,17S)-10-ethyl-3-hydroxy-3-(methoxymethyl)-13-methylhexadecahydro-1H-cyclopenta[a]phenanthren-17-yl)((S)-2-methylpiperidin-1-yl)methanone (II-B11)

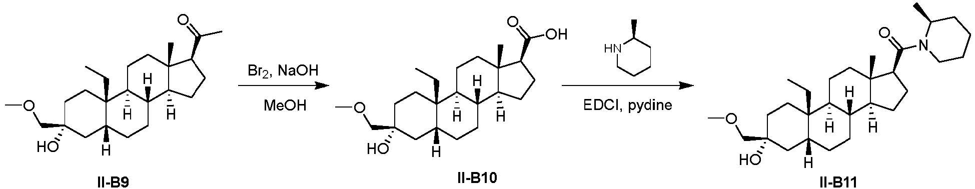

### Synthesis of II-B10

Liquid bromine (1.05 g, 6.60 mmol) was added slowly to a vigorously stirred sodium hydroxide aqueous (5.26 mL, 3 M, 15.8 mmol) at 0°C. When all the bromine was dissolved, the mixture was diluted with cold dioxane (5 mL) and was added slowly to a stirred solution of **II-B9** (500 mg, 1.32 mmol) in dioxane (10 mL) and water (5 mL) and a precipitate was formed, and the reaction mixture was stirred at 15°C for 16 hours. The remaining oxidizing reagent was quenched by Na₂SO₃ aqueous (30 mL) and the mixture was then heated at 80°C until the solid material was dissolved. Acidification of the solution with hydrochloride acid (3 N) furnished a precipitate. The suspension was added EtOAc (20 mL). The organic phase was separated. The aqueous was extracted with EtOAc (2 × 20 mL). The combined organic was washed with brine (20 mL), dried over anhydrous Na₂SO₄, filtered and concentrated under vacuum to afford **II-B10** (550 mg, ) as a solid.

### Synthesis of II-B11

To a solution of **II-B10** (500 mg, 1.32 mmol) in pyridine (10 mL) was added EDCI (1.01 g, 5.28 mmol) and stirred at 25°C for 10 mins. (2S)-2-methylpiperidine (143 mg, 1.45 mmol) was added. The mixture was stirred at 25°C for 16 hrs. To the reaction mixture was added water (20 mL) and EtOAc (20 mL). The organic layer was separated. The aqueous phase was extracted with EtOAc (2 × 10 mL). The combined organic phase was washed with brine (2 × 10 mL), dried over anhydrous Na₂SO₄, filtered, concentrated and purified by HPLC ((column: YMC-Actus Triart C18 100*30mm*5um), gradient: 85-100% B (A= water (0.05% HCl)-ACN), B= MeCN), flow rate: 25 mL/min) to give **II-B11** (136mg, 22 %) as a solid.
**¹H NMR** (400 MHz, CDCl₃) δ 5.00-4.80 (m, 0.7H), 4.65-4.45 (m, 0.3H), 4.35-4.25 (m, 0.3H), 3.80-3.70 (m, 0.7H), 3.40-3.25 (m, 5H), 3.10-2.95 (m, 1H), 2.75-2.60 (m, 2H), 2.35-2.15 (m, 1H), 2.00-1.80 (m, 1H), 1.80-1.55 (m, 12H), 1.55-1.30 (m, 8H), 1.30-1.05 (m, 11H), 0.80-0.65 (m, 6H); **MS ESI** calcd. for C₂₉H₅₀NO₃ [M+H]⁺ 460, found 460.

### Example II-40: Synthesis of 1-((3R,5R,8S,9S,10S,13S,14S,17S)-3-(ethoxymethyl)-10-ethyl-3-hydroxy-13-methylhexadecahydro-1H-cyclopenta[a]phenanthren-17-yl)ethan-1-one (II-C4)

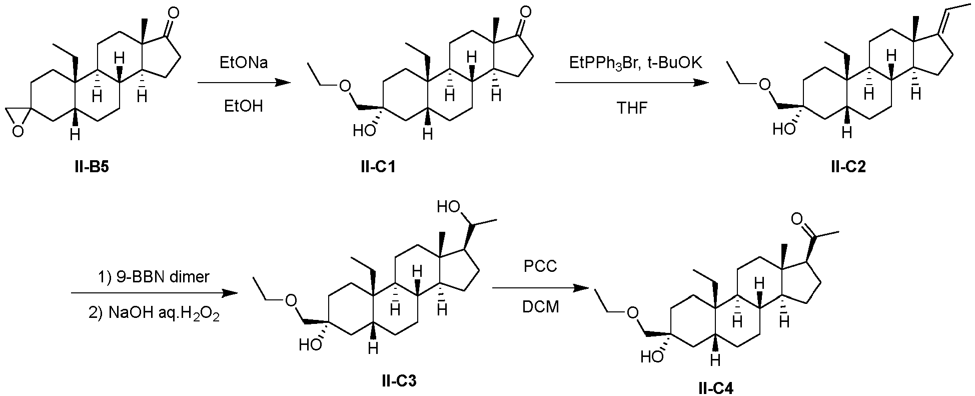

### Synthesis of II-C1

To anhydrous EtOH (200 mL) was added Na (25.4 g, 1107 mmol) at 15°C in ten potions. The reaction mixture was stirred at 75°C for1 h. To the suspension of ethoxysodium was added **II-B5** (22 g, 69.5 mmol) in anhydrous ethanol (50 mL) at 75°C. The reaction mixture was stirred at 75°C for 16 hrs. Water (500ml) was added at 15°C. The reaction mixture was concentrated to remove most of the solvent. The mixture was extracted with EtOAc (2 × 200 mL). The combined organic phase was washed with saturated brine (2 × 100 mL), dried over anhydrous Na₂SO₄, filtered, concentrated. The residue was purified by flash column (0∼20% of EtOAc in PE) to give **II-C1** (12 g, ) as an oil and **II-C1a** (7 g, 28 %) as a solid.

### II-C1:

**¹H NMR**(400 MHz, CDCl₃) δ_{H} 3.60-3.45 (m, 2H), 3.45-3.30 (m, 2H), 2.45-2.30 (m, 1H), 2.10-1.90 (m, 4H), 1.90-1.55 (m, 5H), 1.55-1.30 (m, 7H), 1.30-1.10 (m, 11H), 0.90-0.80 (m, 3H), 0.80-0.60 (m, 3H).

### II-C1a:

**¹H NMR** (400 MHz, CDCl₃) δ_{H} 3.55-3.46 (m, 2H), 3.25-3.15 (m, 2H),2.47-2.35 (m, 1H),2.10-1.95 (m, 4H), 1.95-1.85 (m, 1H), 1.85-1.60 (m, 5H), 1.55-1.35 (m, 6H), 1.35-1.15 (m, 11H), 0.90-0.80 (m, 6H)

### Synthesis of II-C2

To a suspension of bromo(ethyl)triphenylphosphorane (48.9 g, 132 mmol) in THF (200 mL) was added *t*-BuOK (14.7 g, 132 mmol) at 15°C under N₂. The mixture was heated to 45°C and stirred for 1 h. A solution of **II-C1** (12 g, 33.0 mmol) in THF (20 mL) was added at 45°C. The mixture was stirred at 45°C for 16 hrs. The mixture was treated with NH₄Cl (100 mL). The organic layer was separated. The aqueous phase was extracted with EtOAc (2 × 50 mL). The combined organic phase was washed with brine (2 × 40 mL), dried over anhydrous Na₂SO₄, filtered, concentrated in vacuum. The residue triturated from MeOH/H₂O (50mL/50 mL) at 20°C to give **II-C2** (17 g,) as an oil.

### Synthesis of II-C3

To a solution of **II-C2** (17 g, 45.3 mmol) in THF (100 mL) was added 9-BBN dimer (22.1 g, 90.6 mmol) at 15°C. The mixture was stirred at 15°C for 16 hrs. After cooling to 0°C, to the reaction mixture was added ethanol (20.8 g, 453 mmol) and NaOH (90.6 mL, 5 M, 453 mmol) very slowly. After the addition was completed, H₂O₂ (45.3 mL, 453 mmol, 30%) was added slowly and the inner temperature was maintained below 15°C. The resulting solution was stirred at 75°C for 1 hrs. Saturated aqueous Na₂S₂O₃ (100 mL) was added and the mixture was stirred at 0 °C for another 1 hour. The reaction was checked by potassium iodide-starch test paper to confirm excess H₂O₂ was destroyed. The mixture was cooled and added to Water (2 L). The mixture was filtered. The combined filter oil was dissolved in EtOAc (200 mL). The organic phase was washed with brine (50 mL), dried over anhydrous Na₂SO₄, filtered and concentrated under vacuum to give **II-C3** (18 g,) as an oil.

### Synthesis of II-C4

To a solution **of II-C3** (18 g, 45.8 mmol) in DCM (200 mL) was added PCC (19.6 g, 91.6 mmol) and silica gel (15 g) at 15°C. The solution was stirred at 15°C for 3 hrs. The reaction mixture was filtered and the residue was washed with anhydrous DCM (2 × 100 mL). The combined filtrate was concentrated in vacuum. The residue was purified by flash column (0∼20% of EtOAc in PE) to give **II-C4** (8 g, 45%) as a solid.
**¹H NMR** (400 MHz, CDCl₃) δ_{H} 3.60-3.50 (m, 2H), 3.50-3.30 (m, 2H), 2.74 (s, 1H), 2.60-2.45 (m, 1H), 2.20-2.05 (m, 4H), 2.05-1.75 (m, 2H), 1.75-1.50 (m, 7H), 1.50-1.30 (m, 7H), 1.30-1.05 (m, 10H), 0.80-0.65 (m, 3H), 0.59 (s, 3H); MS ESI calcd. for C₂₅H₄₁O₂ [M+H-H₂O]⁺ 373, found 373.

### Example II-43- II-51

The following examples were made from **II-C5** as in Example II-41 replacing 1,3-dimethyl-1H-pyrazol-5-amine with the listed amine and an appropriate coupling agent (oxalic dichloride, EDCI, HATU, etc).

| **Exa mple #** | **amine** | **Name** | **¹H NMR (400 MHz, CDCl₃) / ¹⁹F NMR (376.5 MHz, CDCl3)** | **MS ESI** |
|---|---|---|---|---|
| II-43 | 4-amino-3-methylbenzonitri le | (3R,5R,8S,9S,10S ,13S,14S,17S)-N-(4-cyano-2-methylphenyl)-3-(ethoxymethyl)-10-ethyl-3-hydroxy-13-methylhexadecahy dro-1H-cyclopenta[a]phen anthrene-17-carboxamide (**II-43**) | δ_{H} 8.40-8.30 (m, 1H), 7.50-7..48 (m, 1H), 7.48-7.40 (m, 1H), 6.95 (s, 1H), 3.65-3.48 (m, 2H), 3.48-3.30 (m, 2H), 2.74 (s, 1H), 2.35-2.20 (m, 5H), 2.10-1.50 (m, 9H), 1.50-1.25 (m, 7H), 1.25-1.10 (m, 10H), 0.80-0.75 (m, 3H), 0.72 (s, 3H). | calcd. For C₃₂H₄₇N₂O₃ [M+H]⁺ 507, found 507 |
| II-44 | (2S)-2-methylpiperidine | ((3R,5R,8S,9S,10 S,13S,14S,17S)-3-(ethoxymethyl)-10-ethyl-3- | δ_{H} 4.94 (s, 0.5H), 4.59-4.48 (m, 0.25H), 4.35-4.28 (m, 0.25 H), 3.82 (d, J = 12.0 Hz, 0.5H), 3.54 (q, J = 7.2 | MS ESI calcd. For C₃₀H₅₂NO₃ [M+H] ⁺ |
| | | hydroxy-13-methylhexadecahy dro-1H-cyclopenta[a]phen anthren-17-yl)((S)-2-methylpiperidin-1-yl)methanone (**II-44**) | Hz, 2H), 3.40 (q, J = 13.6 Hz, 2H), 3.10-3.00 (m, 0.5H), 2.69-2.62 (m, 2H), 2.36-2.15 (m, 1H), 1.94 (t, J = 12.0 Hz, 1H), 1.83-1.58 (m, 10H), 1.55-1.36 (m, 11H), 1.30-1.10 (m, 14H), 0.65-0.33 (m, 6H) | 474, found 474 |
| II-45 | (2R)-2-methylpiperidine | ((3R,5R,8S,9S,10 S,13S,14S,17S)-3-(ethoxymethyl)-10-ethyl-3-hydroxy-13-methylhexadecahy dro-1H-cyclopenta[a]phen anthren-17-yl)((R)-2-methylpiperidin-1-yl)methanone (**II-45**) | δ_{H} 5.10-5.05 (m, 0.5H), 4.56-4.48 (m, 0.5H), 4.40-3.30 (brs, 0.5 H), 3.92-3.84 (m, 0.5H), 3.54 (q, *J* = 7.2 Hz, 2H), 3.40 (q, *J* = 13.6 Hz, 2H), 3.12-3.05 (m, 0.5H), 2.69 (s, 2.5H), 2.38-2.30 (m, 1H), 1.94 (t, *J* = 12.0 Hz, 1H), 1.83-1.58 (m, 12H), 1.45-1.09 (m, 21H), 0.77 (t, *J* = 7.6 Hz, 3H), 0.69 (d, *J* = 4.0 Hz, 3H). | calcd. For C₃₀H₅₂NO₃ [M+H] ⁺ 474, found 474 |
| II-46 | aniline | (3R,5R,8S,9S,10S ,13S,14S,17S)-3-(ethoxymethyl)-10-ethyl-3-hydroxy-13-methyl-N-phenylhexadecahy dro-1H-cyclopenta[a]phen anthrene-17-carboxamide (**II-46**) | δ_{H} 7.51 (d, *J* = 8.0 Hz, 2H), 7.31 (t, *J* = 8.0 Hz, 2H), 7.08 (t, *J* = 7.6 Hz, 1H), 6.94 (s, 1H), 3.55 (q, *J* = 7.2 Hz, 2H), 3.42 (q, *J* = 9.6 Hz, 2H), 2.74 (brs, 1H), 2.38-2.18 (m, 2H), 2.06-1.90 (m, 2H), 1.88-1.58 (m, 8H), 1.53-1.51 (m, 1H), 1.50-1.28 (m, 9H), 1.30-1.15 (m , 6H), 0.78 (t, *J=* 7.6 Hz, 3H), 0.73 (s, 3H) | calcd. For C30H46NO 3 [M+H]⁺ 468, found 468 |
| II-47 | 2-methylaniline | (3R,5R,8S,9S,10S ,13S,14S,17S)-3-(ethoxymethyl)-10-ethyl-3-hydroxy-13-methyl-N-(o-tolyl)hexadecahyd ro-1H-cyclopenta[a]phen anthrene-17-carboxamide (**II-47**) | δ_{H} 7.95 (d, *J* = 8.0 Hz, 1H), 7.23-7.14 (m, 2H), 7.08-7.00 (m, 1H), 6.80 (s, 1H), 3.55 (q, *J* = 7.2 Hz, 2H), 3.47-3.36 (m, 2H), 2.73 (s, 1H), 2.37-2.21 (m, 5H), 2.13-2.03 (m, 1H), 2.00-1.90 (m, 1H), 1.88-1.57 (m, 8H), 1.53-1.31 (m, 8H), 1.28-1.15 (m, 8H), 0.83-0.72 (m, 6H). | calcd. for C₃₁H₄₈NO₃ [M+H] ⁺ 482, found 482 |
| II-48 | Ammonium chloride | (3R,5R,8S,9S,10S , 13 S, 14S, 17S)-3-(ethoxymethyl)- | δ_{H} 5.27 (d, *J*=26.8 Hz, 2H), 3.58-3.50 (m, 2H), 3.46-3.35 (m, 2H), 2.74 (s, 1H), | calcd. for C₂₄H₃₂NO₃ [M +H]⁺ |
| | | 10-ethyl-3-hydroxy-13-methylhexadecahy dro-1H-cyclopenta[a]phen anthrene-17-carboxamide (**II-48**) | 2.20-2.10 (m, 2H), 1.98-1.90 (m, 2H), 1.80-1.56 (m, 7H), 1.56-1.15 (m, 17H), 0.80-0.74 (m, 3H), 0.70 (s, 3H) | 392, found 392 |
| II-50 | (R)-2-(4-fluorophenyl)pip eridine | ((3R,5R,8S,9S,10 S,13S,14S,17S)-3-(ethoxymethyl)-10-ethyl-3-hydroxy-13-methylhexadecahy dro-1H-cyclopenta[a]phen anthren-17-yl)((R)-2-(4-fluorophenyl)piper idin-1-yl)methanone (**II-50**) | δ_{H} 7.12 (br s, 2H), 7.01 (br s, 2H), 6.10-5.95 (m, 1H), 3.99-3.82 (m, 1H), 3.54 (q, J = 7.2 Hz, 2H), 3.46-3.35 (m, 2H), 2.98-2.85 (m, 1 H), 2.82-2.73 (m, 1H), 2.70 (br s, 1H), 2.37 (br d, J = 14.0 Hz, 2H), 1.63 (br s, 12H), 1.52-1.14 (m, 19H), 0.85-0.73 (m, 6H). | calcd. for C₃₅H₅₃FNO ₃ [M+H]⁺ 554, found 554 |
| | | | δ_{F}-117.23. | |
| II-51 | (S)-2-(4-fluorophenyl)pip eridine | ((3R,5R,8S,9S,10 S,13S,14S,17S)-3-(ethoxymethyl)-10-ethyl-3-hydroxy-13-methylhexadecahy dro-1H-cyclopenta[a]phen anthren-17-yl)((S)-2-(4-fluorophenyl)piper idin-1-yl)methanone (**II-51**) | δ_{H} 7.21 (s, 1H), 7.11 (s, 2H), 7.03 (d, J = 8.0 Hz, 2H), 6.10-5.95 (m, 0.5H), 5.35-5.25 (m, 0.5H), 4.65 (d, J = 12.0 Hz, 0.5H), 3.92 (d, J = 13.2 Hz, 0.5H), 3.53 (q, J = 6.8 Hz, 2H), 3.40 (q, J = 15.2 Hz, 2H), 2.95-2.80 (m, 1 H), 2.72-2.61 (m, 1 H), 2.59-2.48 (m, 1H), 2.43-2.23 (m, 2H), 1.95-1.55 (m, 12H), 1.49-1.12 (m, 18H), 0.778 (m, 6H). | calcd. for C₃₅H₅₃FNO ₃ [M+H]⁺ 554, found 554 |
| | | | δ_{F} -116.27, -117.01 | |

### Example II-61: Synthesis of 1-((3R,5S,8S,9S,10S,13S,14S,17S)-3-(ethoxymethyl)-10-ethyl-3-hydroxy-13-methylhexadecahydro-1H-cyclopenta[a]phenanthren-17-yl)ethan-1-one (II-D9)

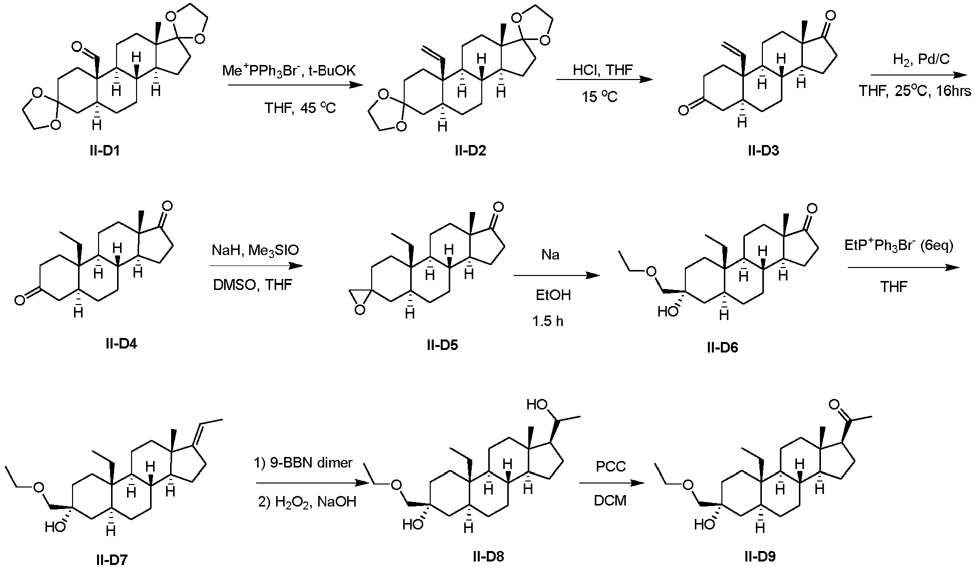

### Synthesis of II-D2

To a suspension of MePPh₃Br (50.3 g, 141 mmol) in THF (200 mL) was added t-BuOK (15.8 g, 141 mmol) at 15°C. After stirring at 45°C for 0.5 hour, a solution of **II-D1** (18.5 g, 47.3 mmol) in THF (100 mL) was added at 45°C and the reaction mixture was stirred at 45°C for 1 h. Saturated NH₄Cl solution (200 mL) was added to the mixture and the organic layer was separated. The aqueous layer was extracted with EtOAc (2 × 100 mL). The combined organic layers were washed with saturated brine (200 mL), filtered and concentrated under reduced pressure. The residue was purified by column chromatography (PE/EtOAc=10/1-4/1) to afford **II-D2** (13.6 g, 74.3%) as a solid.
**¹H NMR** (400 MHz, CDCl₃) δ_{H} 5.88 (dd, *J* = 10.8, 17.6 Hz, 1H), 5.35 (dd, *J* = 11.2 Hz, 1H), 5.02 (dd, *J* = 1.2, 18.0 Hz, 1H), 4.00-3.82 (m, 8H), 2.20-2.12 (m, 1H), 2.00-1.90 (m, 1H), 1.85-1.12 (m, 18H), 1.07-0.92 (m, 1H), 0.90-0.80 (m, 1H), 0.74 (s, 3H).

### Synthesis of II-D3

To a solution of **II-D2**(13.6 g, 35.0 mmol) in THF (80 mL) was added 12M HCl (29.1 mL, 350 mmol). The reaction mixture was stirred at 15°C for 16 hours t. The reaction mixture was diluted with H₂O (50 mL) and adjust to pH = 9 with solid Na₂CO₃ (20 g). The aqueous layer was extracted with EtOAc (3 × 200 mL). The combined organic layers were washed with saturated brine (200 mL), dried over anhydrous Na₂SO₄, filtered and concentrated to give the product (10.5 g). The product was purified by flash column (15∼20% of EtOAc in PE) to give **II-D3**(8.0 g, 76.9%) as a solid.

**¹H NMR** (400 MHz, CDCl₃) δ_{H} 6.03 (dd, *J* = 11.2, 18.0 Hz, 1H), 5.52 (d, *J* = 11.2 Hz, 1H), 5.20 (d, *J* = 18.0 Hz, 1H), 2.55-2.40 (m, 3H), 2.32-1.55 (m, 13H), 1.55-1.15 (m, 4H), 1.10-0.95 (m, 1H), 0.93-0.83 (m, 1H), 0.80 (s, 3H).

### Synthesis of II-D4

To a mixture of **II-D3** (8.0 g, 26.6 mmol) in THF (200 mL) was added Pd-C (wet, 50%, 2.0 g) under Ar. The suspension was degassed under vacuum and purged with H₂ for three times. 206843 Pa (30 psi) of hydrogen at 25°C for 16 hrs was applied to the resulting solution. The reaction mixture was filtered through a pad of Celite and washed with THF (2 × 200 mL). The filtrate was concentrated under reduced pressure. The product was purified by flash column (15∼20% of EtOAc in PE) to give **II-D4** (8.0 g, 99.5%) as a solid.
**¹H NMR** (400 MHz, CDCl₃) δ_{H} 2.55-2.25 (m, 5H), 2.15-1.15 (m, 17H), 1.10 (t, *J* = 8.0 Hz, 3H), 1.05-0.95 (m, 1H), 0.91 (s, 3H), 0.85-0.65 (m, 1H); **MS ESI** calcd. for C₂₀H₃₁O₂ [M+H]⁺ 303, found 303.

### Synthesis of II-D5

To a stirred solution of MesSIO (4.35 g, 19.8 mmol) in DMSO (30 mL) and THF (30 mL) was aded NaH (791 mg, 19.8 mmol, 60 % in oil) at 0°C in portions. The reaction mixture was stirred for 1 h under N₂. The mixture was added to a solution of **II-D4** (5.0 g, 16.5 mmol) in DMSO (30 mL). The reaction mixture was stirred at 15°C for 16 hrs. The reaction mixture was poured into ice-water (300 mL). The mixture was extracted with EtOAc (2 × 100 mL). The combined organic phase was washed with water (2 × 100 mL) and brine (100 mL), dried over anhydrous Na₂SO₄, filtered, and concentrated to give the product **II-D5** (5.0 g,) as an oil (yielding the equatorial methylene selectively).
**¹H NMR** (400 MHz, CDCl₃) δ_{H} 2.61 (s, 2H), 2.42 (dd, *J* = 8.8, 19.6 Hz, 1H), 2.15-1.40 (m, 11H), 1.35-1.05 (m, 8H), 1.02-0.70 (m, 10H).

### Synthesis of II-D6

To a fresh prepared solution of ethoxysodium (31.5 mL, 31.5 mmol, 1M in EtOH) was added **II-D5** (1.0 g, 3.15 mmol) in anhydrous ethanol (20 mL) at 75°C. The reaction mixture was stirred at 75°C for 16 hrs. Water (50 mL) was added. The reaction mixture was concentrated to remove most of the solvent. The mixture was extracted with EtOAc (2 × 30 mL). The combined organic phase was washed with saturated brine (50 mL), dried over anhydrous Na₂SO₄, filtered and concentrated. The residue was purified by silica gel chromatography (0-40% of EtOAc in PE) to give **II-D6** (512 mg, 44.9%) as an oil.

### II-D6:

**¹H NMR** (400 MHz, CDCl₃) δ_{H} 3.52 (q, *J* = 6.8 Hz, 2H), 3.21 (s, 2H), 2.42 (dd, *J* = 8.8, 19.6 Hz, 1H), 2.15-1.50 (m, 9H), 1.50-1.10 (m, 15H), 1.05-0.99 (m, 1H), 0.95 (t, *J* = 7.6 Hz, 3H), 0.90-0.65 (m, 4H); **MS ESI** calcd. for C₂₃H₃₈O₃Na [M+Na]⁺ 385, found 385.

### Synthesis of II-D7

To a suspension of bromo(ethyl)triphenylphosphorane (22.4 g, 60.4 mmol) in THF (100 mL) was added t-BuOK (6.76 g, 60.4 mmol) at 15°C under N₂. The mixture was heated to 45°C and stirred for 1 h. A solution **of II-D6** (5.5 g, 15.1 mmol) in THF (100 mL) was added at 45°C. The mixture was stirred at 45°C for 16 hrs. The mixture was treated with NH₄Cl (300 mL). The organic layer was separated. The aqueous phase was extracted with EtOAc (2 × 150 mL). The combined organic phase was washed with brine (2 × 100 mL), dried over anhydrous Na₂SO₄, filtered, concentrated in vacuum. The residue was purified by flash column (0-15% of EtOAc in PE) to give **II-D7** (5.0 g, 88%) as an oil.
**¹H NMR** (400 MHz, CDCl₃) δ_{H} 5.15-5.05 (m, 1H), 3.52 (q, *J* = 6.8 Hz, 2H), 3.21 (s, 2H), 2.40-2.10 (m, 4H), 1.85-1.75(m, 1H), 1.70-1.40 (m, 7H), 1.35-1.10 (m, 14H), 1.10-0.80 (m, 11H).

### Synthesis of II-D8

To a solution of **II-D7** (3.0 g, 8.0 mmol) in THF (50 mL) was added 9-BBN dimer (3.90 g, 16.0 mmol) at 15°C. The mixture was stirred at 15°C for 16 hrs. After cooling to 0°C, the mixture was added ethanol (3.68 g, 80.0 mmol) and NaOH (16 mL, 5 M, 80.0 mmol) very slowly. After the addition was complete, H₂O₂ (8.01 mL, 80.0 mmol, 30%) was added slowly and the inner temperature was maintained below 15°C. The resulting solution was stirred at 75°C for 1 hrs. The solution was workup and purified together with another batch done in similar manner. Saturated aqueous Na₂S₂O₃ (300 mL) was added and the mixture was stirred at 0°C for another 1 hour. The reaction was checked by potassium iodide-starch test paper to confirm excess H₂O₂ was destroyed. The mixture was cooled and added to water (200 mL). The mixture was extracted with EtOAc (3x100 mL), washed with saturated brine (200 mL), dried over anhydrous Na₂SO₄, filtered and concentrated under vacuum to give **II-D8** (5.0 g,) as an oil.
**¹H NMR** (400 MHz, CDCl₃) δ_{H} 3.75-3.65 (m, 1H), 3.51 (q, *J* = 6.8 Hz, 2H), 3.20 (s, 2H), 1.95-1.75 (m, 12H), 1.74-1.40 (m, 13H), 1.39-0.95 (m, 8H), 0.92 (t, *J* = 7.6 Hz, 3H), 0.66 (s, 3H).

### Synthesis of II-D9

To a solution of **II-D8** (0.8 g, 2.0 mmol) in DCM (20 mL) was added PCC (872 mg, 4.1 mmol) and silica gel (1 g) at 18°C. The solution was stirred at 18°C for 3h. The reaction mixture was filtered and the residue was washed with anhydrous DCM (2 × 50 mL). The combined filtrate was concentrated in vacuum. The residue was purified by flash column (0∼20% of EtOAc in PE) to give **II-D9** (0.75 g, 94.6%) as a solid.
**¹H NMR** (400 MHz, CDCl₃) δ_{H} 3.52 (q, *J* = 6.8 Hz, 2H), 3.21 (s, 2H), 2.52 (t, *J* = 8.8 Hz, 1H), 2.25-2.10 (m, 5H), 2.05-1.95 (m, 1H), 1.90-1.80 (m, 1H), 1.75-1.65 (m, 4H), 1.55-1.05 (m, 18H), 1.00-0.80 (m, 5H), 0.62 (s, 3H); **MS ESI** calcd. for C₂₅H₄₁O₂ [M-H₂O+H]⁺ 373, found 373.

### Example II-62: Synthesis of ((3R,5S,8S,9S,10S,13S,14S,17S)-3-(ethoxymethyl)-10-ethyl-3-hydroxy-13-methylhexadecahydro-1H-cyclopenta[a]phenanthren-17-yl)((S)-2-methylpiperidin-1-yl)methanone (II-D11)

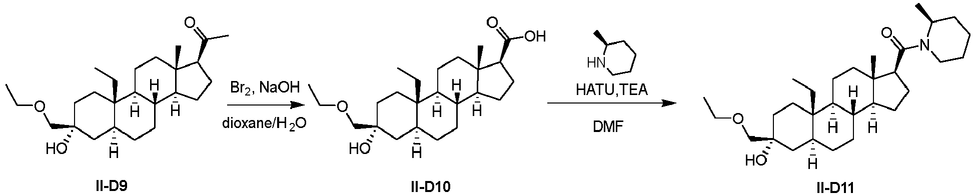

### Synthesis of II-D10

Liquid bromine (1.22 g, 7.7 mmol) was added slowly to a vigorously stirred aqueous sodium hydroxide (10.2 mL, 3 M, 30.7 mmol) at 0°C. When all the bromine was dissolved, the mixture was added slowly to a stirred solution of **II-D9** (1.0 g, 2.6 mmol) in dioxane (18 mL) and water (6 mL). The reaction mixture was stirred at 15°C for 16 hours. The remaining oxidizing reagent was quenched by aqueous Na₂SO₃ solution (30 mL). The solution with hydrochloride acid (3 N) furnished a precipitate. To the suspension was added EtOAc (50 mL). The organic phase was separated. The aqueous phase was extracted EtOAc (2 × 50 mL). The combined organic layers were washed with saturated brine (50 mL), dried over anhydrous Na₂SO₄, filtered and concentrated in vacuum to give **II-D10** (0.8 g, 80%) as a solid.
**¹H NMR** (400 MHz, CDCl₃) δ_{H} 3.71 (s, 1H), 3.52 (q, *J* = 6.8 Hz, 2H), 3.21 (s, 2H), 2.37 (t, *J* = 9.2 Hz, 1H), 2.15-2.00 (m, 3H), 1.90-1.70 (m, 2H), 1.60-1.10 (m, 20H), 1.00-0.65 (m, 5H), 0.74 (s, 3H).

### Synthesis of II-D11

To a solution of **II-D10** (200 mg, 0.5 mmol) in DMF (5 mL) were added HATU (382 mg, 1.0 mmol), TEA (256 mg, 2.5 mmol) and (2S)-2-methylpiperidine (100 mg, 1.0 mmol) at 20°C. The mixture was stirred at 20°C for 16 hrs to give brown solution. The reaction was poured into water (50 mL) and extracted with EtOAc (2 × 20 mL). The combined organic phase was washed with saturated brine (2 × 20 mL), dried over anhydrous Na₂SO₄, filtered and concentrated in vacuum. The residue was purified by flash column (10-20% of EtOAc in PE) to afford the product. The product was purified by prep-HPLC (Column: Agela DuraShell 150mm_25mm_5µm; Condition: water (10mM NH₄HCO₃)-ACN; Begin B: 83; End B: 99; Gradient Time (min): 8.5; 100%B Hold Time (min): 2) to afford **II-D11** (53.3 mg, 22%) as a solid.
**¹H NMR** (400 MHz, CDCl₃) δ_{H} 5.00-4.90 (m, 1H), 4.60-4.25 (m, 1H), 3.82 (d, *J* = 11.6 Hz, 1H), 3.52 (q, *J* = 6.8 Hz, 2H), 3.21 (s, 2H), 3.03 (t, *J* = 12.0 Hz, 1H), 2.63 (t, *J* = 8.8 Hz, 1H), 2.40-2.15 (m, 1H), 2.12 (s, 1H), 1.90-1.80 (m, 2H), 1.65-1.60 (m, 5H), 1.58-1.40 (m, 10H), 1.38-1.00 (m, 15H), 0.95-0.65 (m, 8H); **MS ESI** calcd. for C₃₀H₅₂NO₃ [M+H]⁺ 474, found 474.

The following example was made from **II-D10** as in Example II-62 replacing (2S)-2-methylpiperidine with the listed amine and an appropriate coupling agent (oxalic dichloride, EDCI, HATU, etc).

| **Exampl e #** | **amine** | **Name** | **¹H NMR (400 MHz, CDCl₃)** | **MS ESI** |
|---|---|---|---|---|
| II-63 | 4-amino-3-methylb enzonitri le | (3R,5S,8S,9S,10S,13 S,14S,17S)-N-(4-cyano-2-methylphenyl)-3-(ethoxymethyl)-10-ethyl-3-hydroxy-13-methylhexadecahydro -1H-cyclopenta[a]phenant hrene-17-carboxamide (**II-63**) | δ_{H} 8.33 (d, *J* = 8.4 Hz, 1H), 7.49 (d, *J* = 8.4 Hz, 1H), 7.45 (s, 1H), 6.98 (s, 1H), 3.52 (q, *J* = 6.8 Hz, 2H), 3.21 (s, 2H), 2.40-2.20 (m, 5H), 2.15 (s, 1H), 2.05-1.95 (m, 1H), 1.90-1.65 (m, 5H), 1.60-1.10 (m, 18H), 1.00-0.80 (m, 5H), 0.76 (s, 3H). | calcd. for C₃₂H₄₇N₂O₃ [M+H]⁺ 507, found 507 |

### Example II-70: Synthesis of 1-((3R,5S,8S,9S,10S,13S,14S,17S)-10-ethyl-3-hydroxy-3-(methoxymethyl)-13-methylhexadecahydro-1H-cyclopenta[a]phenanthren-17-yl)ethan-1-one (II-E4)

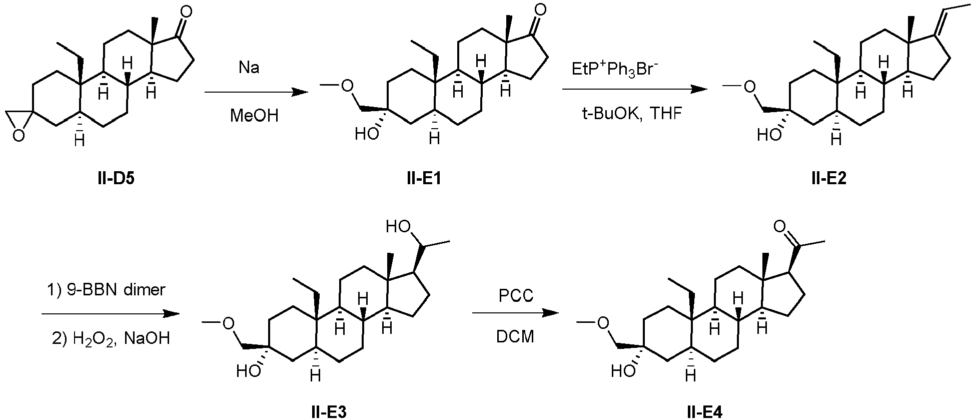

### Synthesis of II-E1

Fresh Na (1.45 g, 63 mmol) was carefully added to MeOH (63 mL) in portions at 0°C. After stirring at 20°C for 3h, Na was dissolved completely. To a solution of MeONa (63 mL, 63.0 mmol, 1M in MeOH) was added **II-D5** (2.0 g, 6.3 mmol) in anhydrous MeOH (40 mL) at 65°C. The reaction mixture was stirred at 65°C for 2 hrs. Water (100 mL) was added to the above mixture. The reaction mixture was concentrated to remove most of the solvent. The aqueous layer was extracted with EtOAc (2 × 100 mL). The combined organic phase was washed with saturated brine (100 mL), dried over anhydrous Na₂SO₄, filtered and concentrated under reduced pressure. The residue was purified by flash column (15-20% EtOAc in PE) to afford **II-E1** (0.8 g, 37%) as an oil and **II-E1a.**

### II-E1:

**¹H NMR** (400 MHz, CDCl₃) δ_{H} 3.39 (s, 3H), 3.18 (s, 2H), 2.43 (dd, *J* = 8.4, 19.2 Hz, 1H), 2.15-1.86 (m, 4H), 1.85-1.75 (m, 3H), 1.75-1.45 (m, 6H), 1.44-1.13 (m, 10H), 1.12-0.93 (m, 4H), 0.92-0.82 (m, 3H).

### Synthesis of II-E2

To a suspension of bromo(ethyl)triphenylphosphorane (3.4 g, 9.2 mmol) in THF (10 mL) was added t-BuOK (1.0 g, 9.2 mmol) at 15°C under N₂. The mixture was heated to 60°C and stirred for 1h. A solution of **II-E1** (0.8 g, 2.3 mmol) in THF (5 mL) was added at 60°C. The mixture was stirred at 60°C for 16 hrs. The mixture was treated with NH₄Cl (20 mL). The organic layer was separated. The aqueous phase was extracted with EtOAc (3 × 20 mL). The combined organic phase was washed with brine (20 mL), dried over anhydrous Na₂SO₄, filtered and concentrated under reduced pressure. The residue was purified by flash column (0-15% of EtOAc in PE) to give **II-E2** (630 mg, 76%) as an oil.

**¹H NMR** (400 MHz, CDCl₃) δ_{H} 5.12 (q, *J* = 6.8 Hz, 1H), 3.38 (s, 3H), 3.18 (s, 2H), 2.45-2.12 (m, 3H), 1.88 (d, *J* = 12.8 Hz, 1H), 1.77-1.40 (m, 12H), 1.39-1.08 (s, 11H), 0.95 (t, *J* = 7.6 Hz, 3H), 0.93-0.80 (m, 4H).

### Synthesis of II-E3

To a solution **of II-E2** (630 mg, 1.7 mmol) in THF (10 mL) was added 9-BBN dimer (849 mg, 3.5 mmol) at 15°C. The mixture was stirred at 15°C for 16 hrs. After cooling to 0°C, ethanol (800 mg, 17.4 mmol) and 5M NaOH (3.47 mL, 17.4 mmol) were added slowly to the mixture. After the addition was complete, H₂O₂ (1.74 mL, 17.4 mmol, 30%) was added slowly and the inner temperature was maintained below 15°C. The resulting solution was stirred at 75°C for 1 hrs. The aqueous layer was extracted with EtOAc (3 × 20 mL). The combined organic layer was washed with saturated brine (20 mL), dried over anhydrous Na₂SO₄, filtered and concentrated under reduced pressure. The residue was purified by flash column (30%~50% EtOAc in PE) to afford **II-E3** (540 mg, 82%) as a solid.
**¹H NMR** (400 MHz, CDCl₃) δ_{H} 3.77-3.15 (m, 1H), 3.38 (s, 3H), 3.18 (s, 2H), 1.95-1.80 (m, 3H), 1.70-1.40 (m, 11H), 1.40-1.00 (m, 15H), 0.98-0.90 (m, 3H), 0.89-0.77 (m, 1H), 0.76 (s, 1H), 0.64 (s, 2H).

### Synthesis of II-E4

To a solution **of II-E3** (0.54 g, 1.4 mmol) in DCM (10 mL) was added PCC (610 mg, 2.8 mmol) and silica gel (1 g) at 18°C. The solution was stirred at 18°C for 1h. The reaction mixture was filtered and the residue was washed with DCM (2 × 50 mL). The combined filtrate was concentrated under reduced pressure to afford **II-E4** (534 mg) as an oil. Purification by chromatography yield pure **II-E4**.
**¹H NMR** (400 MHz, CDCl₃) δ_{H} 3.38 (s, 3H), 3.18 (s, 2H), 2.52 (t, *J* = 8.4 Hz, 1H), 2.20-2.08 (m, 4H), 2.05-1.95 (m, 2H), 1.93-1.83 (m, 1H), 1.77-1.55 (m, 4H), 1.54-1.08 (m, 15H), 1.05-0.82 (m, 5H), 0.62 (s, 3H); **MS ESI** calcd. for C₂₄H₄₀O₃Na [M+Na]⁺ 399, found 399.

### Example II-71: Synthesis of (3R,5S,8S,9S,10S,13S,14S,17S)-N-(4-cyano-2-methylphenyl)-10-ethyl-3-hydroxy-3-(methoxymethyl)-13-methylhexadecahydro-1H-cyclopenta[a]phenanthrene-17-carboxamide (II-E6)

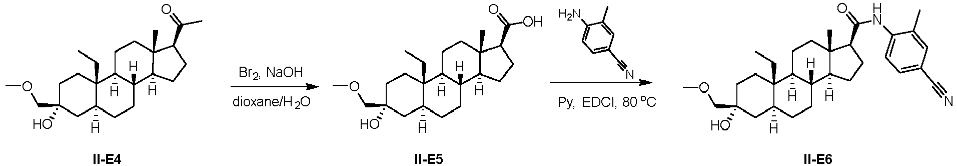

### Synthesis of II-E5

Liquid bromine (127 mg, 0.8 mmol) was added slowly to a vigorously stirred aqueous sodium hydroxide solution (1.05 mL, 3M, 3.2 mmol) at 0°C. When all the bromine was dissolved, the mixture was added slowly to a stirred solution of **II-E4** (100 mg, 0.3 mmol) in dioxane (6 mL) and water (2 mL). The reaction mixture was stirred at 15°C for 16 hours. The remaining oxidizing reagent was quenched by aqueous Na₂SO₃ solution (3 mL), and the solution with hydrochloride acid (5 mL, 3 N) furnished a precipitate. To the suspension was added EtOAc (10 mL). The organic phase was separated. The aqueous phase was extracted EtOAc (2 × 10 mL). The combined organic phase was washed with saturated brine (30 mL), dried over anhydrous Na₂SO₄, filtered and concentrated in vacuum to give **II-E5** (120 mg,) as a solid.
**¹H NMR** (400 MHz, CDCl₃) δ_{H} 3.38 (s, 3H), 3.18 (s, 2H), 2.37 (t, *J* = 9.2Hz, 1H), 2.12-1.99 (m, 2H), 1.93-1.64 (m, 5H), 1.62-1.40 (m, 5H), 1.36-1.09 (m, 11H), 1.07-0.79 (m, 6H), 0.73 (s, 3H).

### Synthesis of II-E6

To a solution of **II-E5** (110 mg, 0.3 mmol) in pyridine (5 mL) was added EDCI (167 mg, 0.9 mmol) and stirred for 10 mins. 4-amino-3-methylbenzonitrile (76.7 mg, 0.6 mmol) was added. The mixture was stirred at 80°C for 16 hrs to give a brown solution. The reaction mixture was poured into water (50 mL) and extracted with EtOAc (2 × 20 mL). The combined organic phase was washed with saturated brine (2 × 20 mL), dried over anhydrous Na₂SO₄, filtered and concentrated in vacuum. The residue was purified by silica gel chromatography (0-50% of EtOAc in PE) to give the product **II-E6** (51.1 mg, 36%) as a solid.
**¹H NMR** (400 MHz, CDCl₃) δ_{H} 8.35 (d, *J* = 8.8 Hz, 1H), 7.53-7.48 (m, 1H), 7.45 (s, 1H), 6.95 (s, 1H), 3.39 (s, 3H), 3.19 (s, 2H), 2.36-2.23 (m, 5H), 2.06-1.99 (m, 2H), 1.91-1.67 (m, 5H), 1.65-1.56 (m, 2H), 1.53-1.41 (m, 3H), 1.36-1.13 (m, 10H), 1.02-0.85 (m, 5H), 0.76 (s, 3H); **MS ESI** calcd. for C₃₁H₄₅N₂O₃ [M+H] ⁺ 493, found 493.

### Example II-72: Synthesis of 1-(2-((3R,SS,8S,9S,lOS,13S,14S,17S)-10-ethyl-3-hydroxy-3-(methoxymethyl)-13-methylhexadecahydro-1H-cyclopenta[a]phenanthren-17-yl)-2-oxoethyl)-1H-pyrazole-3-carbonitrile (II-E8)

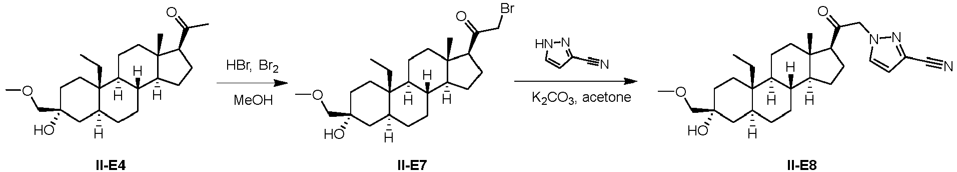

### Synthesis of II-E7

To a solution of **II-E4** (100 mg, 0.27 mmol) in MeOH (5 mL) was added HBr (10.7 mg, 0.05 mmol, 40%) and Br₂ (46.6 mg, 0.3 mmol) at 15°C. The reaction mixture was stirred at 15°C for 1 hours. Saturated NaHCO₃ (10 mL) and saturated Na₂SO₃ solution (10 mL) were added to the mixture. The aqueous layer was extracted with EtOAc (3 × 10 mL). The combined organic layers were washed with saturated brine (10 mL), dried over anhydrous Na₂SO₄, filtered and concentrated to give **II-E7** (120 mg,) as an oil.

### Synthesis of II-E8

To a solution of **II-E7** (120 mg, 0.26 mmol) in acetone (3 mL) were added K₂ CO₃ (54.6 mg, 0.4 mmol) and 1H-pyrazole-4-carbonitrile (36.7 mg, 0.4 mmol) at 15°C. The reaction mixture was stirred at 15°C for 16 hours. The reaction mixture was concentrated and purified by flash column (20-40% of EtOAc in PE) to afford **II-E8** (46 mg, 39%) as a solid.
**¹H NMR** (400 MHz, CDCl₃) δ_{H} 7.46 (d, *J* = 2.4 Hz, 1H), 6.70 (d, *J* = 2.0 Hz, 1H), 4.96 (dd, *J* = 18.0, 42.8 Hz, 2H), 3.37 (s, 3H), 3.17 (s, 2H), 2.58 (t, *J* = 8.8 Hz, 1H), 2.24-2.00 (m, 3H), 1.86 (d, *J* = 12.4 Hz, 1H), 1.80-1.43 (m, 9H), 1.42-1.10 (m, 10H), 1.05-0.85 (m, 5H), 0.66 (s, 3H); **MS ESI** calcd. for C₂₈H₄₁N₃O₃Na [M+Na]⁺ 490, found 490.

### Example II-73: Synthesis of 1-(2-((3R,5S,8S,9S,10S,13S,14S,17S)-3-(ethoxymethyl)-10-ethyl-3-hydroxy-13-methylhexadecahydro-1H-cyclopenta[a]phenanthren-17-yl)-2-oxoethyl)-1H-pyrazole-3-carbonitrile (II-D13)

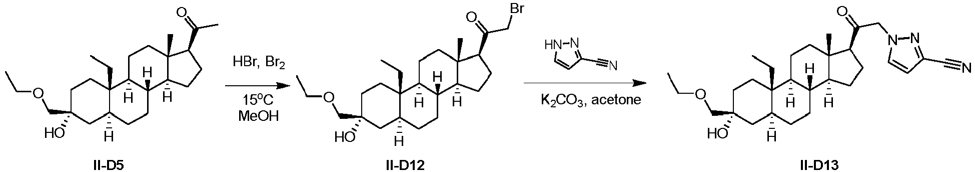

### Synthesis of II-D12

To a solution of **II-D5** (150 mg, 0.38 mmol) in MeOH (5 mL) was added HBr (15.3 mg, 0.077 mmol, 40%) and Br₂ (67.5 mg, 0.42 mmol) at 15°C. The reaction mixture was stirred for 16 hours at 15°C. The reaction mixture was added into saturated NaHCO₃ (20 mL), then extracted with EtOAc (3 × 20 mL). The combined organic layer was washed with saturated brine (50 mL), dried over Na₂SO₄, filtered and concentrated to give **II-D12** (165 mg,) as an oil.
**¹H NMR** (400 MHz, CDCl₃) δ_{H} 4.00-3.85 (m, 2H), 3.52 (q, *J* = 6.8 Hz, 2H), 3.21 (s, 2H), 2.80 (t, *J* = 9.2 Hz, 1H), 2.25-2.10 (m, 1H), 1.90-1.80 (m, 2H), 1.75-1.40 (m, 10H), 1.40-1.10 (m, 12H), 1.00-0.80 (m, 6H), 0.65 (s, 3H).

### Synthesis of II-D13

To a solution of **II-D12** (165 mg, 0.4 mmol) in acetone (5 mL) was added K₂CO₃ (96.9 mg, 0.7 mmol) and 1H-pyrazole-4-carbonitrile (65.4 mg, 0.7 mmol) at 15°C. The reaction mixture was stirred for 16 hours at 15°C. The reaction mixture was added into saturated NH₄Cl (20 mL). The aqueous layer was extracted with EtOAc (3 × 20 mL). The combined organic layer was washed with saturated brine (50 mL), dried over anhydrous Na₂SO₄, filtered and concentrated to give product. The residue was purified by combi flash (0-20% of EtOAc in PE) to give **II-D13** (74.6 mg, 44.1%) as a solid.
**¹H NMR** (400 MHz, CDCl₃) δ_{H} 7.47 (d, *J* = 2.4 Hz, 1H), 6.72 (d, *J* = 2.4 Hz, 1H), 4.96 (dd, *J* = 17.6, 51.2 Hz, 2H), 3.52 (q, *J* = 6.8 Hz, 2H), 3.21 (s, 2H), 2.59 (t, *J* = 8.8, 1H), 2.25-2.10 (m, 2H), 1.90-1.60 (m, 6H), 1.55-1.10 (m, 18H), 1.05-0.80 (m, 5H), 0.68 (s, 3H); **MS ESI** calcd. for C₂₉H₄₂N₃O₂ [M-H₂O+H]⁺ 464, found 464.

### Example II-74: Synthesis of 1-(2-((3R,5R,8S,9S,10S,13S,14S,17S)-3-(ethoxymethyl)-10-ethyl-3-hydroxy-13-methylhexadecahydro-1H-cyclopenta[a]phenanthren-17-yl)-2-oxoethyl)-1H-pyrazole-4-carbonitrile (II-C8)

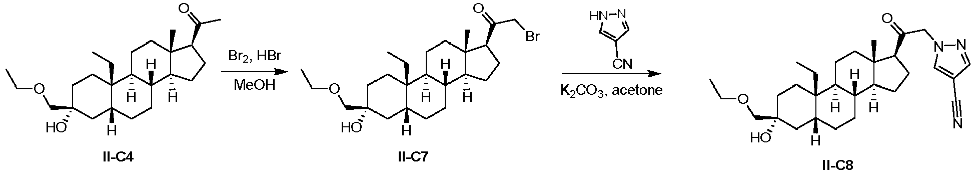

### Synthesis of II-C7

To a solution of **II-C4** (500 mg, 1.3 mmol) in MeOH (10 mL) was added HBr (10 mg, 40%) and Br₂ (224 mg, 1.4 mmol) at 15°C. The reaction mixture was stirred for 16 hours at 15°C. The reaction mixture was added into saturated NaHCO₃ (50 mL). The aqueous layer was extracted with EtOAc (3 × 50 mL). The combined organic layer was washed with saturated brine (100 mL), dried over anhydrous Na₂SO₄, filtered and concentrated to give **II-C7** (220 mg,) as an oil.
**¹H NMR** (400 MHz, CDCl₃) δ_{H} 3.95-3.88 (m, 2H), 3.54 (q, *J* = 6.8 Hz, 2H), 3.41 (q, *J* = 11.2 Hz, 2H), 2.85-2.75 (m, 1H), 2.74-2.60 (m, 1H), 2.25-2.10 (m, 1H), 1.95-1.81 (m, 2H), 1.78-1.59 (m, 9H), 1.38-1.10 (m, 13H), 0.98-0.78 (m, 2H), 0.78 (t, *J* = 6.8 Hz, 3H), 0.62 (s, 3H).

### Synthesis of II-C8

To a solution of **II-C7** (70 mg, 0.15 mmol) in acetone (5 mL) were added K₂CO₃ (41 mg, 0.3 mmol) and 1H-pyrazole-4-carbonitrile (28 mg, 0.3 mmol) at 15°C. The reaction mixture was stirred for 16 hours at 15°C. The reaction mixture was added into saturated NH₄Cl (50 mL), and the aqueous layer was extracted with EtOAc (3 × 50 mL). The combined organic layer was washed with saturated brine (100 mL), dried over anhydrous Na₂SO₄, filtered and concentrated to give product. The residue was purified by combi flash (0-20% of EtOAc in PE) to give **II-C8** (45 mg, 63%) as a solid.
**¹H NMR** (400 MHz, CDCl₃) δ_{H} 7.85 (s, 1H), 7.81 (s, 1H), 5.02 (d, *J* = 18.0 Hz, 2H), 4.89 (d, *J* = 18.0 Hz, 2H), 3.54 (q, *J* = 6.8 Hz, 2H), 3.41 (q, *J* = 9.2 Hz, 2H), 2.75 (s, 1H), 2.63-2.53 (m, 1H), 2.25-2.10 (m, 1H), 2.09-1.85 (m, 2H), 1.80-1.58 (m, 7H), 1.51-1.25 (m, 11H), 1.24-1.10 (m, 6H), 0.78 (t, *J* = 7.2 Hz, 3H), 0.64 (s, 3H); **MS ESI** calcd. for C₂₉H₄₂N₃O₂ [M+H-H₂O]⁺ 464, found 464.

### Examples II-75 to II-79

Examples II-75-II-76 were made from α-halo ketone (**II-C7)**, examples II-77-II-70 were made from α-halo ketone **II-D12**) as in **Examples II-72 to II-74** replacing 1H-pyrazole-4-carbonitrile with the listed nucleophile.

| **Exa mple #** | **nucleophile** | **Name** | **¹H NMR (400 MHz, CDCl₃)** | **MS ESI** |
|---|---|---|---|---|
| II-75 | 1,2,3-triazole | 1-((3R,5R,8S,9S,1 0S,13S,14S,17S) -3-(ethoxymethyl)-10-ethyl-3-hydroxy-13-methylhexadeca | δ_{H} 7.68 (s, 2H), 5.23 (s, 2H), 3.54 (q, *J* = 6.8 Hz, 2H), 3.41 (q, *J* = 9.2 Hz, 2H), 2.73 (s, 1H), 2.56 (t, *J* = 9.6 Hz, 1H), 2.24-2.15 (m, 1H), 2.09-2.05 (m, 1H), 1.97- | calcd. for C₂₇H₄₄ N₃O₃ [M+H]⁺ 458, found 458 |
| | | hydro-1H-cyclopenta[a]ph enanthren-17-yl)-2-(2H-1,2,3-triazol-2-yl)ethan-1-one (**II-75**) | 1.88 (m, 2H), 1.78-1.69 (m, 5H), 1.56-1.53 (m, 5H), 1.41-1.38 (m, 3H), 1.23-1.20 (m, 6H), 0.89-0.86 (m, 4H), 0.78 (t, *J* = 7.6 Hz, 3H), 0.68 (s, 3H). | |
| II-76 | 1,2,3-triazole | 1-((3R,5R,8S,9S,1 0S,13S,14S,17S) -3-(ethoxymethyl)-10-ethyl-3-hydroxy-13-methylhexadeca hydro-1H-cyclopenta[a]ph enanthren-17-yl)-2-(1H-1,2,3-triazol-1-yl)ethan-1-one (**II-76**) | δ_{H} 7.76 (s, 1H), 7.64 (s, 1H), 5.27 (d, *J* = 18.0 Hz, 1H), 5.12 (d, *J* = 18.0 Hz, 1H), 3.54 (q, *J* = 6.8 Hz, 2H), 3.40 (q, *J* = 9.2 Hz, 2H), 2.74 (s, 1H), 2.64 (t, *J* = 6.6 Hz, 1H), 2.26-2.17 (m, 1H), 2.11-2.04 (m, 1H), 1.94 (t, *J* = 13.2, 1H), 1.78-1.71 (m, 3H), 1.66 - 1.61 (m, 3H), 1.57-1.48 (m, 6H), 1.43- 1.38 (m, 3H), 1.32-1.27 (m, 3H), 1.24-1.20 (m, 5H), 0.94-0.85 (m, 1H), 0.78 (t, *J* = 11.6 Hz, 3H), 0.65 (s, 3H). | calcd. for C₂₇H₄₄ N₃O₃ [M+H]⁺ 458, found 458 |
| II-77 | 5-methyl-2H-tetrazole | 1-((3R,5R,8S,9S,1 0S,13S,14S,17S) -3-(ethoxymethyl)-10-ethyl-3-hydroxy-13-methylhexadeca hydro-1H-cyclopenta[a]ph enanthren-17-yl)-2-(5-methyl-2H-tetrazol-2-yl)ethan-1-one (**II-77**) | δ_{H} 5.35-5.25 (m, 2H), 3.53 (q, *J* = 6.8 Hz, 2H), 3.21 (s, 2H), 2.65 (t, *J* = 8.8 Hz, 1H), 2.56 (s, 3H), 2.25-2.05 (m, 3H), 1.90-1.65 (m, 6H), 1.60-1.10 (m, 17H), 1.05-0.85 (m, 5H), 0.72 (s, 3H). | calcd. for C₂₇H₄₅ N₄O₃ [M+H]⁺ 473, found 473 |
| II-78 | 5-methyl-2H-tetrazole | 1-((3R,5S,8S,9S,1 0S,13S,14S,17S) -3-(ethoxymethyl)-10-ethyl-3-hydroxy-13-methylhexadeca hydro-1H-cyclopenta[a]ph enanthren-17-yl)-2-(5-methyl-1H-tetrazol-1-yl)ethan-1-one (**II-78**) | δ_{H} 5.09 (dd, *J* = 18.4, 36.4 Hz, 2H), 3.53 (q, *J* = 6.8 Hz, 2H), 3.22 (s, 2H), 2.70-2.60 (m, 3H), 2.48 (s, 3H), 2.25-1.40 (m, 10H), 1.35-1.10 (m, 13H), 1.05-0.65 (m, 5H), 0.70 (s, 3H) | calcd. for C₂₇H₄₅ N₄O₃ [M+H]⁺ 473, found 473 |
| II-79 | 1H-pyrazole-3-carbonitrile | 1-(2-((3R,5R,8S,9S,1 0S,13S,14S,17S) -3-(ethoxymethyl)-10-ethyl-3-hydroxy-13-methylhexadeca hydro-1H-cyclopenta[a]ph enanthren-17-yl)-2-oxoethyl)-1H-pyrazole-3-carbonitrile (**II-79**) | δ_{H} 7.48 (d, *J* = 2.4 Hz, 1H), 6.73 (d, *J* = 2.4 Hz, 1H), 5.03 (d, *J* = 17.6 Hz, 1H), 4.91 (d, *J* = 17.6 Hz, 1H), 3.54 (q, *J* = 6.8 Hz, 2H), 3.41 (q, *J* = 9.6 Hz, 2H), 2.75 (s, 1H), 2.63-2.53 (m, 1H), 2.25-2.10 (m, 1H), 2.09-2.03 (m, 1H), 2.00-1.85 (m, 1H), 1.80-1.40 (m, 11H), 1.39-1.10 (m, 13H), 0.78 (t, *J* = 7.2 Hz, 3H), 0.65 (s, 3H) | calcd. for C₂₉H₄₂ N₃O₂ [M+H-H₂O]⁺ 464, found 464 |

### EXAMPLE II-80: Synthesis of 1-((3R,5R,8S,9S,10R,13S,14S,17S)-3-hydroxy-10-((R)-1-methoxyethyl)-3,13-dimethylhexadecahydro-1H-cyclopenta[a]phenanthren-17-yl)ethan-1-one (II-E10)

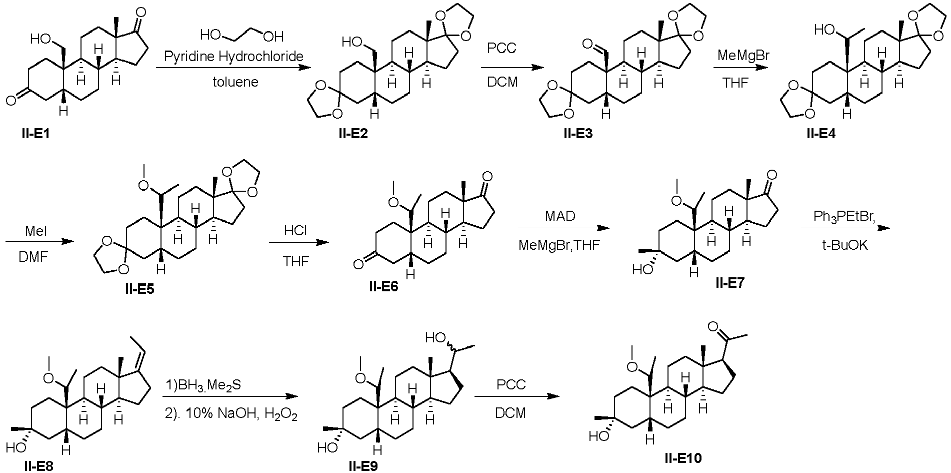

### Synthesis of II-E2

To a solution of **II-E2** (6 g, 19.7 mmol) in toluene (100 mL) were added pyridine hydrochloride (453 mg, 3.94 mmol) and ethane-1,2-diol (6.1 g, 98.4 mmol). After stirring at 130°C for 16 h, the mixture was concentrated in vacuum. The residue was purified by silica gel column eluted with (PE/EtOAc = 5/1) to afford **II-E2** (5.4 g, 66%) as a solid. **¹H NMR** (400 MHz, CDCl₃) δ 3.98-3.80 (m, 9H), 3.58-3.55 (m, 1H), 2.10-2.01 (m, 1H), 1.99-1.91 (m, 2H), 1.84-1.60 (m, 5H), 1.59-1.33 (m, 10H), 1.30-1.17 (m, 4H), 1.15-1.02 (m, 1H), 0.82 (s, 3H).

### Synthesis of II-E3

To a solution of **II-E2** (5.3 g, 13.5 mmol) in DCM (60 mL) was added PCC (4.34 g, 20.2 mmol) at 25°C. After stirring at 25°C for 30 min, the solution was filtered, and the filtered cake was washed with DCM (2 × 100 mL). The combined filtrate was washed with saturated NaHCO₃ (100 mL) and brine (100 mL), dried over Na₂SO₄, filtered and concentrated in vacuum. The residue was purified by silica gel column eluted with (PE/EtOAc = 8/1) to afford **II-E3** (3.1 g, 53%) as an oil. **¹H NMR** (400 MHz, CDCl₃) δ 9.58 (s, 1H), 4.00-3.84 (m, 8H), 2.25-2.15 (m, 1H), 2.03-1.88 (m, 2H), 1.84-1.59 (m, 7H), 1.56-1.32 (m, 10H), 1.30-1.17 (m, 1H), 1.13-0.99 (m, 1H), 0.92 (s, 3H).

### Synthesis of II-E4

To a solution of **II-E4** (3 g, 7.68 mmol) in THF (50 mL) was added MeMgBr (5.1 mL, 15.3 mmol, 3M in ethyl ether) at 0°C. After stirring at 25°C for 2 h, the reaction was poured into water (100 mL) and extracted with EtOAc (2 × 100 mL). The combined organic solution was washed with brine (100 mL), dried over Na₂SO₄, filtered and concentrated in vacuum to afford **II-E4** (2.8 g, 81%) as an oil.
**¹H NMR** (400 MHz, CDCl₃) δ 4.42 (m, 1H), 3.97-3.81 (m, 8H), 2.03-1.92 (m, 2H), 1.90-1.74 (m, 5H), 1.71-1.64 (m, 1H), 1.60-1.34 (m, 9H), 1.30-1.22 (m, 4H), 1.21-1.11 (m, 5H), 0.87 (s, 3H).

### Synthesis of II-E5

To a solution of **II-E4** (2.7 g, 6.64 mmol) in DMF (20 mL) was added NaH (795 mg, 19.9 mmol, 60%) at 25°C. After stirring at 50°C for 30 min, MeI (2.82 g, 19.9 mmol) was added dropwise to the reaction. After stirring at 50°C for 2 h, additional MeI (2.82 g, 19.9 mmol) was added dropwise. After stirring at 50°C for 1 h, the mixture was poured into ice water (50 mL) and extracted with EtOAc (2 × 60 mL). The combined organic solution was washed with saturated brine (100 mL), dried with anhydrous Na₂SO₄, filtered and concentrated in vacuum. The residue was purified by column chromatography on silica gel (PE/EtOAc = 10/1) to afford **II-E5** (2 g, 64%) as a solid. **¹H NMR** (400 MHz, CDCl₃) δ 3.97-3.83 (m, 8H), 3.79-3.74 (m, 1H), 3.29 (s, 3H), 2.08-1.93 (m, 2H), 1.91-1.75 (m, 3H), 1.73-1.57 (m, 3H), 1.54-1.36 (m, 8H), 1.31-1.05 (m, 9H), 0.88 (s, 3H).

### Synthesis of II-E6

To a solution of **II-E5** (2 g, 4.75 mmol) in THF (30 mL) was added aq. HCl (4.75 mL, 4M, 19 mmol). After stirring at 25°C for 16 h, the mixture was poured into water (100 mL) and extracted with EtOAc (2 × 50 mL). The combined organic solution was washed with saturated aqueous NaHCO₃ (50 mL), brine (50 mL), dried over Na₂SO₄ and concentrated in vacuum to afford **II-E6** (1.4 g, 80%) as a solid. **¹H NMR** (400 MHz, CDCl₃) δ 3.86-3.82 (m, 1H), 3.31 (s, 3H), 2.77-2.67 (m, 1H), 2.54-2.43 (m, 1H), 2.32-2.19 (m, 3H), 2.16-1.94 (m, 4H), 1.91-1.78 (m, 3H), 1.75-1.60 (m, 4H), 1.55-1.35 (m, 2H), 1.30-1.16 (m, 4H), 1.10-1.05 (m, 3H), 0.93 (s, 3H).

### Synthesis of II-E7

To a solution of BHT (5.56 g, 25.2 mmol) in toluene (50 mL) was added dropwise AlMe₃ (6.3 mL, 12.6 mmol, 2 M in toluene) at 0°C. After stirring at 25°C for 1 h, a solution of **II-E6** (1.4 g, 4.21 mmol) in toluene (20 mL) was added dropwise to the mixture at -65°C. After stirring at -65°C for 1 h, MeMgBr (4.19 mL, 12.6 mmol, 3M in ethyl ether) was added dropwise at -65°C. After stirring at -65°C for 3 h, the reaction was quenched by saturated aqueous NH₄Cl (50 mL) at -65°C. After stirring at 25°C for 0.5 h, the resulting mixture was filtered through a celite pad and the pad was washed with EtOAc (100 mL). The combined organic solution was separated, washed with brine (2 × 100 mL), dried over Na₂SO₄, filtered and concentrated in vacuum. The residue was purified by silica gel column eluted with PE/EtOAc = 5/1 to give **II-E7** (1.1 g, 68%) as a solid. **¹H NMR** (400 MHz, CDCl₃) δ 3.80-3.75 (m, 1H), 3.29 (s, 3H), 2.48-2.41 (m, 1H), 2.15-2.02 (m, 1H), 2.00-1.77 (m, 4H), 1.74-1.63 (m, 4H), 1.57-1.33 (m, 7H), 1.29-1.13 (m, 9H), 1.08 (d, *J* = 6.4 Hz, 3H), 0.89 (s, 3H); **LC-ELSD/MS** purity 99%, MS ESI calcd. For C₂₁H₃₁O [M+H-H₂O-MeOH] ⁺ 299, found 299.

### Synthesis of II-E8

To a solution of PPh₃EtBr (3.18 g, 8.58 mmol) in THF (15 mL) was added t-BuOK (962 mg, 8.58 mmol) at 25°C. After stirring at 60°C for 1 h, a solution of **II-E7** (1 g, 2.86 mmol) in THF (5 mL) was added dropwise. After stirring at 60°C for 16 h, the mixture was poured into ice-water (100 mL) and extracted with EtOAc (2 × 50 mL). The combined organic solution was washed with brine (50 mL), dried over Na₂SO₄ and filtered, concentrated in vacuum. The residue was purified by silica gel column eluted with PE/EtOAc = 15/1 to afford **II-E8** (1 g, 78%) as a solid. **¹H NMR** (400 MHz, CDCl₃) δ 5.18-4.99 (m, 1H), 3.83-3.71 (m, 1H), 3.30 (s, 3H), 2.45-2.09 (m, 3H), 2.01-1.81 (m, 3H), 1.68-1.58 (m, 6H), 1.58-1.37 (m, 7H), 1.31-1.12 (m, 10H), 1.08-1.03 (m, 3H), 0.91 (s, 3H).

### Synthesis of II-E9

To a solution of **II-E8** (1 g, 2.77 mmol) in THF (20 mL) was added dropwise a solution of BH₃-Me₂S (2.77 mL, 27.7 mmol) at 0°C. After stirring at 25°C for 16 h, the solution was cooled to 0°C and NaOH (9.23 mL, 3M) was added very slowly. After the addition was complete, H₂O₂ (4.5 mL, 33%) was added slowly and the inner temperature was maintained below 10°C. After stirring at 25°C for 2 h, the resulting solution was extract with EtOAc (2 × 20 mL). The combined organic solution was washed with saturated aqueous Na₂S₂O₃ (2 × 50 mL), brine (50 mL), dried over Na₂SO₄, filtered and concentrated in vacuum to give **II-E9** (0.9 g) as a solid, which used for the next step without further purification.

### Synthesis of II-E10

To a solution of **II-E9** (800 mg, 2.11 mmol) in DCM (10 mL) was added PCC (907 mg, 4.22 mol) at 25°C. After stirring at 25°C for 2 h, the solution was filtered and the filter cake was washed with DCM (2 × 50 mL). The combined filtrate was concentrated in vacuum. The residue was purified by silica gel column eluted with (PE/EtOAc = 5/1) to afford **II-E10** (600 mg, 68%) as a solid. **¹H NMR** (400 MHz, CDCl₃) δ 3.78-3.74 (m, 1H), 3.27 (s, 3H), 2.56-2.51 (m, 1H), 2.22-2.16 (m, 1H), 2.12 (s, 3H), 2.06-1.94 (m, 2H), 1.91-1.79 (m, 1H), 1.74-1.61 (m, 6H), 1.50-1.32 (m, 6H), 1.29-1.10 (m, 10H), 1.06 (d, *J* = 6.0 Hz, 3H), 0.64 (s, 3H); **LC-ELSD/MS** purity 99%, MS ESI calcd. For C₂₃H₃₅O [M+H-H₂O-MeOH] ⁺ 327, found 327.

### EXAMPLE II-81: Synthesis of 1-((3R,5R,8S,9S,10R,13S,14S,17S)-3-hydroxy-10-((R)-1-methoxyethyl)-3,13-dimethylhexadecahydro-1H-cyclopenta[a]phenanthren-17-yl)-2-(5-methyl-2H-tetrazol-2-yl)ethan-1-one (II-E12) & 1-((3R,5R,8S,9S,10R,13S,14S,17S)-3-hydroxy-10-((R)-1-methoxyethyl)-3,13-dimethylhexadecahydro-1H-cyclopenta[a]phenanthren-17-yl)-2-(5-methyl-1H-tetrazol-1-yl)ethan-1-one (II-E13)

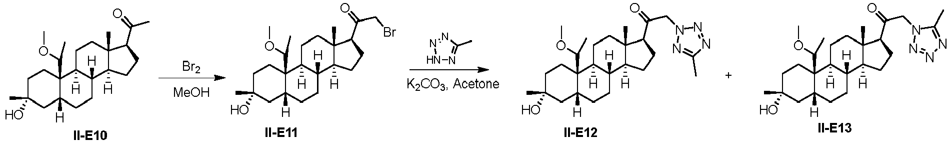

### Synthesis of II-E11

To a solution of **II-E10** (300 mg, 0.796 mmol) and HBr (0.1 mL, 48% in water) in MeOH (5 mL) was added dropwise bromine (190 mg, 1.19 mmol). After stirring at 25°C for 2 h, the reaction was quenched with saturated aqueous NaHCO₃ (20 mL) and the pH was adjusted to 7~8. The mixture was extracted with EtOAc (2 × 30 mL). The combined organic solution was washed with brine (50 mL), dried over Na₂SO₄, filtered and concentrated in vacuum. The residue was purified by silica gel column eluted with (PE/EtOAc = 8/1) to afford **II-E11** (240 mg, 60%) as a solid. **¹H NMR** (400 MHz, CDCl₃) δ 3.98-3.86 (m, 2H), 3.77-3.73 (m, 1H), 3.26 (s, 3H), 2.85-2.83 (m, 1H), 2.27-2.13 (m, 1H), 2.02-1.83 (m, 3H), 1.77-1.61 (m, 5H), 1.54-1.33 (m, 7H), 1.31-1.11 (m, 8H), 1.08-1.02 (m, 3H), 0.91-0.80 (m, 2H), 0.67 (s, 3H).

### Synthesis of II-E12 & II-E13

To a solution of **II-E11** (120 mg, 0.263 mmol) in acetone (3 mL) were added K₂CO₃ (72.5 mg, 0.526 mmol) and 5-methyl-2H-tetrazole (44.2 mg, 0.526 mmol) at 25°C. After stirring at 25°C for 16 h, the mixture was poured into water (30 mL) and extracted with EtOAc (2 × 30 mL). The combined organic solution was washed with brine (20 mL), dried over Na₂SO₄, filtered and concentrated in vacuum. The residue was purified by prep-HPLC separation (column: Boston Green ODS 150*30 5u, gradient: 60-90% B (A= 0.05%HCl-ACN, B= acetonitrile), flow rate: 25 mL/min) to give **II-E12** (15 mg, 12%) and **II-E12** (32 mg, 27%) both as solids.
**II-E12: ¹H NMR** (400 MHz, CDCl₃) δ 5.43-5.29 (m, 2H), 3.79-3.74 (m, 1H), 3.27 (s, 3H), 2.65-2.60 (m, 1H), 2.57 (s, 3H), 2.30-2.16 (m, 1H), 2.11 -2.08 (m, 1H), 2.01-1.95 (m, 1H), 1.76-1.65 (m, 1H), 1.80-1.63 (m, 6H), 1.52-1.34 (m, 6H), 1.33-1.10 (m, 10H), 1.07 (d, *J* = 6.4 Hz, 3H), 0.74 (s, 3H); **LC-ELSD/MS** purity 99%, MS ESI calcd. for C₂₆H₄₁N₄O₂ [M+H-H₂O] ⁺ 441, found 441.
**II-E13: ¹H NMR** (400 MHz, CDCl₃) δ 5.21-5.02 (m, 2H), 3.79-3.74 (m, 1H), 3.28 (s, 3H), 2.71-2.61 (m, 1H), 2.48 (s, 3H), 2.29-2.17 (m, 1H), 2.09-2.06 (m, 1H), 2.03-1.93 (m, 1H), 1.90-1.87 (m, 1H), 1.83-1.62 (m, 6H), 1.53-1.41 (m, 6H), 1.39-1.11 (m, 10H), 1.07 (d, *J* = 6.4 Hz, 3H), 0.71 (s, 3H); **LC-ELSD/MS** purity 99%, MS ESI calcd. for C₂₆H₄₁N₄O₂ [M+H-H₂O] ⁺ 441, found 441.

**Formula III Abbreviations:** PE: petroleum ether; EtOAc: ethyl acetate; THF: tetrahydrofuran; PCC: pyridinium chlorochromate; TLC: thin layer chromatography; PCC: pyridinium chlorochromate; t-BuOK: potassium tert-butoxide; 9-BBN: 9-borabicyclo[3.3.1]nonane; Pd(*t*-Bu₃P)₂: bis(tri-tert-butylphosphine)palladium(0); AcCl: acetyl chloride; *i*-PrMgCl: Isopropylmagnesium chloride; TBSCl: tert-Butyl(chloro)dimethylsilane; (*i*-PrO)₄Ti: titanium tetraisopropoxide; BHT: 2,6-di-t-butyl-4-methylphenoxide; Me: methyl; *i*-Pr: iso-propyl; *t*-Bu: tert-butyl; Ph: phenyl; Et: ethyl; Bz: benzoyl; BzCl: benzoyl chloride; CsF: cesium fluoride; DCC: dicyclohexylcarbodiimide; DCM: dichloromethane; DMAP: 4-dimethylaminopyridine; DMP: Dess-Martin periodinane; EtMgBr: ethylmagnesium bromide; EtOAc: ethyl acetate; TEA: triethylamine; AlaOH: alanine; Boc: t-butoxycarbonyl. Py: pyridine; TBAF: tetra-n-butylammonium fluoride; THF: tetrahydrofuran; TBS: t-butyldimethylsilyl; TMS: trimethylsilyl; TMSCF₃: (Trifluoromethyl)trimethylsilane; Ts: p-toluenesulfonyl; Bu: butyl; Ti(OiPr)₄: tetraisopropoxytitanium; LAH: Lithium Aluminium Hydride; LDA: lithium diisopropylamide; LiOH.H₂O: lithium hydroxide hydrates; MAD: methyl aluminum bis(2,6-di-t-butyl-4-methylphenoxide); MeCN: acetonitrile; NBS: N-bromosuccinimide; Na₂SO₄: sodium sulfate; Na₂S₂O₃: sodium thiosulfate; PE: petroleum ether; MeCN: acetonitrile; MeOH: methanol; Boc: t-butoxycarbonyl; MTBE: methyl tert-butyl ether; K-selectride: Potassium tri(s-butyl)borohydride.

### EXAMPLE III-1: Synthesis of 1-((3R,5R,8R,9R,10S,13S,14S,17S)-13-ethyl-3-hydroxy-3-methylhexadecahydro-1H-cyclopenta[a]phenanthren-17-yl)ethan-1-one (III-A8)

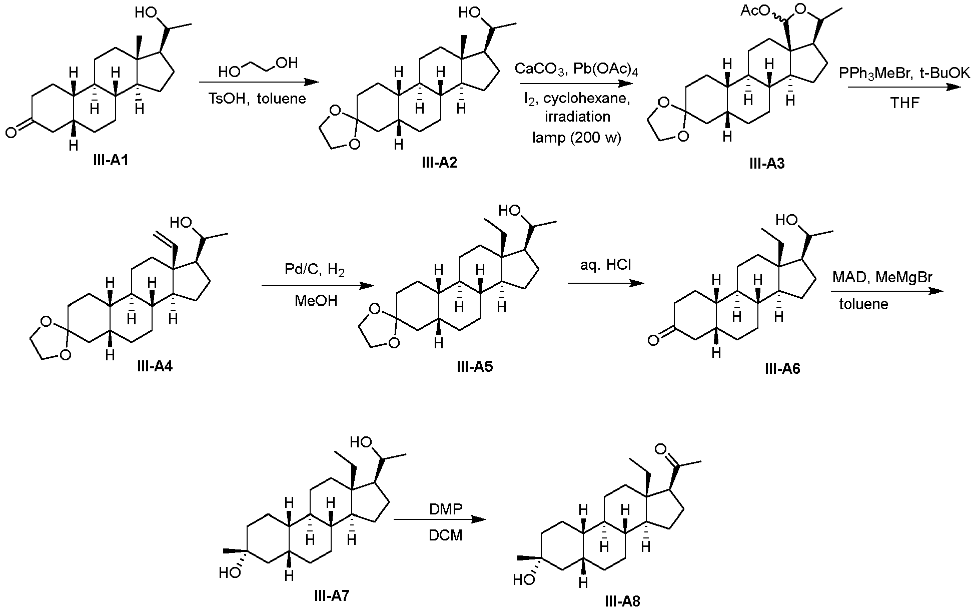

### Synthesis of III-A2

To a solution of **III-A1** (described in WO 2013056181) (14 g, 45.9 mmol), TsOH (787 mg, 4.6 mmol) and ethane-1,2-diol (28.4 g, 458 mol) was added to toluene (200 mL) at 25°C under N₂. The mixture was stirred at 120°C for 4 hours. The mixture was poured into water (100 mL) and extracted with ethyl acetate (2 × 100 mL). The combined organic solution was washed with brine (100 mL), dried over Na₂SO₄, filtered and concentrated in vacuum. The residue was purified by flash chromatography (5-10% EtOAc in PE) to give **III-A2** (8 g, 50%) as a solid.
**¹H NMR** (400 MHz, CDCl3) δ_{H} 3.93 (s, 4H), 3.76-3.63 (m, 1H), 2.03-1.95 (m, 1H), 1.93-1.80 (m, 4H), 1.64-1.58 (m, 3H), 1.56-1.48 (m, 2H), 1.44-1.30 (s, 6H), 1.27-0.95 (m, 12H), 0.66 (s, 3H).

### Synthesis of III-A3

To a solution of **III-A2** (2 g, 5.7 mmol) in cyclohexane (100 mL) stirred under N₂ were added CaCO₃ (1.71 g, 17.1 mmol), Pb(OAc)₄ (7.58 g, 17.1 mmol), and I₂ (2.89 g, 11.4 mmol) and irradiated with a high intensity tungsten lamp for 130 min. The solvent was refluxed during irradiation. The reaction was allowed cooled to 25°C, filtered and the cyclohexane solution washed with 10% aqueous Na₂S₂O₃ (30 mL), brine (50 mL), dried Na₂SO₄ and concentrated. The residue was purified by flash column (0-15% of EtOAc in PE) to give **III-A3** (900 mg, 39%) as an oil.
**¹H NMR** (400 MHz, CDCl3) δ_{H} 5.95 (s, 1H), 4.36-4.27 (m, 1H), 3.93 (s, 4H), 2.38-2.35 (m, 1H), 2.08 (s, 3H), 2.05-1.96 (m, 2H), 1.91-1.73 (m, 4H), 1.70-1.60 (m, 2H), 1.41-1.32 (m, 4H), 1.31-1.19 (m, 7H), 1.18-0.98 (m, 4H), 0.89-0.80 (m, 3H).

### Synthesis of III-A4

To a solution of PPh₃MeBr (5.28 g, 14.8 mmol) in THF (15 mL) was added t-BuOK (1.66 g, 14.8 mmol) at 25°C. After stirring at 50°C for 1 h, a solution of **III-A3** (600 mg, 1.48 mmol) in THF (5 mL) was added drop wise at 50°C. After stirring at 50°C for 3 h, the mixture was poured into saturated NH₄Cl (50 mL) and extracted with EtOAc (2 × 50 mL). The organic layer was washed with brine (100 mL), dried over Na₂SO₄ and filtered, concentrated in vacuum. The residue was purified by flash column (0-30% of EtOAc in PE) to give **III-A4** (420 mg, 79%) as an oil.
**¹H NMR** (400 MHz, CDCl3) δ_{H} 5.84-5.70 (m, 1H), 5.34-5.23 (m, 1H), 5.18-5.13 (m, 1H), 3.94 (s, 4H), 3.87-3.75 (m, 1H), 2.39-2.34 (m, 1H), 2.03-1.93 (m, 1H), 1.90-1.73 (m, 5H), 1.63-1.56 (m, 4H), 1.44-1.21 (m, 10H), 1.14-1.09 (m, 4H), 1.16-1.09 (m, 1H), 0.90-0.86 (m, 2H).

### Synthesis of III-A5

To a solution of **III-A4** (420 mg, 1.2 mmol) in MeOH (10 mL) was added Pd/C (200 mg, <1% water). Then the solution was hydrogenated under 103421 Pa (15 psi) of hydrogen at 25°C for 16 hrs. The mixture was filtered through a pad of celite and the filtrate was concentrated in vacuum to give **III-A5** (420 mg, 100%) as a solid.
**¹H NMR** (400 MHz, CDCl3) δ_{H} 3.94 (s, 4H), 3.85-3.82 (m, 1H), 2.15-2.11 (m, 1H), 2.03-1.97 (m, 1H), 1.90-1.84 (m, 3H), 1.65-1.51 (m, 10H), 1.45-1.31 (m, 6H), 1.28-1.26 (m, 3H), 1.18-1.06 (m, 3H), 0.98-0.89 (m, 2H), 0.84 (t, *J* = 8 Hz, 3H).

### Synthesis of III-A6

To a solution of **III-A5** (420 mg, 1.2 mmol) in THF (10 mL) was added aq. HCl (1.15 mL, 4M, 4.6 mmol). After stirring at 25°C for 2 h, the mixture was poured into water (100 mL) and extracted with EtOAc (2 × 50 ml). The combined organic solution was washed with saturated aqueous NaHCO₃ (50 mL), brine (50 mL), dried over Na₂SO₄ and concentrated in vacuum to afford **III-A6** (240 mg, 66%) as a solid.
**¹H NMR** (400 MHz, CDCl3) δ_{H} 3.87-3.84 (m, 1H), 2.60 (t, *J* = 16Hz, 1H), 2.29-2.14 (m, 4H), 2.11-2.03 (m, 1H), 1.92-1.84 (m, 1H), 1.75-1.64 (m, 4H), 1.61-1.56 (m, 3H), 1.54-1.45 (m, 2H), 1.45-1.31 (m, 4H), 1.29-1.26 (m, 3H), 1.24-1.09 (m, 5H), 1.07-0.92 (m, 2H), 0.87 (t, *J* = 8Hz, 3H).

### Synthesis of III-A7

To a solution of BHT (1.65 g, 7.52 mmol) in toluene (10 mL) was added dropwise AlMe₃ (2 M in toluene, 1.88 mL, 3.76 mmol) under nitrogen at 0°C. The mixture was stirred at 25°C for 1 h. **III-A6** (240 mg, 0.75 mmol) in toluene (5 mL) was added drop wise to the solution at -65°C. After stirring at -65°C for 1 h, MeMgBr (1 mL, 3.0 mmol, 3M in ethyl ether) was added drop wise at -65°C. The resulting solution was stirred at -65°C for 1 h. The reaction was quenched by saturated aqueous NH₄Cl (50 mL) at -65°C. After stirring at 25°C for 0.5 h, the resulting mixture was filtered through a celite pad and the pad was washed with EtOAc (20 mL). The combined organic layer was separated, washed with brine (2 × 20 mL), dried over Na₂SO₄, filtered and concentrated in vacuum. The residue was purified by flash column (0-30% EtOAc in PE) to give **III-A7** (215 mg, 86%) as a solid.
**¹H NMR** (400 MHz, CDCl3) δ_{H} 3.85-3.81 (m, 1H), 2.15-2.11 (m, 1H), 1.92-1.76 (m, 4H), 1.64-1.61 (m, 1H), 1.48-1.18 (m, 20H), 1.11-1.04 (m, 4H), 0.98-0.88 (m, 1H), 0.87-0.79 (m, 4H).

### Synthesis of III-A8

To a solution of **III-A7** (215 mg, 0.64 mmol) in DCM (5 mL) was added DMP (542 mg, 1.28 mmol). The reaction mixture was stirred at 30°C for 30 min. The reaction mixture was quenched with saturated NaHCO₃ aqueous (50 mL) until pH of the aqueous layer became about pH 9 and filtered. The DCM layer was separated and the aqueous phase was extracted with DCM (10 mL). The combined organic phase was washed with saturated Na₂S₂O₃ aqueous (2 × 20mL), sat. NaHCO₃ (40 mL), brine (40 mL), dried over Na₂SO₄, filtered and concentrated. The residue was purified by flash column (0-30% EtOAc in PE) to give **III-A8** (138 mg, 65%) as a solid.
**¹H NMR** (400 MHz, CDCl3) δ_{H} 2.46 (t, *J* = 8Hz, 1H), 2.33-2.21 (m, 2H), 2.20 (s, 3H), 1.87-1.80 (m, 3H), 1.74-1.59 (m, 4H), 1.52-1.33 (m, 7H), 1.32-1.04 (m, 12H), 0.96-0.81 (m, 1H), 0.62 (t, *J* = 8Hz, 3H); **LC-ELSD/MS** purity 99%, **MS ESI** calcd. for C₂₂H₃₅O [M-H₂O+H]⁺ 315, found 315.

### EXAMPLE III-2: Synthesis of 1-(2-((3R,5R,8R,9R,10S,13S,14S,17S)-13-ethyl-3-hydroxy-3-methylhexadecahydro-1H-cyclopenta[a]phenanthren-17-yl)-2-oxoethyl)-1H-pyrazole-4-carbonitrile (III-A10)

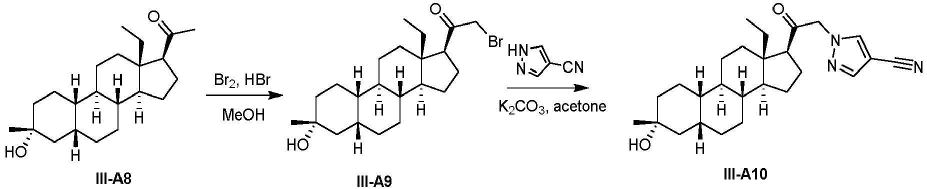

### Synthesis of III-A9

To a solution **of III-A8** (107 mg, 0.32 mmol) and concentrated HBr (0.1 mL, 48% in water) in MeOH (2 mL) was added bromine (61.5 mg, 0.39 mmol). The reaction mixture was stirred at 25°C for 2 hrs. The reaction was quenched by saturated aqueous NaHCO₃ and the pH was adjusted to 7~8 and extracted with EtOAc (2 × 30 mL). The combined organic phase was washed with saturated Na₂S₂O₃ (50 mL) and brine (50 mL), dried over anhydrous Na₂SO₄, filtered and concentrated in vacuum to afford **III-A9** (130 mg, ) as a solid.
**¹H NMR** (400 MHz, CDCl3) δ_{H} 4.01-3.91 (m, 2H), 2.67 (t, *J* = 8 Hz 1H), 2.26-2.13 (m, 2H), 1.79-1.71 (m, 4H), 1.67-1.56 (m, 4H), 1.43-1.29 (m, 8H), 1.22-1.08 (m, 10H), 0.86-0.73 (m, 1H), 0.52 (t, *J* = 8Hz, 3H).

### Synthesis of III-A10

To a solution of **III-A9** (130 mg, 0.32 mmol) in acetone (3 mL) was added K₂CO₃ (108 mg, 0.79 mmol) and 1H-pyrazole-4-carbonitrile (43.9 mg, 0.47 mmol). The mixture was stirred at 25°C for 3 hours. The mixture was poured into water (30 mL) and extracted with ethyl acetate (2 × 30 mL). The combined organic layers was washed with brine (50 mL), dried over Na₂SO₄, filtered and concentrated in vacuum. The residue was purified by flash chromatography (15-35% EtOAc in PE) to give **III-A10** (43 mg, 32%) as a solid.
**¹H NMR** (400 MHz, CDCl3) δ_{H} 7.86 (s, 1H), 7.81 (s, 1H), 5.20-4.88 (m, 2H), 2.52-2.46 (m, 1H), 2.36-2.25 (m, 2H), 1.89-1.62 (m, 7H), 1.51-1.33 (m, 10H), 1.29-1.07 (m, 9H), 0.98-0.85 (m, 1H), 0.61 (t, *J* = 8Hz, 3H); **LC-ELSD/MS** purity 99%, **MS ESI** calcd. for C₂₆H₃₇N₃O₂Na [M+Na]⁺ 446, found 446.

### EXAMPLE III-3: Synthesis of 1-((3R,5S,8R,9R,10S,13S,14S,17S)-13-ethyl-3-hydroxy-3-(methoxymethyl)hexadecahydro-1H-cyclopenta[a]phenanthren-17-yl)ethan-1-one (III-A20)

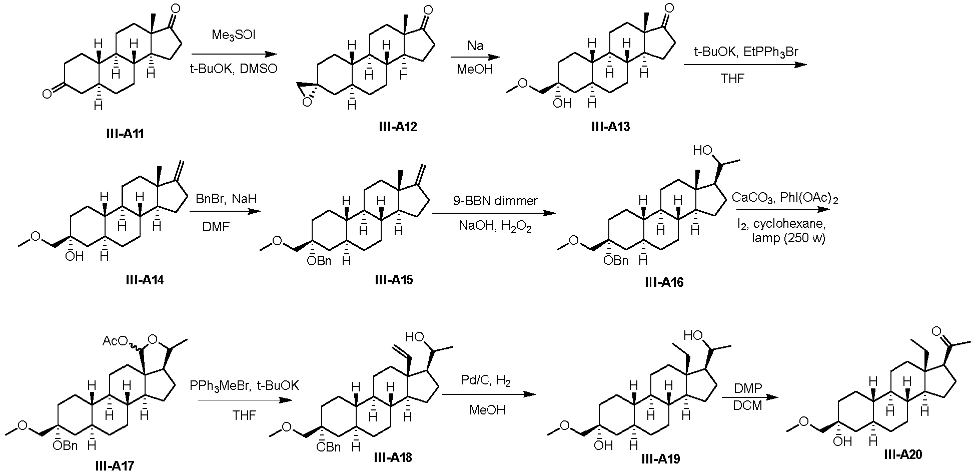

### Synthesis of III-A12

A stirred solution of trimethylsulfoxonium iodide (880 mg, 4.00 mmol) and t-BuOK (448 mg, 4.00 mmol) in DMSO (10 mL) was heated at 40°C for 1 h under N₂. The reaction mixture was added to **III-A11** (1 g, 3.64 mmol, CAS# 5696-58-2) in DMSO (10 mL) and stirred at 40°C. After 10 min, the reaction was treated with water (100 mL), extracted with EtOAc (3 × 50 mL). The combined organic phase was washed with water (100mL), brine (50 mL), dried over anhydrous Na₂SO₄, filtered, and concentrated in vacuum to afford **III-A12** (1.8 g, ) as a solid.
**¹H NMR** (400 MHz, CDCl₃) δ_{H} 2.43 (dd, *J*=8.3, 19.3 Hz, 1H), 2.13 - 2.05 (m, 1H), 2.00 - 1.73 (m, 5H), 1.66 - 1.28 (m, 6H), 1.22 - 0.98 (m, 4H), 0.91 - 0.72 (m, 4H).

### Synthesis of III-A13

To a suspension of **III-A12** (1.8 g, 6.24 mmol) in MeOH (20 mL) was added methoxysodium (1.34 g, 24.9 mmol) at 25°C under N₂. After refluxing for 16 h, the mixture was quenched with H₂O (300 mL) and extracted with EtOAc (2 × 200 mL). The combined organic phase was washed with brine (2 × 200 mL), dried over Na₂SO₄, filtered, concentrated to give a residue. The residue was purified by silica gel chromatography (0-20% of EtOAc in PE) to give **III-A13** (900 mg, 45.2 %) as a solid.
**¹H NMR** (400 MHz, CDCl₃) δ_{H} 3.38 (s, 3H), 3.19 (s, 2H), 2.52 - 2.32 (m, 1H), 2.16 - 2.00 (m, 2H), 1.98 - 1.85 (m, 2H), 1.84 - 1.70 (m, 4H), 1.68 - 1.62 (m, 2H), 1.55 - 1.41 (m, 2H), 1.37 - 1.18 (m, 5H), 1.15 - 0.96 (m, 4H), 0.87 (s, 3H), 0.81 - 0.67 (m, 2H).

### Synthesis of III-A14

To a solution of PPh₃EtBr (20.7 g, 56 mmol) in THF (100 mL) was added t-BuOK (6.28 g, 56 mmol) at 25°C. After stirring at 50°C for 1 h, a solution of **III-A13** (9 g, 28 mmol) in THF (50 mL) was added drop wise at 50°C. After stirring at 50°C for 16 h, the mixture was poured into saturated NH₄Cl (500 mL) and extracted with EtOAc (2 × 100 mL). The organic layer was washed with brine (300 mL), dried over Na₂SO₄ and filtered, concentrated in vacuum. The residue was purified by flash column (0-30% of EtOAc in PE) to give **III-A14** (8.5 g, 91.2%) as an oil.
**¹H NMR** (400 MHz, CDCl₃) δ_{H} 5.14-5.07 (m, 1H), 3.38 (s, 3H), 3.18 (s, 2H), 2.38-2.29 (m, 1H), 2.25-2.12 (m, 2H), 1.86-1.67 (m, 3H), 1.60-1.49 (m, 4H), 1.33-0.95(m, 12H), 0.89-0.83 (m, 6H), 0.75-0.65 (m, 2H).

### Synthesis of III-A15

To a solution of **III-A14** (7.5 g, 22.5 mmol) in DMF (100 mL) was added NaH (2.68 g, 67.5 mmol, 60% in mineral oil) in three portions at 25°C. After stirring at 25°C for 30 min, BnBr (11.5 g, 67.5 mmol) was added to the solution. After stirring at 25°C for 16 h, the mixture was poured into ice-water (500 mL) and extracted with ethyl acetate (2 × 200 mL). The combined organic solutions were washed with brine (200 mL), dried over Na₂SO₄, filtered and concentrated in vacuum. The residue was purified by flash chromatography (5-10% EtOAc in PE) to give **III-A15** (8 g, ) as an oil.
**¹H NMR** (400 MHz, CDCl₃) δ_{H} 7.30-7.18 (m, 5H), 5.06-5.01 (m, 1H), 4.50 (s, 1H), 4.44-4.38 (m, 2H), 3.32-3.24 (m, 5H), 2.31-2.25 (m, 1H), 2.17-1.92 (m, 3H), 1.80-1.52 (m, 9H), 1.45-0.88 (m, 9H), 0.79-0.76 (m, 4H), 0.67-0.65 (m, 2H).

### Synthesis of III-A16

A solution of **III-A15** (8.0 g, 18.9 mmol) in THF (150 mL) was added 9-BBN dimer (9.22 g, 37.8 mmol) and stirred at 25°C for 12 hours. To the resulting mixture was added ethanol (50 mL) at 15°C, followed by NaOH aqueous (37.8 mL, 5.0 M, 189 mmol) at 0°C. Hydrogen peroxide (18.9 mL, 10 M, 189 mmol) was added drop-wise at 0°C. The reaction mixture was stirred at 78°C for 1 hour. After cooled to 15°C, the mixture was added to the water (100 mL) and extracted with EtOAc (2 × 200 mL). The combined organic layer was washed with Na₂S₂O₃ (2 × 100 mL) and brine (200 mL), dried over Na₂SO₄, filtered and concentrated in vacuum. The residue was purified by flash column (0-15% EtOAc in PE) to give **III-A16** (8.2 g, ) as an oil. The **III-A16** (8 g, ) was repurified by flash column (0-5% EtOAc in PE) to give **III-A16** (5.6 g, 70%) as a solid.
**¹H NMR** (400 MHz, CDCl₃) δ_{H} 7.39-7.29 (m, 4H), 7.26-7.21 (m, 1H), 4.50-4.45 (m, 2H), 3.75-3.66 (m, 1H), 3.40-3.30 (m, 5H), 2.03-1.96 (m, 1H), 1.93-1.79 (m, 3H), 1.77-1.60 (m, 6H), 1.42-1.18 (m, 8H), 1.17-0.91 (m, 9H), 0.75-0.69 (m, 1H), 0.66 (s, 3H).

### Synthesis of III-A17

To a solution of **III-A16** (1 g, 2.26 mmol) in cyclohexane (100 mL) were added CaCO₃ (677 mg, 6.77 mmol), PhI(OAc)₂ (2.18 g, 6.77 mmol), I₂ (1.14 mg, 4.52 mmol) at 25°C. The reaction mixture was heated to reflux (80°C) by irradiation with infrared lamp (250 W) for 15 min under N₂ atmosphere, then cooled to ambient. The reaction mixture was poured into ice-water saturated aqueous Na₂S₂O₃ (200 mL) and extracted with EtOAc (2 × 200 mL) and the combined organic layers were dried over Na₂SO₄, concentrated below 35°C to give **III-A17** (1.1 g, ) as an oil and used directly for the next step.

### Synthesis of III-A18

To a mixture of MePPh₃Br (15.7 g, 44.2 mmol) in THF (100 mL) was added t-BuOK (4.95 g, 44.2 mmol) at 25°C under N₂. After stirring at 50°C for 30 min, **III-A17** (2.2 g, 4.42 mmol) was added in portions below 50°C. The reaction mixture was stirred at 50°C for 16 hours to give a suspension. The reaction mixture was quenched with 10% NH₄Cl aqueous (500 mL). The aqueous solution was extracted with EtOAc (2 × 300 mL) and the combined organic layers were washed with brine (150 mL), dried over Na₂SO₄, filtered and concentrated. The residue was purified by flash column (0~10% of EtOAc in PE) to give **III-A18** (1.3 g, 65%) as an oil.
**¹H NMR** (400 MHz, CDCl₃) δ_{H} 7.38-7.30 (m, 4H), 7.26-7.21 (m, 1H), 5.78 (dd, *J*=11.2, 18.4 Hz, 1H), 5.32-5.12 (m, 2H), 4.47 (s, 2H), 3.86-3.77 (m, 1H), 3.37-3.29 (m, 5H), 2.40-2.31 (m, 1H), 2.01-1.94 (m, 1H), 1.85-1.65 (m, 5H), 1.56-1.23 (m, 9H), 1.22-0.93 (m, 10H), 0.78-0.62 (m, 2H).

### Synthesis of III-A19

To a solution of **III-A18** (220 mg, 0.49 mmol) in MeOH (10 mL) was added Pd/C (500 mg) (10% Pd, 50% water), the mixture was stirred at 25°C under H₂ (103421 Pa = 15 psi) for 15 hours. The reaction mixture was filtered through a pad of Celite and washed with MeOH (3 × 10 mL). The filtrate was concentrated to give **III-A19** (170 mg, 96%) as a solid. The solid (170 mg) was purified by flash column (0~20% of EtOAc in PE) to give **III-A19** (140 mg) as a solid.
**¹H NMR** (400 MHz, CDCl₃) δ 3.87-3.78 (m, 1H), 3.38 (s, 3H), 3.18 (s, 2H), 2.15-2.08 (m, 1H), 1.93-1.69 (m, 5H), 1.64-1.58 (m, 5H), 1.46-1.30 (m, 4H), 1.26 (d, *J*=6.0 Hz, 3H), 1.24-1.15 (m, 3H), 1.13-1.05 (m, 3H), 1.03-0.87 (m, 5H), 0.84 (t, *J*=8.0 Hz, 3H), 0.75-0.63 (m, 2H). **LC-ELSD/MS** purity 99%, **MS ESI** calcd. for C₂₃H₃₇O [M-2H₂O+H]⁺ 329.3, found 329.3.

### Synthesis of III-A20

To a solution of **III-A19** (1 g, 2.74 mmol) in DCM (20 mL) was added DMP (2.32 g, 5.48 mmol), the mixture was stirred at 25°C for 2 hours. The mixture was quenched by saturated NaHCO₃ aqueous (150 mL) and Na₂S₂O₃ aqueous (150 mL), The aqueous phase was extracted with DCM (3 × 100 mL). The combined organic phase was washed with brine (200 mL), dried over Na₂SO₄, filtered and concentrated. The residue was purified by flash column (0~15% of EtOAc in PE) to give **III-A20** (840 mg, 84.5%) as a solid.
**¹H NMR** (400 MHz, CDCl₃) δ 3.38 (s, 3H), 3.18 (s, 2H), 2.46 (t, *J*=8.8 Hz, 1H), 2.36-2.20 (m, 2H), 2.19 (s, 3H), 1.87-1.60 (m, 7H), 1.58-1.08 (m, 11H), 1.05-0.86 (m, 4H), 0.81-0.66 (m, 2H), 0.62 (t, *J*=7.6 Hz, 3H); **LC-ELSD/MS** 99%, **MS ESI** calcd. for C₂₃H₃₇O₂ [M-H₂O+H]⁺ 345.3, found 345.3.

### EXAMPLE III-4: Synthesis of 1-((3R,5S,8R,9R,10S,13S,14S,17S)-13-ethyl-3-hydroxy-3-(methoxymethyl)hexadecahydro-1H-cyclopenta[a]phenanthren-17-yl)-2-(5-methyl-2H-tetrazol-2-yl)ethan-1-one (III-A22) & 1-((3R,5S,8R,9R,10S,13S,14S,17S)-13-ethyl-3-hydroxy-3-(methoxymethyl)hexadecahydro-1H-cyclopenta[a] phenanthren-17-yl)-2-(5-methyl-1H-tetrazol-1-yl)ethan-1-one (III-A23)

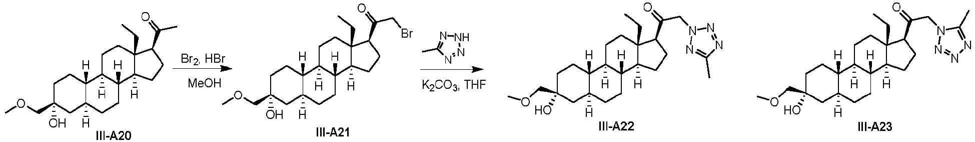

### Synthesis of III-A21

To a solution of **III-A20** (150 mg, 0.41 mmol) in MeOH (2 mL) was added HBr (16.7 mg, 0.08 mmol, 40% in water) and Br₂ (66.1 mg, 0.41 mmol) at 25°C. After stirring at 25°C for 2 h, the mixture was quenched by saturated aqueous NaHCO₃ (10 mL), treated with water (30 mL), extracted with EtOAc (2 × 50 mL). The combined organic phase was washed with brine (30 mL), dried over anhydrous Na₂SO₄, filtered, concentrated to give **III-A21** (180 mg, ) as an oil and used directly for the next step.
**¹H NMR** (400 MHz, CDCl₃) δ 4.01 (s, 2H), 3.39 (s, 3H), 3.18 (s, 2H), 2.74 (t, *J*=8.8 Hz, 1H), 2.32-2.17 (m, 2H), 1.87-1.69 (m, 4H), 1.61-1.54 (m, 1H), 1.47-1.15 (m, 13H), 0.96-0.65 (m, 6H), 0.59 (t, *J*=7.6 Hz, 3H).

### Synthesis of III-A22 & III-A23

To a solution of **III-A21** (180 mg, 0.41 mmol) in THF (3 mL) was added K₂CO₃ (168 mg, 1.22 mmol) and 5-methyl-2*H*-1,2,3,4-tetrazole(52 mg, 0.62 mmol). After stirring at 25°C for 14 h, the mixture was added water (20 mL) and extracted with EtOAc (2 × 30 mL). The organic layer was separated, dried over Na₂SO₄, filtered and concentrated. The residue was purified by flash column (5~90% of EtOAc in PE) to give **III-A22** (50 mg, ) and **III-A23** (50 mg, ).
**III-A22** (50 mg, ) was purified by HPLC to give **III-A22** (4 mg, 2.2%) as a solid.
**¹H NMR** (400 MHz, CDCl₃) δ 5.54-5.32 (m, 2H), 3.39 (s, 3H), 3.19 (s, 2H), 2.60-2.47 (m, 4H), 2.41-2.23 (m, 2H), 2.09-1.96 (m, 1H), 1.94-1.61 (m, 7H), 1.49-1.17 (m, 9H), 1.07-0.90 (m, 4H), 0.84-0.67 (m, 5H); **LC-ELSD/MS** purity 99%, **MS ESI** calcd. for C₂₅H₄₀N₄O₃ [M+H]⁺ 445.3, found 445.3.
**III-A23** (50 mg, ) was purified by HPLC to give **III-A23** (4 mg, 2.2%) as a solid.
**¹H NMR** (400 MHz, CDCl₃) δ_{H} 5.48-4.89 (m, 2H), 3.39 (s, 3H), 3.19 (s, 2H), 2.45 (t, *J*=8.4 Hz, 1H), 2.49 (s, 3H), 2.43-2.36 (m, 1H), 2.32-2.22 (m, 1H), 1.95-1.64 (m, 8H), 1.48-1.18 (m, 8H), 1.11-0.67 (m, 7H), 0.63 (t, *J*=7.6 Hz, 3H); **LC-ELSD/MS** purity 99%, **MS ESI** calcd. for C₂₅H₄₀N₄O₃ [M+H]⁺ 445.3, found 445.3.

### EXAMPLE III-6: Synthesis of 1-((3R,5R,8R,9S,10S,13S,14S,17S)-13-ethyl-3-hydroxy-3,10-dimethylhexadecahydro-1H-cyclopenta[a]phenanthren-17-yl)ethan-1-one (III-A30)

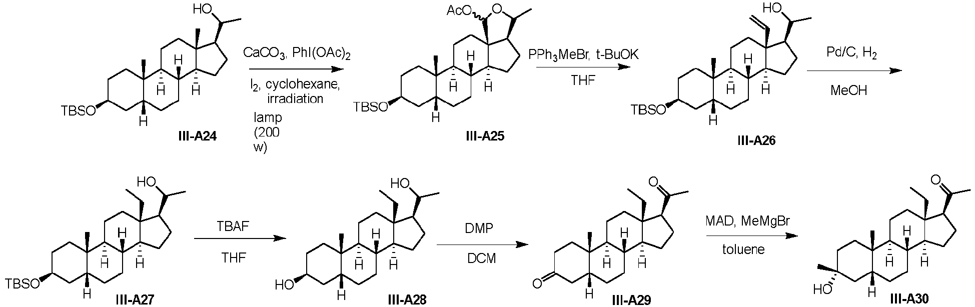

### Synthesis of III-A25

To a solution **of III-A24** (1.0 g, 2.3 mmol) in cyclohexane (100 mL) were added CaCO₃ (688 mg, 6.89 mmol), PhI(OAc)₂ (2.21 g, 6.89 mmol), I₂ (1.16 g, 4.6 mmol) at 25°C. The reaction mixture was heated to reflux (80°C) by irradiation with infrared lamp (250 W) for 15 min under N₂ atmosphere. Then the reaction mixture was cooled to ambient temperature. The reaction mixture was poured into ice-water saturated aqueous Na₂S₂O₃ (100 mL) and separated. The aqueous layer was extracted with EtOAc (2×50 mL) and the combined organic layers were washed with brine, dried over Na₂SO₄, concentrated below 40°C to give **III-A25** (2.5 g, ) as a solid and used directly for the next step.

### Synthesis of III-A26

To a mixture of MePPh₃Br (9.07 g, 25.4 mmol) in THF (50 mL) was added t-BuOK (2.85 g, 25.4 mmol) at 25°C under N₂. After stirring at 50°C for 30 min, **III-A25** (2.5 g, 5.09 mmol) was added in portions below 50°C. After stirring at 50°C for 14 h, the reaction mixture was quenched with 10% NH₄Cl aqueous (100 mL) at 15°C. The mixture was extracted with EtOAc (2 × 50 mL). The organic layer was separated, dried over Na₂SO₄, filtered and concentrated. The residue was purified by flash column (0~10% of EtOAc in PE) to give **III-A26** (410 mg, 18.0%) as a solid.
**¹H NMR** (400 MHz, CDCl₃) δ_{H} 5.73-5.57 (m, 1H), 5.36-5.25 (m, 1H), 5.23-5.12 (m, 1H), 3.70-3.48 (m, 2H), 2.54-2.43 (m, 1H), 1.87-1.66 (m, 5H), 1.61-1.44 (m, 6H), 1.40-1.30 (m, 8H), 1.28-1.13 (m, 4H), 1.12-1.08 (m, 3H), 0.90-0.80 (s, 12H), 0.06 (s, 6H) .

### Synthesis of III-A27

To a solution of **III-A26** (560 mg, 1.25 mmol) in MeOH (20 mL) was added Pd/C (500 mg, 10%, 50% water wet). The solution was hydrogenated under 103421 Pa (15 psi) of hydrogen at 25°C for 16 h. The reaction mixture was filtered through a pad of Celite and washed with THF (3 × 50 mL). The filtrate was concentrated to give **III-A27** (600 mg, ) as a solid used directly for the next step.
**¹H NMR** (400 MHz, CDCl₃) δ_{H} 3.81-3.71 (m, 1 H), 3.64-3.53 (m, 1H), 2.33-2.18 (m, 1H), 1.90-1.71 (m, 3H), 1.70-1.58 (m, 2H), 1.54-1.14 (m, 14H), 1.14-1.11 (m, 3H), 1.10-1.01 (m, 3H), 1.00-0.92 (m, 5H), 0.91-0.88 (m, 12H), 0.06 (s, 6H).

### Synthesis of III-A28

**III-A27** (562 mg, 1.25 mmol) was dissolved in TBAF (6.26 mL, 6.26 mmol, 1M in THF). The mixture was stirred at 25°C for 16 hrs. The mixture was quenched with 10%NH₄Cl (30 mL) and extracted with EtOAc (2 × 10 mL). The combined organic phase was washed with 10% NH₄Cl (3 × 20 mL), dried over Na₂SO₄, filtered and concentrated to give **III-A28** (400 mg, ) as an oil.

### Synthesis of III-A29

To a solution of **III-A28** (300 mg, 0.89 mmol) in DCM (5 mL) was added Dess-martin (1.14 g, 2.69 mmol) at 30 °C. After stirring at 30 °C and stirred for 10 min, the mixture was quenched by saturated NaHCO₃/Na₂S₂O₃ aqueous (1:1, 50 mL) at 25 °C. The organic phase was separated and washed with saturated NaHCO₃/Na₂S₂O₃ aqueous (1:1, 50 mL), brine (50 mL), dried over Na₂SO₄, filtered and concentrated under vacuum to give **III-A29** (230 mg) as a solid and used directly for the next step.
**¹H NMR** (400 MHz, CDCl₃) δ_{H} 2.74-2.62 (m, 1 H), 2.52-2.43 (m, 1H), 2.41-2.23 (m, 3H), 2.21 (s, 3H), 2.09-0.99 (m, 2H), 1.96-1.78 (m, 2H), 1.72-1.58 (m, 3 H), 1.55-1.47 (m, 3H), 1.47-1.37 (m, 1H), 1.37-1.05 (m, 9H), 1.02 (s, 3H), 0.65 (t, *J*=7.6Hz, 3H); **LC-ELSD/MS** purity 99%, **MS ESI** calcd. for C₂₂H₃₄O₂ [M+H]⁺331.2, found 331.2.

### Synthesis of III-A30

To a MAD (3.38 mmol in 3 mL toluene) solution was added a solution of **III-A29** (280 mg, 0.85 mmol) in DCM (2 mL) dropwise at -70°C. After stirring at -70°C for 1 h under N₂, MeMgBr (0.85 mL, 2.55 mmol, 3M in ethyl ether) was added drop wise at -70°C. The resulting solution was stirred at -70°C for another 4 hrs. The reaction mixture was poured into saturated aqueous citric acid (50 mL) at below 10°C and extracted with EtOAc (2 x 50mL). The combined organic layer was dried over Na₂SO₄, filtered and concentrated in vacuum. The residue was purified by a silica gel column (PE/EtOAc= 0-20%) to give **III-A30** (110 mg) as a solid.
**¹H NMR** (400 MHz, CDCl₃) δ_{H} 2.49-2.39 (m, 1H), 2.36-2.21 (m, 2H), 2.19 (s, 3H), 2.01-1.92 (m, 1H), 1.91-1.79 (m, 1H), 1.79-1.70 (m, 1H), 1.68-1.57 (m, 3H), 1.53-1.32 (m, 9H), 1.31-1.19 (m, 11H), 1.14-1.00 (m, 3H), 0.93 (s, 3H), 0.62 (t, *J*=7.6Hz, 3H); **LC-ELSD/MS** purity 99%, **MS ESI** calcd. for C₂₃H₃₈O₂ [M+H]⁺329.3, found 329.3.

### EXAMPLE III-7 & III-8: Synthesis of 1-((3R,5R,8R,9S,10S,13S,14S,17S)-13-ethyl-3-hydroxy-3,10-dimethylhexadecahydro-1H-cyclopenta[a]phenanthren-17-yl)-2-(5-methyl-1H-tetrazol-1-yl)ethan-1-one (III-A32) & 1-((3R,5R,8R,9S,10S,13S,14S,17S)-13-ethyl-3-hydroxy-3,10-dimethylhexadecahydro-1H-cyclopenta[a]phenanthren-17-yl)-2-(5-methyl-2H-tetrazol-2-yl)ethan-1-one (III-A33)

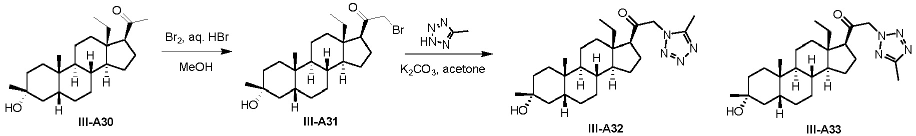

### Synthesis of III-A31

To a solution of **III-A30** (99 mg, 0.28 mmol) in MeOH (2 mL) was added HBr (0.01 mL, 40% in water) and Br₂ (54.7 mg, 0.3427 mmol) at 25 °C. After stirring at 25 °C for 2 h, the mixture was quenched by sat.aq NaHCO₃ (10 mL), extracted with EtOAc (2 × 30 mL). The combined organic phase was washed with brine (30 mL), dried over anhydrous Na₂SO₄, filtered, concentrated in vacuum to afford **III-A31** (100 mg, ) as a solid and used directly for the next step.

### Synthesis of III-A32 & III-A33

To a solution of **III-A31** (100 mg, 0.23 mmol) in acetone (3 mL) was added 1H-pyrazole-4-carbonitrilev (23.7 mg, 0.282 mmol) and K₂CO₃(60.2 mg, 0.47 mmol). After stirring at 25°C for 14 hours, the mixture was added water (20 mL) and extracted with EtOAc (2 × 30 mL). The organic layer was separated, dried over Na₂SO₄, filtered and concentrated. The residue was purified by flash column (20~50% of EtOAc in PE) to give **III-A32** (20 mg, 19.9%) and **III-A33** (16.5 mg, 16.5%) as a solid.
**III-A32: ¹H NMR** (400 MHz, CDCl₃) δ_{H} 5.36 (d, *J*=18.0 Hz, 1H), 4.96 (d, *J*=18.0 Hz, 1H), 2.57-2.50 (m, 1H), 2.49 (s, 3H), 2.44-2.17 (m, 2H), 2.03-1.81 (m, 2H), 1.81-1.62 (m, 3H), 1.51-1.40 (m, 5H), 1.39-1.22 (m, 12H), 1.22-0.99 (m, 4H), 0.95 (s, 3H), 0.63 (t, J=7.6Hz, 3H); **LC-ELSD/MS** purity 99%, **MS ESI** calcd. for C₂₅H₄₀N₄O₂ [M-H₂O +H]⁺411.3, found 411.3.
**III-A33: ¹H NMR** (400 MHz, CDCl₃) δ_{H} 5.48 (d, *J*=17.6Hz, 1H) 5.37 (d, *J*=17.6Hz, 1H), 2.56 (s, 3H), 2.54-2.47 (m, 1H), 2.41-2.20 (m, 2H), 2.06-1.79 (m, 2H), 1.79-1.63 (m, 3H), 1.52-1.39 (m, 6H), 1.39-1.20 (m, 14H), 1.19-0.99 (m, 4H), 0.95 (s, 3H), 0.71 (t, *J*=7.6Hz, 3H); **LC-ELSD/MS** purity 99%, **MS ESI** calcd. for C₂₅H₄₀N₄O₂ [M-H₂O +H]⁺411.3, found 411.3.

### EXAMPLE III-9: Synthesis of 1-((3R,5R,8R,9R,10S,13S,14S,17S)-13-ethyl-3-hydroxy-3-(methoxymethyl)hexadecahydro-1H-cyclopenta[a]phenanthren-17-yl)ethan-1-one (III-B15)

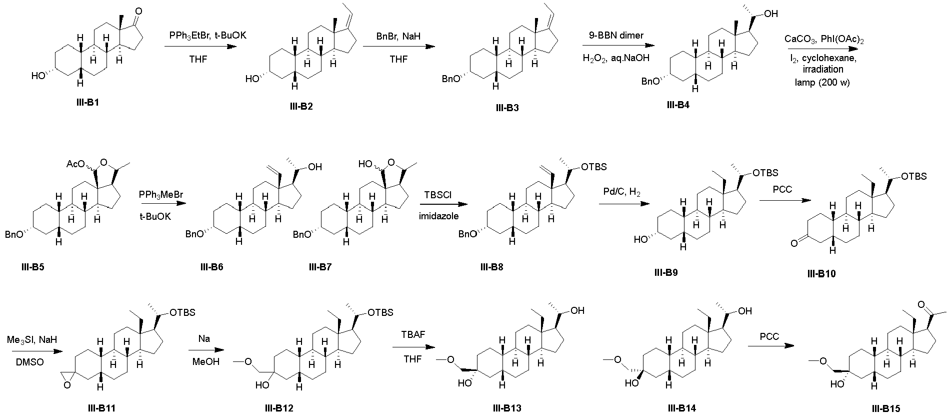

### Synthesis of III-B2

To a suspension of bromo(ethyl)triphenylphosphorane (160 g, 432 mmol) in THF (300 mL) was added t-BuOK (48.3 g, 432 mmol) at 25°C under N₂. After stirring at 45°C and for 1 h, a solution of **III-B1** (30 g, 108 mmol, CAS: 33036-33-8) in THF (100 mL) was added at 45°C. After stirring at 45°C for 16 h, the mixture was cooled, diluted with saturated NH₄Cl solution (1000 mL) and extracted with EtOAc (2 × 250 mL). The combined organic solution was washed with brine (2 × 250 mL), dried over anhydrous Na₂SO₄, filtered, concentrated (30 g) and purified together with another two batches (from 20 g & 30 g). The residue (80 g) was purified by flash column (0~25% of EtOAc in PE) to give **III-B2** (50 g, 63%) as a solid.
**¹H NMR** (400 MHz, CDCl₃) δ_{H} 5.20-5.05 (m, 1H), 3.70-3.60 (m, 1H), 2.40-2.30 (m, 1H), 2.25-2.10 (m, 2H), 1.95-1.85 (m, 1H), 1.85-1.40 (m, 12H), 1.40-1.05 (m, 11H), 0.87 (s, 3H).

### Synthesis of III-B3

To a solution of **III-B2** (15 g, 51.9 mmol) in DMF (100 mL) was added NaH (6.18 g, 155 mmol, 60% in oil) in portions at 25°C. After stirring at 25°C for 30 mins, BnBr (26.5 g, 155 mmol) was added. After stirring at 25°C for 3 h, the mixture was poured into ice-water (500 mL) and extracted with ethyl acetate (2 × 100 mL). The combined organic solution was washed with brine (100 mL), dried over anhydrous Na₂SO₄, filtered and concentrated in vacuum. The residue was purified by flash chromatography (PE) to give **III-B3** (19 g) as an oil. **¹H NMR** (400 MHz, CDCl₃) δ_{H} 7.43-7.26 (m, 5H), 5.20-5.05 (m, 1H), 4.57 (s, 2H), 3.45-3.35 (m, 1H), 2.45-2.10 (m, 4H), 2.00-1.93 (m, 1H), 1.85-1.55 (m, 8H), 1.50-1.00 (m, 13H), 0.88 (s, 3H).

### Synthesis of III-B4

To a solution of **III-B3** (10 g, 26.4 mmol) in THF (100 mL) was added 9-BBN dimer (12.8 g, 52.8 mmol) at 25°C. After stirring at 25°C for 12 h, the reaction was cooled to 0°C and ethanol (12.1 g, 264 mmol) and NaOH (52.8 mL, 5 M, 264 mmol) were added very slowly. After the addition was complete, H₂O₂ (26.4 mL, 264 mmol, 30%) was added slowly maintaining the inner temperature below 15°C. After stirring at 75°C for 1 h, saturated aqueous Na₂S₂O₃ solution (500 mL) was added and the mixture was stirred at 0°C for another 1 hour. The mixture was diluted with water (500 mL) and extracted with EtOAc (3 × 200 mL). The combined organic solution was washed with saturated brine (500 mL), dried over anhydrous Na₂SO₄, filtered, concentrated (10 g, ) and purified by flash column (0~15% of EtOAc in PE) to give **III-B4** (8 g, 80%) as an oil. **¹H NMR** (400 MHz, CDCl₃) δ_{H} 7.42-7.20 (m, 5H), 4.56 (s, 2H), 3.90-3.80 (m, 2H), 3.75-3.65 (m, 1H), 3.45-3.30 (m, 1H), 2.00-1.75 (m, 9H), 1.70-1.55 (m, 4H), 1.40-0.95 (m, 13H), 0.66 (s, 3H).

### Synthesis of III-B5

To a suspension of **III-B4** (2.5 g, 6.3 mmol), PhI(OAc)₂ (6.08 g, 18.9 mmol) and CaCO₃ (1.89 g, 18.9 mmol) in cyclohexane (250 mL) was added diiodine (3.19 g, 12.6 mmol) at 25°C. After heating to 80°C by irradiation with infrared lamp (275 W) for 10 min, the mixture was added into saturated Na₂S₂O₃ solution (500 mL) and extracted with EtOAc (3 × 50 mL). The combined organic solution was washed with saturated brine (200 mL), dried over anhydrous Na₂SO₄, filtered and concentrated to give **III-B5** (2.85 g) as am oil, which was used directly to the next step without further purifiaction.

### Synthesis of III-B6 & III-B7

After stirring at 50°C for 1h, a suspension of PPh₃MeBr (11.2 g, 31.4 mmol) and t-BuOK (3.51 g, 31.4 mmol) in THF (30 mL) was added a solution of **III-B7** (2.85 g, 6.29 mmol) in THF (20 mL). After stirring at 50°C for 12 h, the mixture was added into saturated NH₄Cl (100 mL) and extracted with EtOAc (3 × 50 mL). The combined organic solution was washed with saturated brine (100 mL), dried over anhydrous Na₂SO₄, filtered and concentrated (2.5 g) as an oil, which was purified together with other three batches (from 2.85 g & 2.85g & 2.35 g). The residue (9.5 g) was purified by flash column (0-15% of EtOAc in PE) to give mixture of **III-B6 & III-B7** (~1:1) (5 g) as an oil.

### Synthesis of III-B8

To a solution of **III-B6 & III-B7** (~1:1) (5.0 g, 12.2 mmol) in DCM (50 mL) were added imidazole (2.48 g, 36.5 mmol) and TBSCl (5.50 g, 36.5 mmol) at 25°C. After stirring at 25°C for 12 h, the mixture was filtered and the filtrate was concentrated. The product was dissolved in DCM (70 mL), washed with saturated aqueous NH₄Cl (50 mL), saturated brine (50 mL). The organic solution was dried over sodium sulfate, filtered and the filtrate was concentrated and purified by flash column (0-15% of EtOAc in PE) to give **III-B8** (3.5 g, 55%) and **III-B7** (2 g, 40%) both as oils.
**III-B8: ¹H NMR** (400 MHz, CDCl₃) δ_{H} 7.40-7.20 (m, 5H), 5.57 (dd, *J* = 11.2, 17.6 Hz, 1H), 5.18 (dd, *J* = 1.6, 12.8 Hz, 1H), 4.98 (dd, *J* = 1.6, 18.0 Hz, 1H), 4.55 (s, 2H), 3.50-3.30 (m, 2H), 2.40-2.25 (m, 1H), 2.05-1.75 (m, 3H), 1.55-1.00 (m, 14H), 0.95-0.75 (m, 12H), 0.02 (s, 3H), 0.01 (s, 3H).
**III-B7: ¹H NMR** (400 MHz, CDCl₃) δ_{H} 7.40-7.20 (m, 5H), 5.04 (s, 1H), 4.56 (s, 2H), 4.45-4.35 (m, 1H), 3.45-3.30 (m, 1H), 2.55-2.30 (m, 2H), 2.00-1.45 (m, 15H), 1.44-0.95 (m, 12H).

### Synthesis of III-B9

To a solution of **III-B8** (3.5 g, 6.7 mmol) in MeOH (30 mL) was added Pd/C (1 g, 10% Pd, 50% water). After stirring at 25°C under H₂ (103421 Pa = 15 psi) for 12 h, the reaction mixture was filtered through a pad of Celite and washed with MeOH (3 × 10 mL). The filtrate was concentrated to give **III-B9** (2.50 g, 86%) as an oil. **¹H NMR** (400 MHz, CDCl₃) δ_{H} 3.80-3.73 (m, 1H), 3.65-3.55 (m, 1H), 2.15-2.05 (m, 1H), 1.95-1.70 (m, 6H), 1.55-1.35 (m, 6H), 1.30-0.95 (m, 15H), 0.90-0.75 (m, 14H), 0.05 (s, 3H), 0.04 (s, 3H).

### Synthesis of III-B10

To a solution of **III-B9** (2.5 g, 5.8 mmol) in DCM (20 mL) was added PCC (2.47 g, 11.5 mmol) and silica gel (5 g) at 25°C. After stirring at 25°C for 1 h, the reaction mixture was filtered and the residue was washed with anhydrous DCM (2 × 20 mL). The combined filtrate was concentrated in vacuum to give **III-B10** (2.2 g, 88%) as a solid. **¹H NMR** (400 MHz, CDCl₃) δ_{H} 3.85-3.75 (m, 1H), 2.61 (t, *J* = 14.0 Hz, 1H), 2.30-2.10 (m, 6H), 1.95-1.83 (m, 1H), 1.78-1.60 (m, 2H), 1.50-1.25 (m, 7H), 1.25-0.95 (m, 11H), 0.90-0.80 (m, 13H), 0.05 (s, 3H), 0.04 (s, 3H).

### Synthesis of III-B11

To a stirred solution of Me₃SI (1.24 g, 6.1 mmol) in DMSO (10 mL) and THF (10 mL) was aded NaH (243 mg, 6.1 mmol, 60 % in oil) at 0°C. After stirring for 1 h under N₂, the mixture was added to a solution of **III-B10** (2.2 g, 5.1 mmol) in DMSO (10 mL). After stirring at 25°C for 16 h, the reaction mixture was poured into ice-water (100 mL) and extracted with EtOAc (2 × 50 mL). The combined organic solution was washed with water (2 × 50 mL) and brine (50 mL), dried over anhydrous Na₂SO₄, filtered, and concentrated to give **III-B11** (2 g) as an oil, which was used directly for the next step without further purification. **¹H NMR** (400 MHz, CDCl₃) δ_{H} 3.80-3.70 (m, 1H), 2.65-2.50 (m, 2H), 2.30-2.05 (m, 2H), 2.00-1.80 (m, 3H), 1.55-1.30 (m, 12H), 1.30-0.75 (m, 24H), 0.05 (s, 3H), 0.04 (s, 3H).

### Synthesis of III-B12

Fresh Na (1.02 g, 44.7 mmol) was carefully added to MeOH (44.7 mL) in portions. After stirring at 25°C for 3h, a solution of **III-B11** (2 g, 4.47 mmol) in anhydrous MeOH (30 mL). After stirring at 75°C for 16 h, the reaction was diluted with water (50 mL), concentrated to remove most of the solvent and extracted with EtOAc (2 × 50 mL). The combined organic solution was washed with saturated brine (100 mL), dried over anhydrous Na₂SO₄, filtered and concentrated. The residue was purified by silica gel chromatography (0-20% of EtOAc in PE) to give the product **III-B12** (2 g, 93%) as an oil.

### Synthesis of III-B13 & III-B14

To a solution of **III-B12** (2 g, 4.2 mmol) in THF (30 mL) was added TBAF.3H₂O (1.95 g, 6.3 mmol) at 25°C. After stirring at 55°C for 12 h, the mixture was poured into water (50 mL) and extracted with EtOAc (2 × 30 mL). The organic solution was washed with saturated brine (2 × 20 mL), dried over anhydrous Na₂SO₄, filtered and concentrated in vacuum. The residue was purified by silica gel chromatography (0-20% of EtOAc in PE) to give **III-B13** (620 mg, 42%) and **III-B14** (570 mg, 38%) both as oils.
**III-B13: ¹H NMR** (400 MHz, CDCl₃) δ_{H} 3.93-3.76 (m, 1H), 3.48-3.35 (m 5H), 2.58 (s, 1H), 2.17-2.08 (m, 1H), 1.93-1.60 (m, 8H), 1.55-1.02 (m, 19H), 1.00-0.78 (m, 5H); **LC-ELSD/MS** purity 99%, 100% de based on H-NMR. MS ESI calcd. for C₂₃H₃₇O [M-H₂O-H₂O+H]⁺329.3, found 329.3.
**III-B14: ¹H NMR** (400 MHz, CDCl₃) δ_{H} 3.93-3.76 (m, 1H), 3.39 (s, 3H), 3.20 (s, 2H), 3.18-3.10 (m 2H), 2.00 (s, 1H), 1.95-1.82 (m, 1H), 1.75-1.58 (m, 5H), 1.54-0.85 (m, 25H); **LC-ELSD/MS** purity 99%, 100% de based on H-NMR. MS ESI calcd. for C₂₃H₃₇O [M-H₂O-H₂O+H]⁺329.3, found 329.3.

### Synthesis of III-B15

To a solution of **III-B13** (600 mg, 1.6 mmol) in DCM (20 mL) were added silica gel (1.5 g) and PCC (1.05 g, 4.9 mmol) in portions at 25°C. After stirring at 25°C for 0.5 h, the mixture was filtered and the filter cake was washed with DCM (30 mL). The combined filtrate was concentrated and purified by silica gel chromatography (0-20% of EtOAc in PE) to give **III-B15** (550 mg, 93%) as an oil. **¹H NMR** (400 MHz, CDCl₃) δ_{H} 3.45-2.35 (m, 5H), 2.61 (s, 1H), 2.53-2.42 (m, 1H), 2.35-2.18 (m, 5H), 1.85-1.10 (m, 22H), 0.96-0.82 (m, 1H), 0.62 (t, *J* = 7.6 Hz, 3H); **LC-ELSD/MS** purity 99%, MS ESI calcd. for C₂₃H₃₇O₂ [M-H₂O+H]⁺345.3, found 345.3.

### EXAMPLE III-10: Synthesis of 1-(2-((3R,5R,8R,9R,10S,13S,14S,17S)-13-ethyl-3-hydroxy-3-(methoxymethyl)hexadecahydro-1H-cyclopenta[a]phenanthren-17-yl)-2-oxoethyl)-1H-pyrazole-4-carbonitrile (III-B17)

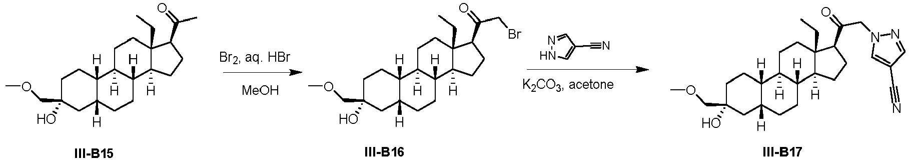

### Synthesis of III-B16

To a solution of **III-B15** (80 mg, 0.22 mmol) in MeOH (5 mL) was added HBr (8.80 mg, 0.044 mmol) and Br₂ (35.2 mg, 0.22 mmol) at 25°C. After stirring for 2 h at 25°C, the reaction mixture was added into saturated NaHCO₃ (10 mL) and extracted with EtOAc (3 x 10 mL). The combined organic solution was washed with saturated brine (20 mL), dried over anhydrous Na₂SO₄, filtered and concentrated to give **III-B16** (90 mg) as an oil, which was used directly to the next step without further purification.

### Synthesis of III-B17

To a solution of **III-B16** (90 mg, 0.2 mmol) and 1H-pyrazole-4-carbonitrile (37.9 mg, 0.4 mmol) in acetone (5 mL) was added K₂CO₃ (56.2 mg, 0.4 mmol). After stirring at 25°C for 2 h, the reaction mixture was extracted with EtOAc (3 × 30 mL). The combined organic solution was washed with saturated brine (30 mL), dried over anhydrous Na₂SO₄, filtered and concentrated. The residue was purified by flash column (0-30% of EtOAc in PE) and then by
Prep-HPLC (Column: Waters Xbridge Prep OBD C18 150*30 5 µm; Condition: water (10 mM NH₄HCO₃)-ACN; Begin B: 70; End B: 90; Gradient Time(min): 7; 100%B Hold Time(min): 1) to give **III-B17** (32.6 mg, 53%) as a solid. **¹H NMR** (400 MHz, CDCl₃) δ_{H} 7.86 (s, 1H), 7.81 (s, 1H), 5.04 (dd, *J* = 18.0, 95.6 Hz, 2H), 3.45-3.33 (m, 5H), 2.50 (t, *J* = 8.8 Hz, 1H), 2.40-2.23 (m, 2H), 1.88-1.10 (m, 23H), 1.00-0.83 (m, 1H), 0.60 (t, *J* = 7.6 Hz, 3H); **LC-ELSD/MS** purity 99%, MS ESI calcd. for C₂₇H₃₈N₃O₂ [M-H₂O+H]⁺436.3, found 436.3.

### EXAMPLE III-11: Synthesis of 1-((3R,5R,8R,9R,10S,13R,14S,17S)-3-hydroxy-13-(methoxymethyl)-3-methylhexadecahydro-1H-cyclopenta[a]phenanthren-17-yl)ethan-1-one (III-C11)

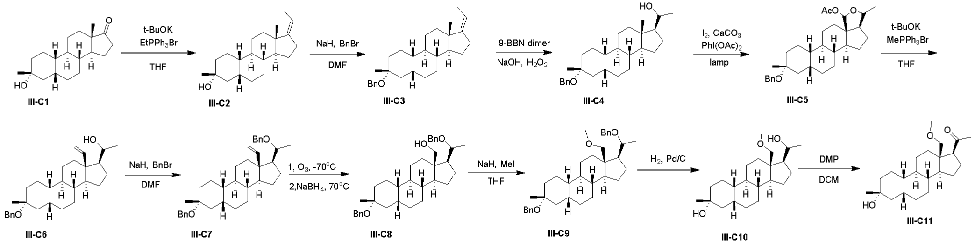

### Synthesis of III-C2

To a mixture of EtPh₃PBr (63.4 g, 171 mmol) in THF (400 mL) was added t-BuOK (19.1 g, 171 mmol) at 25°C. After stirring at 50°C for 30 min, **III-C1** (20 g, 68.8 mmol) was added. After stirring at 50°C for 2 h, the mixture was added into saturated NH₄Cl (400 mL) and extracted with EtOAc (3 × 200 mL). The combined organic solution was washed with saturated brine (500 mL), dried over anhydrous Na₂SO₄, filtered and concentrated. The residue was triturated from MeOH/water (700 mL, 1:1) to give **III-C2** (25 g, ) as an oil. **¹H NMR** (400 MHz, CDCl₃) δ_{H} 5.25-5.05 (m, 1H), 2.42-2.10 (m, 3H), 1.92-1.55 (m, 7H), 1.53-1.25 (m, 14H), 1.24-1.06 (m, 6H), 0.87 (s, 3H).

### Synthesis of III-C3

To a mixture of **III-C2** (25 g, 82.6 mmol) in DMF (300 mL) was added NaH (8.23 g, 206 mmol, 60%) at 0°C. After stirring at 25°C for 1h, BnBr (35.2 g, 206 mmol) was added. After stirring at 60°C for 20 h, the mixture was added into water (500 mL) and extracted with EtOAc (3 × 200 mL). The combined organic solution was washed with LiCl (2 × 500 mL. 4% in water), saturated brine (500 mL), dried over anhydrous Na₂SO₄, filtered and concentrated. The residue was purified by flash column (100% of PE) to give **III-C3** (30 g, 93%) as a solid.
**¹H NMR** (400 MHz, CDCl₃) δ_{H} 7.40-7.27 (m, 5H), 5.15-5.05 (m, 1H), 4.50 (s, 2H), 2.40-2.10 (m, 3H), 1.99-1.59 (m, 9H), 1.52-1.34 (m, 6H), 1.33 (s, 3H), 1.32-1.05 (m, 8H), 0.87 (s, 3H).

### Synthesis of III-C4

To a solution of **III-C3** (28 g, 71.3 mmol) in THF (300 mL) was added 9-BBN dimer (34.3 g, 142 mmol) at 0°C. After stirring at 25°C for 1 h, ethanol (200 mL) at 25°C and then NaOH aqueous (142 mL, 5 M, 713 mmol) was added at 0°C followed by H₂O₂ (121 g, 30%, 1069 mmol) dropwise. After stirring at 70°C for 1 h, the reaction was diluted with water (300 mL) and stirred at 25°C for 1 h. The solid was filtered and washed with water (2 × 300 mL), dried under vacuum to give **III-C4** (19.1 g, 65%) as a solid. **¹H NMR** (400 MHz, CDCl₃) δ_{H} 7.45-7.28 (m, 5H), 4.50 (s, 2H), 3.75-3.62 (m, 1H), 2.00-1.59 (m, 12H), 1.52-1.32 (m, 10H), 1.31-1.00 (m, 9H), 0.67 (s, 3H).

### Synthesis of III-C5

To a solution of **III-C4** (3 g, 7.3 mmol) in cyclohexane (300 mL) at 25°C under N₂ were added CaCO₃ (2.19 g, 21.9 mmol), PhI(OAc)₂ (7.05 g, 21.9 mmol), I₂ (3.70 g, 14.6 mmol). After heating to reflux (80°C) by irradiated with infrared lamp (250 W) for 30 min, the mixture was added into saturated Na₂S₂O₃ (500 mL) and extracted with EtOAc (3 × 100 mL). The combined organic solution was washed with saturated brine (500 mL), dried over anhydrous Na₂SO₄, filtered and concentrated. The residue was purified by flash column (0-5% of EtOAc in PE) to give **III-C5** (4.0 g) as an oil. **¹H NMR** (400 MHz, CDCl₃) δ_{H} 7.40-7.20 (m, 4H), 7.25-7.20 (m, 1H), 6.00 (s, 1H), 5.07 (s, 1H), 4.50 (s, 2H), 4.40-4.25 (m, 2H), 2.20-2.08 (m, 1H), 2.00-1.65 (m, 13H), 1.50-1.40 (m, 10H), 1.25-0.95 (m, 5H), 0.85-0.70 (m, 2H).

### Synthesis of III-C6

To a solution of MePh₃PBr (30.5 g, 85.7 mmol) in THF (200 mL) was added t-BuOK (9.59 g, 85.7 mmol) at 25°C. After stirring at 50°C for 1h, a solution of **III-C5** (4 g, 8.6 mmol) in THF (20 mL) was added. After stirring at 50°C for 16 h, the mixture was added into saturated NH₄Cl (200 mL) and extracted with EtOAc (3 × 100 mL). The combined organic solution was washed with saturated brine (200 mL), dried over anhydrous Na₂SO₄, filtered and concentrated. The residue was triturated from MeOH/water (400 mL, 1:1) at 25°C to give **III-C6** (3.6 g) as an oil. **¹H NMR** (400 MHz, CDCl₃) δ_{H} 7.42-7.27 (m, 5H), 5.85-5.70 (m, 1H), 5.35-5.25 (m, 1H), 5.20-5.10 (m, 1H), 4.50 (s, 2H), 3.90-3.75 (m, 1H), 2.40-2.30 (m, 1H), 1.95-1.60 (m, 9H), 1.55-1.30 (m, 14H), 1.28-0.90 (m, 7H).

### Synthesis of III-C7

To a solution of **III-C6** (3.6 g, 8.5 mmol) in DMF (20 mL) was added NaH (1.70 g, 42.5 mmol, 60% in oil) at 25°C. After stirring at 25°C for 1h, BnBr (4.36 g, 25.5 mmol) was added. After stirring at 60°C for 16 h, the mixture was added into water (200 mL) and extracted with EtOAc (3 × 50 mL). The combined organic solution was washed with LiCl (2 × 100 mL, 4% in water), saturated brine (100 mL), dried over anhydrous Na₂SO₄, filtered and concentrated. The residue was purified by flash column (0-2% of EtOAc in PE) to give **III-**C7 (1 g, 23%) as an oil. **¹H NMR** (400 MHz, CDCl₃) δ_{H} 7.45-7.28 (m, 10H), 5.70-5.55 (m, 1H), 5.25-5.15 (m, 1H), 5.05-4.95 (m, 1H), 4.57-4.45 (m, 3H), 4.33-4.25 (m, 1H), 3.27-3.15 (m, 1H), 2.41-2.32 (m, 1H), 2.15-2.05 (m, 1H), 1.95-1.59 (m, 7H), 1.52-1.25 (m, 11H), 1.20-0.80 (m, 10H).

### Synthesis of III-C8

To a solution **of III-C7** (1 g, 1.9 mmol) in DCM/MeOH (10 mL/10 mL) were added NaHCO₃ (1 g, 11.9 mmol) at 25°C. After stirring under O₃ (1 atm) for 20 minutes at - 70°C, NaBH₄ (296 mg, 7.8 mmol) was added to the mixture at 0°C. After stirring for 1h at 70°C, the mixture was added into saturated NH₄Cl (100 mL) and extracted with EtOAc (3 × 50 mL). The combined organic solution was washed with saturated brine (100 mL), dried over anhydrous Na₂SO₄, filtered and concentrated to give **III-C8** (300 mg, ) as an oil, which was used for next step directly.

### Synthesis of III-C9

To a solution of **III-C8** (300 mg, 0.58 mmol) in THF (5 mL) was added NaH (92.6 mg, 2.3 mmol, 60%) at 0°C. After stirring at 25°C for 1h, MeI (329 mg, 2.3 mmol) was added. After stirring at 25°C for 1h, the mixture was added into saturated NH₄Cl (100 mL) and extracted with EtOAc (3 × 50 mL). The combined organic solution was washed with saturated brine (100 mL), dried over anhydrous Na₂SO₄, filtered and concentrated. The residue was purified by flash column (0-2% of EtOAc in PE) to give **III-C9** (200 mg) as an oil. **¹H NMR** (400 MHz, CDCl₃) δ_{H} 7.45-7.28 (m, 8H), 7.25-7.21 (m, 2H), 4.65-4.55 (m, 1H), 4.50 (s, 2H), 4.40-4.30 (m, 1H), 3.55-3.43 (m, 1H), 3.40-3.30 (m, 1H), 3.21 (s, 3H), 3.15-3.05 (m, 1H), 2.40-2.30 (m, 1H), 2.20-1.59 (m, 11H), 1.52-1.20 (m, 11H), 1.18-0.80 (m, 7H).

### Synthesis of III-C10

To a solution of **III-C9** (200 mg, 0.38 mmol) in MeOH (5 mL) was added Pd/C (50 mg, 10% in water) at 25°C. After stirring at rt for 16 h under H₂ (344738 Pa = 50 psi), the mixture was filtered and the mother liquor was concentrated. The residue was purified by flash column (0-30% of EtOAc in PE) to give **III-C10** (50 mg, 38%) as an oil. **¹H NMR** (400 MHz, CDCl₃) δ_{H} 4.20-4.11 (m, 1H), 4.10-4.00 (m, 1H), 3.40-3.25 (m, 5H), 2.35-2.25 (m, 1H), 1.95-1.80 (m, 4H), 1.78-1.59 (m, 4H), 1.52-1.35 (m, 5H), 1.33-1.23 (m, 7H), 1.30-1.00 (m, 6H), 0.98-0.80 (m, 4H).

### Synthesis of III-C11

A solution of **III-C10** (50 mg, 0.14 mmol) and DMP (120 mg, 0.29 mmol) in DCM (2 mL) was stirred at 25°C for 1h. The mixture was added into saturated NaHCO₃ (50 mL) and extracted with DCM (3 × 20 mL). The combined organic solution was washed with saturated Na₂S₂O₃ (2 × 50 mL), saturated brine (50 mL), dried over anhydrous Na₂SO₄, filtered and concentrated. The residue was purified by flash column (0-30% of EtOAc in PE) to give **III-C11** (30 mg, 60%) as a solid. **¹H NMR** (400 MHz, CDCl₃) δ_{H} 3.48-3.40 (m, 1H), 3.09 (s, 3H), 2.88-2.80 (m, 1H), 2.65-2.55 (m, 1H), 2.50-2.40 (m, 1H), 2.38-2.28 (m, 1H), 2.15 (s, 3H), 1.95-1.59 (m, 7H), 1.52-1.25 (m, 13H), 1.24-1.00 (m, 5H); **LC-ELSD/MS** purity 99%, MS ESI calcd. for C₂₂H₃₇O₃ [M+H]⁺ 349.3, found 349.3.

### EXAMPLE III-12: Synthesis of 1-(2-((3R,5R,8R,9R,10S,13R,14S,17S)-3-hydroxy-13-(methoxymethyl)-3-methylhexadecahydro-1H-cyclopenta[a]phenanthren-17-yl)-2-oxoethyl)-1H-pyrazole-4-carbonitrile (III-C13)

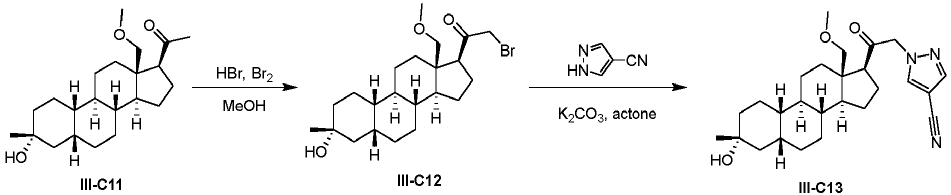

### Synthesis of III-C12

To a solution of **III-C11** (25 mg, 0.072 mmol) in MeOH (5 mL) were added HBr (2 mg, 40%) at 25°C and then Br₂ (11.4 mg, 0.072 mmol) at 0°C. After stirring at 25°C for 5 h, the mixture was added into saturated NaHCO₃ (50 mL) and extracted with EtOAc (3 × 20 mL). The combined organic solution was washed with saturated brine (50 mL), dried over anhydrous Na₂SO₄, filtered and concentrated to give **III-C12** (40 mg) as an oil **¹H NMR** (400 MHz, CDCl₃) δ_{H} 4.05-3.95 (m, 2H), 3.55-3.45 (m, 1H), 3.06 (s, 3H), 2.85-2.75 (m, 2H), 2.52-2.38 (m, 2H), 1.92-1.59 (m, 5H), 1.52-1.30 (m, 12H), 1.27 (s, 3H), 1.25-0.80 (m, 5H).

### Synthesis of III-C13

A solution of **III-C12** (40 mg, 0.094 mmol), K₂CO₃ (17.4 mg, 0.19 mmol) and 1H-pyrazole-4-carbonitrile (25.8 mg, 0.19 mmol) was stirred at 25°C for 2 hours. The mixture was added into water (100 mL) and extracted with EtOAc (3 × 20 mL). The combined organic solution was washed with water (2 × 100 mL), saturated brine (100 mL), dried over anhydrous Na₂SO₄, filtered and concentrated. The residue was purified by flash column (0-50% of EtOAc in PE) to give **III-C13** (5 mg, 12%) as a solid. **¹H NMR** (400 MHz, CDCl₃) δ_{H} 7.82 (s, 1H), 7.80 (s, 1H), 5.28-5.20 (m, 1H), 4.92-4.85 (m, 1H), 3.55-3.45 (m, 1H), 3.02 (s, 3H), 2.92-2.82 (m, 1H), 2.65-2.35 (m, 3H), 1.90-1.59 (m, 7H), 1.52-1.30 (m, 11H), 1.28 (s, 3H), 1.25-1.02 (m, 4H); **LC-ELSD/MS** purity 99%, MS ESI calcd. for C₂₆H₃₈N₃O₃ [M+H]⁺ 440.3, found 440.3.

### EXAMPLE III-13: Synthesis of 1-((3R,5R,8R,9R,10S,13R,14S,17S)-3-hydroxy-13-isobutyl-3-(methoxymethyl)hexadecahydro-1H-cyclopenta[a]phenanthren-17-yl)ethan-1-one (III-D20)

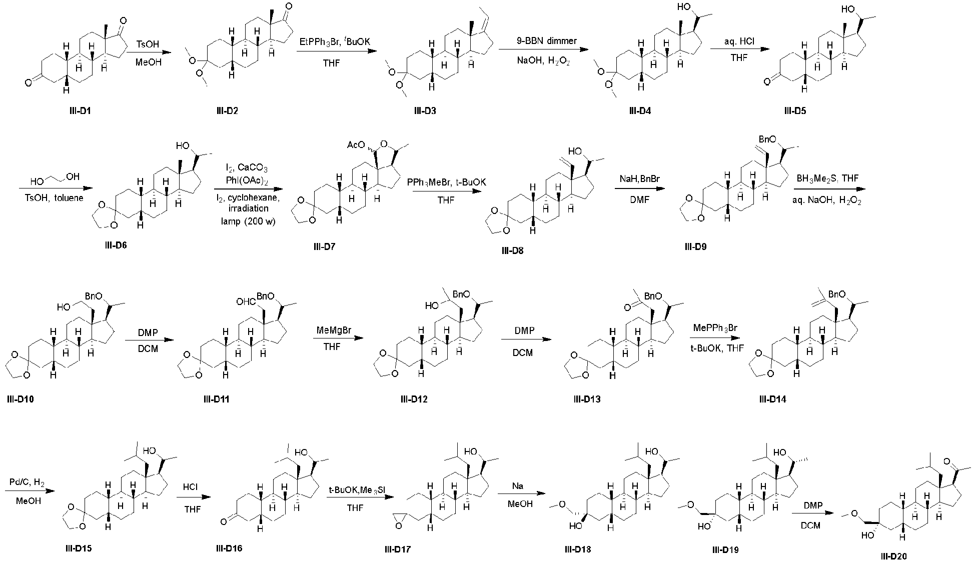

### Synthesis of III-D2

To a solution of **III-D1** (300 g, 1093 mmol) in MeOH (2 L) was added 4-methylbenzenesulfonic acid (18.7 g, 109 mmol) at 25°C. After stirring at 65°C for 1h, the reaction mixture was cooled, and the precipitate was collected by filtration and washed with methanol (2 × 300 mL) to give **III-D2** (230 g) as a solid. **¹H NMR** (400 MHz, CDCl₃) δ_{H} 3.19 (s, 3H), 3.14 (s, 3H), 2.60-2.39 (m, 2H), 2.25-2.00 (m, 2H), 1.97-1.90 (m, 2H), 1.86-1.75 (m, 6H), 1.70-1.60 (m, 5H), 1.56-1.49 (m, 4H), 1.47-1.35 (m, 10H), 1.30-1.22 (m, 5H), 1.15-1.00 (m, 2H), 0.86 (s, 3H).

### Synthesis of III-D3

To a suspension of EtPPh₃Br (798 g, 2.15 mol) in THF (1.5 L) was added t-BuOK (241 g, 2.15 mol) at 25°C under N₂. After stirring at 50°C for 30 min, a solution of **III-D2** (230 g, 717 mmol) in THF (500 mL) was added at 50°C. After stirring at 50°C for 16 h, the reaction was cooled to 25°C, diluted with sat NH₄Cl (500 mL) and extracted with EtOAc (2 × 500 mL). The combined organic solution was washed with brine (2 × 500 mL), dried over anhydrous Na₂SO₄, filtered and concentrated. The residue was triturated from methanol (1 L) and water (1 L) to yield **III-D3** (290 g) as a solid. **¹H NMR** (400 MHz, CDCl₃) δ_{H} 3.19 (s, 3H), 3.14 (s, 3H), 2.40-2.10 (m, 4H), 1.95-1.35 (m, 13H), 1.33-1.05 (m, 10H), 0.87 (s, 3H).

### Synthesis of III-D4

To a solution of **III-D3** (275 g, 826 mmol) in THF (2 L) was added 9-BBN dimer (402 g, 1.65 mol) at 25°C. After stirring at 50°C for 2 h, the reaction was cooled to 0°C and ethanol (379 g, 8.26mol) and NaOH (1.65 L, 5 M, 8.26mol) were added very slowly. After the addition was complete, H₂O₂ (825 mL, 8.26 mol, 30%) was added slowly maintaining the inner temperature below 15°C. After stirring at 75°C for 1 h, the reaction was quenched with saturated aqueous Na₂S₂O₃ (260 mL) and stirred at 0°C for 1 h. The mixture was diluted with water (2 L) and filtered. The filter cake was washed with water (3 × 700 mL), dried under vacuum to give **III-D4** (285 g) as a solid. **¹H NMR** (400 MHz, CDCl₃) δ_{H} 3.17-3.09 (m, 6H), 1.96-1.77 (m, 8H), 1.64-1.29 (m, 11H), 1.24-0.91 (m, 10H), 0.63 (s, 3H).

### Synthesis of III-D5

To a solution of **III-D4** (285 g, 813 mmol) in THF (3 L) was added aq HCl (1.62 L, 1.62 mol, 1 M) at 20°C. After stirring for 1 h, the mixture was diluted with water (700 mL) and extracted with DCM (2 × 500 mL). The combined organic solution was washed with brine (2 × 500 mL), dried over anhydrous Na₂SO₄, filtered, concentrated to afford **III-D5** (280 g) as a solid. **¹H NMR** (400 MHz, CDCl₃) δ_{H} 3.75-3.65 (m, 1H), 2.65-2.55 (m, 1H), 2.30-2.10 (m, 1H), 2.00-1.80 (m, 5H), 1.75-1.42 (m, 10H), 1.40-1.28 (m, 4H), 1.29-1.15 (m, 7H), 0.66 (s, 3H).

### Synthesis of III-D6

To a solution **of III-D6** (14 g, 45.9 mmol) toluene (200 mL) were added, TsOH (787 mg, 4.6 mmol) and ethane-1,2-diol (28.4 g, 458 mol) at 25°C under N₂. After stirring at 120°C for 4 h, the mixture was poured into water (100 mL) and extracted with ethyl acetate (2 × 100 mL). The combined organic solution was washed with brine (100 mL), dried over Na₂SO₄, filtered and concentrated in vacuum. The residue was purified by flash chromatography (5-10% EtOAc in PE) to give **III-D6** (8 g, 50%) as a solid. **¹H NMR** (400 MHz, CDCl₃) δ_{H} 3.93 (s, 4H), 3.76-3.63 (m, 1H), 2.03-1.95 (m, 1H), 1.93-1.80 (m, 4H), 1.64-1.58 (m, 3H), 1.56-1.48 (m, 2H), 1.44-1.30 (s, 6H), 1.27-0.95 (m, 12H), 0.66 (s, 3H).

### Synthesis of III-D7

To a solution **of III-D6** (2.0 g, 5.7 mmol), PhI(OAc)₂ (5.50 g, 17.1 mmol) and CaCO₃ (1.71 g, 17.1 mmol) in cyclohexane (200 mL) was added I₂ (2.89 g, 11.4 mmol) at 25°C. The mixture was heated to 80°C by irradiation with infrared lamp (200 W) for 10 minutes. The mixture was purified together with another similar reaction (2 g). The mixture was added into saturated Na₂S₂O₃ (1000 mL) and extracted with EtOAc (3 × 300 mL). The combined organic solution was washed with saturated brine (1000 mL), dried over anhydrous Na₂SO₄, filtered and concentrated to give **III-D7** (4.6 g) as an oil. **¹H NMR** (400 MHz, CDCl₃) δ_{H} 5.95 (s, 1H), 4.35-4.40 (m, 1H), 3.92 (s, 4H), 2.40-2.30 (m, 1H), 2.25-2.05 (m, 3H), 2.00-1.70 (m, 12H), 1.50-1.35 (m, 7H), 1.30-1.00 (m, 6H), 0.90-0.70 (m, 1H).

### Synthesis of III-D8

To a solution of PPh₃MeBr (20.2 g, 56.8 mmol) and t-BuOK (6.36 g, 56.8 mmol) in THF (30 mL) stirred for 1h at 50°C was added a solution of **III-D7** (2.3 g, 5.7 mmol) in THF (20 mL). After stirring for 12 hours at 50°C, the solution was purified together with another batch (from 2.3 g). The mixture was added into saturated NH₄Cl (200 mL) and extracted with EtOAc (3 × 50 mL). The combined organic solution was washed with saturated brine (100 mL), dried over anhydrous Na₂SO₄, filtered and concentrated. The residue was purified by silica gel chromatography (0-20% of EtOAc in PE) to give **III-D8** (2.3 g) as an oil. **¹H NMR** (400 MHz, CDCl₃) δ_{H} 5.77 (dd, *J* = 11.2,18.0 Hz, 1H), 5.28 (d, *J* = 10.8 Hz, 1H), 5.14 (d, *J* = 18.0 Hz, 1H), 3.93 (s, 4H), 3.90-3.85 (m, 1H), 2.40-2.30 (m, 1H), 2.00-1.90 (m, 1H), 1.90-1.65 (m, 6H), 1.60-1.50 (m, 3H), 1.50-1.40 (m, 1H), 1.35-0.80 (m, 16H).

### Synthesis of III-D9

To a solution of **III-D8** (2.0 g, 5.5 mmol) in DMF (20 mL) was added NaH (438 mg, 11.0 mmol, 60% in mineral oil) at 0°C. After stirring at 20°C for 1 h, BnBr (2.36 g, 13.8 mmol) was added into the mixture at 20°C. After stirring at 60°C for 10 h, the mixture was poured into water (50 mL) and extracted with EtOAc (2 × 50 mL). The combined organic solution was washed with brine (3 × 50 mL), dried over anhydrous Na₂SO₄, filtered and concentrated. The residue was purified by flash column (0-40% of EtOAc in PE) to give **III-D9** (2.0 g, 80.3%) as an oil. **¹H NMR** (400 MHz, CDCl₃) δ_{H} 7.34-7.25 (m, 5H), 5.66-5.56 (m, 1H), 5.27-5.17 (m, 1H), 5.08-5.01 (m, 1H), 4.57-4.26 (m, 2H), 3.93 (s, 4H), 3.27-3.14 (m, 1H), 2.39-2.34 (m, 1H), 1.96 (s, 2H), 1.90-1.78 (m, 3H), 1.61-1.54 (m, 4H), 1.48-1.34 (m, 10H), 1.20-1.14 (m, 5H), 1.06-0.99 (m, 2H).

### Synthesis of III-D10

To a solution of **III-D9** (2.0 g, 4.4 mmol) in THF (20 mL) was added BH₃.Me₂S (2.21 mL, 10 M, 22.1 mmol). After stirring at 25°C for 16 h, EtOH (2.55 mL, 44.3 mmol) followed by aq. NaOH (1.77 g in 8.86 mL water, 44.3 mmol) and aq. H₂O₂ (4.43 mL, 10 M, 44.3 mmol) were added slowly. After stirring at 70°C for 1 h, the mixture was quenched by Na₂SO₃ (80 mL, 10%) and extracted with EtOAc (2 × 100 mL). The combined organic solution was dried over Na₂SO₄, filtered and concentrated and purified by flash column (0~30% of EtOAc in PE) to give **III-D10** (1.4 g, 67.6%) as a solid. **¹H** NMR (400 MHz, CDCl₃) δ 7.37-7.31 (m, 4H), 7.28-7.24 (m, 1H), 4.66 (d, *J*=11.2 Hz, 1H), 4.32 (d, *J*=11.2 Hz, 1H), 3.93 (s, 4H), 3.68-3.46 (m, 3H), 2.02-1.74 (m, 6H), 1.70-1.57 (s, 5H), 1.52-1.33 (m, 9H), 1.28-1.23 (m, 2H), 1.21-1.03 (m, 5H), 1.02-0.92 (m, 3H)

### Synthesis of III-D11

To a solution of **III-D10** (1.4 g, 2.98 mmol) in DCM (50 mL) was added DMP (2.52 g, 5.96 mmol). After stirring at 20°C for 1h, the mixture was added into saturated NaHCO₃ (200 mL). and extracted with DCM (3 × 50 mL). The combined organic solution was washed with saturated Na₂S₂O₃ (2 × 200 mL), saturated brine (200 mL), dried over anhydrous Na₂SO₄, filtered and concentrated to give **III-D11** (1.4 g) as an oil. **¹H NMR** (400 MHz, CDCl₃) δ_{H} 9.66 (s, 1H), 7.40-7.27 (m, 4H), 7.25-7.15 (m, 1H), 4.56 (d, *J* = 11.6 Hz, 1H), 4.23 (d, *J* = 11.6 Hz, 1H), 3.93 (s, 4H), 3.80-3.70 (m, 1H), 2.55-2.40 (m, 2H), 2.30-2.20 (m, 1H), 2.10-1.60 (m, 9H), 1.52-1.25 (m, 8H), 1.22 (d, *J* = 6.0 Hz, 3H), 1.20-0.92 (m, 6H).

### Synthesis of III-D12

To a solution of **III-D11** (1.4 g, 3.0 mmol) in THF (20 mL) was added MeMgBr (5 mL, 3 M in Et₂O, 15.0 mmol) at 0°C. After stirring at 20°C for 1h, the mixture was added into saturated NH₄Cl (200 mL) and extracted with EtOAc (3 × 50 mL). The combined organic solution was washed with saturated brine (200 mL), dried over anhydrous Na₂SO₄, filtered and concentrated to give **III-D12** (1.3 g) as an oil. **¹H NMR** (400 MHz, CDCl₃) δ_{H} 7.35-7.28 (m, 4H), 7.25-7.16 (m, 1H), 4.61 (d, *J* = 12.0 Hz, 1H), 4.34 (d, *J* = 11.6 Hz, 1H), 3.93 (s, 4H), 3.91-3.75 (m, 1H), 3.54-3.40 (m, 1H), 2.40-2.25 (m, 1H), 2.00-1.60 (m, 8H), 1.52-1.20 (m, 14H), 1.18-0.80 (m, 10H).

### Synthesis of III-D13

To a solution of **III-D12** (1.8 g, 3.7 mmol) in DCM (40 mL) was added Dess-Martin periodinane (DMP) (3.15 g, 7.4 mmol). After stirring at 20°C for 1h, the mixture was added into saturated NaHCO₃ (300 mL) and extracted with DCM (3 × 50 mL). The combined organic solution was washed with saturated Na₂S₂O₃ (2 × 300 mL), saturated brine (300 mL), dried over anhydrous Na₂SO₄, filtered and concentrated to give **III-D13** (1.8 g) as an oil. **¹H NMR** (400 MHz, CDCl₃) δ_{H} 7.40-7.28 (m, 4H), 7.25-7.16 (m, 1H), 4.51 (d, *J* = 12.0 Hz, 1H), 4.11 (d, *J* = 12.0 Hz, 1H), 3.93 (s, 4H), 3.13-3.02 (m, 1H), 2.44 (d, *J* = 18.4 Hz, 1H), 2.29 (d, *J* = 19.2 Hz, 1H), 2.10-1.78 (m, 8H), 1.76 (s, 3H), 1.52-1.11 (m, 15H), 1.10-0.75 (m, 4H).

To a solution of MePh₃PBr (6.67 g, 18.7 mmol) in THF (30 mL) was added t-BuOK (2.09 g, 18.7 mmol) at 20°C. After stirring at 50°C for 1h, a solution of **III-D13** (1.8 g, 3.7 mmol) in THF (20 mL) was added to the reaction below 50°C. After stirring at 50°C for 16 h, the mixture was added into saturated NH₄Cl (200 mL) and extracted with EtOAc (3 × 50 mL). The combined organic solution was washed with saturated brine (200 mL), dried over anhydrous Na₂SO₄, filtered and concentrated. The residue was purified by flash column (0-5% of EtOAc in PE) to give **III-D14** (1.2 g, 67%) as an oil. **¹H NMR** (400 MHz, CDCl₃) δ_{H} 7.40-7.28 (m, 4H), 7.25-7.16 (m, 1H), 4.77 (s, 1H), 4.71 (s, 1H), 4.57 (d, *J* = 11.6 Hz, 1H), 4.34 (d, *J* = 11.2 Hz, 1H), 3.93 (s, 4H), 3.90-3.75 (m, 1H), 2.36 (d, *J* = 11.6 Hz, 1H), 2.20-1.80 (m, 9H), 1.78-1.59 (m, 4H), 1.52-1.25 (m, 9H), 1.23-1.19 (m, 3H), 1.15-0.80 (m, 6H).

### Synthesis of III-D15 & III-D15a

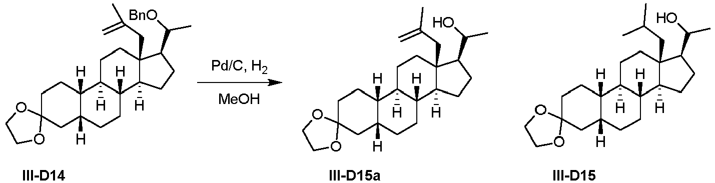

To a solution of **III-D14** (1.2 g, 2.5 mmol) in MeOH (20 mL) was added Pd/C (200 mg, 10% in water) at 25°C. After stirring at 25°C for 16 h under H₂ (344738 Pa = 50 psi), the mixture was filtered and the mother liquor was concentrated. The residue was purified by flash column (0-10% of EtOAc in PE) to give **III-D15a** (400 mg, 41%) and **III-D15** (300 mg, 31%) both as oils.

To a solution of **III-15a** (400 mg, 1.0 mmol) in MeOH (10 mL) was added Pd/C (100 mg, 10% in water) at 25°C. After stirring at 50°C for 16 h under H2 (344738 Pa = 50 psi), the mixture was filtered and the filter cake was washed with MeOH (3 × 30 mL). The mother liquor was concentrated to give **III-D15** (300 mg) as an oil. ¹H **NMR** (400 MHz, CDCl₃) δ_{H} 3.93 (s, 4H), 3.84-3.71 (m, 1H), 2.25-2.15 (m, 1H), 2.08-1.60 (m, 8H), 1.52-1.33 (m, 9H), 1.31 (d, *J* = 6.0 Hz, 3H), 1.28-1.02 (m, 10H), 0.97 (d, *J* = 6.4 Hz, 3H), 0.92 (d, *J* = 6.8 Hz, 3H).

### Synthesis of III-D16

A solution of **III-D15** (600 mg, 1.5 mmol) and aq. HCl (3 mL, 2 M, 6.0 mmol) in THF (5 mL) was stirred at 20°C for 1h. The mixture was added into saturated NaHCO₃ (150 mL) and extracted with EtOAc (3 × 50 mL). The combined organic solution was washed with saturated brine (150 mL), dried over anhydrous Na₂SO₄, filtered and concentrated to give **III-D16** (500 mg) as an oil. **¹H NMR** (400 MHz, CDCl₃) δ_{H} 3.90-3.75 (m, 1H), 2.59 (t, *J* = 14.0 Hz, 1H), 2.35-2.05 (m, 6H), 2.00-1.60 (m, 5H), 1.50-1.35 (m, 4H), 1.32 (d, *J* = 5.6 Hz, 3H), 1.30-1.03 (m, 12H), 1.00 (d, *J* = 6.4 Hz, 3H), 0.94 (d, *J* = 6.8 Hz, 3H).

### Synthesis of III-D17

To a solution of t-BuOK (581 mg, 5.2 mmol) and Me₃SI (1.05 g, 5.2 mmol) in THF (15 mL) was added **III-D16** (600 mg, 1.7 mmol). After stirring at 20°C for 1h, the reaction was diluted with water (200 mL) and extracted with EtOAc (3 × 50 mL). The combined organic solution was washed with saturated brine (200 mL), dried over anhydrous Na₂SO₄, filtered and concentrated to give **III-D17** (540 mg) as an oil. **¹H NMR** (400 MHz, CDCl₃) δ_{H} 3.85-3.70 (m, 1H), 2.65-2.55 (m, 2H), 2.30-2.15 (m, 2H), 2.03-1.59 (m, 9H), 1.52-1.35 (m, 5H), 1.32 (d, *J* = 6.0 Hz, 3H), 1.30-1.02 (m, 10H), 0.99 (d, *J* = 6.4 Hz, 3H), 0.93 (d, *J* = 6.8 Hz, 3H), 0.91-0.80 (m,2H).

### Synthesis of III-D18 & III-D19

Na (716 mg, 29.8 mmol) was added into MeOH (30 mL) at 20°C. After stirring at 70°C for 3 h, a solution of **III-D17** (540 mg, 1.5 mmol) in MeOH (10 mL) was added. After stirring at 70°C for 5 h, the mixture was added into saturated NH₄Cl (150 mL) and extracted with EtOAc (3 × 50 mL). The combined organic solution was washed with saturated brine (150 mL), dried over anhydrous Na₂SO₄, filtered and concentrated. The residue was purified by flash column (0-25% of EtOAc in PE) to give **III-D18** (150 mg, 26%) and **III-D19** (250 mg, 43%, ) both as oils. The stereochemistry of C3 was assigned based on H-NMR.
**III-D18: ¹H NMR** (400 MHz, CDCl₃) δ_{H} 3.90-3.75 (m, 1H), 3.39 (s, 3H), 3.20 (s, 2H), 2.25-2.10 (m, 2H), 1.99 (s, 1H), 1.98-1.58 (m, 8H), 1.52-1.34 (m, 6H), 1.31 (d, *J* = 6.0 Hz, 3H), 1.28-1.02 (m, 12H), 0.98 (d, *J* = 6.4 Hz, 3H), 0.92 (d, *J* = 6.8 Hz, 3H).
**III-D19: ¹H NMR** (400 MHz, CDCl₃) δ_{H} 3.88-3.72 (m, 1H), 3.48-3.30 (m, 5H), 2.57 (s, 1H), 2.19 (d, *J* = 9.2 Hz, 1H), 2.00-1.60 (m, 8H), 1.50-1.34 (m, 6H), 1.31 (d, *J* = 5.6 Hz, 3H), 1.28-1.02 (m, 12H), 0.98 (d, *J* = 6.4 Hz, 3H), 0.92 (d, *J* = 6.4 Hz, 3H), 0.90-0.85 (m, 1H); **LC-ELSD/MS** purity 99%, 100% de based on H-NMR; MS ESI calcd. for C₂₅H₄₁O [M-2H₂O+H]⁺ 357.3, found 357.3.

### Synthesis of III-D20

To a solution of **III-D19** (230 mg, 0.59 mmol) in DCM (10 mL) was added DMP (496 mg, 1.2 mmol). After stirring at 20°C for 1h, the mixture was added into saturated NaHCO₃ (100 mL) and extracted with DCM (3 × 50 mL). The combined organic solution was washed with saturated Na₂S₂O₃ (2 × 100 mL), saturated brine (100 mL), dried over anhydrous Na₂SO₄, filtered and concentrated. The residue was purified by flash column (0-25% of EtOAc in PE) to give **III-D20** (132 mg, 58%) as a solid. **¹H NMR** (400 MHz, CDCl₃) δ_{H} 3.45-3.35 (m, 5H), 2.61 (s, 1H), 2.48-2.36 (m, 2H), 2.30-2.15 (m, 4H), 1.90-1.59 (m, 8H), 1.52-1.32 (m, 7H), 1.30-1.05 (m, 9H), 0.93 (d, *J* = 6.4 Hz, 3H), 0.78 (d, *J* = 6.4 Hz, 3H); **LC-ELSD/MS** purity 99%, MS ESI calcd. for C₂₅H₄₁O₂ [M-H₂O+H]⁺ 373.3, found 373.3.

### EXAMPLE III-14 Synthesis of 1-(2-((3R,5R,8R,9R,10S,13R,14S,17S)-3-hydroxy-13-isobutyl-3-(methoxymethyl)hexadecahydro-1H-cyclopenta[a]phenanthren-17-yl)-2-oxoethyl)-1H-pyrazole-4-carbonitrile (III-D22)

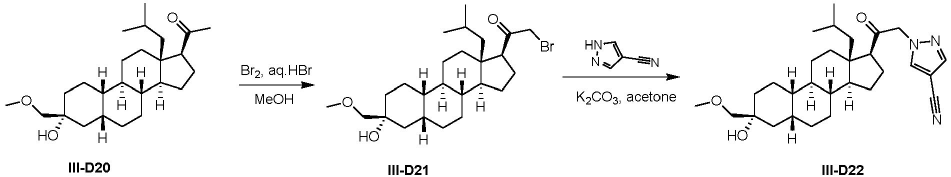

### Synthesis of III-D21

To a solution of **III-D21** (117 mg, 0.3 mmol) and HBr (11.9 mg, 0.06 mmol, 40%) in MeOH (5 mL) was added Br₂ (47.9 mg, 0.3 mmol) at 0°C. After stirring at 20°C for 3 h, the mixture was added into saturated NaHCO₃ (100 mL) and extracted with EtOAc (3 × 50 mL). The combined organic solution was washed with saturated brine (100 mL), dried over anhydrous Na₂SO₄, filtered and concentrated to give **III-D21** (180 mg) as an oil, which was used as is.

### Synthesis of III-D22

To a solution of **III-D22** (180 mg, 0.38 mmol) in acetone (5 mL) were added 1H-pyrazole-4-carnonotrile (106 mg, 1.1 mmol) and K₂CO₃ (157 mg, 1.1 mmol). After stirring at 20°C for 16 h, the mixture was added into saturated NH₄Cl (100 mL) and extracted with EtOAc (3 × 30 mL). The combined organic solution was washed with water (2 × 100 mL), saturated brine (100 mL), dried over anhydrous Na₂SO₄, filtered and concentrated. The residue was purified by flash column (0-40% of EtOAc in PE) to give **III-D2** (24.5 mg, 13%) as a solid. **¹H NMR** (400 MHz, CDCl₃) δ_{H} 7.86 (s, 1H), 7.80 (s, 1H), 5.22 (d, *J* = 18.0 Hz, 1H), 4.90 (d, *J* = 17.6 Hz, 1H), 3.48-3.35 (m, 5H), 2.50-2.40 (m, 2H), 2.35-2.22 (m, 1H), 1.95-1.55 (m, 13H), 1.53-1.00 (m, 12H), 0.95 (d, *J* = 6.4 Hz, 3H), 0.67 (d, *J* = 6.4 Hz, 3H); **LC-ELSD/MS** purity 99%, MS ESI calcd. for C₂₉H₄₂N₃O₂ [M-H₂O+H]⁺ 464.3, found 464.3.

### Steroid Inhibition of TBPS Binding

[³⁵S]-t-Butylbicyclophosphorothionate (TBPS) binding assays using rat brain cortical membranes in the presence of 5 mM GABA has been described (Gee et al, J. Pharmacol. Exp. Ther. 1987, 241, 346-353; Hawkinson et al, Mol. Pharmacol. 1994, 46, 977-985; Lewin, A.H et al., Mol. Pharmacol. 1989, 35, 189-194).

Briefly, cortices are rapidly removed following decapitation of carbon dioxide-anesthetized Sprague-Dawley rats (200-250 g). The cortices are homogenized in 10 volumes of ice-cold 0.32 M sucrose using a glass/teflon homogenizer and centrifuged at 1500 × g for 10 min at 4 °C. The resultant supernatants are centrifuged at 10,000 × g for 20 min at 4 °C to obtain the P2 pellets. The P2 pellets are resuspended in 200 mM NaCl/50 mM Na-K phosphate pH 7.4 buffer and centrifuged at 10,000 × g for 10 min at 4 °C. This washing procedure is repeated twice and the pellets are resuspended in 10 volumes of buffer. Aliquots (100 mL) of the membrane suspensions are incubated with 3 nM [³⁵S]-TBPS and 5 mL aliquots of test drug dissolved in dimethyl sulfoxide (DMSO) (final 0.5%) in the presence of 5 mM GABA. The incubation is brought to a final volume of 1.0 mL with buffer. Nonspecific binding is determined in the presence of 2 mM unlabeled TBPS and ranged from 15 to 25 %. Following a 90 min incubation at room temp, the assays are terminated by filtration through glass fiber filters (Schleicher and Schuell No. 32) using a cell harvester (Brandel) and rinsed three times with ice-cold buffer. Filter bound radioactivity is measured by liquid scintillation spectrometry. Non-linear curve fitting of the overall data for each drug averaged for each concentration is done using Prism (GraphPad). The data are fit to a partial instead of a full inhibition model if the sum of squares is significantly lower by F-test. Similarly, the data are fit to a two component instead of a one component inhibition model if the sum of squares is significantly lower by F-test. The concentration of test compound producing 50% inhibition (IC₅₀) of specific binding and the maximal extent of inhibition (Iₘₐₓ) are determined for the individual experiments with the same model used for the overall data and then the means ± SEM.s of the individual experiments are calculated. Picrotoxin serves as the positive control for these studies as it has been demonstrated to robustly inhibit TBPS binding.

Various compounds are or can be screened to determine their potential as modulators of [³⁵S]-TBPS binding *in vitro.* These assays are or can be performed in accordance with the above.

### Example I-10: TBPS Binding

In **Table 1-1** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table 1-1. TBPS binding of the exemplary compounds.**

| **Example** | **Compound ID** | **Structure** | **IC50 (µM)** |
|---|---|---|---|
| I-1 | **I-A2** | 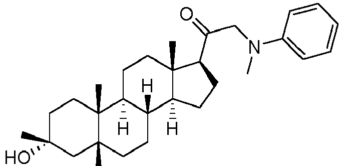 | C |
| I-2 | **I-A3** | 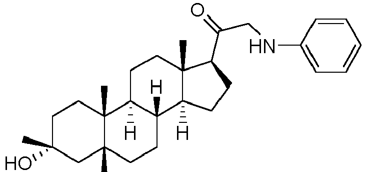 | C |
| I-3 | **I-B6** | 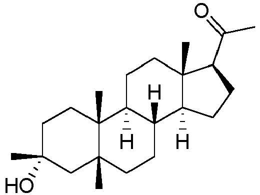 | C |
| I-4 | **I-A4** | 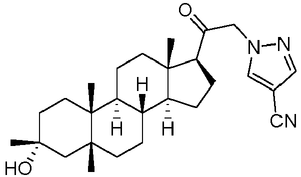 | C |
| I-5 | **I-A5** | 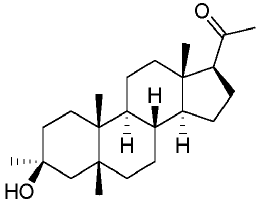 | D |
| I-6 | **I-A6** | 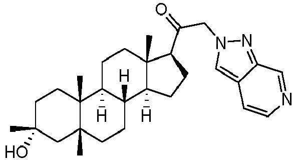 | C |
| I-7 | **I-A7** | 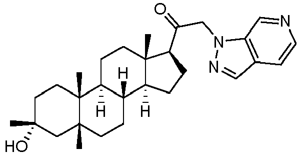 | C |
| I-8 | **I-A8** | 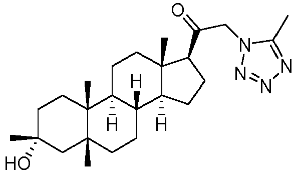 | C |
| I-9 | **I-A9** | 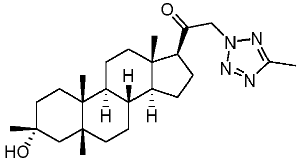 | C |

### EXAMPLE 11-82:

In **Table II-2** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table II-2**

| **Example** | **Compound ID** | **STRUCTURE** | **IC50 (µM)** |
|---|---|---|---|
| II-1 | II**-1** | 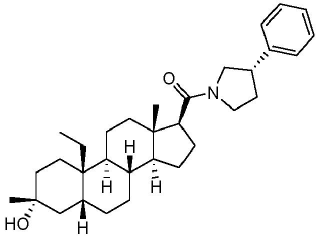 | B |
| II-2 | II-**A4** | 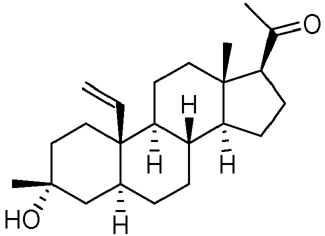 | B |
| II-3 | II-**A5** | 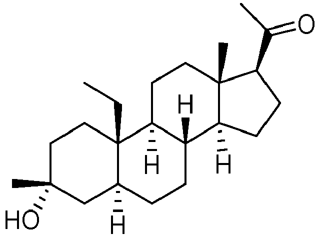 | B |
| II-4 | II-**A7** | 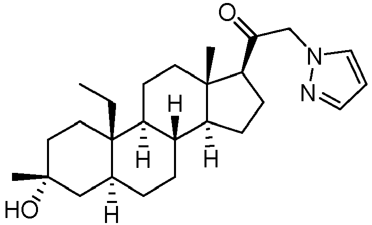 | B |
| II-5 | II-**A8** | 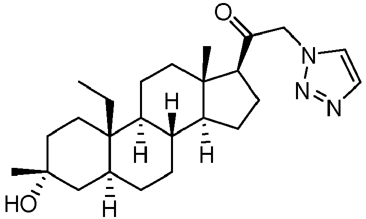 | A |
| II-5 | II-**A8a** | 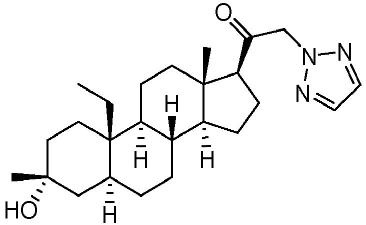 | B |
| II-6 | II-**A9** | 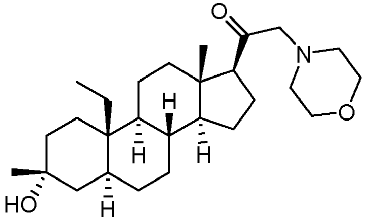 | B |
| II-7 | II-**A10** | 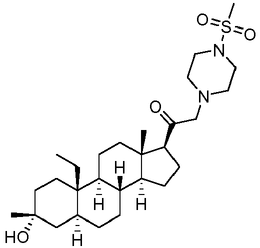 | B |
| II-8 | II-**A11** | 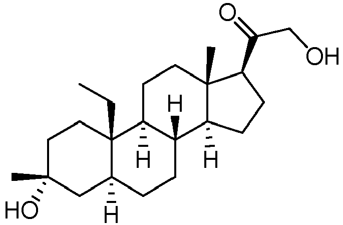 | B |
| II-9 | II-**E12** | 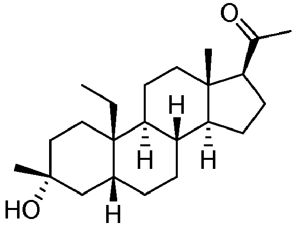 | A |
| II-10 | II-**G3** | 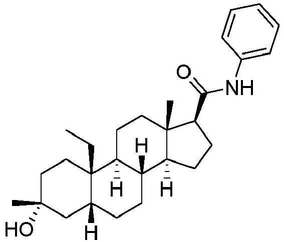 | A |
| II-11 | II-**M2** | 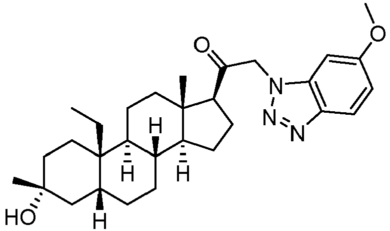 | A |
| II-11 | II-**M2a** | 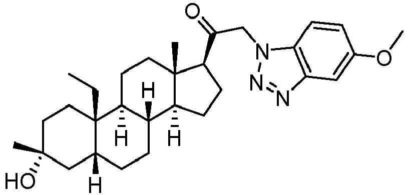 | A |
| II-11 | II-**M2b** | 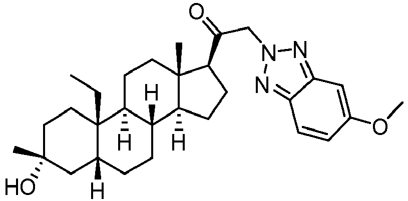 | A |
| II-12 | II-**12** | 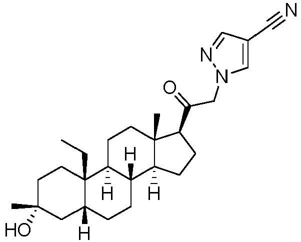 | A |
| II-13 | II-**13** | 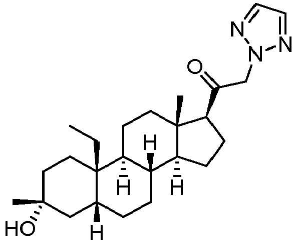 | A |
| II-13a | II-**13a** | 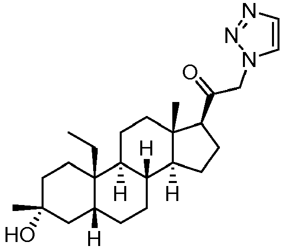 | A |
| II-14 | II-**14** | 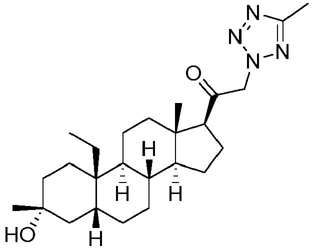 | A |
| II-14a | II-**14a** | 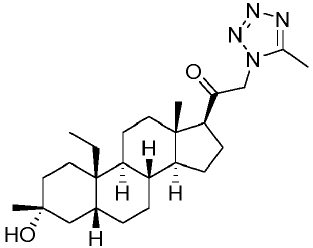 | A |
| II-20 | II-**20** | 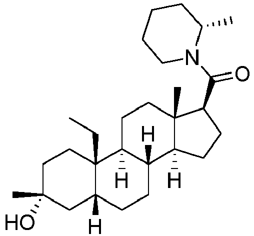 | B |
| II-21 | II-**21** | 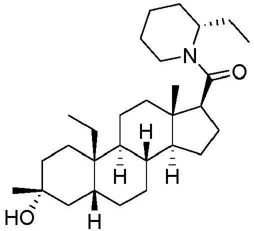 | B |
| II-21a | II-**21a** | 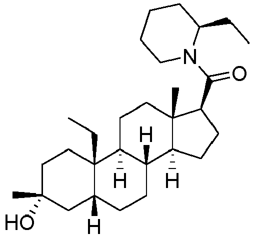 | B |
| II-22 | II-**22** | 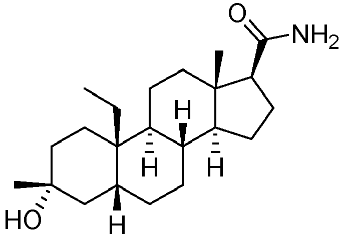 | C |
| II-30 | II-**G5** | 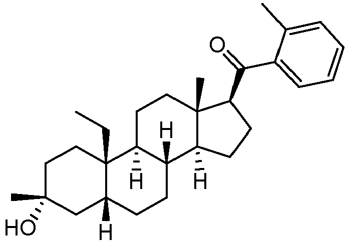 | B |
| II-35 | II-**B9** | 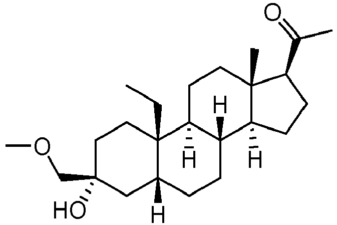 | |
| II-36 | II-**B11** | 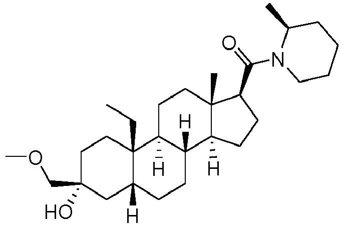 | B |
| II-40 | II-**C4** | 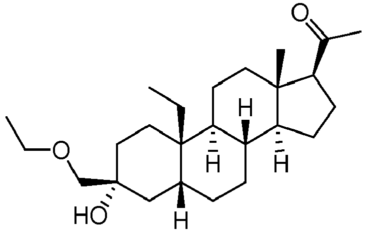 | B |
| II-43 | II-**43** | 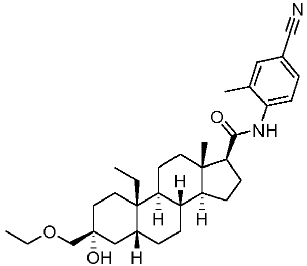 | A |
| II-44 | II-**44** | 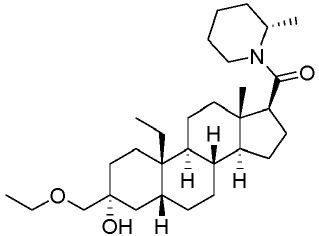 | C |
| II-45 | II-**45** | 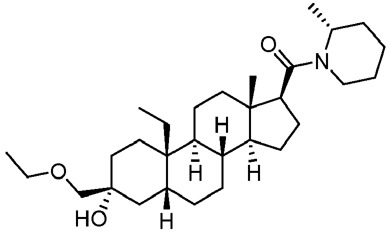 | B |
| II-46 | II-**46** | 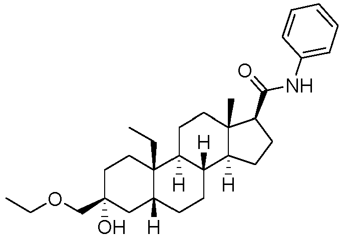 | B |
| II-47 | II-**47** | 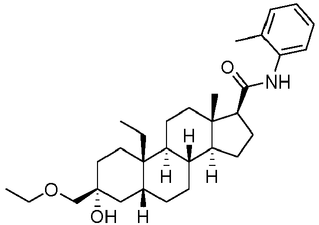 | A |
| II-48 | II-**48** | 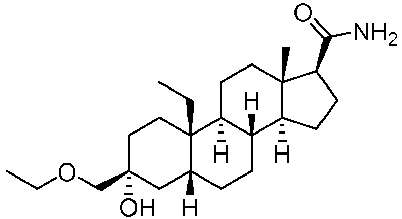 | C |
| II-50 | II-**50** | 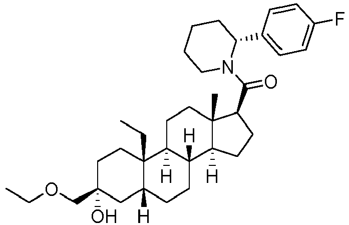 | C |
| II-51 | II-**51** | 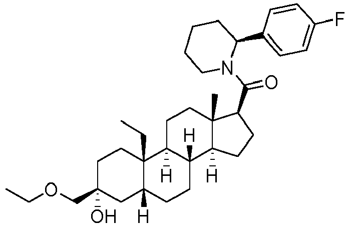 | B |
| II-61 | II-**D9** | 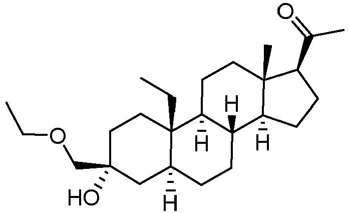 | B |
| II-62 | II-**D11** | 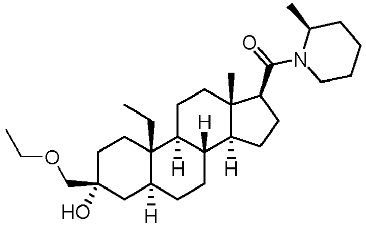 | C |
| II-63 | II-**63** | 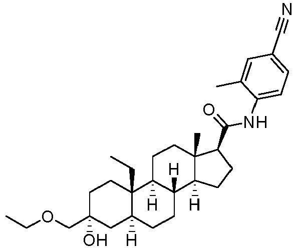 | B |
| II-70 | II-**E4** | 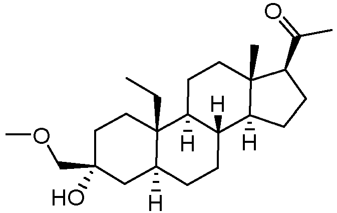 | |
| II-71 | II-**E6** | 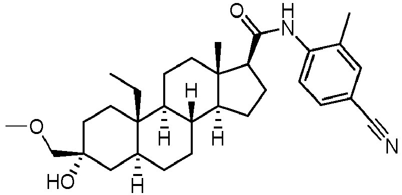 | A |
| II-72 | II-**E8** | 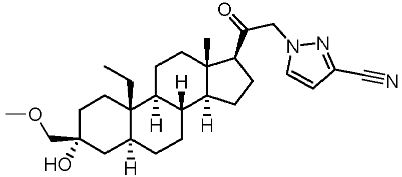 | A |
| II-73 | II-**D13** | 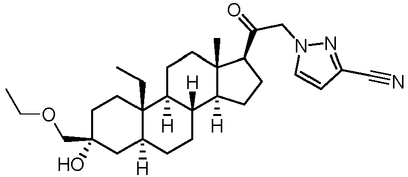 | B |
| II-74 | II-**C8** | 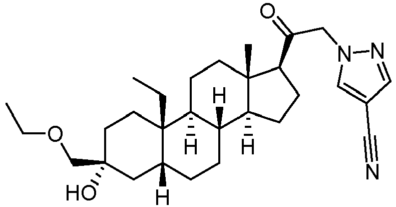 | A |
| II-75 | II-**75** | 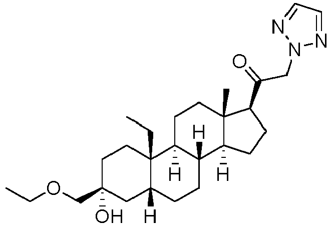 | B |
| II-76 | II-**76** | 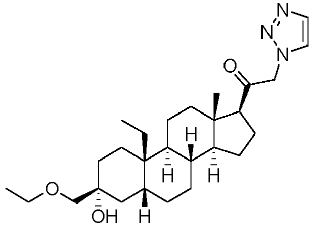 | B |
| II-77 | II-77 | 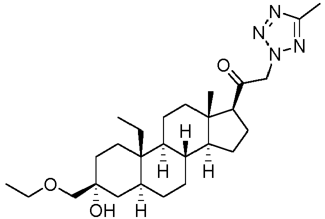 | B |
| II-78 | II-**78** | 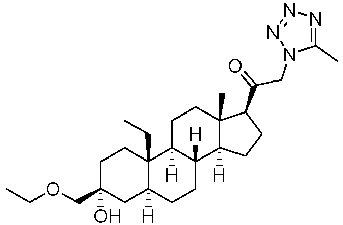 | C |
| II-79 | II-**79** | 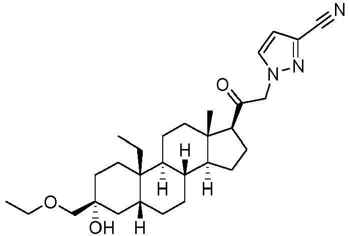 | A |
| II-80 | II-E10 | 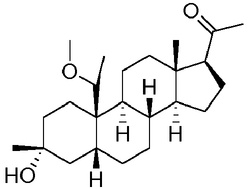 | B |
| II-81 | II-E12 | 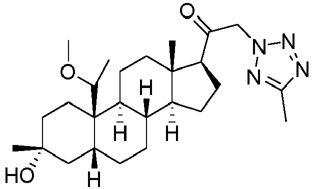 | B |
| II-81 | II-E13 | 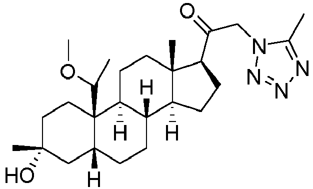 | C |

### EXAMPLE III-14: Biological Data

In **Table III-1** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table 111-1:**

| **Example** | **Compound ID** | **STRUCTURE** | **IC50 (µM)** |
|---|---|---|---|
| **III-1** | **III-A8** | 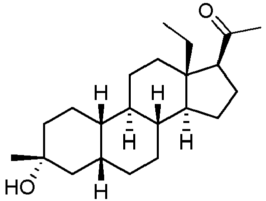 | **A** |
| **III-2** | **III-A10** | 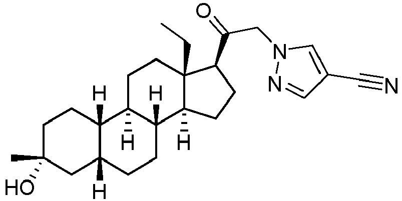 | **A** |
| **III-3** | **III-A20** | 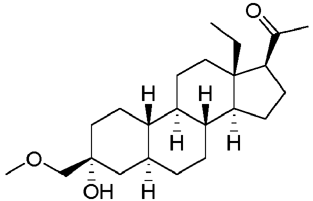 | **B** |
| **III-4** | **III-A22** | 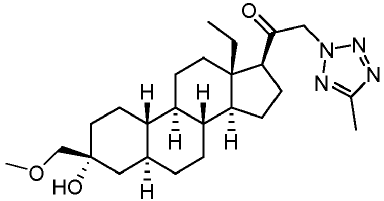 | **A** |
| **III-4a** | **III-A23** | 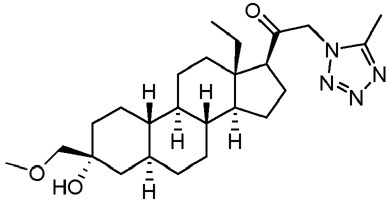 | **C** |
| **III-6** | **III-A30** | 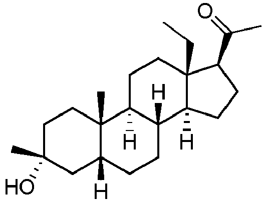 | **A** |
| **III-7** | **III-A32** | 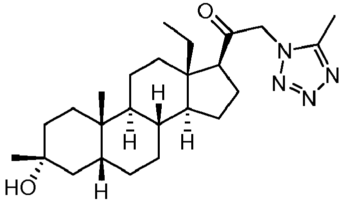 | **C** |
| **III-8** | **III-A33** | 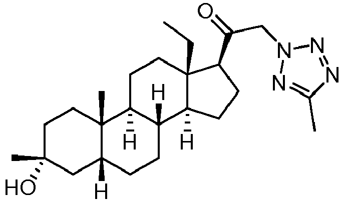 | **A** |
| **III-9** | **III-B15** | 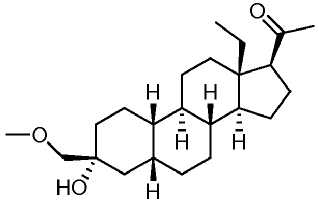 | **B** |
| **III-10** | **III-B17** | 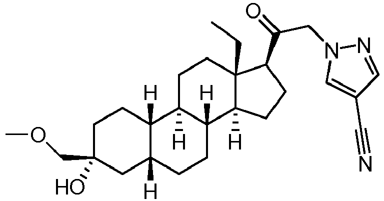 | **B** |
| **III-11** | **III-C11** | 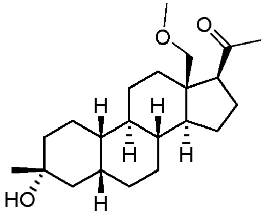 | **B** |
| **III-12** | **III-C13** | 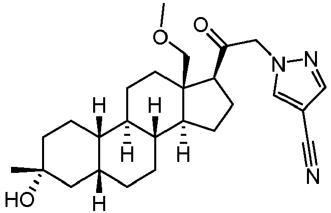 | **A** |
| **III-13** | **III-D20** | 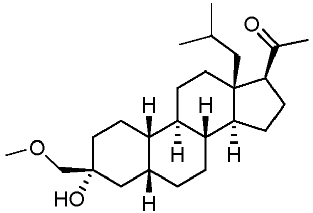 | |
| **III-14** | **III-D22** | 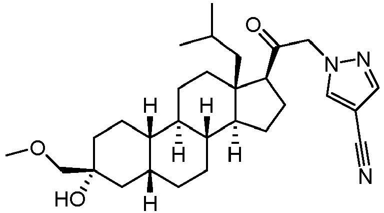 | |

## Claims

1. A compound of Formula (II-Ia): 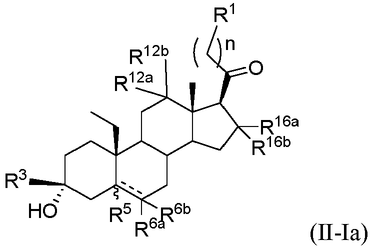 or a pharmaceutically acceptable salt thereof;
wherein:
n is 0, 1 or 2;
R⁵ is hydrogen or methyl, or when 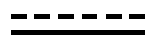 is a double bond, R⁵ is absent;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
each of R^{6a} and R^{6b} is independently hydrogen, halogen, cyano, hydroxyl, substituted or unsubstituted alkyl, substituted or unsubstituted alkenyl, or substituted or unsubstituted alkynyl, or R^{6a} and R^{6b} are joined to form an oxo (=O) group;
R¹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂,-OC(=O)R^{A1}, -OC(=O)OR^{A1}, -OC(=O)SR^{A1}, -OC(=O)N(R^{A1})₂, -SC(=O)R^{A2}, -SC(=O)OR^{A1}, -SC(=O)SR^{A1}, -SC(=O)N(R^{A1})₂, -NHC(=O)R^{A1}, -NHC(=O)OR^{A1}, -NHC(=O)SR^{A1}, -NHC(=O)N(R^{A1})₂, -OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or -S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
each of R^{12a} and R^{12b} is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1}, -OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or R^{12a} and R^{12b} are joined to form an oxo (=O) group;
each of R^{16a} and R^{16b} is independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}), -CN(R^{A1})₂, -C(O)R^{A1}, -OC(=O)R^{A1}, -OC(=O)OR^{A1}, -OC(=O)SR^{A1}, -OC(=O)N(R^{A1})₂, -SC(=O)R^{A2}, -SC(=O)OR^{A1}, -SC(=O)SR^{A1}, -SC(=O)N(R^{A1})₂, -NHC(=O)R^{A1}, -NHC(=O)OR^{A1}, -NHC(=O)SR^{A1}, -NHC(=O)N(R^{A1})₂, -OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or -S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl; and
wherein 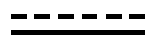 represents a single or double bond, provided if a double bond is present in Ring B, then one of R^{6a} or R^{6b} is absent.

2. The compound or pharmaceutically acceptable salt of claim 1, wherein R⁵ is hydrogen or methyl in the cis position, relative to the C19 position.

3. The compound or pharmaceutically acceptable salt of claim 1, wherein R⁵ is hydrogen or methyl in the trans position, relative to the C19 position.

4. The compound or pharmaceutically acceptable salt of any one of claims 1-3, wherein R³ is hydrogen, substituted or unsubstituted alkyl, substituted or unsubstituted alkenyl, or substituted or unsubstituted alkynyl.

5. The compound or pharmaceutically acceptable salt of any one of claims 1-4, wherein R³ is substituted or unsubstituted C₁-C₆ alkyl.

6. The compound or pharmaceutically acceptable salt of any one of claims 1-5, wherein R³ is methyl.

7. The compound or pharmaceutically acceptable salt of any one of claims 1-6, wherein both R^{6a} and R^{6b} are hydrogen.

8. The compound or pharmaceutically acceptable salt of any one of claims 1-7, wherein each of R^{12a} and R^{12b} is independently hydrogen, substituted or unsubstituted C₁-C₆ alkyl, -OH, or R^{12a} and R^{12b} together form oxo.

9. The compound or pharmaceutically acceptable salt of any one of claims 1-8, wherein each of R^{16a} and R^{16b} is independently hydrogen, substituted or unsubstituted C₁-C₆ alkyl, -OR^{D1}, -OC(=O)R^{D1}, -NH2, -N(R^{D1})2, or -NR^{D1}C(=O)R^{D1}, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

10. The compound or pharmaceutically acceptable salt of any one of claims 1-9, wherein each of R^{16a} and R^{16b} is independently hydrogen or substituted or unsubstituted C₁-C₆ alkyl.

11. The compound or pharmaceutically acceptable salt of any one of claims 1-10, wherein both of R^{16a} and R^{16b} are hydrogen.

12. The compound or pharmaceutically acceptable salt of any one of claims 1-11, wherein n is 0 or 1, and R¹ is -N(R^{A1})₂, -OR^{A1}, substituted or unsubstituted alkyl, substituted or unsubstituted carbocyclyl, substituted or unsubstituted heterocyclyl, substituted or unsubstituted aryl, substituted or unsubstituted heteroaryl.

13. The compound or pharmaceutically acceptable salt of any one of claims 1-12, wherein R¹ is 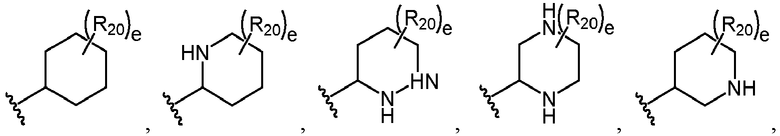 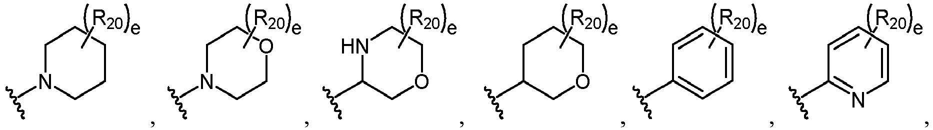 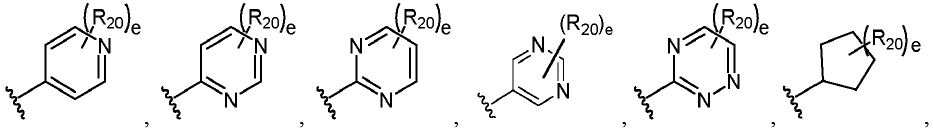 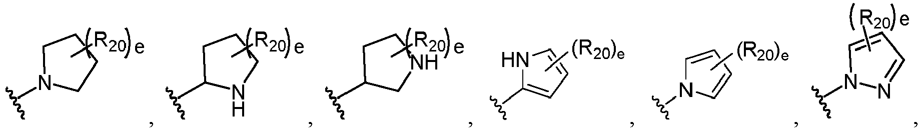 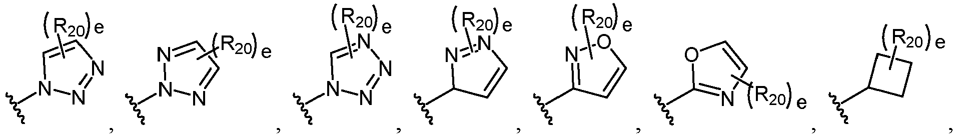 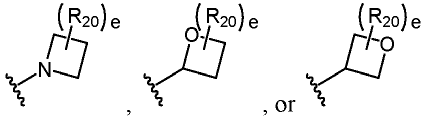 wherein each instance of R₂₀ is, independently hydrogen, halogen, -NO₂, -CN, -OR^{GA}, -N(R^{GA})₂, -C(=O)R^{GA}, -C(=O)OR^{GA}, -C(=O)N(R^{GA})₂, -OC(=O)R^{GA}, -OC(=O)OR^{GA}, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, -S(=O)₂N(R^{GA})₂, or -N(R^{GA})S(=O)₂R^{GA}, substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocylyl, substituted or unsubstituted 3- to 4- membered heterocylyl, C₅₋₁₀ substituted or unsubstituted aryl, substituted or unsubstituted 5- to 10- membered heteroaryl, or optionally two R^{GA} are taken with the intervening atoms to form a substituted or unsubstituted 3-to 4-membered carbocyclic or heterocyclic ring;
wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring; and
e is 0, 1,2, 3, 4, or 5.

14. The compound or pharmaceutically acceptable salt of claim 1, wherein the compound is a compound of Formula (II-Ib) or Formula (II-Ic) 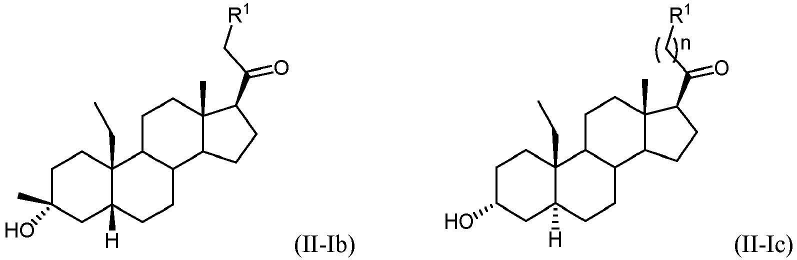 or a pharmaceutically acceptable salt thereof.

15. The compound or pharmaceutically acceptable salt of claim 1, wherein the compound is
(a) a compound Formula (II-Ie) 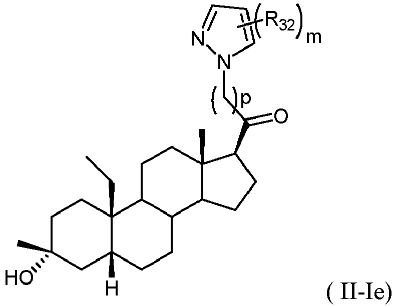
wherein m is 0, 1, 2 or 3;
p is 0, 1, or 3;
each R₃₂ is independently halogen, alkyl, hydroxyl, or cyano;
or a pharmaceutically acceptable salt thereof;
(b) a compound of Formula (II-Ig) 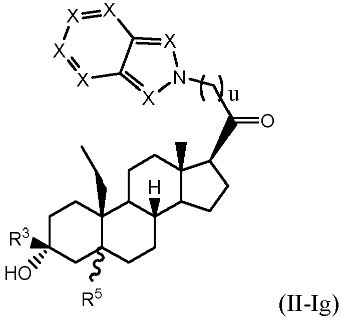
wherein u is 0, 1, or 2; each X is independently -C(R^{N})-, -C(R^{N})₂-, -S-, -N-, or -N(R^{N})- wherein R^{N} is independently hydrogen, substituted or unsubstituted C₁₋₆ alkyl, -C(=O)R^{GA}, -C(=O)OR^{GA}, -C(=O)N(R^{GA})₂, -S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof;
(c) a compound of Formula (II-Iga) 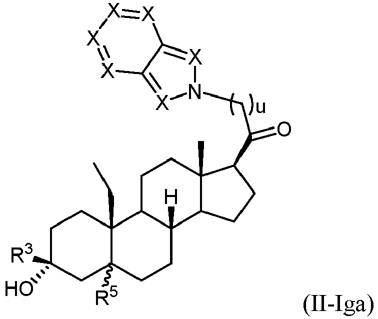
wherein u is 0, 1, or 2; each X is independently -C(R^{N})-, -C(R^{N})₂-, -S-, -N-, or -N(R^{N})- wherein R^{N} is independently hydrogen, substituted or unsubstituted C₁₋₆ alkyl, -C(=O)R^{GA}, -C(=O)OR^{GA}, -C(=O)N(R^{GA})₂, -S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof;
(d) a compound of Formula (II-Ih) 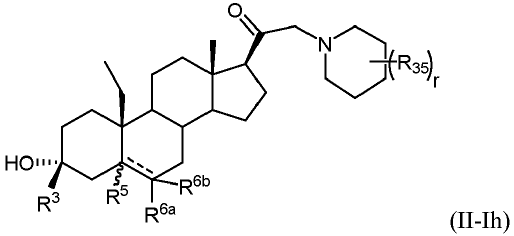 wherein each R₃₅ is independently halogen, alkyl, hydroxyl, or cyano; and r is 0, 1, 2 or 3; or a pharmaceutically acceptable salt thereof; or
(e) a compound of Formula (II-Ii) 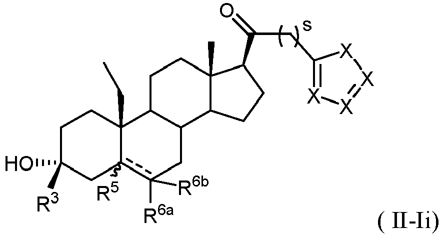
wherein s is 0, 1, or 2; each X is independently -C(R^{N})-, -C(R^{N})₂-, -O-, -S-, -N-, or -N(R^{N})- wherein R^{N} is independently hydrogen, substituted or unsubstituted C₁₋₆ alkyl, -C(=O)R^{GA}, -C(=O)OR^{GA}, -C(=O)N(R^{GA})₂, -S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring; or a pharmaceutically acceptable salt thereof.

16. A compound selected from
| **Compound ID** | **STRUCTURE** |
|---|---|
| II-1 | 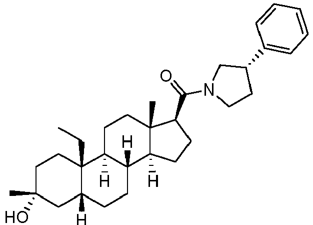 |
| II-A4 | 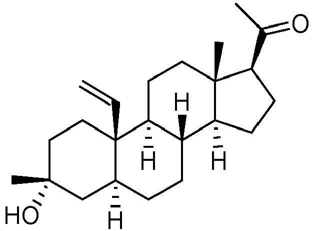 |
| II-A5 | 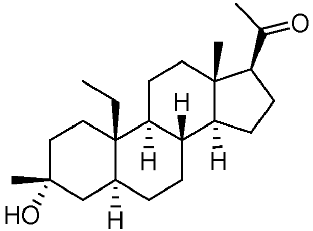 |
| II-A7 | 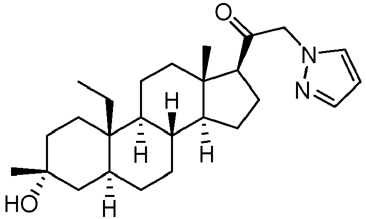 |
| II-A8 | 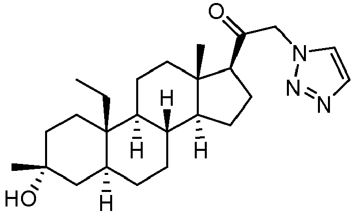 |
| II-A8a | 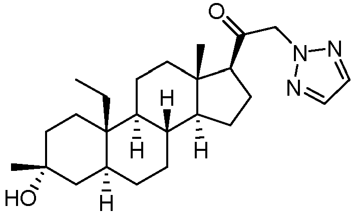 |
| II-A9 | 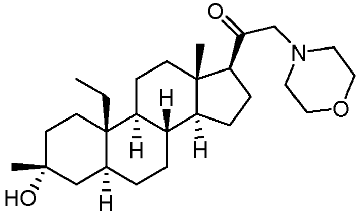 |
| II-A10 | 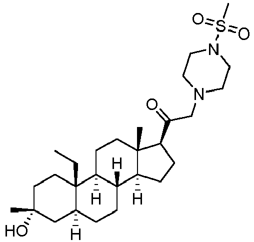 |
| II-A11 | 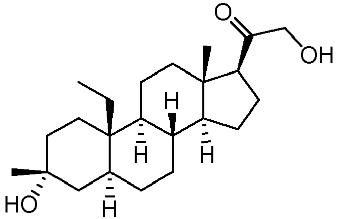 |
| II-E12 | 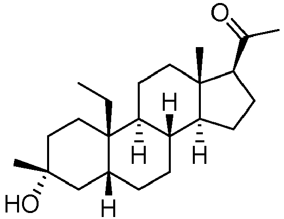 |
| II-G3 | 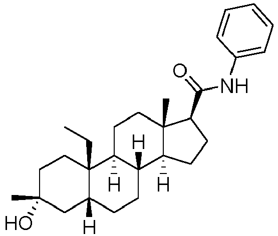 |
| II-M2 | 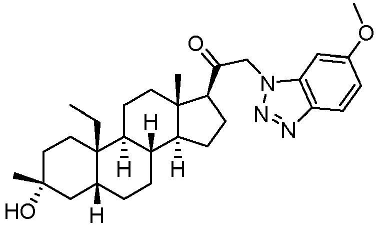 |
| II-M2a | 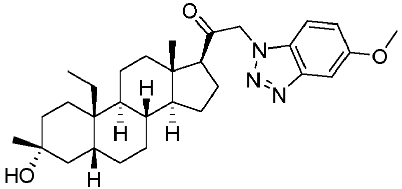 |
| II-M2b | 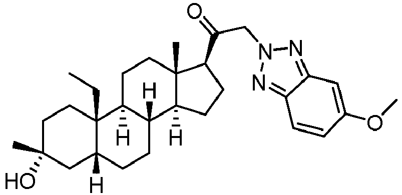 |
| II-12 | 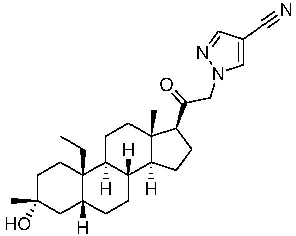 |
| II-13 | 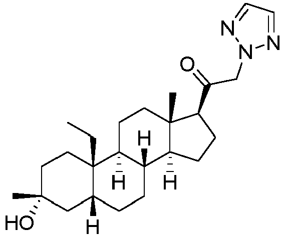 |
| II-13a | 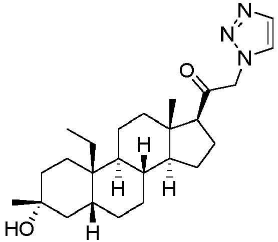 |
| II-14 | 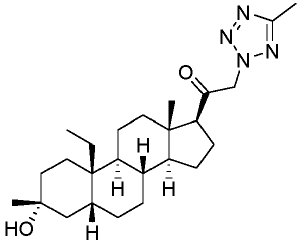 |
| II-14a | 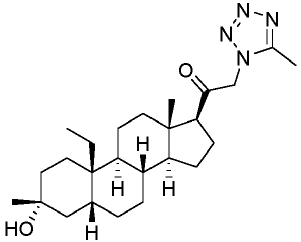 |
| II-20 | 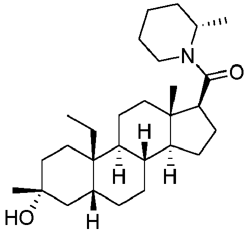 |
| II-21 | 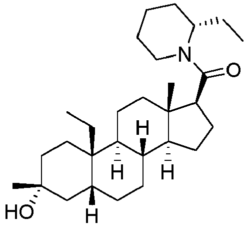 |
| II-21a | 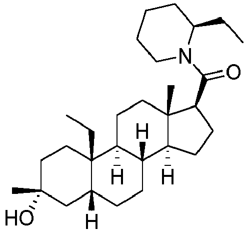 |
| II-22 | 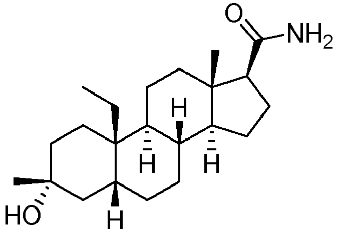 |
| II-G5 | 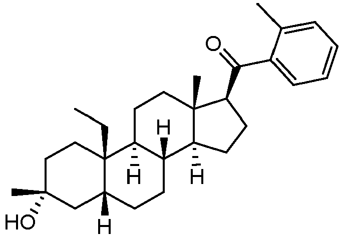 |
| II-B9 | 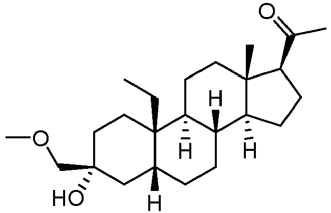 |
| II-B11 | 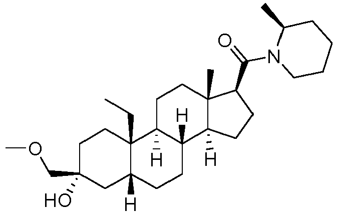 |
| II-C4 | 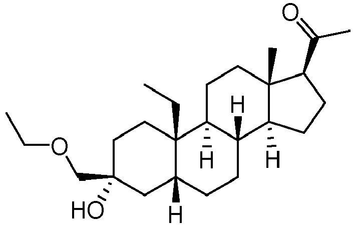 |
| II-43 | 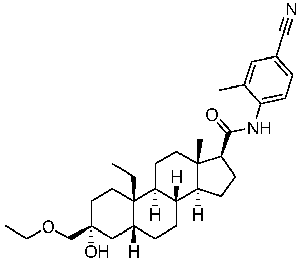 |
| II-44 | 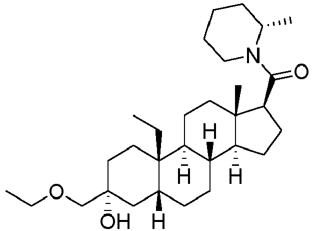 |
| II-45 | 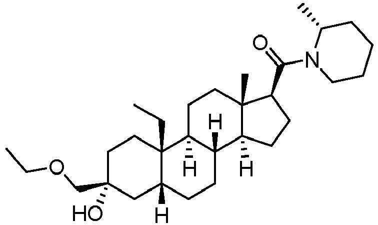 |
| II-46 | 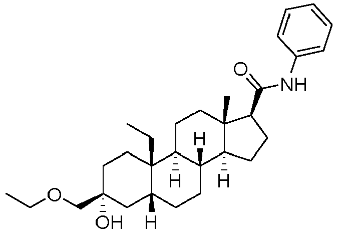 |
| II-47 | 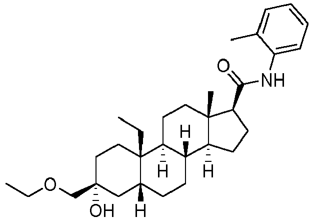 |
| II-48 | 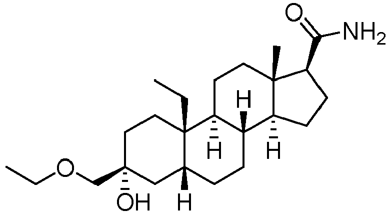 |
| II-50 | 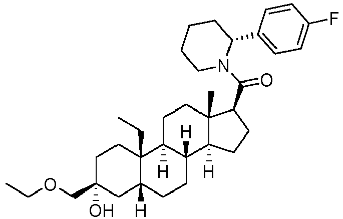 |
| II-51 | 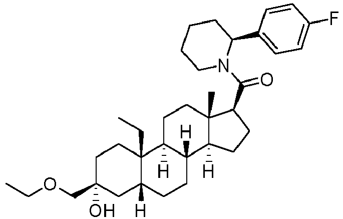 |
| II-D9 | 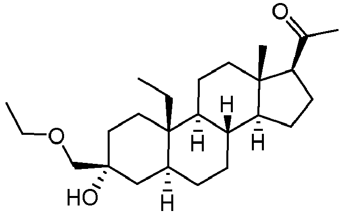 |
| II-D11 | 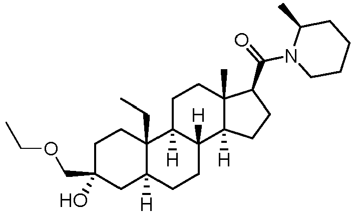 |
| II-63 | 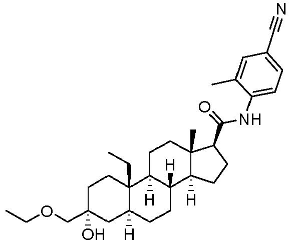 |
| II-E4 | 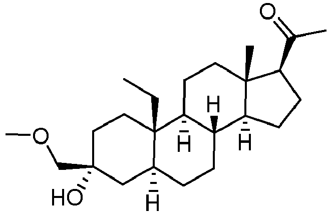 |
| II-E6 | 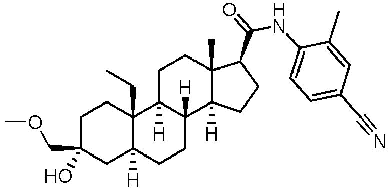 |
| II-E8 | 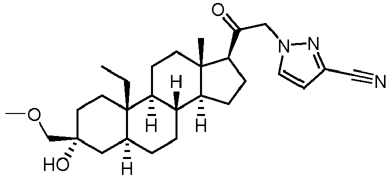 |
| II-D13 | 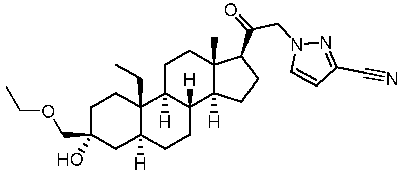 |
| II-C8 | 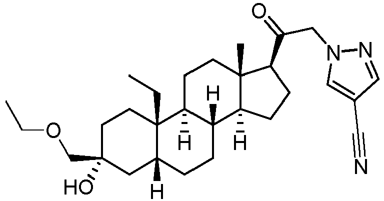 |
| II-75 | 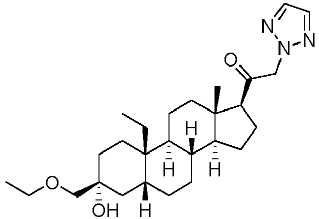 |
| II-76 | 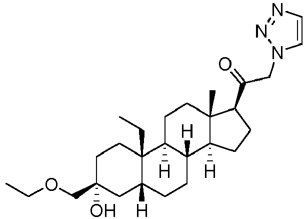 |
| II-77 | 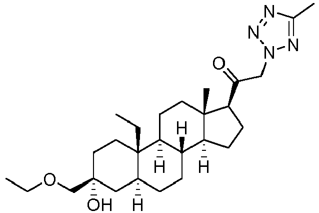 |
| II-78 | 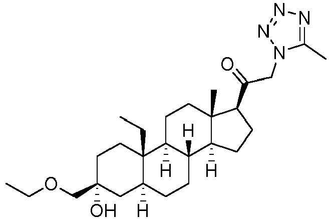 |
| II-79 | 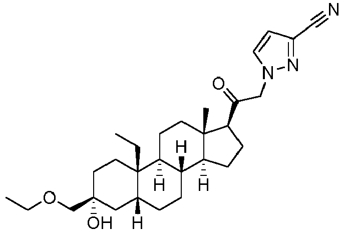 |
or a pharmaceutically acceptable salt thereof.

17. A pharmaceutical composition comprising a compound of any one of claims 1-16 or pharmaceutically acceptable salt thereof, and a pharmaceutically acceptable excipient.

18. A compound according to any one of claims 1-16 or a pharmaceutically acceptable salt thereof, or a pharmaceutical composition according to claim 17, for use in treating a CNS-related disorder in a subject.

19. A compound according to any one of claims 1-16 or a pharmaceutically acceptable salt thereof, or a pharmaceutical composition according to claim 17, for use according to claim 18, wherein the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus.

20. A compound according to any one of claims 1-16 or a pharmaceutically acceptable salt thereof, or a pharmaceutical composition according to claim 17, for use according to claim 19, wherein the CNS-related disorder is depression.

21. A compound according to any one of claims 1-16 or a pharmaceutically acceptable salt thereof, or a pharmaceutical composition according to claim 17, for use according to claim 19, wherein the depression is postpartum depression.

22. A compound according to any one of claims 1-16 or a pharmaceutically acceptable salt thereof, or a pharmaceutical composition according to claim 17, for use according to claim 19, wherein the CNS-related disorder is major depressive disorder.

23. A compound according to any one of claims 1-16 or a pharmaceutically acceptable salt thereof, or a pharmaceutical composition according to claim 17, for use according to claim 22, wherein the major depressive disorder is moderate major depressive disorder.

24. A compound according to any one of claims 1-16 or a pharmaceutically acceptable salt thereof, or a pharmaceutical composition according to claim 17, for use according to claim 22, wherein the major depressive disorder is severe major depressive disorder.

## Patentansprüche

1. Verbindung der Formel (II-Ia) 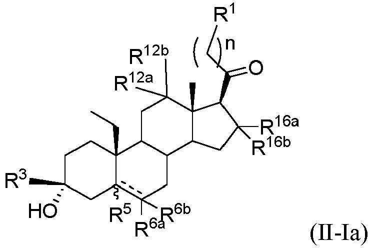 oder ein pharmazeutisch unbedenkliches Salz davon;
wobei:
n für 0, 1 oder 2 steht;
R⁵ für Wasserstoff oder Methyl steht oder dann, wenn ^{- - - - - -} für eine Doppelbindung steht, R⁵ fehlt;
R³ für Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl steht; R^{6a} und R^{6b} jeweils für Wasserstoff, Halogen, Cyano, Hydroxyl, substituiertes oder unsubstituiertes Alkyl, substituiertes oder unsubstituiertes Alkenyl oder substituiertes oder unsubstituiertes Alkinyl stehen oder R^{6a} und R^{6b} zusammen eine Oxogruppe (=O) bilden;
R¹ für substituiertes oder unsubstituiertes Alkyl, substituiertes oder unsubstituiertes Alkenyl, substituiertes oder unsubstituiertes Alkinyl, substituiertes oder unsubstituiertes Carbocylyl, substituiertes oder unsubstituiertes Heterocyclyl, substituiertes oder unsubstituiertes Aryl, substituiertes oder unsubstituiertes Heteroaryl, -OR^{A1}, - SR^{A1}, -N(R^{A1})₂, -OC(=O) R^{A1}, -OC(=O) OR^{A1}, -OC(=O) SR^{A1}, - OC(=O) N(R^{A1})₂, -SC (=O) R^{A2}, -SC (=O) OR^{A1}, -SC (=O) SR^{A1}, - SC(=O) N(R^{A1})₂, -NHC (=O) R^{A1}, -NHC (=O)OR^{A1}, -NHC (=O) SR^{A1}, - NHC(=O)N(R^{A1})₂, -OS(=O)₂R^{A2}, -OS(=O) ₂OR^{A1}, -S-S(=O)₂R^{A2}, -S-S(=O) ₂OR^{A1}, -S(=O) R^{A2}, -SO₂R^{A2} oder -S(=O) ₂OR^{A1} steht, wobei jedes Vorkommen von R^{A1} unabhängig für Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, eine Sauerstoff-Schutzgruppe, wenn es an ein Sauerstoffatom gebunden ist, eine Schwefel-Schutzgruppe, wenn es an ein Schwefelatom gebunden ist, eine Stickstoff-Schutzgruppe, wenn es an ein Stickstoffatom gebunden ist, steht oder zwei R^{A1}-Gruppen zusammengenommen einen substituierten oder unsubstituierten heterocyclischen Ring oder Heteroarylring bilden; und R^{A2} für substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, oder substituiertes oder unsubstituiertes Heteroaryl steht;
R^{12a} und R^{12b} jeweils unabhängig für Wasserstoff, Halogen, Cyano, Hydroxyl, substituiertes oder unsubstituiertes Alkyl, substituiertes oder unsubstituiertes Alkenyl, substituiertes oder unsubstituiertes Heterocyclyl oder substituiertes oder unsubstituiertes Alkinyl, -OR^{D1}, - OC(=O)R^{D1}, -NH₂, -N(R^{D1})₂ oder -NR^{D1}C(=O)R^{D1} stehen, wobei jedes Vorkommen von R^{D1} unabhängig für Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl, eine Sauerstoff-Schutzgruppe, wenn es an ein Sauerstoffatom gebunden ist, eine Stickstoff-Schutzgruppe, wenn es an ein Stickstoffatom gebunden ist, steht oder zwei R^{D1}-Gruppen zusammengenommen einen substituierten oder unsubstituierten heterocyclischen Ring bilden; oder R^{12a} und R^{12b} zusammengenommen eine Oxogruppe (=O) bilden;
R^{16a} und R^{16b} jeweils unabhängig für Wasserstoff, Halogen, Cyano, Hydroxyl, substituiertes oder unsubstituiertes Alkyl, substituiertes oder unsubstituiertes Alkenyl, substituiertes oder unsubstituiertes Alkinyl, substituiertes oder unsubstituiertes Carbocylyl, substituiertes oder unsubstituiertes Heterocyclyl, substituiertes oder unsubstituiertes Aryl, substituiertes oder unsubstituiertes Heteroaryl, -OR^{A1}, - SR^{A1}, -N(R^{A1})₂, -N(R^{A1}), -CN(R^{A1})₂, -C(O)R^{A1}, -OC(=O)R^{A1}, - OC(=O) OR^{A1}, -OC(=O) SR^{A1}, -OC(=O) N(R^{A1})₂, -SC(=O) R^{A2}, - SC(=O) OR^{A1}, -SC (=O) SR^{A1}, -SC(=O)N(R^{A1})₂, -NHC(=O) R^{A1}, - NHC(=O) OR^{A1}, -NHC (=O) SR^{A1}, -NHC (=O) N(R^{A1})₂, -OS(=O) ₂R^{A2}, - OS(=O)₂OR^{A1}, -S-S(=O)₂R^{A2}, -S-S(=O)₂OR^{A1}, -S(=O) R^{A2}, -SO₂R^{A2}, oder -S(=O) ₂OR^{A1} stehen, wobei jedes Vorkommen von R^{A1} unabhängig für Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, eine Sauerstoff-Schutzgruppe, wenn es an ein Sauerstoffatom gebunden ist, eine Schwefel-Schutzgruppe, wenn es an ein Schwefelatom gebunden ist, eine Stickstoff-Schutzgruppe, wenn es an ein Stickstoffatom gebunden ist, -SO₂R^{A2}, -C(O)R^{A2} steht oder zwei R_{A1}-Gruppen zusammengenommen einen substituierten oder unsubstituierten heterocyclischen Ring oder Heteroarylring bilden; und R^{A2} für substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, oder substituiertes oder unsubstituiertes Heteroaryl steht; und
wobei 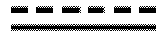 für eine Einfach- oder Doppelbindung steht, mit der Maßgabe, dass dann, wenn in Ring B eine Doppelbindung vorliegt, eines von R^{6a} oder R^{6b} fehlt.

2. Verbindung oder pharmazeutisch unbedenkliches Salz nach Anspruch 1, wobei R⁵ für Wasserstoff oder Methyl in der cis-Position zur C19-Position steht.

3. Verbindung oder pharmazeutisch unbedenkliches Salz nach Anspruch 1, wobei R⁵ für Wasserstoff oder Methyl in der trans-Position zur C19-Position steht.

4. Verbindung oder pharmazeutisch unbedenkliches Salz nach einem der Ansprüche 1-3, wobei R³ für Wasserstoff, substituiertes oder unsubstituiertes Alkyl, substituiertes oder unsubstituiertes Alkenyl oder substituiertes oder unsubstituiertes Alkinyl steht.

5. Verbindung oder pharmazeutisch unbedenkliches Salz nach einem der Ansprüche 1-4, wobei R³ für substituiertes oder unsubstituiertes C₁-C₆-Alkyl steht.

6. Verbindung oder pharmazeutisch unbedenkliches Salz nach einem der Ansprüche 1-5, wobei R³ für Methyl steht.

7. Verbindung oder pharmazeutisch unbedenkliches Salz nach einem der Ansprüche 1-6, wobei sowohl R^{6a} als auch R^{6b} für Wasserstoff stehen.

8. Verbindung oder pharmazeutisch unbedenkliches Salz nach einem der Ansprüche 1-7, wobei R^{12a} und R^{12b} jeweils unabhängig für Wasserstoff, substituiertes oder unsubstituiertes C₁-C₆-Alkyl, -OH stehen oder R^{12a} und R^{12b} zusammen Oxo bilden.

9. Verbindung oder pharmazeutisch unbedenkliches Salz nach einem der Ansprüche 1-8, wobei R^{16a} und R^{16b} jeweils unabhängig für Wasserstoff, substituiertes oder unsubstituiertes C₁-C₆-Alkyl, -OR^{D1}, -OC(=O)R^{D1}, -NH2, - N(R^{D1}) 2 oder -NR^{D1}C(=O)R^{D1} stehen, wobei jedes Vorkommen von R^{D1} unabhängig für Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl steht.

10. Verbindung oder pharmazeutisch unbedenkliches Salz nach einem der Ansprüche 1-9, wobei R^{16a} und R^{16b} jeweils unabhängig für Wasserstoff oder substituiertes oder unsubstituiertes C₁-C₆-Alkyl steht.

11. Verbindung oder pharmazeutisch unbedenkliches Salz nach einem der Ansprüche 1-10, wobei beide von R^{16a} und R^{16b} für Wasserstoff stehen.

12. Verbindung oder pharmazeutisch unbedenkliches Salz nach einem der Ansprüche 1-11, wobei n für 0 oder 1 steht und R¹ für -N(R^{A1})₂, -OR^{A1}, substituiertes oder unsubstituiertes Alkyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl steht.

13. Verbindung oder pharmazeutisch unbedenkliches Salz nach einem der Ansprüche 1-12, wobei R¹ für 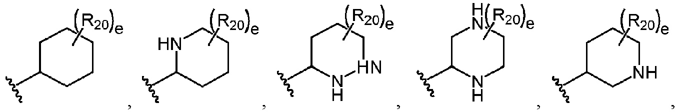 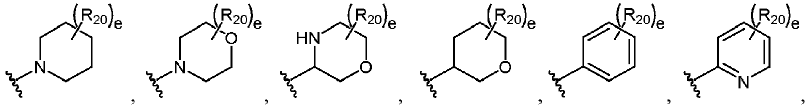 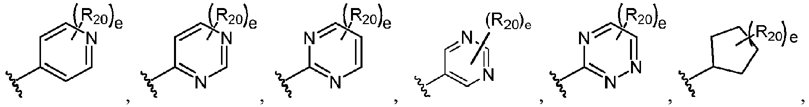 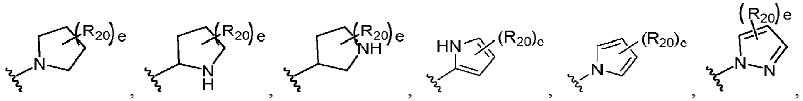 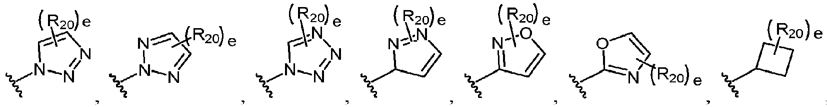 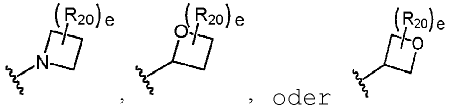
steht, wobei jedes Vorkommen von R₂₀ unabhängig für Wasserstoff, Halogen, -NO₂, -CN, -OR^{GA}, -N(R^{GA})₂, - C(=O)R^{GA}, -C(=O)OR^{GA}, -C(=O)N(R^{GA})₂, -OC(=O)R^{GA}, - OC(=O)OR^{GA}, -N(R^{GA})C(=O)R^{GA}, -OC(=O)N(R^{GA})₂, - N(R^{GA})C(=O) OR^{GA}, -S(=O)₂R^{GA}, -S(=O)₂OR^{GA}, -OS (=O) ₂R^{GA}, - S(=O)₂N(R^{GA})₂ oder -N(R^{GA})S(=O)₂R^{GA}, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₄-Carbocyclyl, substituiertes oder unsubstituiertes 3- bis 4-gliedriges Heterocyclyl, substituiertes oder unsubstituiertes C₅₋₁₀-Aryl, substituiertes oder unsubstituiertes 5- bis 10-gliedriges Heteroaryl steht oder gegebenenfalls zwei R^{GA} mit den dazwischenliegenden Atomen einen substituierten oder unsubstituierten 3- bis 4-gliedrigen carbocyclischen oder heterocyclischen Ring bilden; wobei jedes Vorkommen von R^{GA} unabhängig für Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₆-Carbocyclyl, substituiertes oder unsubstituiertes 3- bis 6-gliedriges Heterocyclyl, substituiertes oder unsubstituiertes Aryl, substituiertes oder unsubstituiertes Heteroaryl, eine Sauerstoff-Schutzgruppe, wenn es an Sauerstoff gebunden ist, eine Stickstoff-Schutzgruppe, wenn es an Stickstoff gebunden ist, steht oder zwei R^{GA}-Gruppen zusammen mit den dazwischenliegenden Atomen einen substituierten oder unsubstituierten carbocyclischen oder heterocyclischen Ring bilden; und
e für 0, 1, 2, 3, 4 oder 5 steht.

14. Verbindung oder pharmazeutisch unbedenkliches Salz nach Anspruch 1, wobei es sich bei der Verbindung um eine Verbindung der Formel (II-Ib) oder Formel (II-Ic) 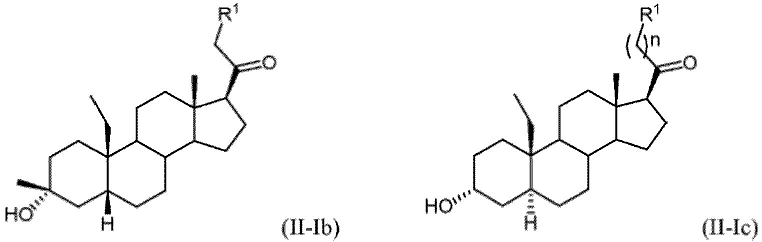 oder ein pharmazeutisch unbedenkliches Salz davon handelt.

15. Verbindung oder pharmazeutisch unbedenkliches Salz nach Anspruch 1, wobei es sich bei der Verbindung um Folgendes handelt:
(a) eine Verbindung der Formel (II-Ie) 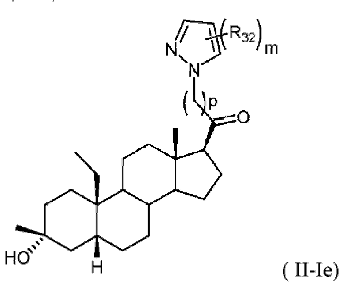
wobei m für 0, 1, 2 oder 3 steht;
p für 0, 1 oder 3 steht;
R₃₂ jeweils unabhängig für Halogen, Alkyl, Hydroxyl oder Cyano steht;
oder ein pharmazeutisch unbedenkliches Salz davon;
(b) eine Verbindung der Formel (II-Ig) 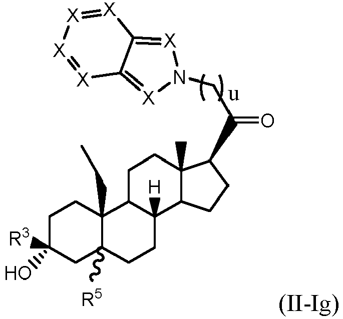
wobei u für 0, 1 oder 2 steht; X jeweils unabhängig für -C(R^{N})-, -C(R^{N})₂-, -S-, -N- oder -N(R^{N})- steht, wobei R^{N} unabhängig für Wasserstoff, substituiertes oder unsubstituiertes C₁-₆-Alkyl, -C(=O)R^{GA}, -C(=O)OR^{GA}, - C(=O)N(R^{GA})₂, -S(=O)₂R^{GA} oder -S(=O)₂N(R^{GA})₂ steht; und
jedes Vorkommen von R^{GA} unabhängig für Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₆-Carbocyclyl, substituiertes oder unsubstituiertes 3- bis 6-gliedriges Heterocyclyl, substituiertes oder unsubstituiertes Aryl, substituiertes oder unsubstituiertes Heteroaryl, eine Sauerstoff-Schutzgruppe, wenn es an Sauerstoff gebunden ist, oder eine Stickstoff-Schutzgruppe, wenn es an Stickstoff gebunden ist, steht oder zwei R^{GA}-Gruppen zusammen mit den dazwischenliegenden Atomen einen substituierten oder unsubstituierten Heterocyclyl- oder Heteroarylring bilden; oder ein pharmazeutisch unbedenkliches Salz davon;
(c) eine Verbindung der Formel (II-Iga) 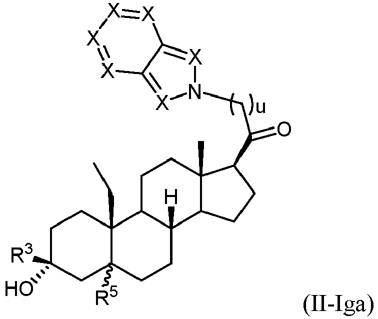 wobei u für 0, 1 oder 2 steht; X jeweils unabhängig für -C(R^{N})-, -C(R^{N})₂-, -S-, -N- oder -N(R^{N}) - steht, wobei R^{N} unabhängig für Wasserstoff, substituiertes oder unsubstituiertes C₁-₆-Alkyl, -C(=O)R^{GA}, -C(=O)OR^{GA}, - C(=O)N(R^{GA})₂, -S(=O)₂R^{GA} oder -S(=O)₂N(R^{GA})₂ steht; und
jedes Vorkommen von R^{GA} unabhängig für Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₆-Carbocyclyl, substituiertes oder unsubstituiertes 3- bis 6-gliedriges Heterocyclyl, substituiertes oder unsubstituiertes Aryl, substituiertes oder unsubstituiertes Heteroaryl, eine Sauerstoff-Schutzgruppe, wenn es an Sauerstoff gebunden ist, oder eine Stickstoff-Schutzgruppe, wenn es an Stickstoff gebunden ist, steht oder zwei R^{GA}-Gruppen zusammen mit den dazwischenliegenden Atomen einen substituierten oder unsubstituierten Heterocyclyl- oder Heteroarylring bilden; oder ein pharmazeutisch unbedenkliches Salz davon;
(d) eine Verbindung der Formel (II-Ih) 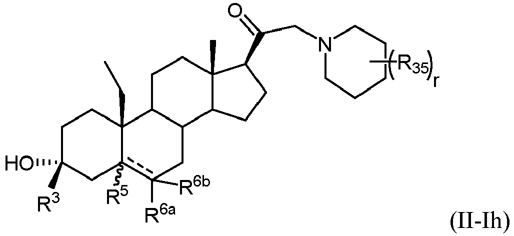 wobei R₃₅ jeweils unabhängig für Halogen, Alkyl, Hydroxyl oder Cyano steht; und r für 0, 1, 2 oder 3 steht; oder ein pharmazeutisch unbedenkliches Salz davon; oder
(e) eine Verbindung der Formel (II-Ii) 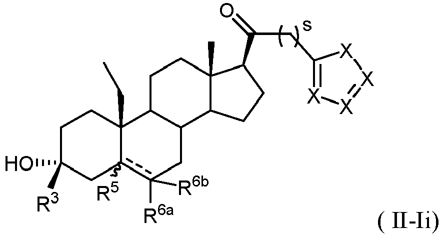 wobei s für 0, 1 oder 2 steht; X jeweils unabhängig für -C(R^{N})-, -C(R^{N})₂-, -O-, -S-, -N- oder -N(R^{N}) - steht, wobei R^{N} unabhängig für Wasserstoff, substituiertes oder unsubstituiertes C₁-₆-Alkyl, -C(=O)R^{GA}, -C(=O)OR^{GA}, - C(=O)N(R^{GA})₂, -S(=O)₂R^{GA} oder -S(=O)₂N(R^{GA})₂ steht; und
jedes Vorkommen von R^{GA} unabhängig für Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₆-Carbocyclyl, substituiertes oder unsubstituiertes 3- bis 6-gliedriges Heterocyclyl, substituiertes oder unsubstituiertes Aryl, substituiertes oder unsubstituiertes Heteroaryl, eine Sauerstoff-Schutzgruppe, wenn es an Sauerstoff gebunden ist, oder eine Stickstoff-Schutzgruppe, wenn es an Stickstoff gebunden ist, steht oder zwei R^{GA}-Gruppen zusammen mit den dazwischenliegenden Atomen einen substituierten oder unsubstituierten Heterocyclyl- oder Heteroarylring bilden; oder ein pharmazeutisch unbedenkliches Salz davon.

16. Verbindung, ausgewählt aus
| Verbindungsbezeichnung | STRUKTUR |
|---|---|
| II-1 | 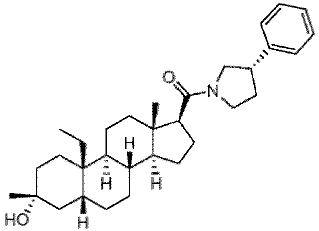 |
| II-A4 | 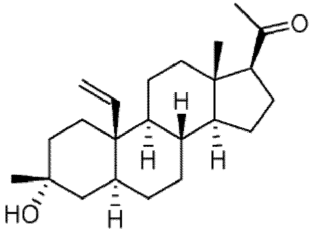 |
| II-A5 | 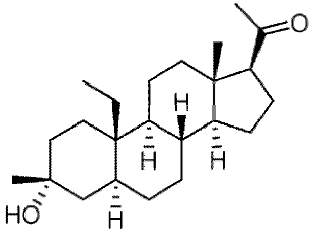 |
| II-A7 | 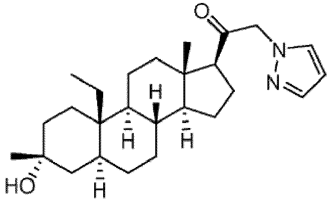 |
| II-A8 | 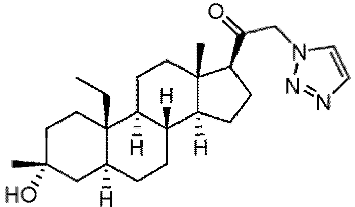 |
| II-A8a | 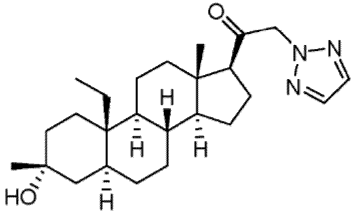 |
| II-A9 | 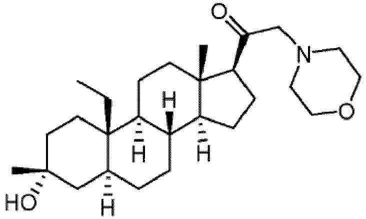 |
| II-A10 | 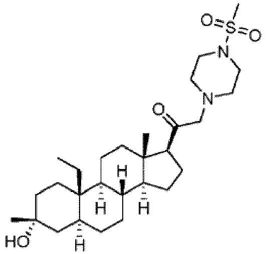 |
| II-A11 | 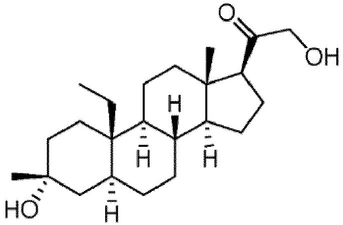 |
| II-E12 | 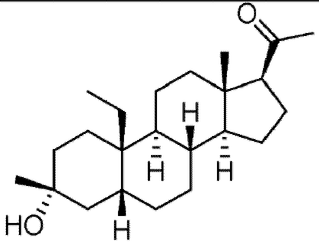 |
| II-G3 | 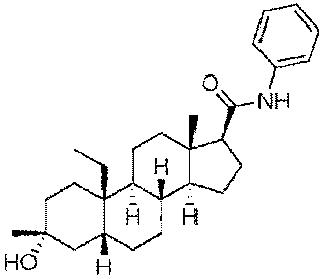 |
| II-M2 | 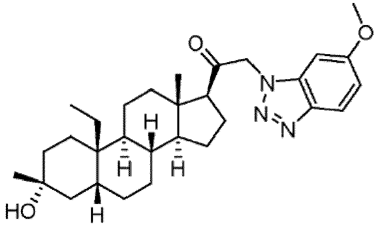 |
| II-M2a | 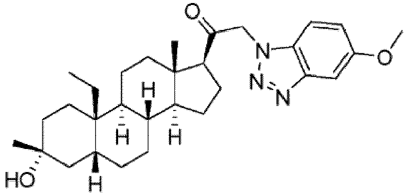 |
| II-M2b | 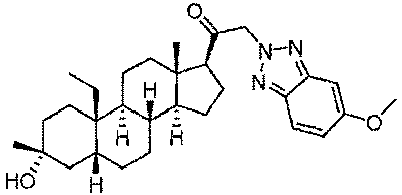 |
| II-12 | 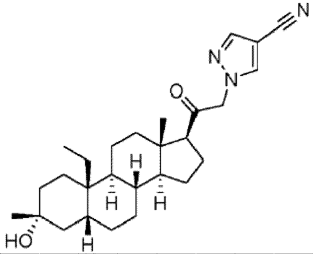 |
| II-13 | 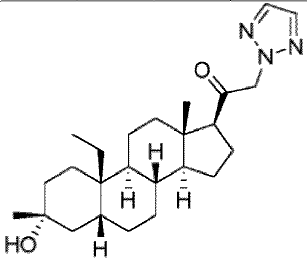 |
| II-13a | 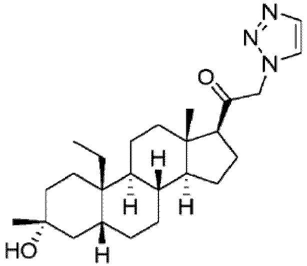 |
| II-14 | 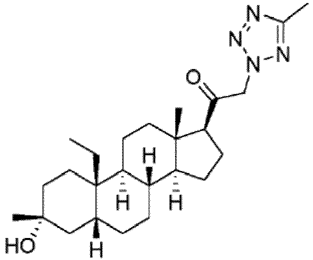 |
| II-14a | 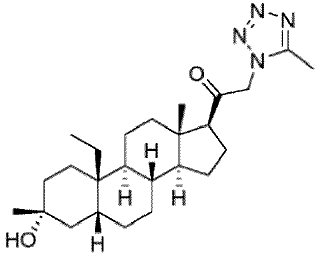 |
| II-20 | 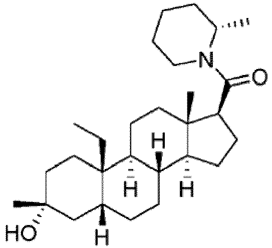 |
| II-21 | 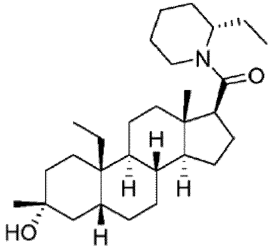 |
| II-21a | 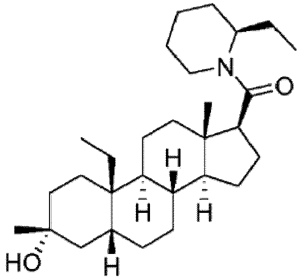 |
| II-22 | 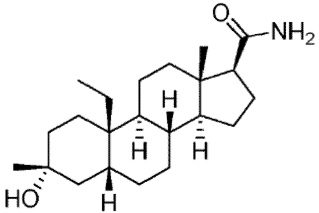 |
| II-G5 | 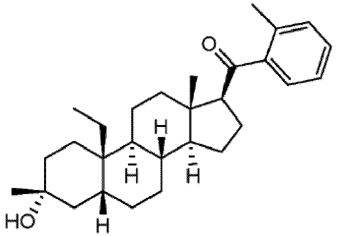 |
| II-B9 | 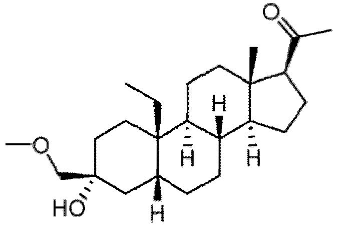 |
| II-B11 | 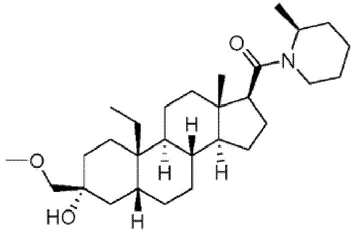 |
| II-C4 | 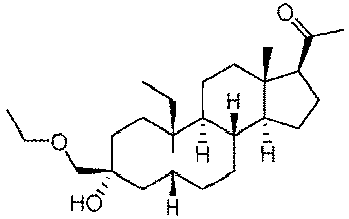 |
| II-43 | 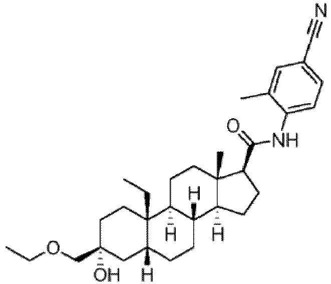 |
| II-44 | 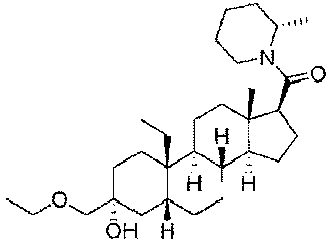 |
| II-45 | 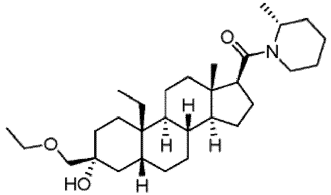 |
| II-46 | 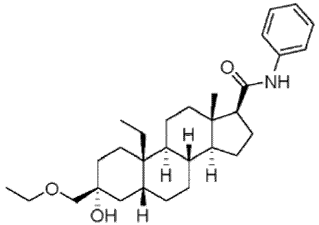 |
| II-47 | 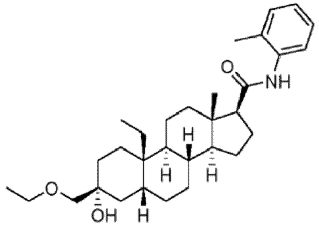 |
| II-48 | 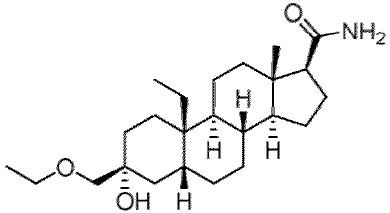 |
| II-50 | 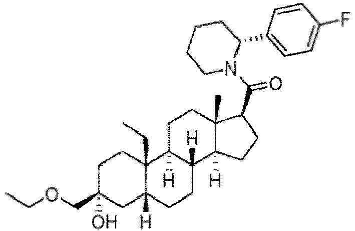 |
| II-51 | 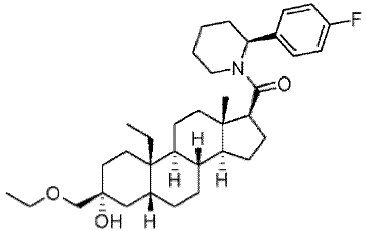 |
| II-D9 | 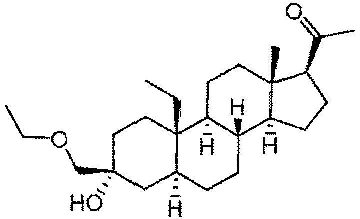 |
| II-D11 | 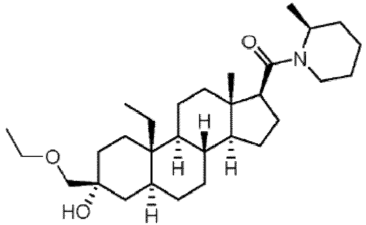 |
| II-63 | 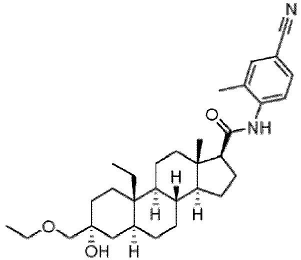 |
| II-E4 | 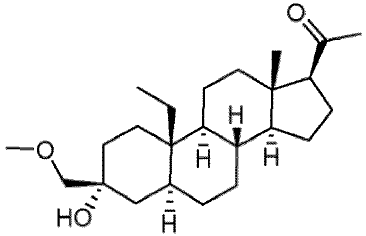 |
| II-E6 | 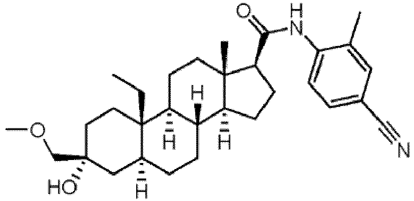 |
| II-E8 | 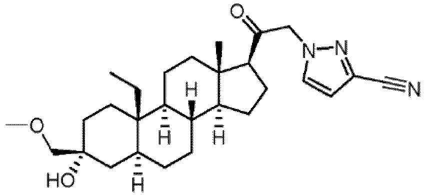 |
| II-D13 | 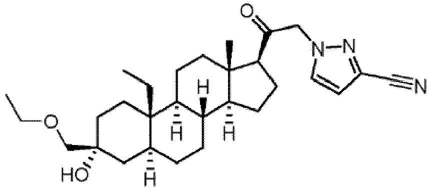 |
| II-C8 | 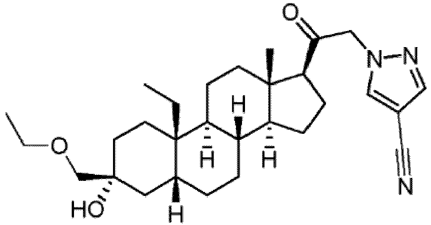 |
| II-75 | 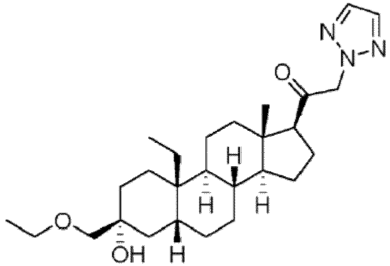 |
| II-76 | 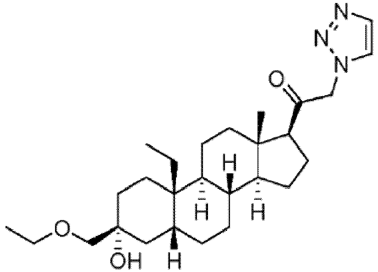 |
| II-77 | 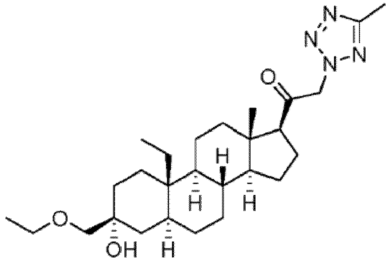 |
| II-78 | 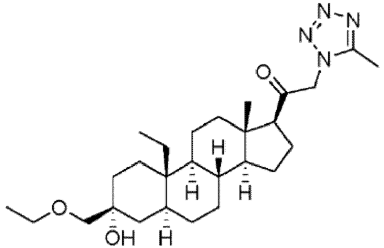 |
| II-79 | 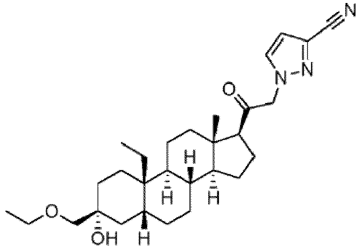 |
oder ein pharmazeutisch unbedenkliches Salz davon.

17. Pharmazeutische Zusammensetzung, umfassend eine Verbindung nach einem der Ansprüche 1-16 oder ein pharmazeutisch unbedenkliches Salz davon und einen pharmazeutisch unbedenklichen Hilfsstoff.

18. Verbindung nach einem der Ansprüche 1-16 oder pharmazeutisch unbedenkliches Salz davon oder pharmazeutische Zusammensetzung nach Anspruch 17 zur Verwendung bei der Behandlung einer mit dem ZNS in Zusammenhang stehenden Störung bei einem Individuum.

19. Verbindung nach einem der Ansprüche 1-16 oder pharmazeutisch unbedenkliches Salz davon oder pharmazeutische Zusammensetzung nach Anspruch 17 zur Verwendung nach Anspruch 18, wobei es sich bei der mit dem ZNS in Zusammenhang stehenden Störung um eine Schlafstörung, eine affektive Störung, eine Störung des Schizophrenie-Spektrums, eine konvulsive Störung, eine Gedächtnis- und/oder Kognitionsstörung, eine Bewegungsstörung, eine Persönlichkeitsstörung, eine Störung des Autismus-Spektrums, Schmerzen, traumatische Hirnverletzung, eine Gefäßerkrankung, eine Substanzmissbrauchsstörung und/oder ein Entzugssyndrom, Tinnitus oder Status epilepticus handelt.

20. Verbindung nach einem der Ansprüche 1-16 oder pharmazeutisch unbedenkliches Salz davon oder pharmazeutische Zusammensetzung nach Anspruch 17 zur Verwendung nach Anspruch 19, wobei es sich bei der mit dem ZNS in Zusammenhang stehenden Störung um Depression handelt.

21. Verbindung nach einem der Ansprüche 1-16 oder pharmazeutisch unbedenkliches Salz davon oder pharmazeutische Zusammensetzung nach Anspruch 17 zur Verwendung nach Anspruch 19, wobei es sich bei der Depression um postpartale Depression handelt.

22. Verbindung nach einem der Ansprüche 1-16 oder pharmazeutisch unbedenkliches Salz davon oder pharmazeutische Zusammensetzung nach Anspruch 17 zur Verwendung nach Anspruch 19, wobei es sich bei der mit dem ZNS in Zusammenhang stehenden Störung um majore depressive Störung handelt.

23. Verbindung nach einem der Ansprüche 1-16 oder pharmazeutisch unbedenkliches Salz davon oder pharmazeutische Zusammensetzung nach Anspruch 17 zur Verwendung nach Anspruch 22, wobei es sich bei der majoren depressiven Störung um moderate majore depressive Störung handelt.

24. Verbindung nach einem der Ansprüche 1-16 oder pharmazeutisch unbedenkliches Salz davon oder pharmazeutische Zusammensetzung nach Anspruch 17 zur Verwendung nach Anspruch 22, wobei es sich bei der majoren depressiven Störung um schwere majore depressive Störung handelt.

## Revendications

1. Composé de formule (II-Ia) : 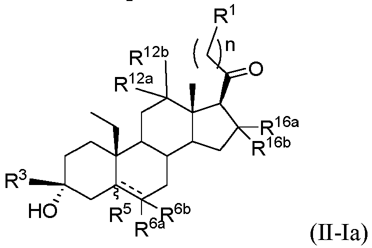
ou sel pharmaceutiquement acceptable correspondant ; n étant 0, 1 ou 2 ;
R⁵ étant hydrogène ou méthyle, ou lorsque 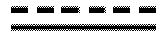 est une double liaison, R⁵ étant absent ;
R³ étant hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué ;
chacun parmi R^{6a} et R^{6b} étant indépendamment hydrogène, halogène, cyano, hydroxyle, alkyle substitué ou non substitué, alcényle substitué ou non substitué ou alcynyle substitué ou non substitué, ou R^{6a} et R^{6b} étant joints pour former un groupe oxo (=O) ;
R¹ étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué, -OR^{A1}, -SR^{A1}, -N(R^{A1})₂, -OC(=O)R^{A1}, -OC(=O)OR^{A1}, - OC(=O)SR^{A1}, -OC(=O)N(R^{A1})₂, -SC (=O)R^{A2}, -SC (=O)OR^{A1}, - SC(=O)SR^{A1}, -SC (=O)N(R^{A1})₂, -NHC(=O)R^{A1}, -NHC(=O)OR^{A1}, - NHC(=O)SR^{A1}, -NHC(=O)N(R^{A1})₂, -OS(=O)₂R^{A2}, -OS (=O)₂OR^{A1}, -S-S(=O)₂R^{A2}, -S-S(=O)₂OR^{A1}, -S(=O)R^{A2}, -SO₂R^{A2}, ou -S(=O)₂OR^{A1}, chaque instance de R^{A1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, un groupe protecteur d'oxygène lorsqu'il est fixé à un atome d'oxygène, un groupe protecteur de soufre lorsqu'il est fixé à un atome de soufre, un groupe protecteur d'azote lorsqu'il est fixé à un atome d'azote, ou deux groupes R^{A1} étant joints pour former un cycle hétérocyclique ou hétéroaryle substitué ou non substitué ; et R^{A2} étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué ;
chacun parmi R^{12a} et R^{12b} étant indépendamment hydrogène, halogène, cyano, hydroxyle, alkyle substitué ou non substitué, alcényle substitué ou non substitué, hétérocyclyle substitué ou non substitué ou alcynyle substitué ou non substitué, -OR^{D1}, -OC(=O)R^{D1}, -NH₂, - N(R^{D1})₂, ou -NR^{D1}C(=O)R^{D1}, chaque instance de R^{D1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué, un groupe protecteur d'oxygène lorsqu'il est fixé à un atome d'oxygène, un groupe protecteur d'azote lorsqu'il est fixé à un atome d'azote, ou deux groupes R^{D1} étant joints pour former un cycle hétérocyclique substitué ou non substitué ; ou R^{12a} et R^{12b} étant joints pour former un groupe oxo (=O) ;
chacun parmi R^{16a} et R^{16b} étant indépendamment hydrogène, halogène, cyano, hydroxyle, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué, -OR^{A1}, -SR^{A1}, -N(R^{A1})₂, -N(R^{A1}), -CN(R^{A1})₂, -C(O)R^{A1}, -OC(=O)R^{A1}, -OC(=O)OR^{A1}, -OC(=O)SR^{A1},-OC (=O)N(R^{A1})₂, -SC (=O)R^{A2}, -SC (=O) OR^{A1}, -SC (=O) SR^{A1}, - SC (=O)N(R^{A1})₂, -NHC (=O)R^{A1}, -NHC (=O) OR^{A1}, -NHC (=O) SR^{A1}, - NHC(=O)N(R^{A1})₂, -OS (=O) ₂R^{A2}, -OS (=O) ₂OR^{A1}, -S-S(=O)₂R^{A2}, -S-S(=O)₂OR^{A1}, -S(=O)R^{A2}, -SO₂R^{A2}, ou -S(=O)₂OR^{A1}, chaque instance de R^{A1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué, un groupe protecteur d'oxygène lorsqu'il est fixé à un atome d'oxygène, un groupe protecteur de soufre lorsqu'il est fixé à un atome de soufre, un groupe protecteur d'azote lorsqu'il est fixé à un atome d'azote, -SO₂R^{A2}, -C(O)R^{A2}, ou deux groupes R^{A1} étant joints pour former un cycle hétérocyclique ou hétéroaryle substitué ou non substitué ; et R^{A2} étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué ou hétéroaryle substitué ou non substitué ; et 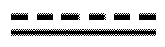
représentant une simple liaison ou une double liaison, à condition que si une double liaison est présente dans le cycle B, alors l'un parmi R^{6a} ou R^{6b} est absent.

2. Composé ou sel pharmaceutiquement acceptable selon la revendication 1, R⁵ étant hydrogène ou méthyle dans la position cis, par rapport à la position de C19.

3. Composé ou sel pharmaceutiquement acceptable selon la revendication 1, R⁵ étant hydrogène ou méthyle dans la position trans, par rapport à la position de C19.

4. Composé ou sel pharmaceutiquement acceptable selon l'une quelconque des revendications 1 à 3, R³ étant hydrogène, alkyle substitué ou non substitué, alcényle substitué ou non substitué ou alcynyle substitué ou non substitué.

5. Composé ou sel pharmaceutiquement acceptable selon l'une quelconque des revendications 1 à 4, R³ étant C₁-C₆ alkyle substitué ou non substitué.

6. Composé ou sel pharmaceutiquement acceptable selon l'une quelconque des revendications 1 à 5, R³ étant méthyle.

7. Composé ou sel pharmaceutiquement acceptable selon l'une quelconque des revendications 1 à 6, à la fois R^{6a} et R^{6b} étant hydrogène.

8. Composé ou sel pharmaceutiquement acceptable selon l'une quelconque des revendications 1 à 7, chacun parmi R^{12a} et R^{12b} étant indépendamment hydrogène, C₁-C₆ alkyle substitué ou non substitué, -OH, ou R^{12a}et R^{12b} formant ensemble oxo.

9. Composé ou sel pharmaceutiquement acceptable selon l'une quelconque des revendications 1 à 8, chacun parmi R^{16a} et R^{16b} étant indépendamment hydrogène, C₁-C₆ alkyle substitué ou non substitué, -OR^{D1}, -OC(=O)R^{D1}, -NH2, - N(R^{D1})2, ou -NR^{D1}C(=O)R^{D1}, chaque instance de R^{D1} étant indépendamment hydrogène, alkyle substitué ou non substitué, carbocyclyle substitué ou non substitué, hétérocyclyle substitué ou non substitué, aryle substitué ou non substitué ou hétéroaryle substitué ou non substitué.

10. Composé ou sel pharmaceutiquement acceptable selon l'une quelconque des revendications 1 à 9, chacun parmi R^{16a} et R^{16b} étant indépendamment hydrogène ou C₁-C₆ alkyle substitué ou non substitué.

11. Composé ou sel pharmaceutiquement acceptable selon l'une quelconque des revendications 1 à 10, à la fois R^{16a} et R^{16b} étant hydrogène.

12. Composé ou sel pharmaceutiquement acceptable selon l'une quelconque des revendications 1 à 11, n étant 0 ou 1, et R¹ étant -N(R^{A1})₂, -OR^{A1}, alkyle substitué ou non substitué, carbocyclyle substitué ou non substitué, hétérocyclyle substitué ou non substitué, aryle substitué ou non substitué ou hétéroaryle substitué ou non substitué.

13. Composé ou sel pharmaceutiquement acceptable selon l'une quelconque des revendications 1 à 12, R¹ étant 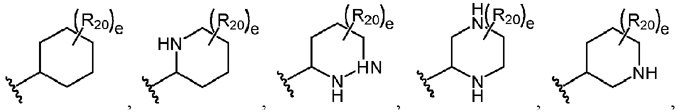 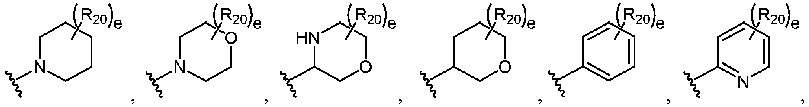 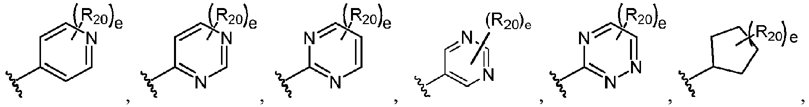 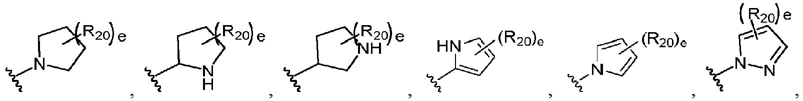 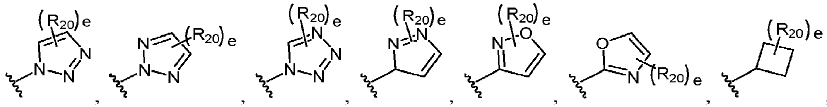 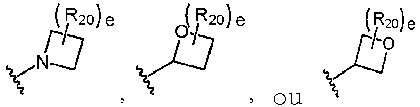 chaque instance de R₂₀ étant indépendamment hydrogène, halogène, -NO₂, -CN, -OR^{GA}, -N(R^{GA})₂, -C(=O)R^{GA}, -C(=O)OR^{GA}, -C(=O)N(R^{GA})₂, -OC(=O)R^{GA}, -OC(=O) OR^{GA}, -N(R^{GA})C (=O) R^{GA}, - OC (=O)N(R^{GA})₂, -N(R^{GA})C (=O) OR^{GA}, -S(=O) ₂R^{GA}, -S(=O)₂OR^{GA}, - OS (=O) ₂R^{GA}, -S (=O) ₂N(R^{GA})₂, ou -N(R^{GA})S (=O) ₂R^{GA}, C₁-C₆ alkyle substitué ou non substitué, C₂₋₆ alcényle substitué ou non substitué, C₂₋₆ alcynyle substitué ou non substitué, C₃₋₄ carbocylyle substitué ou non substitué, hétérocyclyle à 3 à 4 chaînons substitué ou non substitué, C₅₋₁₀ aryle substitué ou non substitué, hétéroaryle à 5 à 10 chaînons substitué ou non substitué, ou éventuellement deux R^{GA} étant pris avec les atomes intervenants pour former un cycle hétérocyclique ou carbocyclique à 3 à 4 chaînons substitué ou non substitué ;
chaque instance de R^{GA} étant indépendamment hydrogène, C₁₋₆ alkyle substitué ou non substitué, C₂₋₆ alcényle substitué ou non substitué, C₂₋₆ alcynyle substitué ou non substitué, C₃₋₆ carbocylyle substitué ou non substitué, hétérocyclyle à 3 à 6 chaînons substitué ou non substitué, aryle substitué ou non substitué, hétéroaryle substitué ou non substitué, un groupe protecteur d'oxygène lorsqu'il est fixé à un oxygène, un groupe protecteur d'azote lorsqu'il est fixé à un azote, ou deux groupes R^{GA} étant pris avec les atomes intervenants pour former un cycle carbocyclique ou hétérocyclique substitué ou non substitué ; et
e étant 0, 1, 2, 3, 4, ou 5.

14. Composé ou sel pharmaceutiquement acceptable selon la revendication 1, le composé étant un composé de formule (II-Ib) ou de formule (II-Ic) 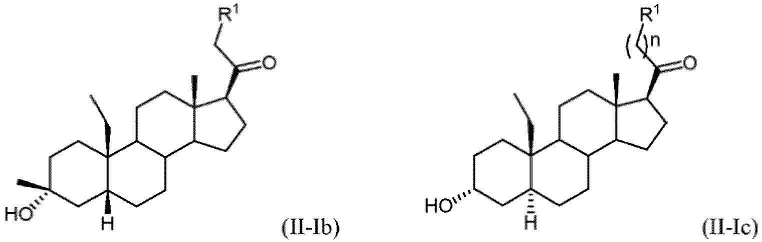 ou sel pharmaceutiquement acceptable correspondant.

15. Composé ou sel pharmaceutiquement acceptable selon la revendication 1, le composé étant
(a) un composé de formule (II-Ie) 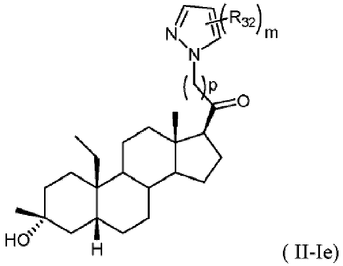
m étant 0, 1, 2 ou 3 ;
p étant 0, 1 ou 3 ;
chaque R₃₂ étant indépendamment halogène, alkyle, hydroxyle, ou cyano ;
ou sel pharmaceutiquement acceptable correspondant ;
(b) un composé de formule (II-Ig) 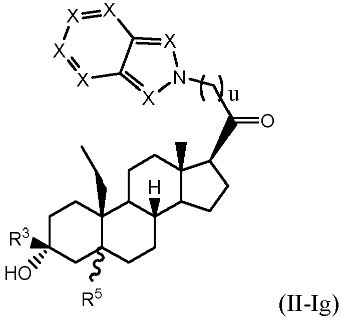
u étant 0, 1 ou 2 ; chaque X étant indépendamment -C(R^{N})-, -C(R^{N})₂-, -S-, -N-, ou -N(R^{N})-, R^{N} étant indépendamment hydrogène, C₁₋₆ alkyle substitué ou non substitué,-C(=O)R^{GA}, -C (=O) OR^{GA}, -C(=O)N(R^{GA})₂, -S(=O)₂R^{GA}, ou - S(=O)₂N(R^{GA})₂ ; et
chaque instance de R^{GA} étant indépendamment hydrogène, C₁₋₆ alkyle substitué ou non substitué, C₂₋₆ alcényle substitué ou non substitué, C₂₋₆ alcynyle substitué ou non substitué, C₃₋₆ carbocylyle substitué ou non substitué, hétérocyclyle à 3 à 6 chaînons substitué ou non substitué, aryle substitué ou non substitué, hétéroaryle substitué ou non substitué, un groupe protecteur d'oxygène lorsqu'il est fixé à un oxygène, un groupe protecteur d'azote lorsqu'il est fixé à un azote, ou deux groupes R^{GA} étant pris avec les atomes intervenants pour former un cycle hétérocyclyle ou hétéroaryle substitué ou non substitué ; ou sel pharmaceutiquement acceptable correspondant ;
(c) un composé de formule (II-Iga) 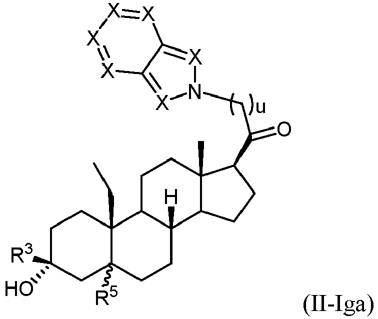
u étant 0, 1 ou 2 ; chaque X étant indépendamment -C(R^{N})-, -C(R^{N})₂-, -S-, -N-, ou -N(R^{N})-, R^{N} étant indépendamment hydrogène, C₁₋₆ alkyle substitué ou non substitué,-C(=O)R^{GA}, -C (=O) OR^{GA}, -C(=O)N(R^{GA})₂, -S(=O)₂R^{GA}, ou - S(=O)₂N(R^{GA})₂ ; et
chaque instance de R^{GA} étant indépendamment hydrogène, C₁₋₆ alkyle substitué ou non substitué, C₂₋₆ alcényle substitué ou non substitué, C₂₋₆ alcynyle substitué ou non substitué, C₃₋₆ carbocylyle substitué ou non substitué, hétérocyclyle à 3 à 6 chaînons substitué ou non substitué, aryle substitué ou non substitué, hétéroaryle substitué ou non substitué, un groupe protecteur d'oxygène lorsqu'il est fixé à un oxygène, un groupe protecteur d'azote lorsqu'il est fixé à un azote, ou deux groupes R^{GA} étant pris avec les atomes intervenants pour former un cycle hétérocyclyle ou hétéroaryle substitué ou non substitué ; ou sel pharmaceutiquement acceptable correspondant ;
(d) un composé de formule (II-Ih) 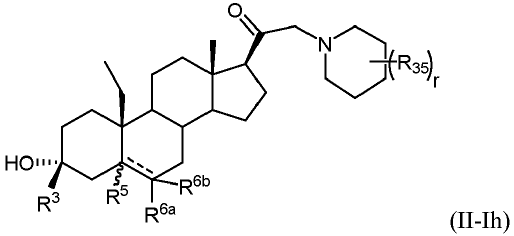 chaque R₃₅ étant indépendamment halogène, alkyle, hydroxyle, ou cyano ; et r étant 0, 1, 2 ou 3 ; ou sel pharmaceutiquement acceptable correspondant ; ou
(e) un composé de formule (II-Ii) 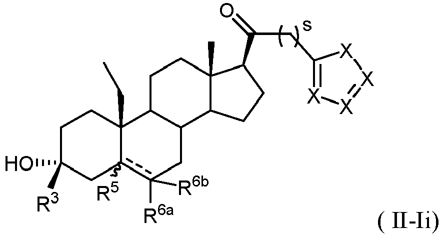
s étant 0, 1 ou 2 ; chaque X étant indépendamment -C(R^{N})-, -C(R^{N})₂-, -O-, -S-, -N-, ou -N(R^{N})-, R^{N} étant indépendamment hydrogène, C₁₋₆ alkyle substitué ou non substitué, -C (=O) R^{GA}, -C (=O) OR^{GA}, -C(=O)N(R^{GA})₂, -S(=O)₂R^{GA}, ou -S(=O)₂N(R^{GA})₂ ; et
chaque instance de R^{GA} étant indépendamment hydrogène, C₁₋₆ alkyle substitué ou non substitué, C₂₋₆ alcényle substitué ou non substitué, C₂₋₆ alcynyle substitué ou non substitué, C₃₋₆ carbocylyle substitué ou non substitué, hétérocyclyle à 3 à 6 chaînons substitué ou non substitué, aryle substitué ou non substitué, hétéroaryle substitué ou non substitué, un groupe protecteur d'oxygène lorsqu'il est fixé à un oxygène, un groupe protecteur d'azote lorsqu'il est fixé à un azote, ou deux groupes R^{GA} étant pris avec les atomes intervenants pour former un cycle hétérocyclyle ou hétéroaryle substitué ou non substitué ; ou sel pharmaceutiquement acceptable correspondant.

16. Composé choisi parmi
| Composé ID | STRUCTURE |
|---|---|
| II-1 | 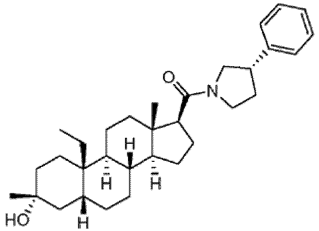 |
| II-A4 | 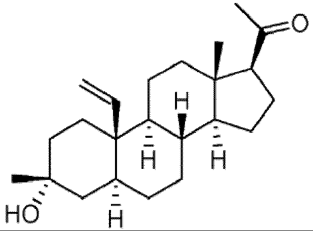 |
| II-A5 | 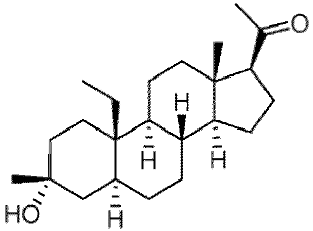 |
| II-A7 | 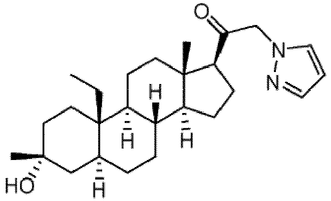 |
| II-A8 | 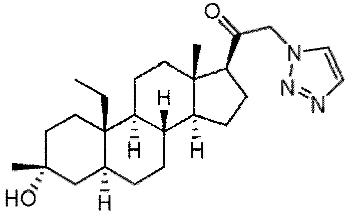 |
| II-A8a | 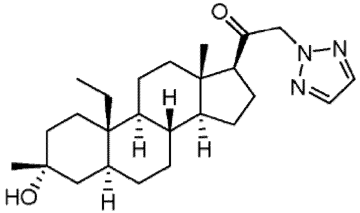 |
| II-A9 | 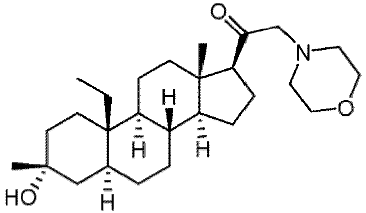 |
| II-A10 | 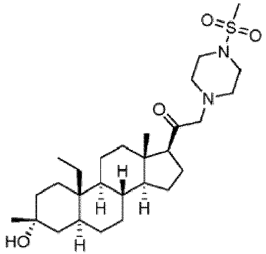 |
| II-A11 | 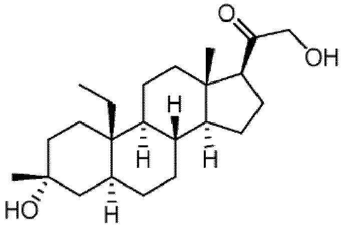 |
| II-E12 | 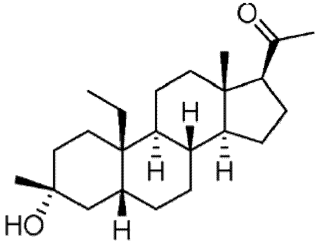 |
| II-G3 | 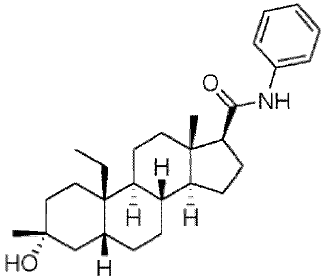 |
| II-M2 | 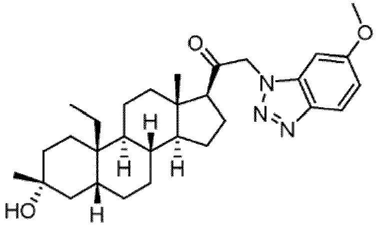 |
| II-M2a | 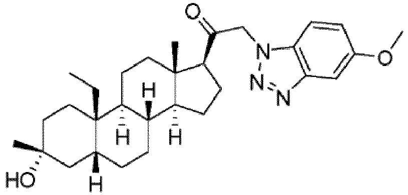 |
| II-M2b | 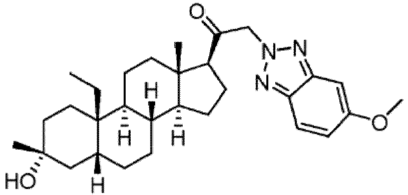 |
| II-12 | 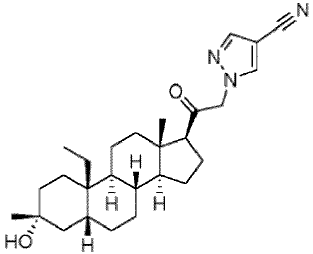 |
| II-13 | 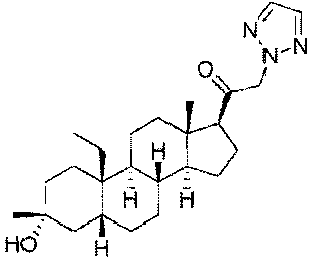 |
| II-13a | 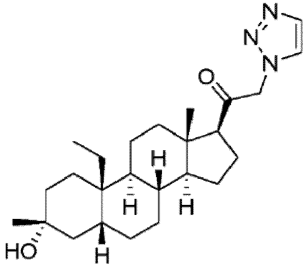 |
| II-14 | 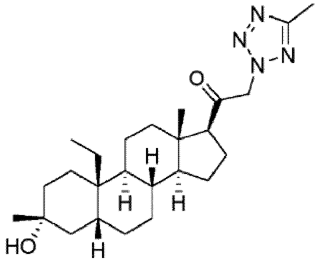 |
| II-14a | 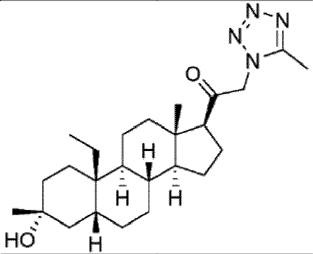 |
| II-20 | 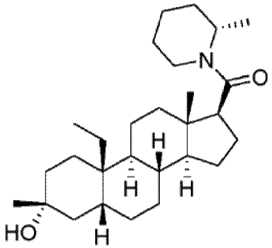 |
| II-21 | 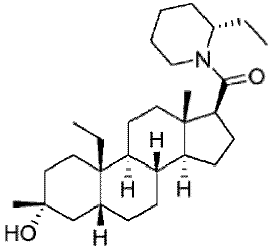 |
| II-21a | 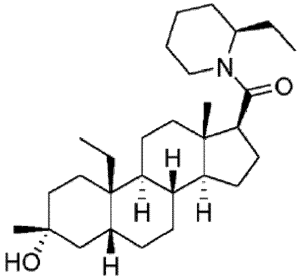 |
| II-22 | 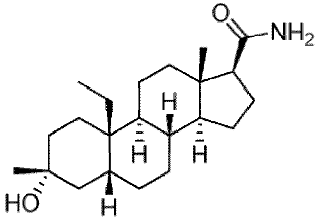 |
| II-G5 | 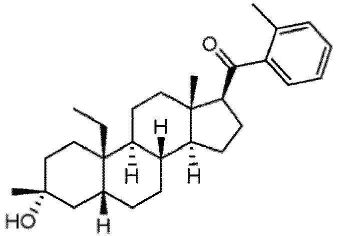 |
| II-B9 | 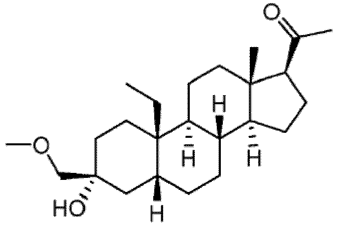 |
| II-B11 | 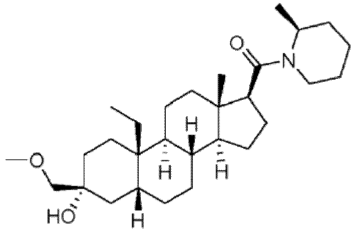 |
| II-C4 | 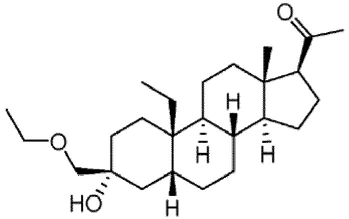 |
| II-43 | 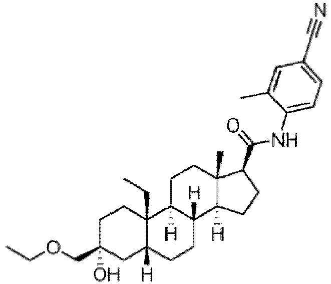 |
| II-44 | 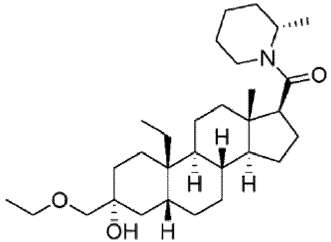 |
| II-45 | 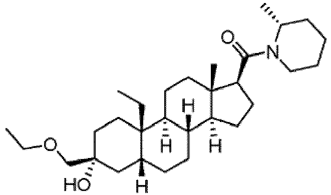 |
| II-46 | 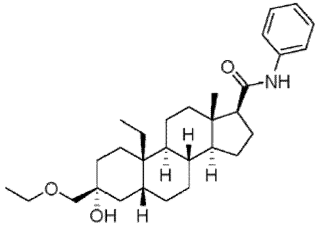 |
| II-47 | 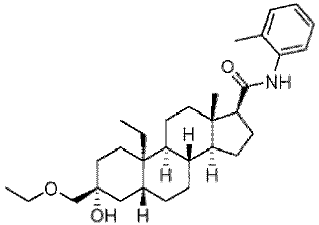 |
| II-48 | 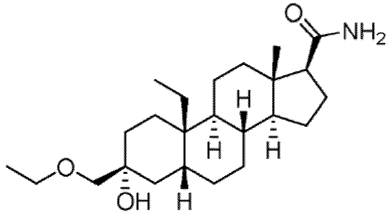 |
| II-50 | 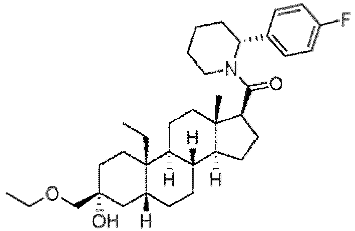 |
| II-51 | 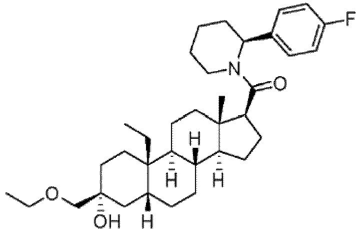 |
| II-D9 | 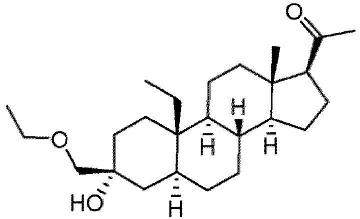 |
| II-D11 | 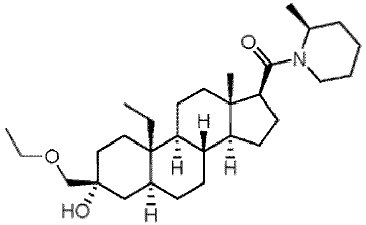 |
| II-63 | 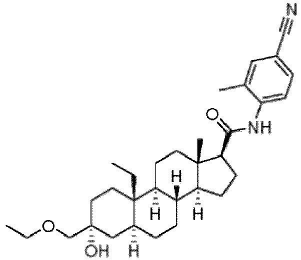 |
| II-E4 | 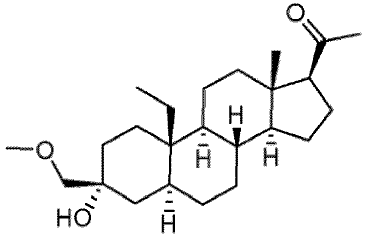 |
| II-E6 | 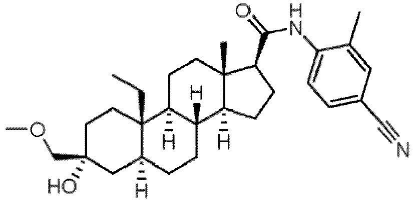 |
| II-E8 | 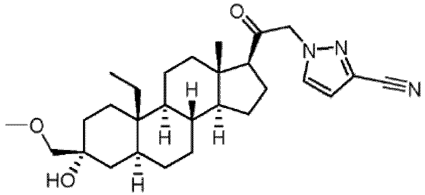 |
| II-D13 | 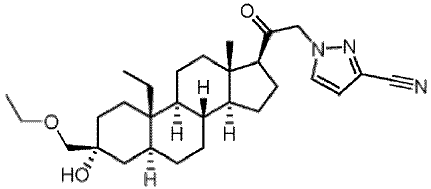 |
| II-C8 | 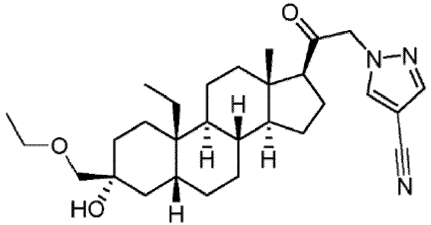 |
| II-75 | 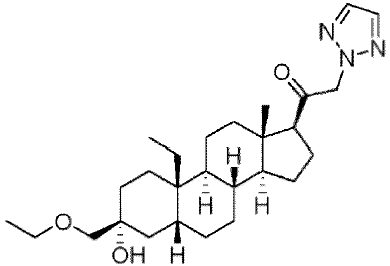 |
| II-76 | 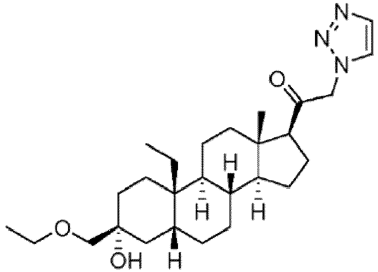 |
| II-77 | 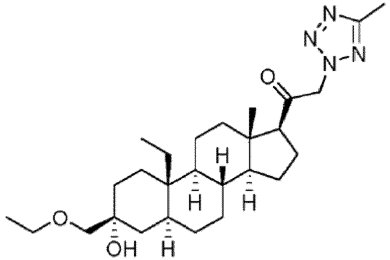 |
| II-78 | 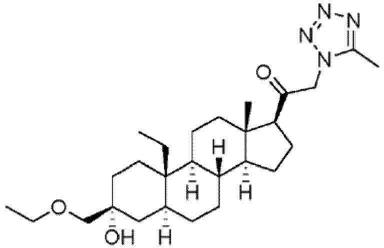 |
| II-79 | 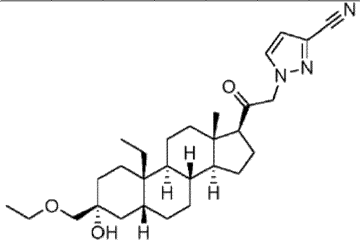 |
ou sel pharmaceutiquement acceptable correspondant.

17. Composition pharmaceutique comprenant un composé selon l'une quelconque des revendications 1 à 16 ou un sel pharmaceutiquement acceptable correspondant, et un excipient pharmaceutiquement acceptable.

18. Composé selon l'une quelconque des revendications 1 à 16 ou sel pharmaceutiquement acceptable correspondant, ou composition pharmaceutique selon la revendication 17, pour une utilisation dans le traitement d'un trouble lié au SNC chez un sujet.

19. Composé selon l'une quelconque des revendications 1 à 16 ou sel pharmaceutiquement acceptable correspondant, ou composition pharmaceutique selon la revendication 17, pour une utilisation selon la revendication 18, le trouble lié au SNC étant un trouble du sommeil, un trouble de l'humeur, un trouble du spectre de la schizophrénie, un trouble convulsif, un trouble de la mémoire et/ou cognitif, un trouble du mouvement, un trouble de la personnalité, un trouble du spectre autistique, la douleur, une lésion traumatique du cerveau, une maladie vasculaire, un trouble lié à l'abus de substances et/ou un syndrome de sevrage, un acouphène, ou un état épileptique.

20. Composé selon l'une quelconque des revendications 1 à 16 ou sel pharmaceutiquement acceptable correspondant, ou composition pharmaceutique selon la revendication 17, pour une utilisation selon la revendication 19, le trouble lié au SNC étant une dépression.

21. Composé selon l'une quelconque des revendications 1 à 16 ou sel pharmaceutiquement acceptable correspondant, ou composition pharmaceutique selon la revendication 17, pour une utilisation selon la revendication 19, la dépression étant une dépression post-partum.

22. Composé selon l'une quelconque des revendications 1 à 16 ou sel pharmaceutiquement acceptable correspondant, ou composition pharmaceutique selon la revendication 17, pour une utilisation selon la revendication 19, le trouble lié au SNC étant un trouble dépressif majeur.

23. Composé selon l'une quelconque des revendications 1 à 16 ou sel pharmaceutiquement acceptable correspondant, ou composition pharmaceutique selon la revendication 17, pour une utilisation selon la revendication 22, le trouble dépressif majeur étant un trouble dépressif majeur modéré.

24. Composé selon l'une quelconque des revendications 1 à 16 ou sel pharmaceutiquement acceptable correspondant, ou composition pharmaceutique selon la revendication 17, pour une utilisation selon la revendication 22, le trouble dépressif majeur étant un trouble dépressif majeur grave.
